# EUROPEAN PATENT SPECIFICATION

(11) **EP 4 413 000 B1**
(45) Date of publication and mention of the grant of the patent: **02.12.2026**
(21) Application number: 22800163.2
(22) Date of filing: 03.10.2022
(51) Int. Cl.: C07D 471/04, A61K 31/437, A61P 35/00, A61P 35/02, A61P 35/04

(54) **N,N-DIMETHYL-4-(7-(N-(1-METHYLCYCLOPROPYL)SULFAMOYL)-IMIDAZO[1,5-A]PYRIDIN-5-YL)PIPERAZINE-1-CARBOXAMIDE DERIVATIVES AND THE CORRESPONDING PYRAZOLO[1,5-A]PYRIDINE DERIVATIVES AS PARG INHIBITORS FOR THE TREATMENT OF CANCER**
N,N-DIMETHYL-4-(7-(N-(1-METHYLCYCLOPROPYL)SULFAMOYL)-IMIDAZO[1,5-A]PYRIDIN-5-YL)PIPERAZIN-1-CARBOXAMID-DERIVATE UND DIE ENTSPRECHENDEN PYRAZOLO[1,5-A]PYRIDIN-DERIVATE ALS PARG INHIBITOREN ZUR BEHANDLUNG VON KREBS
DÉRIVÉS DE N,N-DIMÉTHYL-4-(7-(N-(1-MÉTHYLCYCLOPROPYL)SULFAMOYL)-IMIDAZO[1,5-A]PYRIDIN-5-YL)PIPÉRAZINE-1-CARBOXAMIDE ET LES DÉRIVÉS DE PYRAZOLO[1,5-A]PYRIDINE CORRESPONDANTS EN TANT QU'INHIBITERUS DE PARG POUR LE TRAITEMENT DU CANCER

(30) Priority: 04.10.2021 US 202163251954 P; 21.03.2022 US 202263321975 P
(43) Date of publication of application: 14.08.2024
(73) Proprietor: FoRx Therapeutics AG, 4056 Basel (CH)
(72) Inventor: LUECKING, Ulrich, 10317 Berlin (DE); GOUTOPOULOS, Andreas, Boston, Massachusetts 02116 (US); JIN, Tian, Hubei, 430075 (CN); SOTIRIOU, Sotirios, 4153 Reinach BL (CH); IACOVINO, Luca, 4310 Rheinfelden (CH); FREUDENMANN, Alena, 4125 Riehen (CH); QUEROLLE, Olivier, 68300 Saint Louis (FR)
(74) Representative: Vossius & Partner Patentanwälte Rechtsanwälte mbB
(86) International application number: PCT/EP2022/077476
(87) International publication number: WO 2023/057394

(56) References cited:
- WO-A1-2005/028476
- WO-A1-2010/051549
- WO-A1-2021/055744
- WO-A2-2008/058126
- CN-A- 103 694 242

## Description

### Field of the invention

The present invention relates to a compound of formula (I): 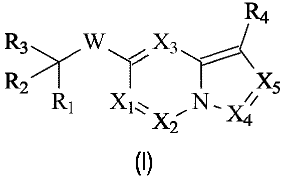 or an enantiomer, diastereoisomer, tautomer, pharmaceutically acceptable solvate, pharmaceutically acceptable crystal form or pharmaceutically acceptable salt thereof. The present invention further relates to the compound of formula (I) of the present invention for use in therapy. Instant compounds are particularly useful as PARG inhibitors, and can be used in a method of treatment of a proliferative disorder, preferably of cancer.

References in the present description to methods of medical treatment or medical uses have to be understood as references to the compounds of the present invention for use in methods of medical treatment. Methods of medical treatment or medical uses are not encompassed by the claimed invention.

### Background of the invention

Cancer is a leading cause of death worldwide. Although progression-free survival and overall survival of cancer patients has improved over the past two decades, millions of cancer patients still have few therapeutic options and poor survival outcomes (Jemal et al., J. Natl. Cancer Inst. 2017, 109, 1975).

DNA replication stress (DRS) is a hallmark of cancer cells and a major source of genomic instability (a) Halazonetis et al., Science 2008, 319, 1352; b) Negrini et al., Nat. Rev. Mol. Cell Biol. 2010, 11, 220). In broad terms, DRS refers to the deregulation of DNA replication and cell cycle progression. DRS can be induced from endogenous or exogenous causes such as oncogene activation and chemotherapeutics, respectively (Zeman and Cimprich, Nat. Cell Biol. 2013, 16, 2). At the level of the replication fork, DRS leads to replication fork stalling, disengagement of the replisome and eventually collapse. Several DNA repair proteins are involved in replication fork stability, protection, and restart under DRS conditions (a) Costantino et al., Science 2014, 343, 88; b) Scully et al., Curr. Opin. Genet. Dev. 2021 71, 154).

Poly(ADP)ribosylation (PARylation) is a transient and reversible post-translational modification that occurs at DNA damaged sites and is catalyzed by the poly (ADP-ribose) polymerase (PARP) family of proteins (Cohen and Chang, Nat. Chem. Biol. 2018, 14, 236). PARylation of various DNA repair proteins leads to their activation. Degradation of the poly(ADP) ribose chains is mediated primarily by the poly(ADP-ribose) glycohydrolase (PARG) protein. DNA damage dependent PARylation/dePARylation is a rapid and dynamic process which needs to be well regulated since imbalances between the two processes can lead to DNA damage.

Human PARG encodes a 111 kDa protein of 976 amino acids. It contains a N-terminal regulatory domain, a catalytic domain and an ADP-ribose binding macrodomain. Five human PARG transcripts have been identified. Full length PARG is mostly nuclear; the smaller isoforms localize primarily to the cytoplasm. PARG functions primarily as an exo-hydrolase and it releases mainly mono(ADP-ribose) by hydrolyzing the α-O-glycosidic ribose-ribose bond in PAR. PARG can also act as an endo-hydrolase. PARG preferentially degrades long and linear PAR chains whereas its activity with small and branched PAR chains is significantly reduced (O'Sullivan et al., Nat. Commun. 2019, 10, 1182).

Although PARG is the dominant cellular PAR degrading enzyme, it cannot act on the terminal protein-ribose bond. Additional hydrolases such as terminal ADP-ribose protein glycohydrolase (TARG1) and ADP-ribosylhydrolase 3 (ARH3) are also known to catalyze PAR-degradation. TARG1 and ARH3 complete the reversal of PARylation by removing protein-bound mono(ADP-ribose) moieties (a) Fontana et al., Elife 2017, doi: 10.7554/eLife.28533; b) Rack et al., Genes Dev. 2020, 34, 263). TARG1 is located in the nucleus and cytoplasm. ARH3 is found primarily in the cytoplasm but it can also be found in the mitochondria and in the nucleus (Rack et al., Genes Dev. 2020, 34, 263).

Genomic aberrations targeting tumor suppressor genes or oncogenes, often make cancer cells dependent on specific DNA repair pathways. For instance, it is well known that PARP inhibitors are particularly effective against tumors carrying mutations in the BRCA1 and BRCA2 genes (a) Bryant et al., Nature 2005, 434, 913; b) Farmer et al., Nature 2005, 434, 917). Targeting synthetic lethal interactions like the one between PARP and BRCA is an attractive novel therapeutic approach for cancer treatment.

PARG participates in DNA replication and in various DNA repair mechanisms including single-strand break (SSB) repair and replication fork restart. PARG inhibitors have shown synthetic lethal phenotype in cells with high levels of DRS caused by low expression of genes involved in DNA replication and/or replication fork stability (Pillay et al., Cancer Cell. 2019, 35, 519). Moreover, PARG inactivation, depletion or inhibition sensitizes cells to irradiation and to DNA damaging agents such as alkylating agents (e.g. temozolomide and methyl methanesulfonate) (a) Fujihara et al., Curr. Cancer Drug Targets 2009, 9, 953; b) Gogola et al., Cancer Cell 2018, 33, 1078; c) Houl et al., Nat Commun. 2019, 10, 5654).

Given the therapeutic potential of PARG inhibitors in cancer treatment, there is an increased need for the development of highly potent and selective PARG inhibitors beyond the ones that have already been described (a) James et al., ACS Chem. Biol. 2016, 11, 3179; b) Waszkowycz et al., J. Med. Chem. 2018, 61, 10767).

Certain compounds that are useful as PARG inhibitors are further disclosed in documents WO 2016/092326, WO 2016/097749 and WO 2021/055744. WO 2010/051549 A1, WO 2008/058126 A2, CN 103 694 242 A, and WO 2005/028476 A1 disclose additional compounds for the treatment of proliferative diseases.

### Summary of the Disclosure

The scope of the claimed invention is defined by the claims. The following embodiments refer to the disclosure of the present description, and not necessarily to the claimed invention.

It was an objective technical problem of the present invention to provide compounds that are cell-permeable inhibitors of PARG. The technical problem of the present invention is solved by the embodiments described herein and as characterized by the claims.

Accordingly, in a first embodiment, the present invention provides a compound of formula (I): 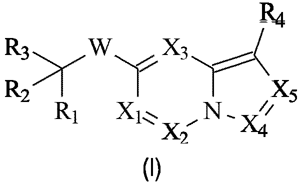 or an enantiomer, diastereoisomer, tautomer, pharmaceutically acceptable solvate, pharmaceutically acceptable crystal form or pharmaceutically acceptable salt thereof. It is understood that thought the present description the term "a compound of formula (I)" preferably encompasses also a compound of formula (la) to (Ibo), unless indicated to the contrary.

A further embodiment of the present invention relates to a pharmaceutical composition comprising the compound of formula (I) or a pharmaceutically acceptable salt, hydrate or solvate thereof, and a pharmaceutically acceptable carrier.

In a further embodiment, the present invention relates to the compound of formula (I) of the present invention or a pharmaceutically acceptable salt, hydrate or solvate thereof, or a pharmaceutical composition of the present invention, for use in therapy.

The compounds of formula (I) are useful for treating a disease or disorder in which PARG activity is implicated.

The compounds of formula (I) are useful for a method of treating a proliferative disorder. In a preferred embodiment of the present invention, the proliferative disorder is cancer, preferably a human cancer.

### Definitions

The following definitions apply throughout the present specification and the claims, unless specifically indicated otherwise.

The term "hydrogen" is herein used to refer to protium, deuterium and/or tritium, preferably to protium. Accordingly, the term "non-hydrogen atom" refers to any atoms that is not hydrogen, i.e. that is not protium, deuterium or tritium.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₅ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₅ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₅ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₅ alkylene" denotes an alkylene group having 1 to 5 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "alkenylene" refers to an alkenediyl group, i.e. a divalent unsaturated acyclic hydrocarbon group which may be linear or branched and comprises one or more (e.g., one or two) carbon-to-carbon double bonds while it does not comprise any carbon-to-carbon triple bond. A "C₂₋₅ alkenylene" denotes an alkenylene group having 2 to 5 carbon atoms. Unless defined otherwise, the term "alkenylene" preferably refers to C₂₋₄ alkenylene (including, in particular, linear C₂₋₄ alkenylene).

As used herein, the term "alkynylene" refers to an alkynediyl group, i.e. a divalent unsaturated acyclic hydrocarbon group which may be linear or branched and comprises one or more (e.g., one or two) carbon-to-carbon triple bonds and optionally one or more (e.g., one or two) carbon-to-carbon double bonds. A "C₂₋₅ alkynylene" denotes an alkynylene group having 2 to 5 carbon atoms. Unless defined otherwise, the term "alkynylene" preferably refers to C₂₋₄ alkynylene (including, in particular, linear C₂₋₄ alkynylene).

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S, N, P and Si, and the remaining ring atoms are carbon atoms, wherein one or more S ring atoms (if present) and/or one or more N ring atoms (if present) and/or one or more P ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

Preferably, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "arylene" refers to an aryl group, as defined herein above, but having two points of attachment, i.e. a divalent aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Arylene" may, e.g., refer to phenylene (e.g., phen-1,2-diyl, phen-1,3-diyl, or phen-1,4-diyl), naphthylene (e.g., naphthalen-1,2-diyl, naphthalen-1,3-diyl, naphthalen-1,4-diyl, naphthalen-1,5-diyl, naphthalen-1,6-diyl, naphthalen-1,7-diyl, naphthalen-2,3-diyl, naphthalen-2,5-diyl, naphthalen-2,6-diyl, naphthalen-2,7-diyl, or naphthalen-2,8-diyl), 1,2-dihydronaphthylene, 1,2,3,4-tetrahydronaphthylene, indanylene, indenylene, anthracenylene, phenanthrenylene, 9H-fluorenylene, or azulenylene. Unless defined otherwise, an "arylene" preferably has 6 to 14 ring atoms, more preferably 6 to 10 ring atoms, even more preferably refers to phenylene or naphthylene, and most preferably refers to phenylene (particularly phen-1,4-diyl).

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "heteroarylene" refers to a heteroaryl group, as defined herein above, but having two points of attachment, i.e. a divalent aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three, or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroarylene" may, e.g., refer to thienylene (i.e., thiophenylene; e.g., thien-2,3-diyl, thien-2,4-diyl, or thien-2,5-diyl), benzo[b]thienylene, naphtho[2,3-b]thienylene, thianthrenylene, furylene (i.e., furanylene; e.g., furan-2,3-diyl, furan-2,4-diyl, or furan-2,5-diyl), benzofuranylene, isobenzofuranylene, chromanylene, chromenylene, isochromenylene, chromonylene, xanthenylene, phenoxathiinylene, pyrrolylene, imidazolylene, pyrazolylene, pyridylene (i.e., pyridinylene), pyrazinylene, pyrimidinylene, pyridazinylene, indolylene, isoindolylene, indazolylene, indolizinylene, purinylene, quinolylene, isoquinolylene, phthalazinylene, naphthyridinylene, quinoxalinylene, cinnolinylene, pteridinylene, carbazolylene, β-carbolinylene, phenanthridinylene, acridinylene, perimidinylene, phenanthrolinylene, phenazinylene, thiazolylene (e.g., thiazol-2,4-diyl, thiazol-2,5-diyl, or thiazol-4,5-diyl), isothiazolylene (e.g., isothiazol-3,4-diyl, isothiazol-3,5-diyl, or isothiazol-4,5-diyl), phenothiazinylene, oxazolylene (e.g., oxazol-2,4-diyl, oxazol-2,5-diyl, or oxazol-4,5-diyl), isoxazolylene (e.g., isoxazol-3,4-diyl, isoxazol-3,5-diyl, or isoxazol-4,5-diyl), oxadiazolylene (e.g., 1,2,4-oxadiazol-3,5-diyl, 1,2,5-oxadiazol-3,4-diyl, or 1,3,4-oxadiazol-2,5-diyl), thiadiazolylene (e.g., 1,2,4-thiadiazol-3,5-diyl, 1,2,5-thiadiazol-3,4-diyl, or 1,3,4-thiadiazol-2,5-diyl), phenoxazinylene, pyrazolo[1,5-a]pyrimidinylene, 1,2-benzoisoxazolylene, benzothiazolylene, benzothiadiazolylene, benzoxazolylene, benzisoxazolylene, benzimidazolylene, benzo[b]thiophenylene (i.e., benzothienylene), triazolylene (e.g., 1H-1,2,3-triazolylene, 2H-1,2,3-triazolylene, 1H-1,2,4-triazolylene, or 4H-1,2,4-triazolylene), benzotriazolylene, 1H-tetrazolylene, 2H-tetrazolylene, triazinylene (e.g., 1,2,3-triazinylene, 1,2,4-triazinylene, or 1,3,5-triazinylene), furo[2,3-c]pyridinylene, dihydrofuropyridinylene (e.g., 2,3-dihydrofuro[2,3-c]pyridinylene or 1,3-dihydrofuro[3,4-c]pyridinylene), imidazopyridinylene (e.g., imidazo[1,2-a]pyridinylene or imidazo[3,2-a]pyridinylene), quinazolinylene, thienopyridinylene, tetrahydrothienopyridinylene (e.g., 4,5,6,7-tetrahydrothieno[3,2-c]pyridinylene), dibenzofuranylene, 1,3-benzodioxolylene, benzodioxanylene (e.g., 1,3-benzodioxanylene or 1,4-benzodioxanylene), or coumarinylene. Unless defined otherwise, the term "heteroarylene" preferably refers to a divalent 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroarylene" refers to a divalent 5 or 6 membered monocyclic ring comprising one or more (e.g., one, two or three) ring heteroatoms independently selected from O, S, and N, wherein one or more S ring atoms (if present) and/or one or more N ring atoms (if present) are optionally oxidized, and wherein one or more carbon ring atoms are optionally oxidized. A "heteroarylene", including any of the specific heteroarylene groups described herein, may be attached through two carbon ring atoms, particularly through those two carbon ring atoms that have the greatest distance from one another (in terms of the number of ring atoms separating them by the shortest possible connection) within one single ring or within the entire ring system of the corresponding heteroarylene.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members (e.g., cyclopropyl or cyclohexyl).

As used herein, the term "cycloalkylene" refers to a cycloalkyl group, as defined herein above, but having two points of attachment, i.e. a divalent saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkylene" may, e.g., refer to cyclopropylene (e.g., cyclopropan-1,1-diyl or cyclopropan-1,2-diyl), cyclobutylene (e.g., cyclobutan-1,1-diyl, cyclobutan-1,2-diyl, or cyclobutan-1,3-diyl), cyclopentylene (e.g., cyclopentan-1,1-diyl, cyclopentan-1,2-diyl, or cyclopentan-1,3-diyl), cyclohexylene (e.g., cyclohexan-1,1-diyl, cyclohexan-1,2-diyl, cyclohexan-1,3-diyl, or cyclohexan-1,4-diyl), cycloheptylene, decalinylene (i.e., decahydronaphthylene), or adamantylene. Unless defined otherwise, "cycloalkylene" preferably refers to a C₃₋₁₁ cycloalkylene, and more preferably refers to a C₃₋₇ cycloalkylene. A particularly preferred "cycloalkylene" is a divalent monocyclic saturated hydrocarbon ring having 3 to 7 ring members (e.g., cyclopropylene or cyclohexylene).

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S, N, P and Si and the remaining ring atoms are carbon atoms, wherein one or more S ring atoms (if present) and/or one or more N ring atoms (if present) and/or one or more P ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, 1,1-dioxothianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

Preferably, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, 1,1-dioxothianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "heterocycloalkylene" refers to a heterocycloalkyl group, as defined herein above, but having two points of attachment, i.e. a divalent saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S, N, P and Si, and the remaining ring atoms are carbon atoms, wherein one or more S ring atoms (if present) and/or one or more N ring atoms (if present) and/or one or more ring P atoms (of present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkylene" may, e.g., refer to aziridinylene, azetidinylene, pyrrolidinylene, imidazolidinylene, pyrazolidinylene, piperidinylene, piperazinylene, azepanylene, diazepanylene (e.g., 1,4-diazepanylene), oxazolidinylene, isoxazolidinylene, thiazolidinylene, isothiazolidinylene, morpholinylene, thiomorpholinylene, oxazepanylene, oxiranylene, oxetanylene, tetrahydrofuranylene, 1,3-dioxolanylene, tetrahydropyranylene, 1,4-dioxanylene, oxepanylene, thiiranylene, thietanylene, tetrahydrothiophenylene (i.e., thiolanylene), 1,3-dithiolanylene, thianylene, 1,1-dioxothianylene, thiepanylene, decahydroquinolinylene, decahydroisoquinolinylene, or 2-oxa-5-aza-bicyclo[2.2.1]hept-5-ylene. Unless defined otherwise, "heterocycloalkylene" preferably refers to a divalent 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkylene" refers to a divalent 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

Preferably, the term "heterocycloalkylene" refers to a heterocycloalkyl group, as defined herein above, but having two points of attachment, i.e. a divalent saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkylene" may, e.g., refer to aziridinylene, azetidinylene, pyrrolidinylene, imidazolidinylene, pyrazolidinylene, piperidinylene, piperazinylene, azepanylene, diazepanylene (e.g., 1,4-diazepanylene), oxazolidinylene, isoxazolidinylene, thiazolidinylene, isothiazolidinylene, morpholinylene, thiomorpholinylene, oxazepanylene, oxiranylene, oxetanylene, tetrahydrofuranylene, 1,3-dioxolanylene, tetrahydropyranylene, 1,4-dioxanylene, oxepanylene, thiiranylene, thietanylene, tetrahydrothiophenylene (i.e., thiolanylene), 1,3-dithiolanylene, thianylene, 1,1-dioxothianylene, thiepanylene, decahydroquinolinylene, decahydroisoquinolinylene, or 2-oxa-5-aza-bicyclo[2.2.1]hept-5-ylene. Unless defined otherwise, "heterocycloalkylene" preferably refers to a divalent 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkylene" refers to a divalent 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "N-heterocycloalkyl" refers to the heterocycloalkyl groups as defined hereinabove wherein said heterocycloalkyl includes at least one nitrogen atom which serves as an attachment point of said heterocycloalkyl.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S, N, P and Si, and the remaining ring atoms are carbon atoms, wherein one or more S ring atoms (if present) and/or one or more N ring atoms (if present) and/or one or more P ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

Preferably, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and **N,** and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "heterocycloalkenylene" refers to a heterocycloalkenyl group, as defined hereinabove, as defined hereinabove, but having two points of attachment, i.e. a divalent unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S, N, P and Si and the remaining ring atoms are carbon atoms, wherein one or more S ring atoms (if present) and/or one or more N ring atoms (if present) and/or one or more P ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring.

Preferably, the term "heterocycloalkenylene" refers to a heterocycloalkenyl group, as defined hereinabove, as defined hereinabove, but having two points of attachment, i.e. a divalent unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring.

As used herein, the term "halogen" refers to fluoro (-F), chloro (-CI), bromo (-Br), or iodo (-I). As it is to be understood for the skilled person, the terms "halogen" and "halo" may be used interchangeably.

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

The terms "bond" and "covalent bond" are used herein synonymously, unless explicitly indicated otherwise or contradicted by context.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

It is to be understood that wherever numerical ranges are provided/disclosed herein, all values and subranges encompassed by the respective numerical range are meant to be encompassed within the scope of the invention. Accordingly, the present invention specifically and individually relates to each value that falls within a numerical range disclosed herein, as well as each subrange encompassed by a numerical range disclosed herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

### Detailed description of the invention

The invention is described in detail in the following. It is to be understood that the present invention specifically relates to each and every combination of features and embodiments described herein, including any combination of general and/or preferred features/embodiments.

In a first embodiment, the present invention relates to a compound of formula (I): 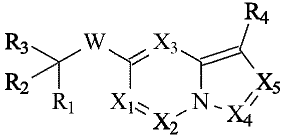 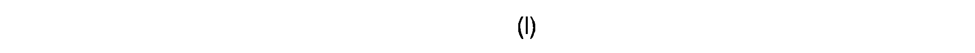 or an enantiomer, diastereoisomer, tautomer, pharmaceutically acceptable solvate, pharmaceutically acceptable crystal form or pharmaceutically acceptable salt thereof.

R₁ is selected from the group consisting of hydrogen, chloro, fluoro, cyano, formyl, (C₁₋₂)alkyl, (C₂)alkenyl, (C₂)alkynyl (C₁₋₂)haloalkyl, -(C₁₋₂ alkylene)-OH and -(C₁₋₂ alkylene)-O-(C₁₋₂ alkyl), preferably from the group consisting of chloro, fluoro, cyano, formyl, (C₁₋₂)alkyl, (C₂)alkenyl, (C₂)alkynyl, (C₁₋₂)haloalkyl, -(C₁₋₂ alkylene)-OH and -(C₁₋₂ alkylene)-O-(C₁₋₂ alkyl). Preferably, R₁ is selected from the group consisting of hydrogen, chloro, fluoro, cyano, formyl, (C₁₋₂)alkyl, (C₂)alkenyl, (C₂)alkynyl and (C₁₋₂)haloalkyl, preferably from the group consisting of chloro, fluoro, cyano, formyl, (C₁₋₂)alkyl, (C₂)alkenyl, (C₂)alkynyl and (C₁₋₂)haloalkyl. More preferably, R₁ is selected from the group consisting of cyano, (C₁₋₂)alkyl, and (C₁₋₂)haloalkyl. Preferably, (C₁₋₂)alkyl as discussed herein is methyl. Preferably, (C₁₋₂)haloalkyl as discussed herein is fluoromethyl. Thus, preferably R₁ is selected from the group consisting of cyano, methyl and fluoromethyl. More preferably, R₁ is cyano. However, in an alternative preferred embodiment, R₁ is methyl, in a particularly preferred alternative embodiment wherein R₁ is methyl, R₁ is CD₃. In again an alternative preferred embodiment, R₁ is fluoromethyl.

R₂ and R₃ are independently each (C₁₋₂)alkyl or (C₁₋₂)haloalkyl, preferably methyl, or R₂ and R₃ together with the carbon atom to which they are attached form cyclopropyl. Preferably, R₂ and R₃ together with the carbon atom to which they are attached form cyclopropyl.

W is selected from -NHS(O)_{y}-, -S(O)_{y}NH-, -NHS(O)(NH)-, -NHS(O)(NCH₃)-, -S(O)(NH)-NH-, - S(O)(NCH₃)-NH-, wherein y is 1 or 2. Preferably, y is 2. Thus, in a preferred embodiment, W is selected from -NHS(O)₂-, -S(O)₂NH-, -NHS(O)(NH)-, and -S(O)(NH)-NH-. More preferably, W is selected from - NHS(O)₂-, and -S(O)₂NH-, even more preferably W is -NHS(O)₂-. Preferably as understood herein, the left side of W as defined herein is attached to the carbon atom that carries R₁, R₂ and R₃, and the right side of W as defined herein is attached to the ring system shown in formula (I). In one preferred embodiment, W is -NHS(O)₂- or -NHS(O)(NCH₃)-. In one preferred embodiment, W is -NHS(O)(NCH₃)-.

X₁ and X₃ are independently selected from the group consisting of N, CH, C(C₁₋₂ alkyl), CCl and CF, preferably independently selected from the group consisting of N, CH and CF. Preferably, X₁ is CF or CH and X₃ is CH, more preferably X₁ and X₃ are each CH. However, in an alternative preferred embodiment, X₁ is CF and X₃ is CH.

X₂ is N or C-Y_{C2}-R_{C2}, preferably X₂ is C-Y_{C2}-R_{C2}.

Y_{C2} is selected from a covalent bond, C₁₋₅ alkylene, C₂₋₅ alkenylene, C₂₋₅ alkynylene, cycloalkylene, cycloalkenylene, heterocycloalkylene and heterocycloalkenylene, wherein said alkylene, said alkenylene and said alkynylene are each optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more -CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-, and further wherein said cycloalkylene, said cycloalkenylene, said heterocycloalkylene and said heterocycloalkenylene are each optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), - (C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl). Preferably, Y_{C2} is selected from a covalent bond, C₁₋₅ alkylene, C₂₋₅ alkenylene, C₂₋₅ alkynylene, cycloalkylene and heterocycloalkylene wherein said alkylene, said alkenylene and said alkynylene are each optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more -CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-, and further wherein said cycloalkylene and said heterocycloalkylene are each optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, - (C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, - (C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl). Preferably, Y_{C2} is selected from a covalent bond, C₁₋₅ alkylene, C₂₋₅ alkenylene, and C₂₋₅ alkynylene, wherein said alkylene, said alkenylene and said alkynylene are each optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more -CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-. More preferably, Y_{C2} is selected from a covalent bond, -(C₁₋₃ alkylene)-, -CO-(C₁₋₃ alkylene)-, (C₁₋₃ alkylene)-CO-, -CONH-(C₁₋₃ alkylene)-, -(C₁₋₃ alkylene)-CONH-, -NHCO-(C₁₋₃ alkylene)-, -(C₁₋₃ alkylene)-NHCO-, -NH-(C₁₋₃ alkylene)-, -(C₁₋₃ alkylene)-NH-, -N(C₁₋₅ alkyl)-, -O-(C₁₋₃ alkylene)-, -(C₁₋₃ alkylene)-O-, -SO₂-(C₁₋₃ alkylene)-, -(C₁₋₃ alkylene)-SO₂-, -CONH-, -NHCO-, -NH-, -O-, -CO- and -SO₂-. C₁₋₃ alkylene is herein preferably a -CH₂-group.

R_{C2} is selected from hydrogen, halo, -OH, -NH₂, -SH, -CN, C₁₋₁₂ alkyl, C₂₋₁₂ alkenyl, C₂₋₁₂ alkynyl, cycloalkyl, cycloalkenyl, heterocycloalkyl, heterocycloalkenyl, aryl, and heteroaryl. Preferably, R_{C2} is selected from hydrogen, halo, -OH, -NH₂, -SH, -CN, C₁₋₁₂ alkyl, C₂₋₁₂ alkenyl, C₂₋₁₂ alkynyl, cycloalkyl, heterocycloalkyl, aryl, and heteroaryl. Preferably, R_{C2} is selected from hydrogen, cycloalkyl, heterocycloalkyl, aryl, and heteroaryl. More preferably, R_{C2} is selected from cycloalkyl, heterocycloalkyl, aryl, and heteroaryl. Even more preferably, R_{C2} is selected from heterocycloalkyl, aryl, and heteroaryl. Said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl)-, - O(C₁₋₅ haloalkyl)-, C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl). Said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), - CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, - (C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, - (C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), - (C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, CN, OH, C₁₋₅ alkyl, C₁₋₅ haloalkyl, O(C₁₋₅ alkyl), -O(C₁₋₅ haloalkyl), SH, S(C₁₅ alkyl), S(O)(C₁₅ alkyl), S(O)₂(C₁₅ alkyl), S(O)(NH)(C₁₅ alkyl), S(O)(N(C₁₅ alkyl))(C₁₅ alkyl), -N=S(O)(C₁₅ alkyl)(C₁₅ alkyl), -S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(N-heterocycloalkyl), -CO(C₁₋₅ alkyl), COO(C₁₋₅ alkyl), CONH₂, CONH(C₁₋₅ alkyl), CON(C₁₋₅ alkyl)(C₁₋₅ alkyl), -CO-(N-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), NHCOO(C₁₋₅ alkyl), N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₅ alkyl)), -P(O)(O(C₁₋₅ alkyl))(C₁₅ alkyl), -(C₁₋₅ alkylene)-CN, -(C₁₋₅ alkylene)OH, -(C₁₋₅ alkylene)O(C₁₋₅ alkyl), -(C₁₋₅ alkylene)-O(C₁₋₅ haloalkyl), -(C₁₋₅ alkylene)SH, -(C₁₋₅ alkylene)S(C₁₅ alkyl), -(C₁₋₅ alkylene)-S(C₁₋₅ haloalkyl), -(C₁₋₅ alkylene)S(O)(C₁₅ alkyl), -(C₁₋₅ alkylene)S(O)₂(C₁₅ **alkyl),** -(C₁₋₅ alkylene)S(O)(NH)(C₁₅ alkyl), -(C₁₋₅ alkylene)S(O)(N(C₁₅ alkyl))(C₁₅ alkyl), -(C₁₋₅ alkylene)-P(O)(C₁₋₅ alkyl)(C₁₅ alkyl), -(C₁₋₅ alkylene)-P(O)(O(C₁₋₅ alkyl))(O(C₁₅ alkyl)), -(C₁₋₅ alkylene)-P(O)(O(C₁₋₅ alkyl))(C₁₋₅ alkyl), -(C₁₋₅ alkylene)NH₂, -(C₁₋₅ alkylene)NH(C₁₋₅ alkyl), -(C₁₋₅ alkylene)-NH(C₁₋₅ haloalkyl), -(C₁₋₅ alkylene)N(C₁₋₅ alkyl)(C₁₋₅ alkyl), -(C₁₋₅ alkylene)N(C₁₋₅ alkyl)(C₁₋₅ haloalkyl), -(C₁₋₅ alkylene)(N-heterocycloalkyl), -(C₁₋₅ alkylene)N(C₁₋₅ haloalkyl)(C₁₋₅ alkyl), -(C₁₋₅ alkylene)-CO(C₁₋₅ alkyl), -(C₁₋₅ alkylene)CONH₂, -(C₁₋₅ alkylene)CONH(C₁₋₅ alkyl), -(C₁₋₅ alkylene)CON(C₁₋₅ alkyl)(C₁₋₅ alkyl), -(C₁₋₅ alkylene)CO-(N-heterocycloalkyl), -(C₁₋₅ alkylene)NHCO-(C₁₋₅ alkyl), -(C₁₋₅ alkylene)N(C₁₋₅ alkyl)-CO-(C₁₋₅ alkyl), -(C₁₋₅ alkylene)NHCONH₂, -(C₁₋₅ alkylene)NHCONH-(C₁₋₅ alkyl), -(C₁₋₅ alkylene)NHCON(C₁₋₅ alkyl)(C₁₋₅ alkyl), -(C₁₋₅ alkylene)N(C₁₋₅ alkyl)CONH₂, -(C₁₋₅ alkylene)N(C₁₋₅ alkyl)CONH-(C₁₋₅ alkyl), -(C₁₋₅ alkylene)N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), (C₁₋₅ alkylene)NHCOO(C₁₋₅ alkyl), and -(C₁₋₅ alkylene)N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N-*heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), - P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

Thus, preferably, -Y_{C2}-R_{C2} is selected from -O-C₁₋₁₂ alkyl, -NH-C₁₋₁₂ alkyl, -N(C₁₋₅ alkyl)-C₁₋₁₂ alkyl, -O-C₂₋₁₂ alkenyl, -NH-C₂₋₁₂ alkenyl, -N(C₁₋₅ alkyl)-C₂₋₁₂ alkenyl, -O-C₂₋₁₂ alkynyl, -NH-C₂₋₁₂ alkynyl, -N(C₁₋₅ alkyl)-C₂₋₁₂ alkynyl, (C₀₋₃ alkylene)-cycloalkyl, -CO-(C₀₋₃ alkylene)cycloalkyl, (C₀₋₃ alkylene)-CO-cycloalkyl, -CONH-(C₀₋₃ alkylene)cycloalkyl, -(C₀₋₃ alkylene)-CONH-cycloalkyl, -NHCO-(C₀₋₃ alkylene)cycloalkyl, (C₀₋₃ alkylene)-NHCO-cycloalkyl, -NH-(C₀₋₃ alkylene)cycloalkyl, -(C₀₋₃ alkylene)-NH-cycloalkyl, -O-(C₀₋₃ alkylene)cycloalkyl, -(C₀₋₃ alkylene)-O-cycloalkyl, -SO₂-(C₀₋₃ alkylene)cycloalkyl, -(C₀₋₃ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, (C₀₋₃ alkylene)-cycloalkenyl, -CO-(C₀₋₃ alkylene)cycloalkenyl, (C₀₋₃ alkylene)-CO-cycloalkenyl, -CONH-(C₀₋₃ alkylene)cycloalkenyl, -(C₀₋₃ alkylene)-CONH-cycloalkenyl, -NHCO-(C₀₋₃ alkylene)cycloalkenyl, (C₀₋₃ alkylene)-NHCO-cycloalkenyl, -NH-(C₀₋₃ alkylene)cycloalkenyl, -(C₀₋₃ alkylene)-NH-cycloalkenyl, -O-(C₀₋₃ alkylene)cycloalkenyl, -(C₀₋₃ alkylene)-O-cycloalkenyl, -SO₂-(C₀₋₃ alkylene)cycloalkenyl, -(C₀₋₃ alkylene)-SO₂-cycloalkenyl, -CONH-cycloalkenyl, -NHCO-cycloalkenyl, -NH-cycloalkenyl, -O-cycloalkenyl, -CO-cycloalkenyl, -SO₂-cycloalkenyl, -(C₀₋₃ alkylene)-heterocycloalkyl, - CO-(C₀₋₃ alkylene)heterocycloalkyl, -(C₀₋₃ alkylene)-CO-heterocycloalkyl, -CONH-(C₀₋₃ alkylene)heterocycloalkyl, -(C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, - (C₀₋₃ alkylene)-NH-heterocycloalkyl, -O-(C₀₋₃ alkylene) heterocycloalkyl, -(C₀₋₃ alkylene)-O-heterocycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)-heterocycloalkenyl, -CO-(C₀₋₃ alkylene)heterocycloalkenyl, -(C₀₋₃ alkylene)-CO-heterocycloalkenyl, -CONH-(C₀₋₃ alkylene)heterocycloalkenyl, -(C₀₋₃ alkylene)-CONH-heterocycloalkenyl, -NHCO-(C₀₋₃ alkylene)heterocycloalkenyl, -(C₀₋₃ alkylene)-NHCO-heterocycloalkenyl, -NH-(C₀₋₃ alkylene)heterocycloalkenyl, -(C₀₋₃ alkylene)-NH-heterocycloalkenyl, -O-(C₀₋₃ alkylene) heterocycloalkenyl, -(C₀₋₃ alkylene)-O-heterocycloalkenyl, -SO₂-(C₀₋₃ alkylene)heterocycloalkenyl, -(C₀₋₃ alkylene)-SO₂-heterocycloalkenyl, -CONH-heterocycloalkenyl, -NHCO-heterocycloalkenyl, -NH-heterocycloalkenyl, -O-heterocycloalkenyl, -CO-heterocycloalkenyl, -SO₂-heterocycloalkenyl, (C₀₋₃ alkylene)aryl, -CO-(C₀₋₃ alkylene)aryl, -(C₀₋₃ alkylene)-CO-aryl, -CONH-(C₀₋₃ alkylene)aryl, -(C₀₋₃ alkylene)-CONH-aryl, -NHCO-(C₀₋₃ alkylene)aryl, -(C₀₋₃ alkylene)-NHCO-aryl, -NH-(C₀₋₃ alkylene)aryl, - (C₀₋₃ alkylene)-NH-aryl, -O-(C₀₋₃ alkylene)aryl, -(C₀₋₃ alkylene)-0-aryl, -SO₂-(C₀₋₃ alkylene)aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)heteroaryl, -CO-(C₀₋₃ alkylene)heteroaryl, -(C₀₋₃ alkylene)-CO-heteroaryl, -CONH-(C₀₋₃ alkylene)heteroaryl, -(C₀₋₃ alkylene)-CONH-heteroaryl, -NHCO-(C₀₋₃ alkylene)heteroaryl, -(C₀₋₃ alkylene)-NHCO-heteroaryl, -NH-(C₀₋₃ alkylene)heteroaryl, (C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, -CO-heteroaryl and -SO₂-heteroaryl, preferably -Y_{C2}-R_{C2} is selected from -O-C₁₋₁₂ alkyl, -NH-C₁₋₁₂ alkyl, -N(C₁₋₅ alkyl)-C₁₋₁₂ alkyl, -O-C₂₋₁₂ alkenyl, -NH-C₂₋₁₂ alkenyl, -N(C₁₋₅ alkyl)-C₂₋₁₂ alkenyl, -O-C₂₋₁₂ alkynyl, -NH-C₂₋₁₂ alkynyl, -N(C₁₋₅ alkyl)-C₂₋₁₂ alkynyl, (C₀₋₃ alkylene)-cycloalkyl, -CO-(C₀₋₃ alkylene)cycloalkyl, (C₀₋₃ alkylene)-CO-cycloalkyl, -CONH-(C₀₋₃ alkylene)cycloalkyl, -(C₀₋₃ alkylene)-CONH-cycloalkyl, -NHCO-(C₀₋₃ alkylene)cycloalkyl, (C₀₋₃ alkylene)-NHCO-cycloalkyl, -NH-(C₀₋₃ alkylene)cycloalkyl, -(C₀₋₃ alkylene)-NH-cycloalkyl, -O-(C₀₋₃ alkylene)cycloalkyl, -(C₀₋₃ alkylene)-O-cycloalkyl, -SO₂-(C₀₋₃ alkylene)cycloalkyl, -(C₀₋₃ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, -(C₀₋₃ alkylene)-heterocycloalkyl, -CO-(C₀₋₃ alkylene)heterocycloalkyl, -(C₀₋₃ alkylene)-CO-heterocycloalkyl, -CONH-(C₀₋₃ alkylene)heterocycloalkyl, -(C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, -(C₀₋₃ alkylene)-NH-heterocycloalkyl, -O-(C₀₋₃ alkylene) heterocycloalkyl, -(C₀₋₃ alkylene)-O-cycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, (C₀₋₃ alkylene)aryl, -CO-(C₀₋₃ alkylene)aryl, -(C₀₋₃ alkylene)-CO-aryl, -CONH-(C₀₋₃ alkylene)aryl, -(C₀₋₃ alkylene)-CONH-aryl, -NHCO-(C₀₋₃ alkylene)aryl, -(C₀₋₃ alkylene)-NHCO-aryl, -NH-(C₀₋₃ alkylene)aryl, -(C₀₋₃ alkylene)-NH-aryl, -O-(C₀₋₃ alkylene)aryl, -(C₀₋₃ alkylene)-O-aryl, -SO₂-(C₀₋₃ alkylene)aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)heteroaryl, -CO-(C₀₋₃ alkylene)heteroaryl, -(C₀₋₃ alkylene)-CO-heteroaryl, - CONH-(C₀₋₃ alkylene)heteroaryl, -(C₀₋₃ alkylene)-CONH-heteroaryl, -NHCO-(C₀₋₃ alkylene)heteroaryl, - (C₀₋₃ alkylene)-NHCO-heteroaryl, -NH-(C₀₋₃ alkylene)heteroaryl, (C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, -CO-heteroaryl and -SO₂-heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₃ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl), -O(C₁₋₅ haloalkyl), -C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl) and wherein said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, **-**(C₁₋₅ alkylene)-O(C₁₋₅ alkyl), **-**(C₁₋₅ alkylene)-O(C₁₋₅ haloalkyl), -(C₁₋₅ alkylene)-SH, **-**(C₁₋₅ alkylene)-S(C₁₋₅ alkyl), **-**(C₁₋₅ alkylene)-S(C₁₋₅ haloalkyl), **-**(C₁₋₅ alkylene)-S(O)(C₁₋₅ alkyl), **-**(C₁₋₅ alkylene)-S(O)₂(C₁₋₅ alkyl), **-**(C₁₋₅ alkylene)-S(O)(NH)(C₁₋₅ alkyl), **-**(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), **-**(C₁₋₅ alkylene)-P(O)(O(C₁₋₅ alkyl))(O(C₁₋₅ alkyl)), **-**(C₁₋₅ alkylene)-P(O)(O(C₁₋₅ alkyl))(C₁₋₅ alkyl), **-**(C₁₋₅ alkylene)-NH₂, **-**(C₁₋₅ alkylene)-NH(C₁₋₅ alkyl), **-**(C₁₋₅ alkylene)-NH(C₁₋₅ haloalkyl), **-**(C₁₋₅ alkylene)-N(C₁₋₅ alkyl)(C₁₋₅ alkyl), **-**(C₁₋₅ alkylene)-N(C₁₋₅ alkyl)(C₁₋₅ haloalkyl), -(C₁₋₅ alkylene)-(N-heterocycloalkyl), **-**(C₁₋₅ alkylene)-N(C₁₋₅ haloalkyl)(C₁₋₅ alkyl), -(C₁₋₅ alkylene)-CO(C₁₋₅ alkyl), **-**(C₁₋₅ alkylene)-CO(C₁₋₅ haloalkyl), -(C₁₋₅ alkylene)-CO-cycloalkyl, -(C₁₋₅ alkylene)-CONH₂, **-**(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), **-**(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), **-**(C₁₋₅ alkylene)-N(C₁₋₅ alkyl)CON(C₁₋₅ alkyl)(C₁₋₅ alkyl), **-**(C₁₋₅ alkylene)-OCONH₂, **-**(C₁₋₅ alkylene)-OCONH-(C₁₋₅ alkyl), **-**(C₁₋₅ alkylene)-OCON(C₁₋₅ alkyl)(C₁₋₅ alkyl), **-**(C₁₋₅ alkylene)-NHCOO(C₁₋₅ alkyl), and **-**(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, - (C₁₋₅ alkylene)-OH, -(C₁₋₅ alkylene)-O(C₁₋₅ alkyl), **-**(C₁₋₅ alkylene)-O(C₁₋₅ haloalkyl), -(C₁₋₅ alkylene)-SH, - (C₁₋₅ alkylene)-S(C₁₋₅ alkyl), **-**(C₁₋₅ alkylene)-S(C₁₋₅ haloalkyl), **-**(C₁₋₅ alkylene)-S(O)(C₁₋₅ alkyl), **-**(C₁₋₅ alkylene)-S(O)₂(C₁₋₅ alkyl), **-**(C₁₋₅ alkylene)-S(O)(NH)(C₁₋₅ alkyl), **-**(C₁₋₅ alkylene)-S(O)(N(C₁₋₅ alkyl))(C₁₋₅ alkyl), **-**(C₁₋₅ alkylene)-P(O)(C₁₋₅ alkyl)(C₁₋₅ alkyl), **-**(C₁₋₅ alkylene)-P(O)(O(C₁₋₅ alkyl))(O(C₁₋₅ alkyl)), **-**(C₁₋₅ alkylene)-P(O)(O(C₁₋₅ alkyl))(C₁₋₅ alkyl), **-**(C₁₋₅ alkylene)-NH₂, **-**(C₁₋₅ alkylene)-NH(C₁₋₅ alkyl), **-**(C₁₋₅ alkylene)-NH(C₁₋₅ haloalkyl), **-**(C₁₋₅ alkylene)-N(C₁₋₅ alkyl)(C₁₋₅ alkyl), **-**(C₁₋₅ alkylene)-N(C₁₋₅ alkyl)(C₁₋₅ haloalkyl), **-**(C₁₋₅ alkylene)-(N-heterocycloalkyl), **-**(C₁₋₅ alkylene)-N(C₁₋₅ haloalkyl)(C₁₋₅ alkyl), **-**(C₁₋₅ alkylene)-CO(C₁₋₅ alkyl), **-**(C₁₋₅ alkylene)-CONH₂, **-**(C₁₋₅ alkylene)-CONH(C₁₋₅ alkyl), **-**(C₁₋₅ alkylene)-CON(C₁₋₅ alkyl)(C₁₋₅ alkyl), **-**(C₁₋₅ alkylene)-CO-(N-heterocycloalkyl), **-**(C₁₋₅ alkylene)-NHCO-(C₁₋₅ alkyl), **-**(C₁₋₅ alkylene)-N(C₁₋₅ alkyl)-CO-(C₁₋₅ alkyl), **-**(C₁₋₅ alkylene)-NHCONH₂, **-**(C₁₋₅ alkylene)-NHCONH-(C₁₋₅ alkyl), **-**(C₁₋₅ alkylene)-NHCON(C₁₋₅ alkyl)(C₁₋₅ alkyl), **-**(C₁₋₅ alkylene)-N(C₁₋₅ alkyl)CONH₂, **-**(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), **-**(C₁₋₅ alkylene)-OCONH₂, **-**(C₁₋₅ alkylene)-OCONH-(C₁₋₅ alkyl), **-**(C₁₋₅ alkylene)-OCON(C₁₋₅ alkyl)(C₁₋₅ alkyl), **-**(C₁₋₅ alkylene)-NHCOO(C₁₋₅ alkyl), and **-**(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), - P(O)(O(C₁₋₅ alkyl))(C₁₋₅ alkyl), -(C₁₋₅ alkylene)-CN, **-**(C₁₋₅ alkylene)-OH, -(C₁₋₅ alkylene)-O(C₁₋₅ alkyl), -(C₁₋₅ alkylene)-O(C₁₋₅ haloalkyl), **-**(C₁₋₅ alkylene)-SH, **-**(C₁₋₅ alkylene)-S(C₁₋₅ alkyl), **-**(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), **-**(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), **-**(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), **-**(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), **-**(C₁₋₅ alkylene)-OH, **-**(C₁₋₅ alkylene)-O(C₁₋₅ alkyl), **-**(C₁₋₅ alkylene)-O(C₁₋₅ haloalkyl), -SH, -S(C₁₋₅ alkyl), -S(C₁₋₅ haloalkyl), **-**(C₁₋₅ alkylene)-SH, **-**(C₁₋₅ alkylene)-S(C₁₋₅ alkyl), **-**(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), **-**(C₁₋₅ alkylene)-NH₂, **-**(C₁₋₅ alkylene)-NH(C₁₋₅ alkyl), **-**(C₁₋₅ alkylene)-NH(C₁₋₅ haloalkyl), **-**(C₁₋₅ alkylene)-N(C₁₋₅ alkyl)(C₁₋₅ alkyl), **-**(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, **-CN,** -OH, C₁₋₅ alkyl, C₁₋₅ haloalkyl, -O(C₁₋₃ alkyl), -O(C₁₋₃ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

More preferably, -Y_{C2}-R_{C2} is selected from -(C₀₋₃ alkylene)-heterocycloalkyl, -CO-(C₀₋₃ alkylene)heterocycloalkyl, -(C₀₋₃ alkylene)-CO-heterocycloalkyl, -CONH-(C₀₋₃ alkylene)heterocycloalkyl, - (C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, -(C₀₋₃ alkylene)-NH-heterocycloalkyl, -O-(C₀₋₃ alkylene) heterocycloalkyl, (C₀₋₃ alkylene)-O-cycloalkyl, (C₀₋₃ alkylene)-O-heterocycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)-heterocycloalkenyl, -CO-(C₀₋₃ alkylene)heterocycloalkenyl, -(C₀₋₃ alkylene)-CO-heterocycloalkenyl, -CONH-(C₀₋₃ alkylene)heterocycloalkenyl, -(C₀₋₃ alkylene)-CONH-heterocycloalkenyl, -NHCO-(C₀₋₃ alkylene)heterocycloalkenyl, -(C₀₋₃ alkylene)-NHCO-heterocycloalkenyl, -NH-(C₀₋₃ alkylene)heterocycloalkenyl, -(C₀₋₃ alkylene)-NH-heterocycloalkenyl, -O-(C₀₋₃ alkylene) heterocycloalkenyl, (C₀₋₃ alkylene)-0-heterocycloalkenyl, -SO₂-(C₀₋₃ alkylene)heterocycloalkenyl, -(C₀₋₃ alkylene)-SO₂-heterocycloalkenyl, -CONH-heterocycloalkenyl, -NHCO-heterocycloalkenyl, -NH-heterocycloalkenyl, -O-heterocycloalkenyl, -CO-heterocycloalkenyl, -SO₂-heterocycloalkenyl, -(C₀₋₃ alkylene)aryl, -CO-(C₀₋₃ alkylene)aryl, -(C₀₋₃ alkylene)-CO-aryl, -CONH-(C₀₋₃ alkylene)aryl, -(C₀₋₃ alkylene)-CONH-aryl, -NHCO-(C₀₋₃ alkylene)aryl, -(C₀₋₃ alkylene)-NHCO-aryl, -NH-(C₀₋₃ alkylene)aryl, - (C₀₋₃ alkylene)-NH-aryl, -O-(C₀₋₃ alkylene)aryl, -(C₀₋₃ alkylene)-O-aryl, -SO₂-(C₀₋₃ alkylene)aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)heteroaryl, -CO-(C₀₋₃ alkylene)heteroaryl, -(C₀₋₃ alkylene)-CO-heteroaryl, -CONH-(C₀₋₃ alkylene)heteroaryl, -(C₀₋₃ alkylene)-CONH-heteroaryl, -NHCO-(C₀₋₃ alkylene)heteroaryl, -(C₀₋₃ alkylene)-NHCO-heteroaryl, -NH-(C₀₋₃ alkylene)heteroaryl, -(C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, -CO-heteroaryl and -SO₂-heteroaryl, preferably -Y_{C2}-R_{C2} is selected from -(C₀₋₃ alkylene)-heterocycloalkyl, -CO-(C₀₋₃ alkylene)heterocycloalkyl, -(C₀₋₃ alkylene)-CO-heterocycloalkyl, -CONH-(C₀₋₃ alkylene)heterocycloalkyl, - (C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, -(C₀₋₃ alkylene)-NH-heterocycloalkyl, -O-(C₀₋₃ alkylene) heterocycloalkyl, (C₀₋₃ alkylene)-O-cycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)aryl, -CO-(C₀₋₃ alkylene)aryl, -(C₀₋₃ alkylene)-CO-aryl, -CONH-(C₀₋₃ alkylene)aryl, -(C₀₋₃ alkylene)-CONH-aryl, -NHCO-(C₀₋₃ alkylene)aryl, -(C₀₋₃ alkylene)-NHCO-aryl, -NH-(C₀₋₃ alkylene)aryl, -(C₀₋₃ alkylene)-NH-aryl, -O-(C₀₋₃ alkylene)aryl, -(C₀₋₃ alkylene)-O-aryl, -SO₂-(C₀₋₃ alkylene)aryl, -(C₀₋₃ alkylene)-SO₂-aryl, -CONH-aryl, - NHCO-aryl, -NH-aryl, -O-aryl, -CO-aryl, -SO₂-aryl, -(C₀₋₃ alkylene)heteroaryl, -CO-(C₀₋₃ alkylene)heteroaryl, -(C₀₋₃ alkylene)-CO-heteroaryl, -CONH-(C₀₋₃ alkylene)heteroaryl, -(C₀₋₃ alkylene)-CONH-heteroaryl, -NHCO-(C₀₋₃ alkylene)heteroaryl, -(C₀₋₃ alkylene)-NHCO-heteroaryl, -NH-(C₀₋₃ alkylene)heteroaryl, -(C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, -CO-heteroaryl and -SO₂-heteroaryl, wherein said heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), - CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), **-**(C₁₋₅ alkylene)-O(C₁₋₅ haloalkyl), -(C₁₋₅ alkylene)-SH, -(C₁₋₅ alkylene)-S(C₁₋₅ alkyl), **-**(C₁₋₅ alkylene)-S(C₁₋₅ haloalkyl), -(C₁₋₅ alkylene)-S(O)(C₁₋₅ alkyl), **-**(C₁₋₅ alkylene)-S(O)₂(C₁₋₅ alkyl), **-**(C₁₋₅ alkylene)-S(O)(NH)(C₁₋₅ alkyl), **-**(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), **-**(C₁₋₅ alkylene)-N(C₁₋₅ alkyl)(C₁₋₅ haloalkyl), **-**(C₁₋₅ alkylene)-(N-heterocycloalkyl), **-**(C₁₋₅ alkylene)-N(C₁₋₅ haloalkyl)(C₁₋₅ alkyl), **-**(C₁₋₅ alkylene)-CO(C₁₋₅ alkyl), **-**(C₁₋₅ alkylene)-CO(C₁₋₅ haloalkyl), - (C₁₋₅ alkylene)-CO-cycloalkyl, **-**(C₁₋₅ alkylene)-CONH₂, **-**(C₁₋₅ alkylene)-CONH(C₁₋₅ alkyl), **-**(C₁₋₅ alkylene)-CON(C₁₋₅ alkyl)(C₁₋₅ alkyl), **-**(C₁₋₅ alkylene)-CO-(N-heterocycloalkyl), **-**(C₁₋₅ alkylene)-NHCO-(C₁₋₅ alkyl), **-**(C₁₋₅ alkylene)-N(C₁₋₅ alkyl)-CO-(C₁₋₅ alkyl), **-**(C₁₋₅ alkylene)-NHCONH₂, **-**(C₁₋₅ alkylene)-NHCONH-(C₁₋₅ alkyl), **-**(C₁₋₅ alkylene)-NHCON(C₁₋₅ alkyl)(C₁₋₅ alkyl), **-**(C₁₋₅ alkylene)-N(C₁₋₅ alkyl)CONH₂, **-**(C₁₋₅ alkylene)-N(C₁₋₅ alkyl)CONH-(C₁₋₅ alkyl), **-**(C₁₋₅ alkylene)-N(C₁₋₅ alkyl)CON(C₁₋₅ alkyl)(C₁₋₅ alkyl), **-**(C₁₋₅ alkylene)-OCONH₂, **-**(C₁₋₅ alkylene)-OCONH-(C₁₋₅ alkyl), **-**(C₁₋₅ alkylene)-OCON(C₁₋₅ alkyl)(C₁₋₅ alkyl), **-**(C₁₋₅ alkylene)-NHCOO(C₁₋₅ alkyl), and **-**(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₃ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), **-**(C₁₋₅ alkylene)-O(C₁₋₅ haloalkyl), -(C₁₋₅ alkylene)-SH, **-**(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), - (C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, (C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

More preferably, -Y_{C2}-R_{C2} is selected from -(C₀₋₃ alkylene)-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)-heterocycloalkenyl, -CONH-heterocycloalkenyl, - NHCO-heterocycloalkenyl, -NH-heterocycloalkenyl, -O-heterocycloalkenyl, -CO-heterocycloalkenyl, -SO₂-heterocycloalkenyl, -(C₀₋₃ alkylene)aryl, -CONH-aryl, -NHCO-aryl, -NH-aryl, -O-aryl, -CO-aryl, -SO₂-aryl, -(C₀₋₃ alkylene)heteroaryl, -CONH-heteroaryl, -NHCO-heteroaryl, -NH-heteroaryl, -O-heteroaryl, -CO-heteroaryl and -SO₂-heteroaryl, preferably -Y_{C2}-R_{C2} is selected from -(C₀₋₃ alkylene)-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)aryl, -CONH-aryl, -NHCO-aryl, -NH-aryl, -O-aryl, -CO-aryl, -SO₂-aryl, -(C₀₋₃ alkylene)heteroaryl, -CONH-heteroaryl, -NHCO-heteroaryl, -NH-heteroaryl, -O-heteroaryl, -CO-heteroaryl and -SO₂-heteroaryl, wherein said heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), - CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₃ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), (C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), - (C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), **-(C₁₋₅** alkylene)-OCON(C₁₋₅ alkyl)(C₁₋₅ alkyl), -(C₁₋₅ alkylene)-NHCOO(C₁₋₅ alkyl), and **-(C₁₋₅** alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₃ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), **-(C₁₋₅** alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), **-(C₁₋₅** alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), - (C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

Even more preferably, -Y_{C2}-R_{C2} is selected from -(C₀₋₃ alkylene)-heterocycloalkyl, -(C₀₋₃ alkylene)-heterocycloalkenyl, -(C₀₋₃ alkylene)aryl, and -(C₀₋₃ alkylene)heteroaryl, preferably -Y_{C2}-R_{C2} is selected from -(C₀₋₃ alkylene)-heterocycloalkyl, -(C₀₋₃ alkylene)aryl, and -(C₀₋₃ alkylene)heteroaryl, wherein said heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), - CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), - (C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₆ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), - (C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₃ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N-*heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₆ alkyl)(C₁₋₅ alkyl).

Even more preferably, -Y_{C2}-R_{C2} is selected from heterocycloalkyl, heterocycloalkenyl, aryl, and heteroaryl, more preferably heterocycloalkyl, aryl, and heteroaryl, more preferably heterocycloalkyl and heteroaryl, even more preferably, heterocycloalkyl, wherein said heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), - CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, - (C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, - (C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₃ alkyl))(O(C₁₋₅ alkyl)), -(C₁₋₅ alkylene)-P(O)(O)(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N*-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁-₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), - (C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, - (C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, - (C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N*-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), - CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), - P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₃ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N-*heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

In one embodiment, -Y_{C2}-R_{C2} is heterocycloalkenyl, wherein said heterocycloalkenyl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₃ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N-*heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N-*heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, - (C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, - (C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O)(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₃ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), - P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O)(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

Preferably, if -Y_{C2}-R_{C2} is aryl, -Y_{C2}-R_{C2} is phenyl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), - CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁-₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), - (C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), - (C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), - P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N-*heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

Preferably, if -Y_{C2}-R_{C2} is heteroaryl, -Y_{C2}-R_{C2} is imidazolyl, pyridazinyl, thiazolyl, pyridinyl, pyrimidinyl, pyrazinyl, or indazolyl, wherein heteroaryl may be optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), - P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), (C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), - (C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), - O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

Preferably, if -Y_{C2}-R_{C2} is heterocycloalkyl, -Y_{C2}-R_{C2} is morpholinyl, 1,1-dioxothiomorpholinyl, azetinyl, pyrrolidinyl, piperidinyl, 6-oxo-1,6- dihydropyridinyl, or piperazinyl, wherein heterocycloalkyl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, - (C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, - (C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), - P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from, halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl). More preferably, -Y_{C2}-R_{C2} is piperazinyl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), - (C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), - CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), - (C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl). Even more preferably, -Y_{C2}-R_{C2} is piperazinyl (preferably N-piperazinyl) optionally substituted (preferably N-substituted) with -CO(C₁₋₅ alkyl), -CONH₂, -CONH(C₁₋₅ alkyl), and -CON(C₁₋₅ alkyl)(C₁₋₅ alkyl). Most preferably, -Y_{C2}-R_{C2} is piperazinyl (preferably N-piperazinyl) substituted (preferably N-substituted, preferably at a different N-atom than that attached to the ring system as shown in formula (I)), with -CON(C₁₋₅ alkyl)(C₁₋₅ alkyl), preferably with -CON(CH₃)₂.

Preferably, if -Y_{C2}-R_{C2} is heterocycloalkenyl, -Y_{C2}-R_{C2} is oxacyclohexenyl or azacyclohexenyl, wherein heterocycloalkenyl is optionally substituted with one or more groups independently selected from halogen, CN, OH, C₁₋₅ alkyl, C₁₋₅ haloalkyl, O(C₁₋₅ alkyl), -O(C₁₋₅ haloalkyl), SH, S(C₁₋₅ alkyl), S(O)(C₁₋₅ alkyl), S(O)₂(C₁₋₅ alkyl), S(O)(NH)(C₁₋₅ alkyl), S(O)(N(C₁₋₅ alkyl))(C₁₋₅ alkyl), -N=S(O)(C₁₋₅ alkyl)(C₁₋₅ alkyl), - S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(N-heterocycloalkyl), -CO(C₁₋₅ alkyl), -CO(C₁₋₅ haloalkyl), -CO-cycloalkyl, COO(C₁₋₅ alkyl), - COO(C₁₋₅ haloalkyl), -COO-cycloalkyl, CONH₂, CONH(C₁₋₅ alkyl), CON(C₁₋₅ alkyl)(C₁₋₅ alkyl), -CO-(N-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), NHCOO(C₁₋₅ alkyl), N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)OH, -(C₁₋₅ alkylene)O(C₁₋₅ alkyl), -(C₁₋₅ alkylene)-O(C₁₋₅ haloalkyl), -(C₁₋₅ alkylene)SH, -(C₁₋₅ alkylene)S(C₁₋₅ alkyl), -(C₁₋₅ alkylene)-S(C₁₋₅ haloalkyl), -(C₁₋₅ alkylene)S(O)(C₁₅ alkyl), -(C₁₋₅ alkylene)S(O)₂(C₁₅ alkyl), -(C₁₋₅ alkylene)S(O)(NH)(C₁₅ alkyl), -(C₁₋₅ alkylene)S(O)(N(C₁₅ alkyl))(C₁₅ alkyl), -(C₁₋₅ alkylene)-P(O)(C₁₋₅ alkyl)(C₁₅ alkyl), -(C₁₋₅ alkylene)-P(O)(O(C₁₋₅ alkyl))(O(C₁₅ alkyl)), -(C₁₋₅ alkylene)-P(O)(O(C₁₋₅ alkyl))(C₁₋₅ alkyl), -(C₁₋₅ alkylene)NH₂, -(C₁₋₅ alkylene)NH(C₁₋₅ alkyl), -(C₁₋₅ alkylene)-NH(C₁₋₅ haloalkyl), -(C₁₋₅ alkylene)N(C₁₋₅ alkyl)(C₁₋₅ alkyl), -(C₁₋₅ alkylene)N(C₁₋₅ alkyl)(C₁₋₅ haloalkyl), -(C₁₋₅ alkylene)(N-heterocycloalkyl), -(C₁₋₅ alkylene)N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), - (C₁₋₅ alkylene)-CO-cycloalkyl, -(C₁₋₅ alkylene)CONH₂, -(C₁₋₅ alkylene)CONH(C₁₋₅ alkyl), -(C₁₋₅ alkylene)CON(C₁₋₅ alkyl)(C₁₋₅ alkyl), -(C₁₋₅ alkylene)CO-(N-heterocycloalkyl), -(C₁₋₅ alkylene)NHCO-(C₁₋₅ alkyl), -(C₁₋₅ alkylene)N(C₁₋₅ alkyl)-CO-(C₁₋₅ alkyl), -(C₁₋₅ alkylene)NHCONH₂, -(C₁₋₅ alkylene)NHCONH-(C₁₋₅ alkyl), -(C₁₋₅ alkylene)NHCON(C₁₋₅ alkyl)(C₁₋₅ alkyl), -(C₁₋₅ alkylene)N(C₁₋₅ alkyl)CONH₂, -(C₁₋₅ alkylene)N(C₁₋₅ alkyl)CONH-(C₁₋₅ alkyl), -(C₁₋₅ alkylene)N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), - (C₁₋₅ alkylene)NHCOO(C₁₋₅ alkyl), and -(C₁₋₅ alkylene)N(C₁₋₅ alkyl)COO-(C₁₋₅ alkyl), preferably selected from halogen, CN, OH, C₁₋₅ alkyl, C₁₋₅ haloalkyl, O(C₁₋₅ alkyl), -O(C₁₋₅ haloalkyl), SH, S(C₁₅ alkyl), S(O)(C₁₅ alkyl), S(O)₂(C₁₅ alkyl), S(O)(NH)(C₁₅ alkyl), S(O)(N(C₁₅ alkyl))(C₁₅ alkyl), -N=S(O)(C₁₅ alkyl)(C₁₅ alkyl), - S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(N-heterocycloalkyl), -CO(C₁₋₅ alkyl), COO(C₁₋₅ alkyl), CONH₂, CONH(C₁₋₅ alkyl), CON(C₁₋₅ alkyl)(C₁₋₅ alkyl), -CO-(N-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), NHCOO(C₁₋₅ alkyl), N(C₁₋₅ alkyl)COO-(C₁₋₅ alkyl), -P(O)(C₁₋₅ alkyl)(C₁₅ alkyl), -P(O)(O(C₁₋₅ alkyl))(O(C₁₅ alkyl)), -P(O)(O(C₁₋₅ alkyl))(C₁₋₅ alkyl), -(C₁₋₅ alkylene)-CN, -(C₁₋₅ alkylene)OH, -(C₁₋₅ alkylene)O(C₁₋₅ alkyl), -(C₁₋₅ alkylene)-O(C₁₋₅ haloalkyl), -(C₁₋₅ alkylene)SH, -(C₁₋₅ alkylene)S(C₁₅ alkyl), -(C₁₋₅ alkylene)-S(C₁₋₅ haloalkyl), -(C₁₋₅ alkylene)S(O)(C₁₅ alkyl), -(C₁₋₅ alkylene)S(O)₂(C₁₅ alkyl), -(C₁₋₅ alkylene)S(O)(NH)(C₁₅ alkyl), -(C₁₋₅ alkylene)S(O)(N(C₁₅ alkyl))(C₁₅ alkyl), -(C₁₋₅ alkylene)-P(O)(C₁₋₅ alkyl)(C₁₅ alkyl), -(C₁₋₅ alkylene)-P(O)(O(C₁₋₅ alkyl))(O(C₁₅ alkyl), -(C₁₋₅ alkylene)-P(O)(O(C₁₋₅ alkyl))(C₁₋₅ alkyl), -(C₁₋₅ alkylene)NH₂, -(C₁₋₅ alkylene)NH(C₁₋₅ alkyl), -(C₁₋₅ alkylene)-NH(C₁₋₅ haloalkyl), -(C₁₋₅ alkylene)N(C₁₋₅ alkyl)(C₁₋₅ alkyl), -(C₁₋₅ alkylene)N(C₁₋₅ alkyl)(C₁₋₅ haloalkyl), -(C₁₋₅ alkylene)(N-heterocycloalkyl), -(C₁₋₅ alkylene)N(C₁₋₅ haloalkyl)(C₁₋₅ alkyl), -(C₁₋₅ alkylene)-CO(C₁₋₅ alkyl), -(C₁₋₅ alkylene)CONH₂, -(C₁₋₅ alkylene)CONH(C₁₋₅ alkyl), -(C₁₋₅ alkylene)CON(C₁₋₅ alkyl)(C₁₋₅ alkyl), -(C₁₋₅ alkylene)CO-(*N*-heterocycloalkyl), -(C₁₋₅ alkylene)NHCO-(C₁₋₅ alkyl), -(C₁₋₅ alkylene)N(C₁₋₅ alkyl)-CO-(C₁₋₅ alkyl), -(C₁₋₅ alkylene)NHCONH₂, -(C₁₋₅ alkylene)NHCONH-(C₁₋₅ alkyl), -(C₁₋₅ alkylene)NHCON(C₁₋₅ alkyl)(C₁₋₅ alkyl), -(C₁₋₅ alkylene)N(C₁₋₅ alkyl)CONH₂, -(C₁₋₅ alkylene)N(C₁₋₅ alkyl)CONH-(C₁₋₅ alkyl), -(C₁₋₅ alkylene)N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)NHCOO(C₁₋₅ alkyl), and -(C₁₋₅ alkylene)N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl), - P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N-*heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from, halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl). More preferably, -Y_{C2}-R_{C2} is azacyclohexenyl, optionally substituted with one or more groups independently selected from halogen, CN, OH, C₁₋₅ alkyl, C₁₋₅ haloalkyl, O(C₁₋₅ alkyl), -O(C₁₋₅ haloalkyl), SH, S(C₁₅ alkyl), S(O)(C₁₅ alkyl), S(O)₂(C₁₅ alkyl), S(O)(NH)(C₁₅ alkyl), S(O)(N(C₁₅ alkyl))(C₁₅ alkyl), -N=S(O)(C₁₅ alkyl)(C₁₅ alkyl), -S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(*N*-heterocycloalkyl), -CO(C₁₋₅ alkyl), -CO(C₁₋₅ haloalkyl), -CO-cycloalkyl, COO(C₁₋₅ alkyl), -COO(C₁₋₅ haloalkyl), -COO-cycloalkyl, CONH₂, CONH(C₁₋₅ alkyl), CON(C₁₋₅ alkyl)(C₁₋₅ alkyl), - CO-(*N*-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), NHCOO(C₁₋₅ alkyl), N(C₁₋₅ alkyl)COO-(C₁₋₅ alkyl), -P(O)(C₁₋₅ alkyl)(C₁₅ alkyl), -P(O)(O(C₁₋₅ alkyl))(O(C₁₅ alkyl), -P(O)(O(C₁₋₅ alkyl))(C₁₋₅ alkyl), -(C₁₋₅ alkylene)-CN, -(C₁₋₅ alkylene)OH, -(C₁₋₅ alkylene)O(C₁₋₅ alkyl), -(C₁₋₅ alkylene)-O(C₁₋₅ haloalkyl), -(C₁₋₅ alkylene)SH, -(C₁₋₅ alkylene)S(C₁₅ alkyl), -(C₁₋₅ alkylene)-S(C₁₋₅ haloalkyl), -(C₁₋₅ alkylene)S(O)(C₁₅ alkyl), -(C₁₋₅ alkylene)S(O)₂(C₁₅ alkyl), -(C₁₋₅ alkylene)S(O)(NH)(C₁₅ alkyl), -(C₁₋₅ alkylene)S(O)(N(C₁₅ alkyl))(C₁₅ alkyl), -(C₁₋₅ alkylene)-P(O)(C₁₋₅ alkyl)(C₁₅ alkyl), -(C₁₋₅ alkylene)-P(O)(O(C₁₋₅ alkyl))(O(C₁₅ alkyl)), -(C₁₋₅ alkylene)-P(O)(O(C₁₋₅ alkyl))(C₁₋₅ alkyl), -(C₁₋₅ alkylene)NH₂, -(C₁₋₅ alkylene)NH(C₁₋₅ alkyl), -(C₁₋₅ alkylene)-NH(C₁₋₅ haloalkyl), -(C₁₋₅ alkylene)N(C₁₋₅ alkyl)(C₁₋₅ alkyl), -(C₁₋₅ alkylene)N(C₁₋₅ alkyl)(C₁₋₅ haloalkyl), -(C₁₋₅ alkylene)(*N-*heterocycloalkyl), -(C₁₋₅ alkylene)N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)CONH₂, -(C₁₋₅ alkylene)CONH(C₁₋₅ alkyl), -(C₁₋₅ alkylene)CON(C₁₋₅ alkyl)(C₁₋₅ alkyl), -(C₁₋₅ alkylene)CO-(*N-*heterocycloalkyl), -(C₁₋₅ alkylene)NHCO-(C₁₋₅ alkyl), -(C₁₋₅ alkylene)N(C₁₋₅ alkyl)-CO-(C₁₋₅ alkyl), -(C₁₋₅ alkylene)NHCONH₂, -(C₁₋₅ alkylene)NHCONH-(C₁₋₅ alkyl), -(C₁₋₅ alkylene)NHCON(C₁₋₅ alkyl)(C₁₋₅ alkyl), - (C₁₋₅ alkylene)N(C₁₋₅ alkyl)CONH₂, -(C₁₋₅ alkylene)N(C₁₋₅ alkyl)CONH-(C₁₋₅ alkyl), -(C₁₋₅ alkylene)N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)NHCOO(C₁₋₅ alkyl), and -(C₁₋₅ alkylene)N(C₁₋₅ alkyl)COO-(C₁₋₅ alkyl), preferably selected from halogen, CN, OH, C₁₋₅ alkyl, C₁₋₅ haloalkyl, O(C₁₋₅ alkyl), -O(C₁₋₅ haloalkyl), SH, S(C₁₅ alkyl), S(O)(C₁₅ alkyl), S(O)₂(C₁₅ alkyl), S(O)(NH)(C₁₅ alkyl), S(O)(N(C₁₅ alkyl))(C₁₅ alkyl), -N=S(O)(C₁₅ alkyl)(C₁₅ alkyl), -S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(*N*-heterocycloalkyl), -CO(C₁₋₅ alkyl), COO(C₁₋₅ alkyl), CONH₂, CONH(C₁₋₅ alkyl), CON(C₁₋₅ alkyl)(C₁₋₅ alkyl), -CO-(*N*-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), NHCOO(C₁₋₅ alkyl), N(C₁₋₅ alkyl)COO-(C₁₋₅ alkyl), -P(O)(C₁₋₅ alkyl)(C₁₅ alkyl), -P(O)(O(C₁₋₅ alkyl))(O(C₁₅ alkyl), -P(O)(O(C₁₋₅ alkyl))(C₁₋₅ alkyl), -(C₁₋₅ alkylene)-CN, -(C₁₋₅ alkylene)OH, -(C₁₋₅ alkylene)O(C₁₋₅ alkyl), -(C₁₋₅ alkylene)-O(C₁₋₅ haloalkyl), -(C₁₋₅ alkylene)SH, -(C₁₋₅ alkylene)S(C₁₅ alkyl), -(C₁₋₅ alkylene)-S(C₁₋₅ haloalkyl), -(C₁₋₅ alkylene)S(O)(C₁₅ alkyl), -(C₁₋₅ alkylene)S(O)₂(C₁₅ alkyl), -(C₁₋₅ alkylene)S(O)(NH)(C₁₅ alkyl), -(C₁₋₅ alkylene)S(O)(N(C₁₅ alkyl))(C₁₅ alkyl), -(C₁₋₅ alkylene)-P(O(C₁₋₅ alkyl)(C₁₅ alkyl), -(C₁₋₅ alkylene)-P(O)(O(C₁₋₅ alkyl))(O(C₁₅ alkyl), -(C₁₋₅ alkylene)-P(O)(O(C₁₋₅ alkyl))(C₁₋₅ alkyl), -(C₁₋₅ alkylene)NH₂, -(C₁₋₅ alkylene)NH(C₁₋₅ alkyl), -(C₁₋₅ alkylene)-NH(C₁₋₅ haloalkyl), -(C₁₋₅ alkylene)N(C₁₋₅ alkyl)(C₁₋₅ alkyl), -(C₁₋₅ alkylene)N(C₁₋₅ alkyl)(C₁₋₅ haloalkyl), -(C₁₋₅ alkylene)(*N*-heterocycloalkyl), -(C₁₋₅ alkylene)N(C₁₋₅ haloalkyl)(C₁₋₅ alkyl), -(C₁₋₅ alkylene)-CO(C₁₋₅ alkyl), -(C₁₋₅ alkylene)CONH₂, -(C₁₋₅ alkylene)CONH(C₁₋₅ alkyl), -(C₁₋₅ alkylene)CON(C₁₋₅ alkyl)(C₁₋₅ alkyl), -(C₁₋₅ alkylene)CO-(*N-*heterocycloalkyl), -(C₁₋₅ alkylene)NHCO-(C₁₋₅ alkyl), -(C₁₋₅ alkylene)N(C₁₋₅ alkyl)-CO-(C₁₋₅ alkyl), -(C₁₋₅ alkylene)NHCONH₂, -(C₁₋₅ alkylene)NHCONH-(C₁₋₅ alkyl), -(C₁₋₅ alkylene)NHCON(C₁₋₅ alkyl)(C₁₋₅ alkyl), - (C₁₋₅ alkylene)N(C₁₋₅ alkyl)CONH₂, -(C₁₋₅ alkylene)N(C₁₋₅ alkyl)CONH-(C₁₋₅ alkyl), -(C₁₋₅ alkylene)N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)NHCOO(C₁₋₅ alkyl), and -(C₁₋₅ alkylene)N(C₁₋₅ alkyl)COO-(C₁₋₅ alkyl), more preferably selected from halogen, -CN, -OH, C₁₋₅ alkyl, C₁₋₅ haloalkyl, -O(C₁-₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), - CO(C₁₋₅ alkyl), -CONH₂, -CONH(C₁₋₅ alkyl), -CON(C₁₋₅ alkyl)(C₁₋₅ alkyl), -CO-(*N-*heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl), - P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl). Even more preferably, -Y_{C2}-R_{C2} is azacyclohexenyl substituted (preferably N-substituted) with -CON(C₁₋₅ alkyl)(C₁₋₅ alkyl), preferably with -CON(CH₃)₂. Preferably, azacyclohexenyl as referred to herein is 1,2,3,6-tetrahydropyridinyl.

In another preferred embodiment, if -Y_{C2}-R_{C2} is heterocycloalkyl, -Y_{C2}-R_{C2} is 2-oxaspiro[3.5]non-6-en-7-yl, 2-oxaspiro[3.5]non-7-yl, 2-oxa-8-azaspiro[4.5]dec-8-yl, 9-oxa-3-azaspiro[5.5]undec-3-yl, 2-oxa-6-azaspiro[3.4]oct-6-yl, 1-oxa-7-azaspiro[3.5]non-7-yl, 1-oxa-8-azaspiro[4.5]dec-8-yl, 6-oxa-2-azaspiro[3.3]hept-2-yl, 2,8-diazaspiro[4.5]dec-8-yl, 7-oxa-3-azabicyclo[3.3.0]oct-3-yl, 8-oxa-3-azabicyclo[4.3.0]non-3-yl, 2-oxa-6-azaspiro[3.5]non-6-yl, 7-oxo-3,6,8-triazabicyclo[4.3.0]non-3-yl, 3-pyrrolino[3,4-c]pyrazol-2-yl, 3,6- diazabicyclo[3.1.1]hept-3-yl, or 2,7-diazaspiro[3.5]non-7-yl.

In one specific embodiment, -Y_{C2}-R_{C2} is selected from: 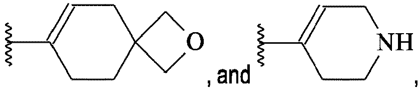 preferably -Y_{C2}-R_{C2} is

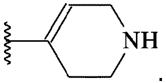

In one specific embodiment, -Y_{C2}-R_{C2} is selected from:

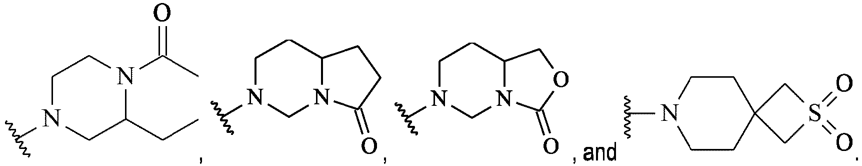

In one specific embodiment, -Y_{C2}-R_{C2} is selected from: 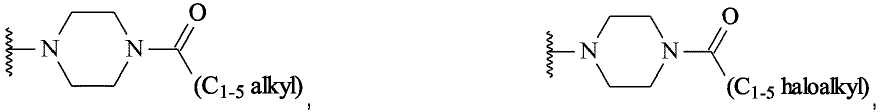 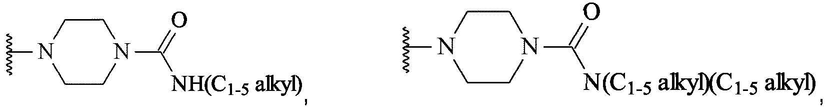 and

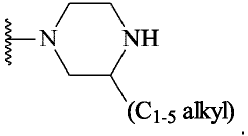

X₄ is N or C-R_{C4}.

R_{C4} is selected from hydrogen, halo, C₁₋₆ alkyl, C₂₋₆ alkynyl, -O(C₁₋₆ alkyl), -S(C₁₋₆ alkyl), -NH(C₁₋₆ alkyl), -N(C₁₋₆ alkyl)(C₁₋₆ alkyl), -CO(C₁₋₆ alkyl), C₁₋₆ haloalkyl, -O(C₁₋₆ haloalkyl), -S(C₁₋₆ haloalkyl), - NH(C₁₋₆ haloalkyl), -N(C₁₋₆ haloalkyl)₂, -CO(C₁₋₆ haloalkyl), -(C₀₋₃ alkylene)cycloalkyl, -O-(C₀₋₃ alkylene)-cycloalkyl, -CO-(C₀₋₃ alkylene)-cycloalkyl, -(C₀₋₃ alkylene)cycloalkenyl, -O-(C₀₋₃ alkylene)-cycloalkenyl, - CO-(C₀₋₃ alkylene)-cycloalkenyl, -(C₀₋₃ alkylene)-heterocycloalkyl, -O-(C₀₋₃ alkylene)-heterocycloalkyl, - CO-(C₀₋₃ alkylene)-heterocycloalkyl, -(C₀₋₃ alkylene)-heterocycloalkenyl, -O-(C₀₋₃ alkylene)-heterocycloalkenyl, -CO-(C₀₋₃ alkylene)-heterocycloalkenyl, -(C₀₋₃ alkylene)-aryl, -O-(C₀₋₃ alkylene)-aryl, - CO-(C₀₋₃ alkylene)-aryl, -(C₀₋₃ alkylene)-heteroaryl, -O-(C₀₋₃ alkylene)-heteroaryl and -CO-(C₀₋₃ alkylene)-heteroaryl, preferably selected from hydrogen, halo, C₁₋₆ alkyl, C₂₋₆ alkynyl, -O(C₁₋₆ alkyl), -S(C₁₋₆ alkyl), - NH(C₁₋₆ alkyl), -N(C₁₋₆ alkyl)(C₁₋₆ alkyl), -CO(C₁₋₆ alkyl), C₁₋₆ haloalkyl, -O(C₁₋₆ haloalkyl), -S(C₁₋₆ haloalkyl), -NH(C₁₋₆ haloalkyl), -N(C₁₋₆ haloalkyl)₂, -CO(C₁₋₆ haloalkyl), -(C₀₋₃ alkylene)cycloalkyl, -O-(C₀₋₃ alkylene)-cycloalkyl, -CO-(C₀₋₃ alkylene)-cycloalkyl, -(C₀₋₃ alkylene)-heterocycloalkyl, -O-(C₀₋₃ alkylene)-heterocycloalkyl, -CO-(C₀₋₃ alkylene)-heterocycloalkyl, -(C₀₋₃ alkylene)-aryl, -O-(C₀₋₃ alkylene)-aryl, -CO-(C₀₋₃ alkylene)-aryl, -(C₀₋₃ alkylene)-heteroaryl, -O-(C₀₋₃ alkylene)-heteroaryl and -CO-(C₀₋₃ alkylene)-heteroaryl. Said alkyl or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably said alkyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). Said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, - (C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, - (C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

Preferably R_{C4} is selected from hydrogen, halo, C₁₋₆ alkyl, C₂₋₆ alkynyl, -O(C₁₋₆ alkyl), -S(C₁₋₆ alkyl), -NH(C₁₋₆ alkyl), C₁₋₆ haloalkyl, -(C₀₋₃ alkylene)-cycloalkyl, -(C₀₋₃ alkylene)-cycloalkenyl, -(C₀₋₃ alkylene)-heterocycloalkyl, -(C₀₋₃ alkylene)-heterocycloalkenyl, -(C₀₋₃ alkylene)-aryl and -(C₀₋₃ alkylene)-heteroaryl, preferably selected from hydrogen, halo, C₁₋₆ alkyl, C₂₋₆ alkynyl, -O(C₁₋₆ alkyl), -S(C₁₋₆ alkyl), - NH(C₁₋₆ alkyl), C₁₋₆ haloalkyl, -(C₀₋₃ alkylene)-cycloalkyl, -(C₀₋₃ alkylene)-heterocycloalkyl, -(C₀₋₃ alkylene)-aryl and -(C₀₋₃ alkylene)-heteroaryl. Said alkyl or alkynyl (preferably said alkyl) is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -O(C₁₋₅ haloalkyl), -SH, -S(C₁₋₅ alkyl), -O(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). Said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), - (C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

Further preferably, R_{C4} is selected from hydrogen, halo, C₁₋₆ alkyl, C₂₋₆ alkynyl, -O-C₁₋₆ alkyl, -S-C₁₋₆ alkyl, -NH-C₁₋₆ alkyl, and C₁₋₆ haloalkyl, more preferably R_{C4} is selected from hydrogen, halo, C₁₋₂ alkyl, and C₂₋₃ alkynyl, even more preferably R_{C4} is selected from hydrogen, halo, and C₁₋₂ alkyl, even more preferably R_{C4} is hydrogen or halo.

In an alternative preferred embodiment, R_{C4} is selected from -(C₀₋₃ alkylene)-cycloalkyl, -(C₀₋₃ alkylene)-cycloalkenyl, -(C₀₋₃ alkylene)-heterocycloalkyl, -(C₀₋₃ alkylene)-heterocycloalkenyl, -(C₀₋₃ alkylene)-aryl and -(C₀₋₃ alkylene)-heteroaryl, preferably selected from -(C₀₋₃ alkylene)-cycloalkyl, -(C₀₋₃ alkylene)-heterocycloalkyl, -(C₀₋₃ alkylene)-aryl and -(C₀₋₃ alkylene)-heteroaryl. Said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), - O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), - (C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). More preferably, R_{C4} is selected from -(C₀₋₃ alkylene)-cycloalkyl, -(C₀₋₃ alkylene)-heterocycloalkyl, and -(C₀₋₃ alkylene)-heteroaryl, preferably from cycloalkyl, heterocycloalkyl, and heteroaryl. Said cycloalkyl, heterocycloalkyl, or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -NH(C₁₋₅ haloalkyl)(C₁₋₅ alkyl), -CONH₂, -CONH(C₁₋₅ alkyl), and -CON(C₁₋₅ alkyl)(C₁₋₅ alkyl). Even more preferably, R_{C4} is selected from heterocycloalkyl, and heteroaryl. Said heterocycloalkyl, or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -NH(C₁₋₅ haloalkyl)(C₁₋₅ alkyl), -CONH₂, -CONH(C₁₋₅ alkyl), and -CON(C₁₋₅ alkyl)(C₁₋₅ alkyl).

In an alternative preferred embodiment, R_{C4} is selected from -CH₂-cycloalkyl, -CH₂-cycloalkenyl, -CH₂-heterocycloalkyl, -CH₂-heterocycloalkenyl, -CH₂-aryl and -CH₂-heteroaryl, preferably selected from -CH₂-cycloalkyl, -CH₂-heterocycloalkyl, -CH₂-aryl and -CH₂-heteroaryl. Said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), - (C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). More preferably R_{C4} is selected from -CH₂-heterocycloalkyl, and -CH₂-heteroaryl. Said heterocycloalkyl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

If R_{C4} is heteroaryl, R_{C4} is preferably imidazolyl, pyridazinyl, thiazolyl, pyridinyl, pyrimidinyl, pyrazinyl, or indazolyl, wherein heteroaryl may be optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), - (C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

If R_{C4} is heterocycloalkyl, R_{C4} is preferably morpholinyl, 1,1-dioxothiomorpholinyl, azetinyl, pyrrolidinyl, piperidinyl, 6-oxo-1,6- dihydropyridinyl, or piperazinyl, wherein heterocycloalkyl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, - (C₁₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, - (C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl, preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). More preferably, R_{C4} is piperazinyl, optionally substituted with one or more groups independently selected from halogen, -CN, -OH, C₁₋₅ alkyl, C₁₋₅ haloalkyl, -O(C₁₋₅ alkyl), - O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N*-heterocycloalkyl), -(C₁₋₅ alkylene)-N(C₁₋₅ haloalkyl)(C₁₋₅ alkyl), - (C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). Even more preferably, R_{C4} is piperazinyl (preferably N-piperazinyl) optionally substituted (preferably N-substituted) with -CONH₂, -CONH(C₁₋₅ alkyl), and -CON(C₁₋₅ alkyl)(C₁₋₅ alkyl). Most preferably, R_{C4} is piperazinyl (preferably N-piperazinyl) substituted (preferably N-substituted, preferably at a different N-atom than that attached to the ring system as shown in formula (I)), with -CON(C₁₋₅ alkyl)(C₁₋₅ alkyl), preferably with -CON(CH₃)₂.

If R_{C4} is heterocycloalkenyl, R_{C4} is oxacyclohexenyl or azacyclohexenyl, preferably R_{C4} is azacyclohexenyl, wherein heterocycloalkenyl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N*-heterocycloalkyl), -(C₁₋₅ alkylene)-N(C₁₋₅ haloalkyl)(C₁₋₅ alkyl), - (C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl, preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

In an alternative embodiment wherein R_{C4} is heterocycloalkyl, R_{C4} is preferably 2-oxaspiro[3.5]non-6-en-7-yl, 2-oxaspiro[3.5]non-7-yl, 2-oxa-8-azaspiro[4.5]dec-8-yl, 9-oxa-3-azaspiro[5.5]undec-3-yl, 2-oxa-6-azaspiro[3.4]oct-6-yl, 1-oxa-7-azaspiro[3.5]non-7-yl, 1-oxa-8-azaspiro[4.5]dec-8-yl, 6-oxa-2-azaspiro[3.3]hept-2-yl, 2,8-diazaspiro[4.5]dec-8-yl, 7-oxa-3-azabicyclo[3.3.0]oct-3-yl, 8-oxa-3-azabicyclo[4.3.0]non-3-yl, 2-oxa-6-azaspiro[3.5]non-6-yl, 7-oxo-3,6,8-triazabicyclo[4.3.0]non-3-yl, 3-pyrrolino[3,4-c]pyrazol-2-yl, 3,6- diazabicyclo[3.1.1]hept-3-yl, or 2,7-diazaspiro[3.5]non-7-yl.

Preferably, if X₂ comprises cycloalkyl, heterocycloalkyl, aryl or heteroaryl, X₄ is C-R_{C4} wherein R_{C4} is selected from hydrogen, halo, C₁₋₆ alkyl, -O(C₁₋₆ alkyl), -S(C₁₋₆ alkyl), -NH(C₁₋₆ alkyl), and C₁₋₆ haloalkyl. More preferably, if X₂ comprises cycloalkyl, heterocycloalkyl, aryl or heteroaryl, X₄ is C-R_{C4} wherein R_{C4} is selected from hydrogen, and halo.

Further preferably, if X₄ comprises cycloalkyl, heterocycloalkyl, aryl or heteroaryl, X₂ does not comprise any of the groups cycloalkyl, heterocycloalkyl, aryl and heteroaryl.

Further preferably, if X₂ comprises cycloalkyl, heterocycloalkyl, aryl or heteroaryl and X₄ comprises cycloalkyl, heterocycloalkyl, aryl or heteroaryl, then together R_{C4} and -Y_{C2}-R_{C2} include not more than 12 non-hydrogen atoms, preferably not more than 10 non-hydrogen atoms.

X₅ is N or C-R_{C5}. Preferably not more than one of X₄ and X₅ is N. In certain preferred embodiments, X₄ is N and X₅ is C-R_{C5}, preferably X₄ is N and X₅ is CH. In certain preferred embodiments, X₄ is C-R_{C4} and X₅ is N, preferably X₄ is CH and X₅ is N. In certain preferred embodiments, X₄ is C-R_{C4} and X₅ is C-R_{C5}. In certain preferred embodiments X₄ is CH and X₅ is CH.

R_{C5} is selected from hydrogen, halo, C₁₋₆ alkyl, -O(C₁₋₆ alkyl), -S(C₁₋₆ alkyl), -NH(C₁₋₆ alkyl), -N(C₁₋₆ alkyl)C₁₋₆ alkyl and C₁₋₆ haloalkyl. Preferably, R_{C5} is selected from hydrogen, halo, C₁₋₃ alkyl, -O(C₁₋₃ alkyl), -S(C₁₋₃ alkyl), -NH(C₁₋₃ alkyl), and C₁₋₃ haloalkyl. More preferably, R_{C5} is selected from hydrogen, halo, C₁₋₃ alkyl, and C₁₋₃ haloalkyl.

R₄ is Y_{R5}-R_{R5}.

Y_{R5} is selected from a covalent bond, C₁₋₄ alkylene, C₂₋₄ alkenylene, and C₂₋₄ alkynylene, wherein said alkylene, said alkenylene and said alkynylene are each optionally substituted with one or more groups independently selected from halogen, -CN, -OH, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), -SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), and -N(C₁₋₅ haloalkyl)(C₁₋₅ alkyl), preferably selected from halogen, -CN, -OH, -O(C₁₋₅ alkyl), -O(C₁₋₅ haloalkyl), -S(C₁₋₅ alkyl), -SO(C₁₋₅ alkyl), -SO₂(C₁₋₅ alkyl), -S(C₁₋₅ haloalkyl), -SO(C₁₋₅ haloalkyl), -SO₂(C₁₋₅ haloalkyl), -NH(C₁₋₅ alkyl), -NH(C₁₋₅ haloalkyl), - N(C₁₋₅ alkyl)(C₁₋₅ alkyl), and -N(C₁₋₅ haloalkyl)(C₁₋₅ alkyl), more preferably selected from halogen, -CN, -OH, -O(C₁₋₅ alkyl), -O(C₁₋₅ haloalkyl), -S(C₁₋₅ alkyl), -S(C₁₋₅ haloalkyl), -NH(C₁₋₅ alkyl), -NH(C₁₋₅ haloalkyl), -N(C₁₋₅ alkyl)(C₁₋₅ alkyl), and -N(C₁₋₅ haloalkyl)(C₁₋₅ alkyl) and further wherein one or more -CH₂- units comprised in said alkylene, said alkenylene or said alkynylene are each optionally replaced by a group independently selected from -O-, -NH-, -N(C₁₋₅ alkyl)-, -CO-, -COO-, -S-, -SO-, and -SO₂-, preferably selected from -O-, -NH-, -N(C₁₋₅ alkyl)-, -CO-, -S-, -SO-, and -SO₂-. Preferably, Y_{R5} is selected from a covalent bond, C₁₋₂ alkylene, -CO-(C₁₋₂ alkylene)-, -(C₁₋₂ alkylene)-CO-, -CONH-(C₁₋₂ alkylene)-, -(C₁₋₂ alkylene)-CONH-, -NHCO-(C₁₋₂ alkylene)-, -(C₁₋₂ alkylene)-NHCO-, -NH-(C₁₋₂ alkylene)-, -(C₁₋₂ alkylene)-NH-, -O-(C₁₋₂ alkylene)-, -(C₁₋₂ alkylene)-O-, -SO₂-(C₁₋₂ alkylene)-, -(C₁₋₂ alkylene)-SO₂-, -CONH-, -CON(C₁₋₅ alkyl)-, -NHCO-, -N(C₁₋₅ alkyl)CO-, -NH-, -O-, -CO-, -COO- and -SO₂-. C₁₋₂ alkylene is herein preferably a -CH₂- group.

R_{R5} is selected from C₁₋₁₂ alkyl, C₂₋₁₂ alkenyl, C₂₋₁₂ alkynyl, cycloalkyl, cycloalkenyl, heterocycloalkyl, heterocycloalkenyl, aryl, and heteroaryl. Preferably R_{R5} is selected from cycloalkyl, heterocycloalkyl, aryl, and heteroaryl, preferably selected from C₁₋₁₂ alkyl, C₂₋₁₂ alkenyl, C₂₋₁₂ alkynyl, cycloalkyl, heterocycloalkyl, aryl, and heteroaryl. More preferably, R_{R5} is selected from heterocycloalkyl, aryl, and heteroaryl. Even more preferably, R_{R5} is selected from aryl and heteroaryl. Most preferably, R_{R5} is heteroaryl. Said alkyl, alkenyl, or alkynyl is optionally substituted with one or more groups independently selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). Said cycloalkyl, cycloalkenyl, heterocycloalkyl, heterocycloalkenyl, aryl or heteroaryl is optionally substituted with one or more groups independently selected from halogen, -CN, -OH, -C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), - SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

Preferably, Y_{R5} is selected from a covalent bond, C₁₋₂ alkylene, -CO-(C₁₋₂ alkylene)-, -(C₁₋₂ alkylene)-CO-, -CONH-(C₁₋₂ alkylene)-, -(C₁₋₂ alkylene)-CONH-, -NHCO-(C₁₋₂ alkylene)-, -(C₁₋₂ alkylene)-NHCO-, -NH-(C₁₋₂ alkylene)-, -(C₁₋₂ alkylene)-NH-, -O-(C₁₋₂ alkylene)-, -(C₁₋₂ alkylene)-O-, -SO₂-(C₁₋₂ alkylene), -(C₁₋₂ alkylene)-SO₂-, -CONH-, -NHCO-, -NH-, -O-, -CO- and -SO₂-. Thus, preferably, R₄ is selected from -(C₀₋₂ alkylene)-cycloalkyl, -CO-(C₀₋₂ alkylene)-cycloalkyl, -(C₀₋₂ alkylene)-CO-cycloalkyl, - CONH-(C₀₋₂ alkylene)-cycloalkyl, -(C₀₋₂ alkylene)-CONH-cycloalkyl, -NHCO-(C₀₋₂ alkylene)-cycloalkyl, - (C₀₋₂ alkylene)-NHCO-cycloalkyl, -NH-(C₀₋₂ alkylene)-cycloalkyl, -(C₀₋₂ alkylene)-NH-cycloalkyl, -O-(C₀₋₂ alkylene)-cycloalkyl, -(C₀₋₂ alkylene)-O-cycloalkyl, -SO₂-(C₀₋₂ alkylene)-cycloalkyl, -(C₀₋₂ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, -(C₀₋₂ alkylene)-cycloalkenyl, -CO-(C₀₋₂ alkylene)-cycloalkenyl, -(C₀₋₂ alkylene)-CO-cycloalkenyl, -CONH-(C₀₋₂ alkylene)-cycloalkenyl, -(C₀₋₂ alkylene)-CONH-cycloalkenyl, -NHCO-(C₀₋₂ alkylene)-cycloalkenyl, -(C₀₋₂ alkylene)-NHCO-cycloalkenyl, -NH-(C₀₋₂ alkylene)-cycloalkenyl, -(C₀₋₂ alkylene)-NH-cycloalkenyl, -O-(C₀₋₂ alkylene)-cycloalkenyl, -(C₀₋₂ alkylene)-O-cycloalkenyl, SO₂-(C₀₋₂ alkylene)-cycloalkenyl, -(C₀₋₂ alkylene)SO₂-cycloalkenyl, -CONH-cycloalkenyl, -NHCO-cycloalkenyl, -NH-cycloalkenyl, -O-cycloalkenyl, -CO-cycloalkenyl, SO₂-cycloalkenyl, -(C₀₋₂ alkylene)-heterocycloalkyl, -CO-(C₀₋₂ alkylene)-heterocycloalkyl, -(C₀₋₂ alkylene)-CO-heterocycloalkyl, -CONH-(C₀₋₂ alkylene)-heterocycloalkyl, -(C₀₋₂ alkylene)-CONH-heterocycloalkyl, -NHCO-(C₀₋₂ alkylene)-heterocycloalkyl, -(C₀₋₂ alkylene)-NHCO-heterocycloalkyl, -NH-(C₀₋₂ alkylene)-heterocycloalkyl, -(C₀₋₂ alkylene)-NH-heterocycloalkyl, -O-(C₀₋₂ alkylene)-heterocycloalkyl, -(C₀₋₂ alkylene)-O-heterocycloalkyl, -SO₂-(C₀₋₂ alkylene)-heterocycloalkyl, -(C₀₋₂ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₂ alkylene)-heterocycloalkenyl, -CO-(C₀₋₂ alkylene)-heterocycloalkenyl, -(C₀₋₂ alkylene)-CO-heterocycloalkenyl, -CONH-(C₀₋₂ alkylene)-heterocycloalkenyl, -(C₀₋₂ alkylene)-CONH-heterocycloalkenyl, -NHCO-(C₀₋₂ alkylene)-heterocycloalkenyl, -(C₀₋₂ alkylene)-NHCO-heterocycloalkenyl, -NH-(C₀₋₂ alkylene)-heterocycloalkenyl, -(C₀₋₂ alkylene)-NH-heterocycloalkenyl, -O-(C₀₋₂ alkylene)-heterocycloalkenyl, -(C₀₋₂ alkylene)-O-heterocycloalkenyl, SO₂-(C₀₋₂ alkylene)-heterocycloalkenyl, -(C₀₋₂ alkylene)SO₂-heterocycloalkenyl, -CONH-heterocycloalkenyl, -NHCO-heterocycloalkenyl, -NH-heterocycloalkenyl, -O-heterocycloalkenyl, -CO-heterocycloalkenyl, SO₂-heterocycloalkenyl, -(C₀₋₂ alkylene)-aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, -SO₂-(C₀₋₂ alkylene)-aryl, -(C₀₋₂ alkylene)-SO₂-aryl, -CONH-aryl, -NHCO-aryl, -NH-aryl, -O-aryl, -CO-aryl, -SO₂-aryl, -(C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)-heteroaryl, -(C₀₋₂ alkylene)-NH-heteroaryl, -O-(C₀₋₂ alkylene)-heteroaryl, -(C₀₋₂ alkylene)-O-heteroaryl, -SO₂-(C₀₋₂ alkylene)-heteroaryl, -(C₀₋₂ alkylene)-SO₂-heteroaryl, -CONH-heteroaryl, -NHCO-heteroaryl, -NH-heteroaryl, -O-heteroaryl, -CO-heteroaryl, and -SO₂-heteroaryl, preferably selected from -(C₀₋₂ alkylene)-cycloalkyl, -CO-(C₀₋₂ alkylene)-cycloalkyl, - (C₀₋₂ alkylene)-CO-cycloalkyl, -CONH-(C₀₋₂ alkylene)-cycloalkyl, -(C₀₋₂ alkylene)-CONH-cycloalkyl, - NHCO-(C₀₋₂ alkylene)-cycloalkyl, -(C₀₋₂ alkylene)-NHCO-cycloalkyl, -NH-(C₀₋₂ alkylene)-cycloalkyl, -(C₀₋₂ alkylene)-NH-cycloalkyl, -O-(C₀₋₂ alkylene)-cycloalkyl, -(C₀₋₂ alkylene)-O-cycloalkyl, SO₂-(C₀₋₂ alkylene)-cycloalkyl, -(C₀₋₂ alkylene)SO₂-cycloalkyl, -CONH-cycloalkyl, -NHCO-cycloalkyl, -NH-cycloalkyl, -O-cycloalkyl, -CO-cycloalkyl, SO₂-cycloalkyl, -(C₀₋₂ alkylene)-heterocycloalkyl, -CO-(C₀₋₂ alkylene)-heterocycloalkyl, -(C₀₋₂ alkylene)-CO-heterocycloalkyl, -CONH-(C₀₋₂ alkylene)-heterocycloalkyl, -(C₀₋₂ alkylene)-CONH-heterocycloalkyl, -NHCO-(C₀₋₂ alkylene)-heterocycloalkyl, -(C₀₋₂ alkylene)-NHCO-heterocycloalkyl, -NH-(C₀₋₂ alkylene)-heterocycloalkyl, -(C₀₋₂ alkylene)-NH-heterocycloalkyl, -O-(C₀₋₂ alkylene)-heterocycloalkyl, -(C₀₋₂ alkylene)-O-heterocycloalkyl, SO₂-(C₀₋₂ alkylene)-heterocycloalkyl, -(C₀₋₂ alkylene)SO₂-heterocycloalkyl, -CONH-heterocycloalkyl, -NHCO-heterocycloalkyl, -NH-heterocycloalkyl, -O-heterocycloalkyl, -CO-heterocycloalkyl, SO₂-heterocycloalkyl, -(C₀₋₂ alkylene)-aryl, - CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, SO₂-(C₀₋₂ alkylene)-aryl, -(C₀₋₂ alkylene)SO₂-aryl, -CONH-aryl, -NHCO-aryl, -NH-aryl, -O-aryl, -CO-aryl, SO₂-aryl, -(C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)-heteroaryl, -(C₀₋₂ alkylene)-NH-heteroaryl, -O-(C₀₋₂ alkylene)-heteroaryl, -(C₀₋₂ alkylene)-O-heteroaryl, SO₂-(C₀₋₂ alkylene)-heteroaryl, -(C₀₋₂ alkylene)SO₂-heteroaryl, -CONH-heteroaryl, -NHCO-heteroaryl, - NH-heteroaryl, -O-heteroaryl, -CO-heteroaryl, and SO₂-heteroaryl. Said cycloalkyl, cycloalkenyl, heterocycloalkyl, heterocycloalkenyl, aryl or heteroaryl is optionally substituted with one or more groups independently selected from halogen, -CN, -OH, -C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), - SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). More preferably, R₄ is selected from -(C₀₋₂ alkylene)-aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, -SO₂-(C₀₋₂ alkylene)-aryl, -(C₀₋₂ alkylene)-SO₂-aryl, -CONH-aryl, -NHCO-aryl, -NH-aryl, -O-aryl, -CO-aryl, -SO₂-aryl, -(C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)-heteroaryl, -(C₀₋₂ alkylene)-NH-heteroaryl, -O-(C₀₋₂ alkylene)-heteroaryl, -(C₀₋₂ alkylene)-O-heteroaryl, -SO₂-(C₀₋₂ alkylene)-heteroaryl, -(C₀₋₂ alkylene)-SO₂-heteroaryl, -CONH-heteroaryl, -NHCO-heteroaryl, -NH-heteroaryl, -O-heteroaryl, -CO-heteroaryl, and -SO₂-heteroaryl, wherein said aryl or heteroaryl is optionally substituted with one or more groups independently selected from halogen, -CN, -OH, -C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), - 8O₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

In certain embodiments, R₄ is selected from -(C₀₋₂ alkylene)-CO-cycloalkyl, preferably -CO-cyclohexyl, and -(C₀₋₂ alkylene)-CO-aryl, preferably -CO-phenyl.

In certain embodiments, R₄ is selected from -COO-(C₁₋₅ alkyl) or -CONH-(C₁₋₅ alkyl).

In one specific embodiment, R₄ is selected from:

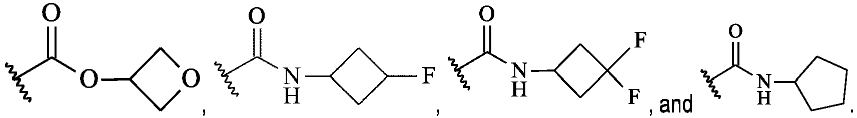

Preferably, Y_{R5} is a covalent bond. Thus, R₄ is preferably selected from C₁₋₁₂ alkyl, C₂₋₁₂ alkenyl, C₂₋₁₂ alkynyl, cycloalkyl, cycloalkenyl, heterocycloalkyl, heterocycloalkenyl, aryl, and heteroaryl, more preferably selected from C₁₋₁₂ alkyl, C₂₋₁₂ alkenyl, C₂₋₁₂ alkynyl, cycloalkyl, heterocycloalkyl, aryl, and heteroaryl. More preferably, R₄ is selected from cycloalkyl, cycloalkenyl, heterocycloalkyl, heterocycloalkenyl, aryl, and heteroaryl. More preferably, R₄ is selected from cycloalkyl, heterocycloalkyl, aryl, and heteroaryl. Even more preferably, R₄ is selected from aryl, and heteroaryl. Most preferably, R₄ is heteroaryl. Said alkyl, alkenyl, or alkynyl is optionally substituted with one or more groups independently selected from halogen, -CN, -OH, -O(C₁₋₅ alkyl), -O(C₁₋₅ haloalkyl), SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). Said cycloalkyl, cycloalkenyl, heterocycloalkyl, heterocycloalkenyl, aryl or heteroaryl is optionally substituted with one or more groups independently selected from halogen, -CN, -OH, -C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), - SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

Preferably, R₄ is a five membered heteroaryl, optionally substituted with one or more groups independently selected from halogen, -CN, -OH, -C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), - SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). The said five membered heteroaryl is preferably selected from imidazolyl, isoxazolyl, pyrazolyl, 1,2,3-triazolyl, 1,2,4-triazolyl, thiazolyl, 1,2,4-oxadiazolyl, 1,3,4-oxadiazolyl, 1,2,4-thiadiazolyl, or 1,3,4-thiadiazolyl. More preferably, said five membered heteroaryl is1,2,4-thiadiazolyl, optionally substituted with one or more groups independently selected from halogen, -CN, -OH, -C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), - SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CONH₂, -CONH(C₁₋₅ alkyl), and -CON(C₁₋₅ alkyl)(C₁₋₅ alkyl), preferably optionally substituted with C₁₋₅ alkyl, C₁₋₅ haloalkyl, -O(C₁₋₅ alkyl), -SH, -S(C₁₋₅ alkyl), more preferably optionally substituted with C₁₋₅ alkyl, C₁₋₅ haloalkyl, even more preferably optionally substituted with C₁₋₅ haloalkyl, preferably selected from -CH₂F, -CHF₂ and CF₃, most preferably optionally substituted with - CHF₂.

In one specific embodiment, R₄ is

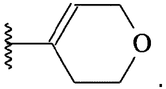

Preferably, the present invention relates to a compound of formula (I) wherein W is -NHS(O)₂-. Thus, in another embodiment, the present invention relates to a compound of formula (la):

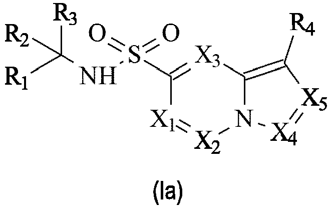

R₁, R₂, R₃, R₄, X₁, X₂, X₃, X₄, and Xs in the compound of formula (la) are as defined hereinabove for the compound of formula (I).

In a preferred embodiment, R₂ and R₃ together with the carbon atom to which they are attached form cyclopropyl. Thus, preferably, the compound of formula (Ia) is a compound of formula (Ib):

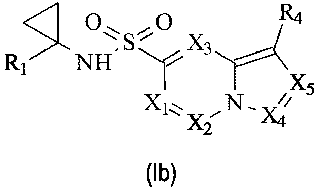

R₁, R₄, X₁, X₂, X₃, X₄, and X₅ in the compound of formula (Ib) are as defined hereinabove for the compound of formula (I).

R₁ is selected from the group consisting of hydrogen, chloro, fluoro, cyano, formyl, (C₁₋₂)alkyl, (C₂)alkenyl, (C₂)alkynyl (C₁₋₂)haloalkyl, -(C₁₋₂ alkylene)-OH and -(C₁₋₂ alkylene)-O-(C₁₋₂ alkyl). Preferably, R₁ is selected from the group consisting of hydrogen, chloro, fluoro, cyano, formyl, (C₁₋₂)alkyl, (C₂)alkenyl, (C₂)alkynyl and (C₁₋₂)haloalkyl. More preferably, R₁ is selected from the group consisting of cyano, (C₁₋₂)haloalkyl and (C₁₋₂)alkyl, preferably cyano, fluoromethyl and methyl. More preferably, R₁ is cyano. Thus, in a preferred embodiment, the compound of formula (Ib) is a compound of formula (Ic):

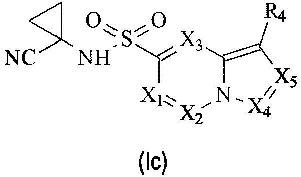

R₄, X₁, X₂, X₃, X₄, and Xs in the compound of formula (Ic) are as defined hereinabove for the compound of formula (I).

Within the scope of the present invention, the compound of formula (I) or the compound of formula (la) or the compound of formula (Ib) wherein R₁ is methyl is also encompassed. In certain preferred embodiments of the present invention, R₁ is methyl. Within the scope of the present invention, the compound of formula (I) or the compound of formula (Ia) or the compound of formula (Ib) wherein R₁ is fluoromethyl is also encompassed. In certain preferred embodiments of the present invention, R₁ is fluoromethyl.

Preferably, within the scope of the present invention, X₁ and X₃ are each CH. Thus, preferably the compound of formula (I) of the present invention is a compound of formula (Id):

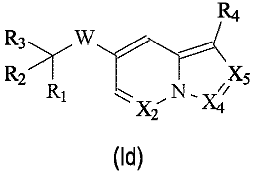

W, R₁, R₂, R₃, R₄, X₂, X₄, and X₅ in the compound of formula (Id) are as defined hereinabove for the compound of formula (I).

Preferably, the present invention relates to a compound of formula (I) wherein W is -NHS(O)₂-. Thus, in another embodiment, the compound of formula (Id) of the present invention is a compound of formula (Ie):

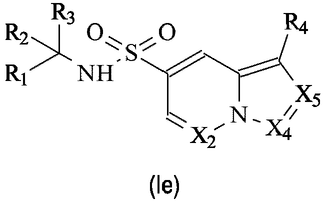

R₁, R₂, R₃, R₄, X₂, X₄, and X₅ in the compound of formula (Ie) are as defined hereinabove for the compound of formula (I).

In a preferred embodiment, R₂ and R₃ together with the carbon atom to which they are attached form cyclopropyl. Thus, preferably, the compound of formula (Ie) of the present invention is a compound of formula (If):

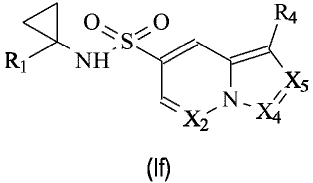

R₁, R₄, X₂, X₄, and X₅ in the compound of formula (If) is defined as defined hereinabove for the compound of formula (I) of the present invention.

Within the scope of the present invention, R₁ is preferably selected from the group consisting of hydrogen, chloro, fluoro, cyano, formyl, (C₁₋₂)alkyl, (C₂)alkenyl, (C₂)alkynyl and (C₁₋₂)haloalkyl. More preferably, R₁ is selected from the group consisting of cyano, (C₁₋₂)haloalkyl and (C₁₋₂)alkyl, preferably cyano, fluoromethyl and methyl. More preferably, R₁ is cyano.

Thus preferably, the compound of formula (If) of the present invention is a compound of formula (Ig):

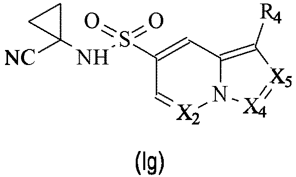

R₄, X₂, X₄, and X₅ in the compound of formula (Ig) are as defined hereinabove for the compound of formula (I) of the present invention.

Within the scope of the present invention, the compound of formula (Id) or the compound of formula (Ie) or the compound of formula (If) wherein R₁ is methyl is also encompassed within the present invention. In certain preferred embodiments of the present invention, R₁ is methyl. Within the scope of the present invention, the compound of formula (Id) or the compound of formula (Ie) or the compound of formula (If) wherein R₁ is fluoromethyl is also encompassed. In certain preferred embodiments of the present invention, R₁ is fluoromethyl.

As encompassed by the present invention, X₄ is N or C-R_{C4} and X₅ is N or G-R_{C5}. Furthermore, as defined hereinabove, preferably not more than one of X₄ and X₅ is N. In certain embodiments of the present invention, X₅ is N. Thus, if X₅ is N, preferably X₄ is C-R_{C4}. Thus, in certain preferred embodiments the compound of formula (I) is a compound of formula (Ih):

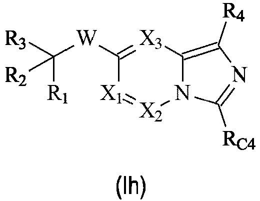

W, R_{C4}, R₁, R₂, R₃, R₄, X₁, X₂, and X₃ in the compound of formula (Ih) are as defined hereinabove for the compound of formula (I).

Preferably, within the scope of the present invention W is -NHS(O)₂-. Thus, preferably, the compound of formula (I) or the compound of formula (la) or the compound of formula (Ih) is a compound of formula (li):

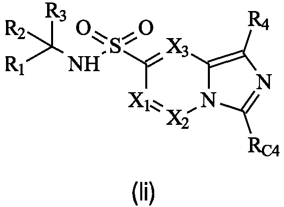

R_{C4}, R₁, R₂, R₃, R₄, X₁, X₂, and X₃ in the compound of formula (li) are as defined hereinabove for the compound of formula (I).

In a preferred embodiment, R₂ and R₃ together with the carbon atom to which they are attached form cyclopropyl. Thus, preferably, the compound of formula (li) of the present invention is a compound of formula (Ij):

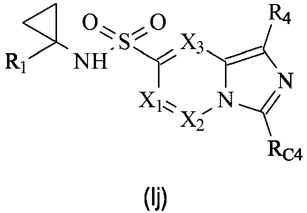

R_{C4}, R₁, R₄, X₁, X₂, and X₃ in the compound of formula (Ij) are as defined hereinabove for the compound of formula (I).

Within the scope of the present invention, preferably R₁ is hydrogen, chloro, fluoro, cyano, formyl, (C₁₋₂)alkyl, (C₂)alkenyl, (C₂)alkynyl and (C₁₋₂)haloalkyl. More preferably, R₁ is selected from the group consisting of cyano, (C₁₋₂)haloalkyl and (C₁₋₂)alkyl, preferably cyano, fluoromethyl and methyl. More preferably, R₁ is cyano.

Thus, preferably within the scope of the present invention the compound of formula (I) or the compound of formula (Ib) or the compound of formula (Ic) or the compound of formula (Ih) or the compound of formula (li) or the compound of formula (Ij) of the present invention is a compound of formula (Ik):

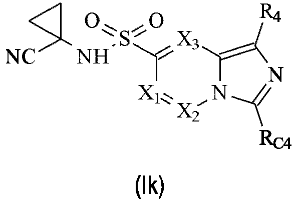

R_{C4}, R₄, X₁, X₂, and X₃ in the compound of formula (Ik) are as defined hereinabove for the compound of formula (I).

In one embodiment of the compound of formula (I) of the present invention, X₁ and X₃ are each CH. Thus, preferably the compound of formula (I) of the present invention is a compound of formula (IL):

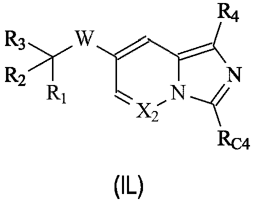

W, R_{C4}, R₁, R₂, R₃, R₄, and X₂ in the compound of formula (IL) are as defined hereinabove for the compound of formula (I).

Preferably, within the scope of the present invention W is -NHS(O)₂-. Thus, preferably, the compound of formula (IL) of the present invention is a compound of formula (Im):

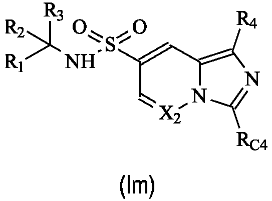

R_{C4}, R₁, R₂, R₃, R₄, and X₂ in the compound of formula (IL) are as defined hereinabove for the compound of formula (I).

Preferably, R₂ and R₃ together with the carbon atom to which they are attached form cyclopropyl. Thus, preferably, the compound of formula (Im) of the present invention is a compound of formula (In):

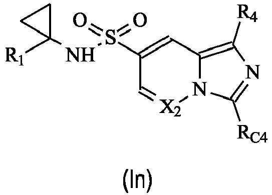

R_{C4}, R₁, R₄, and X₂ in the compound of formula (In) are as defined hereinabove for the compound of formula (I).

Within the scope of the present invention, preferably R₁ is selected from the group consisting of hydrogen, chloro, fluoro, cyano, formyl, (C₁₋₂)alkyl, (C₂)alkenyl, (C₂)alkynyl and (C₁₋₂)haloalkyl. More preferably, R₁ is selected from the group consisting of cyano, (C₁₋₂)haloalkyl and (C₁₋₂)alkyl, preferably cyano, fluoromethyl and methyl. More preferably, R₁ is cyano.

Thus, preferably within the scope of the present invention the compound of formula (In) is a compound of formula (lo):

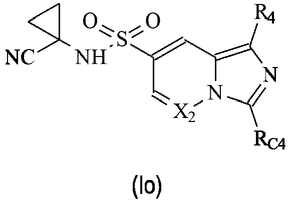

R_{C4}, R₄, and X₂ in the compound of formula (lo) are as defined hereinabove for the compound of formula (I).

Within the scope of the present invention, the compound of formula (IL) or the compound of formula (Im) or the compound of formula (In) wherein R₁ is methyl is also encompassed within the present invention. In certain preferred embodiments of the present invention, R₁ is methyl. Alternatively, within the scope of the present invention, the compound of formula (IL) or the compound of formula (Im) or the compound of formula (In) wherein R₁ is fluoromethyl is also encompassed within the present invention. In certain preferred embodiments of the present invention, R₁ is fluoromethyl.

In one embodiment of the compound of formula (I) of the present invention R₄ is selected from aryl, and heteroaryl. Most preferably, R₄ is heteroaryl. Said aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). Preferably, R₄ is a five membered heteroaryl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). The said five membered heteroaryl is preferably selected from imidazolyl, isoxazolyl, pyrazolyl, 1,2,3-triazolyl, 1,2,4-triazolyl, thiazolyl, 1,2,4-oxadiazolyl, 1,3,4-oxadiazolyl, 1,2,4-thiadiazolyl, or 1,3,4-thiadiazolyl. More preferably, said five membered heteroaryl is1,2,4-thiadiazolyl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably optionally substituted with C₁₋₅ alkyl, C₁₋₅ haloalkyl, -O(C₁₋₅ alkyl), -SH, -S(C₁₋₅ alkyl), more preferably optionally substituted with C₁₋₅ alkyl, C₁₋₅ haloalkyl, even more preferably optionally substituted with C₁₋₅ haloalkyl, preferably selected from -CH₂F, -CHF₂ and CF₃, most preferably optionally substituted with -CHF₂.

Thus, in a preferred embodiment, the compound of formula (I) is a compound of formula (Ip):

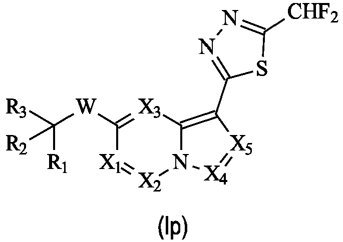

W, R₁, R₂, R₃, X₁, X₂, X₃, X₄, and X₅ in the compound of formula (Ip) are as defined hereinabove for the compound of formula (I).

Preferably W is -NHS(O)₂-. Thus, within the scope of the present invention, the compound of formula (Ip) is a compound of formula (Iq):

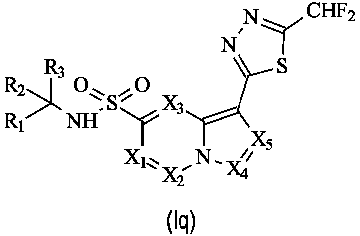

R₁, R₂, R₃, X₁, X₂, X₃, X₄, and X₅ in the compound of formula (lo) are as defined hereinabove for the compound of formula (I).

In a further preferred embodiment, R₂ and R₃ together with the carbon atom to which they are attached form cyclopropyl. Thus, in a preferred embodiment, the compound of formula (Iq) of the present invention is a compound according to formula (Ir):

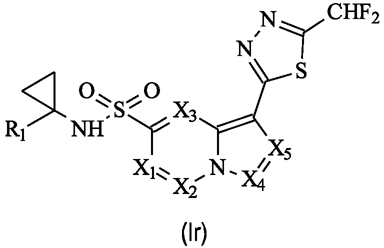

R₁, X₁, X₂, X₃, X₄, and X₅ in the compound of formula (Ir) are as defined hereinabove for the compound of formula (I).

Further preferably, R₁ is selected from the group consisting of hydrogen, chloro, fluoro, cyano, formyl, (C₁₋₂)alkyl, (C₂)alkenyl, (C₂)alkynyl and (C₁₋₂)haloalkyl. Preferably, R₁ is selected from the group consisting of cyano, (C₁₋₂)haloalkyl and (C₁₋₂)alkyl, preferably cyano, fluoromethyl and methyl. More preferably, R₁ is cyano.

Thus, preferably within the scope of the present invention the compound of formula (Ir) is a compound of formula (Is):

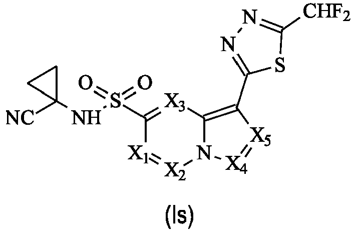

X₁, X₂, X₃, X₄, and X₅ in the compound of formula (Ir) are as defined hereinabove for the compound of formula (I).

It is noted that within the scope of the present invention, the compound of formula (Ip) or the compound of formula (Iq) or the compound of formula (Ir) wherein R₁ is methyl is also encompassed. In certain preferred embodiments of the present invention, R₁ is methyl. It is further noted that alternatively within the scope of the present invention, the compound of formula (Ip) or the compound of formula (Iq) or the compound of formula (Ir) wherein R₁ is fluoromethyl is also encompassed. In certain preferred embodiments of the present invention, R₁ is fluoromethyl.

Further preferred within the scope of the present invention are embodiments wherein X₁ and X₃ are each CH.

Thus, the compound of formula (Ip) of the present invention further relates to an embodiment wherein the compound of formula (Ip) is a compound of formula (It):

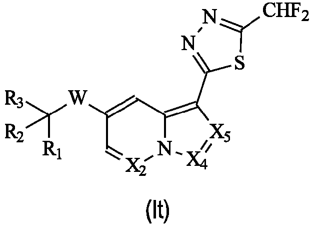

W, R₁, R₂, R₃, X₂, X₄, and X₅ in the compound of formula (It) are as defined hereinabove for the compound of formula (I).

Preferably, W is -NHS(O)₂-. Thus further within the scope of the present invention, the compound of formula (Iq) of the present invention further relates to an embodiment wherein the compound of formula (Iq) is or a compound of formula (lu),

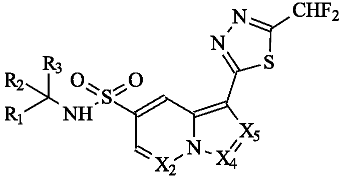

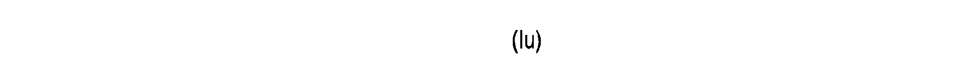

R₁, R₂, R₃, X₂, X₄, and X₅ in the compound of formula (Iu) are as defined hereinabove for the compound of formula (I).

Preferably in the compound of formula (lu) R₂ and R₃ together with the carbon atom to which they are attached form cyclopropyl.

As thus further encompassed by the present invention, the compound of formula (Ir) of the present invention further relates to an embodiment wherein the compound of formula (Ir) is a compound of formula (Iv),

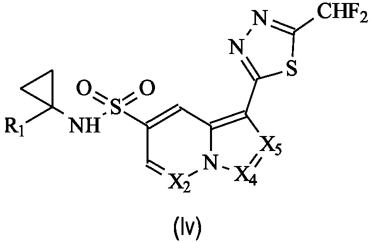

R₁, X₂, X₄, and X₅ in the compound of formula (Iv) are as defined hereinabove for the compound of formula (I).

As preferably R₁ is cyano, the compound of formula (Is) of the present invention further relates to an embodiment wherein the compound of formula (Is) is or a compound of formula (Iw)

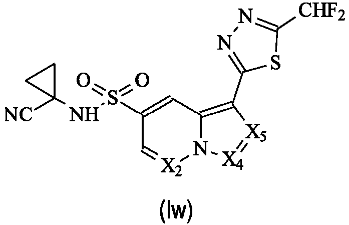

X₂, X₄, and X₅ in the compound of formula (Iw) are as defined hereinabove for the compound of formula (I).

Within the scope of the present invention, the compound of formula (It) or the compound of formula (lu) or the compound of formula (Iv) wherein R₁ is methyl is also encompassed. In certain preferred embodiments of the present invention, R₁ is methyl. Alternatively, within the scope of the present invention, the compound of formula (It) or the compound of formula (lu) or the compound of formula (Iv) wherein R₁ is fluoromethyl is also encompassed. In certain preferred embodiments of the present invention, R₁ is fluoromethyl.

As encompassed by the present invention, X₄ is N or C-R_{C4} and X₅ is N or C-R_{C5}. Furthermore, as defined hereinabove, preferably not more than one of X₄ and X₅ is N. In certain embodiments of the present invention, X₅ is N. Thus, if X₅ is N, preferably X₄ is C-R_{C4}. Thus, in certain preferred embodiments the compound of formula (It) is a compound of formula (Ix):

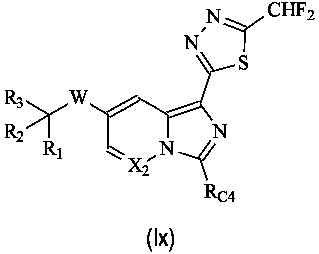

W, R_{C4}, R₁, R₂, R₃, and X₂ in the compound of formula (Ix) are as defined hereinabove for the compound of formula (I).

Further accordingly, as preferably within the scope of the present invention W is -NHS(O)₂-, in certain preferred embodiments the compound of formula (lu) is a compound of formula (ly):

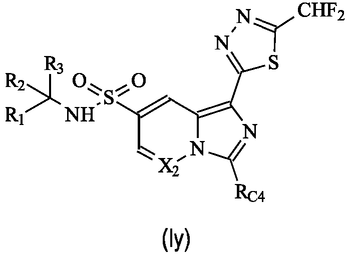

R_{C4}, R₁, R₂, R₃, and X₂ in the compound of formula (ly) are as defined hereinabove for the compound of formula (I).

Preferably R₂ and R₃ together with the carbon atom to which they are attached form cyclopropyl. Thus further accordingly, in certain preferred embodiments the compound of formula (Iv) is a compound of formula (Iz):

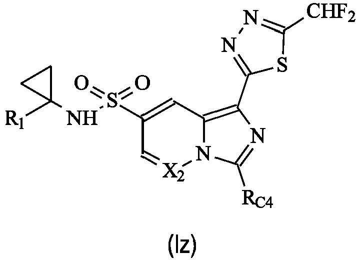

R_{C4}, R₁, and X₂ in the compound of formula (Iz) are as defined hereinabove for the compound of formula (I).

Within the scope of the present invention, preferably R₁ is selected from the group consisting of hydrogen, chloro, fluoro, cyano, formyl, (C₁₋₂)alkyl, (C₂)alkenyl, (C₂)alkynyl and (C₁₋₂)haloalkyl. More preferably, R₁ is selected from the group consisting of cyano, (C₁₋₂)haloalkyl and (C₁₋₂)alkyl, preferably cyano, fluoromethyl and methyl. More preferably, R₁ is cyano. Thus further accordingly, in certain preferred embodiments the compound of formula (Iw) is a compound of formula (laa):

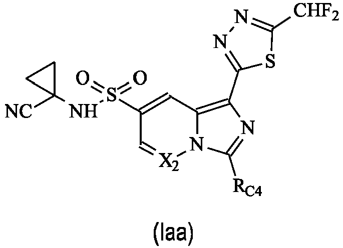

R_{C4}, and X₂ in the compound of formula (laa) are as defined hereinabove for the compound of formula (I).

It is however noted that within the scope of the present invention, the compound of formula (Ix) or the compound of formula (ly) or the compound of formula (Iz) wherein R₁ is methyl is also encompassed. In certain preferred embodiments of the present invention, R₁ is methyl. It is further noted that alternatively within the scope of the present invention, the compound of formula (Ix) or the compound of formula (ly) or the compound of formula (Iz) wherein R₁ is fluoromethyl is also encompassed. In certain preferred embodiments of the present invention, R₁ is fluoromethyl.

Preferably within the scope of the present invention X₂ is C-Y_{C2}-R_{C2}. Most preferably, -Y_{C2}-R_{C2} is piperazinyl (preferably N-piperazinyl) substituted (preferably N-substituted, preferably at a different N-atom than that attached to the ring system as shown in formula (I)), with -CON(C₁₋₅ alkyl)(C₁₋₅ alkyl), preferably with -CON(CH₃)₂.

Thus, preferably the compound of formula (I) of the present invention is a compound of formula (lab):

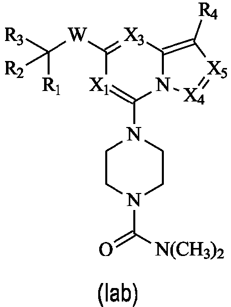

W, R₁, R₂, R₃, R₄, X₁, X₃, X₄, and X₅ in the compound of formula (lab) are as defined for the compound of formula (I) of the present invention.

Preferably W is -NHS(O)₂-. Accordingly, preferably the compound of formula (Ia) of the present invention or the compound of formula (lab) is a compound of formula (lac):

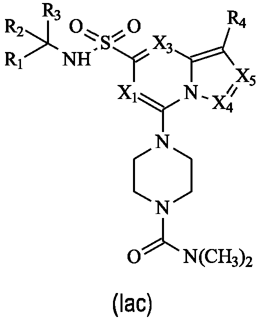

R₁, R₂, R₃, R₄, X₁, X₃, X₄, and X₅ in the compound of formula (lac) are as defined for the compound of formula (I) of the present invention.

Preferably, R₂ and R₃ together with the carbon atom to which they are attached form cyclopropyl. Thus further accordingly, preferably the compound of formula (lac) or the compound of formula (Ib) of the present invention is a compound of formula (lad):

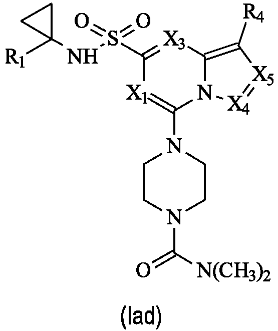

R₁, R₄, X₁, X₃, X₄, and X₅ in the compound of formula (lad) are as defined for the compound of formula (I) of the present invention.

Further accordingly, preferably, R₁ is cyano, thus preferably the compound of formula (Ic) of the present invention or the compound of formula (lad) of the present invention is a compound of formula (lae):

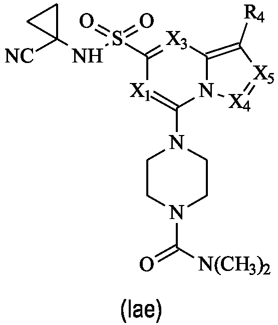

R₄, X₁, X₃, X₄, and X₅ in the compound of formula (lae) are as defined for the compound of formula (I) of the present invention.

It is however noted that within the scope of the present invention, the compound of formula (lab) or the compound of formula (lac) or the compound of formula (lad) wherein R₁ is methyl is also encompassed. In certain preferred embodiments of the present invention, R₁ is methyl. Alternatively, it is noted that within the scope of the present invention, the compound of formula (lab) or the compound of formula (lac) or the compound of formula (lad) wherein R₁ is fluoromethyl is also encompassed. In certain preferred embodiments of the present invention, R₁ is fluoromethyl.

Further preferred within the scope of the present invention are embodiments wherein X₁ and X₃ are each CH.

Thus, accordingly the compound of formula (lac) of the present invention is preferably a compound of formula (lag):

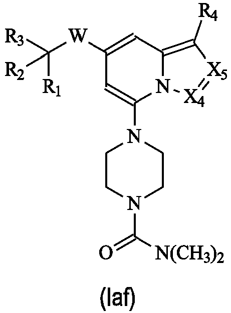

W, R₁, R₂, R₃, R₄, X₄, and X₅ in the compound of formula (Iaf) are as defined for the compound of formula (I) of the present invention.

Preferably W is -NHS(O)₂-. Further accordingly, the compound of formula (lad) of the present invention or the compound of formula (Iaf) as defined herein is preferably a compound of formula (lag):

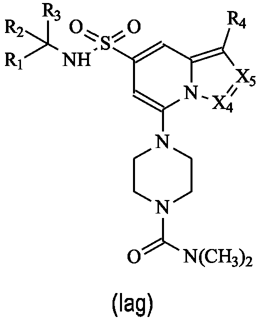

R₁, R₂, R₃, R₄, X₄, and X₅ in the compound of formula (lag) are as defined for the compound of formula (I) of the present invention.

Preferably, R₂ and R₃ together with the carbon atom to which they are attached form cyclopropyl. Further accordingly, the compound of formula (lae) or the compound of formula (lag) of the present invention is preferably a compound of formula (lah):

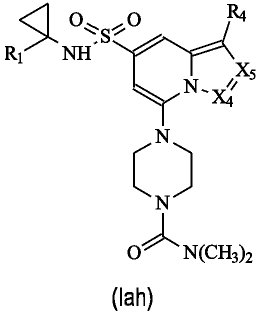

R₁, R₄, X₄, and X₅ in the compound of formula (lah) are as defined for the compound of formula (I) of the present invention.

Preferably, R₁ is cyano. Thus, further accordingly, the compound of formula (laf) of the present invention or the compound of formula (lah) of the present invention is preferably a compound of formula (lai):

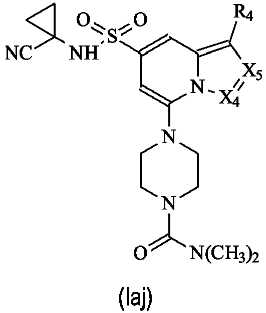

R₄, X₄, and X₅ in the compound of formula (lai) are as defined for the compound of formula (I) of the present invention.

Within the scope of the present invention, the compound of formula (laf) or the compound of formula (lag) or the compound of formula (lah) wherein R₁ is methyl is also encompassed. In certain preferred embodiments of the present invention, R₁ is methyl. Alternatively, within the scope of the present invention, the compound of formula (laf) or the compound of formula (lag) or the compound of formula (lah) wherein R₁ is fluoromethyl is also encompassed. In certain preferred embodiments of the present invention, R₁ is fluoromethyl.

As further encompassed by the present invention, X₄ is N or C-R_{C4} and X₅ is N or C-Rcs. Furthermore, as defined hereinabove, preferably not more than one of X₄ and X₅ is N. In certain embodiments of the present invention, X₅ is N. Thus, if Xs is N, preferably X₄ is C-R_{C4}.

Thus accordingly, the compound of formula (lab) of the present invention is preferably a compound of formula (laj):

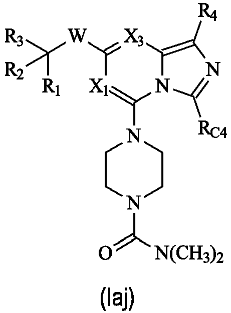

W, R_{C4}, R₁, R₂, R₃, R₄, X₁, and X₃ in the compound of formula (laj) are as defined for the compound of formula (I) of the present invention.

W is preferably -NHS(O)₂-. Thus further accordingly, the compound of formula (lac) or the compound of formula (laj) of the present invention is preferably a compound of formula (lak):

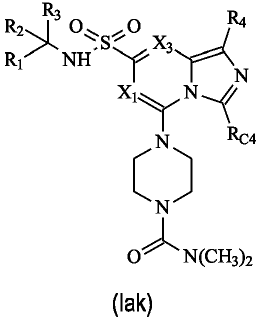

R_{C4}, R₁, R₂, R₃, R₄, X₁, and X₃ in the compound of formula (lak) are as defined for the compound of formula (I) of the present invention.

As disclosed herein, preferably R₂ and R₃ together with the carbon atom to which they are attached form cyclopropyl. Thus further accordingly, the compound of formula (lad) or the compound of formula (lak) of the present invention is preferably a compound of formula (laL):

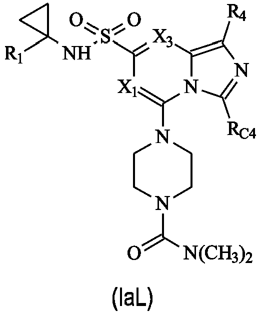

R_{C4}, R₁, R₄, X₁, and X₃ in the compound of formula (laL) are as defined for the compound of formula (I) of the present invention.

Further preferably R₁ is cyano. Thus further accordingly, the compound of formula (lae) or the compound of formula (laL) of the present invention is preferably a compound of formula (lam):

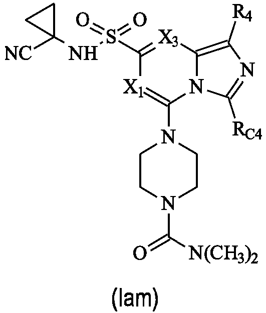

R_{C4}, R₄, X₁, and X₅ in the compound of formula (lam) are as defined for the compound of formula (I) of the present invention.

Within the scope of the present invention, the compound of formula (laj) or the compound of formula (lak) or the compound of formula (laL) wherein R₁ is methyl is also encompassed. In certain preferred embodiments of the present invention, R₁ is methyl. Further within the scope of the present invention, the compound of formula (laj) or the compound of formula (lak) or the compound of formula (laL) wherein R₁ is fluoromethyl is also encompassed. In certain preferred embodiments of the present invention, R₁ is fluoromethyl.

Further accordingly, the compound of formula (laf) of the present invention is preferably a compound of formula (lan):

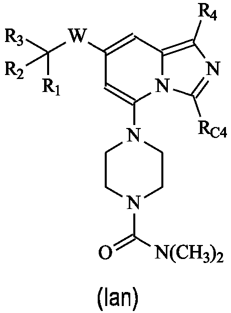

W, R₁, R₂, R₃, R₄, and R_{C4} in the compound of formula (lan) are as defined for the compound of formula (I) of the present invention.

Further accordingly, the compound of formula (lag) of the present invention is preferably a compound of formula (lao):

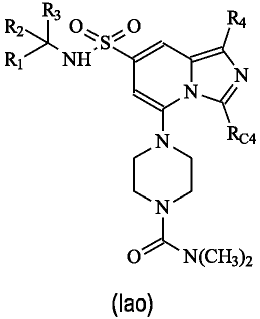

R₁, R₂, R₃, R₄, and R_{C4} in the compound of formula (lao) are as defined for the compound of formula (I) of the present invention.

Further accordingly, the compound of formula (lah) of the present invention is preferably a compound of formula (lap):

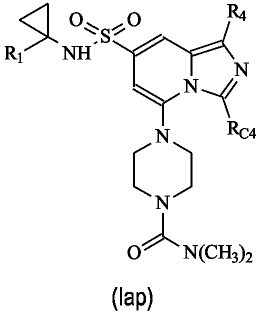

R₁, R₄, and R_{C4} in the compound of formula (lap) are as defined for the compound of formula (I) of the present invention.

Further accordingly, the compound of formula (lai) of the present invention is preferably a compound of formula (laq):

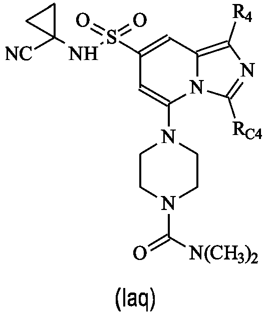

R₄, and R_{C4} in the compound of formula (laq) are as defined for the compound of formula (I) of the present invention.

As further encompassed by the present invention, X₄ is N or C-R_{C4} and X₅ is N or C-Rcs. Furthermore, as defined hereinabove, preferably not more than one of X₄ and X₅ is N. In certain embodiments of the present invention, X₄ is N. Thus, if X₄ is N, preferably X₅ is C-Rcs.

Thus accordingly, in certain preferred embodiments of the present invention, the compound of formula (I) of the present invention is a compound of formula (lar):

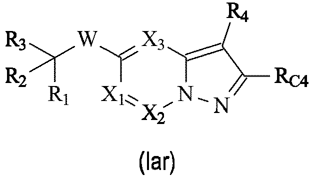

W, R₁, R₂, R₃, R₄, X₁, X₂, X₃, and R_{C5} in the compound of formula (lar) are as defined for the compound of formula (I) of the present invention.

Further accordingly, as W is preferably -NHS(O)₂-, in certain preferred embodiments of the present invention, the compound of formula (la) of the present invention is a compound of formula (las):

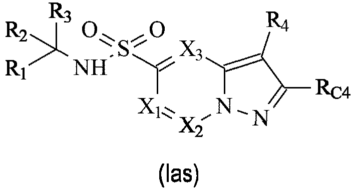

R₁, R₂, R₃, R₄, X₁, X₂, X₃, and R_{C5} in the compound of formula (lar) are as defined for the compound of formula (I) of the present invention.

Further accordingly, as preferably R₂ and R₃ together with the carbon atom to which they are attached form cyclopropyl, in certain preferred embodiments of the present invention, the compound of formula (Ib) of the present invention is a compound of formula (lat):

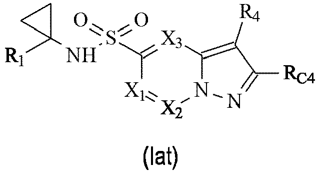

R₁, R₄, X₁, X₂, X₃, and R_{C5} in the compound of formula (lat) are as defined for the compound of formula (I) of the present invention.

Further accordingly, as preferably R₁ is cyano, in certain preferred embodiments of the present invention, the compound of formula (Ic) of the present invention is a compound of formula (lau):

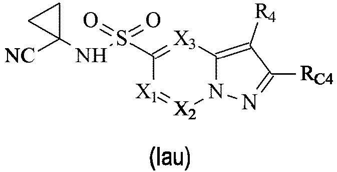

R₄, X₁, X₂, X₃, and R_{C5} in the compound of formula (lau) are as defined for the compound of formula (I) of the present invention.

However, within the scope of the present invention, the compound of formula (lar) or the compound of formula (las) or the compound of formula (lat) wherein R₁ is methyl is also encompassed. In certain preferred embodiments of the present invention, R₁ is methyl. Further within the scope of the present invention, the compound of formula (lar) or the compound of formula (las) or the compound of formula (lat) wherein R₁ is fluoromethyl is also encompassed. In certain preferred embodiments of the present invention, R₁ is fluoromethyl.

Further accordingly, in certain preferred embodiments of the present invention, the compound of formula (Id) of the present invention is a compound of formula (lav):

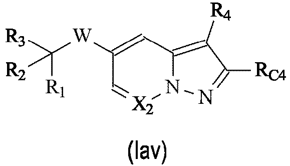

W, R₁, R₂, R₃, R₄, X₂, and R_{C5} in the compound of formula (lav) are as defined for the compound of formula (I) of the present invention.

Further accordingly, in certain preferred embodiments of the present invention, the compound of formula (Ie) of the present invention is a compound of formula (law):

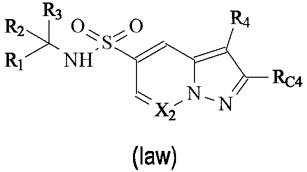

R₁, R₂, R₃, R₄, X₂, and R_{C5} in the compound of formula (law) are as defined for the compound of formula (I) of the present invention.

Further accordingly, in certain preferred embodiments of the present invention, the compound of formula (If) of the present invention is a compound of formula (lax):

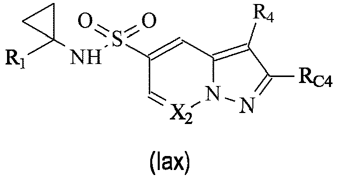

R₁, R₄, X₂, and R_{C5} in the compound of formula (lax) are as defined for the compound of formula (I) of the present invention.

Further accordingly, in certain preferred embodiments of the present invention, the compound of formula (Ig) of the present invention is a compound of formula (lay):

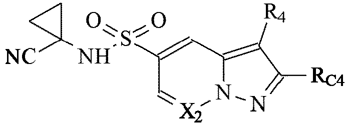

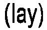

R₄, X₂, and R_{C5} in the compound of formula (lay) are as defined for the compound of formula (I) of the present invention.

Further accordingly, in certain preferred embodiments of the present invention, the compound of formula (Ip) of the present invention is a compound of formula (laz):

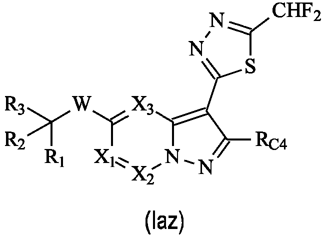

W, R₁, R₂, R₃, X₁, X₂, X₃, and R_{C5} in the compound of formula (laz) are as defined for the compound of formula (I) of the present invention.

Further accordingly, as preferably W is -NHS(O)₂-, in certain preferred embodiments of the present invention, the compound of formula (Iq) of the present invention is a compound of formula (Iba):

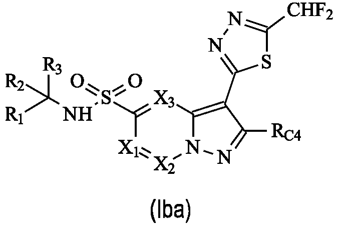

R₁, R₂, R₃, X₁, X₂, X₃, and R_{C5} in the compound of formula (Iba) are as defined for the compound of formula (I) of the present invention.

Further accordingly, as preferably R₂ and R₃ together with the carbon atom to which they are attached form cyclopropyl, in certain preferred embodiments of the present invention, the compound of formula (Ir) of the present invention is a compound of formula (Ibb):

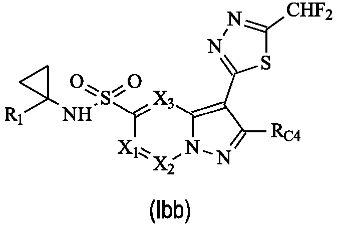

R₁, X₂, and R_{C5} in the compound of formula (Ibb) are as defined for the compound of formula (I) of the present invention.

Further accordingly, as preferably R₁ is cyano, in certain preferred embodiments of the present invention, the compound of formula (Is) of the present invention is a compound of formula (Ibc):

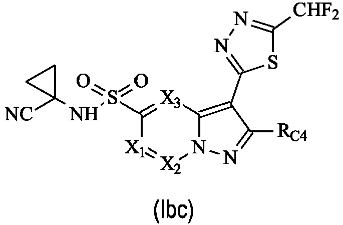

Further preferred within the scope of the present invention are embodiments wherein X₁ and X₃ are each CH. Thus, in certain preferred embodiments, the compound of formula (It) is a compound of formula (Ibd):

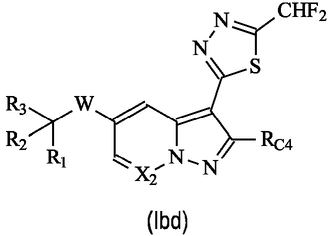

W, R₁, R₂, R₃, X₂, and R_{C5} in the compound of formula (Ibd) are as defined for the compound of formula (I) of the present invention.

Further accordingly, in certain preferred embodiments of the present invention, the compound of formula (lu) of the present invention is a compound of formula (Ibe):

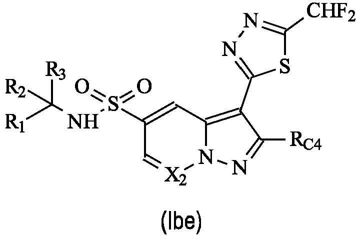

R₁, R₂, R₃, X₂, and R_{C5} in the compound of formula (Ibe) are as defined for the compound of formula (I) of the present invention.

Further accordingly, in certain preferred embodiments of the present invention, the compound of formula (Iv) of the present invention is a compound of formula (Ibf):

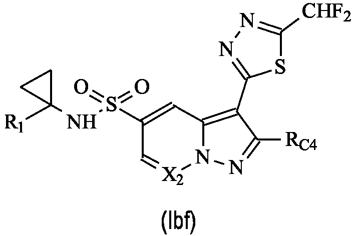

R₁, X₂, and R_{C5} in the compound of formula (Ibf) are as defined for the compound of formula (I) of the present invention.

Further accordingly, in certain preferred embodiments of the present invention, the compound of formula (Iw) of the present invention is a compound of formula (Ibg):

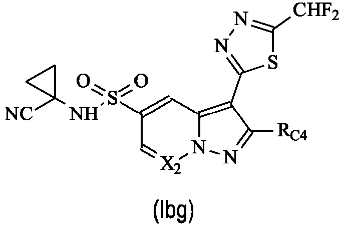

X₂ and R_{C5} in the compound of formula (Ibf) are as defined for the compound of formula (I) of the present invention.

In certain embodiments of the present invention, preferably X₂ is C-Y_{C2}-R_{C2}. Most preferably, - Y_{C2}-R_{C2} is piperazinyl (preferably N-piperazinyl) substituted (preferably N-substituted, preferably at a different N-atom than that attached to the ring system as shown in formula (I)), with -CON(C₁₋₅ alkyl)(C₁₋₅ alkyl), preferably with -CON(CH₃)₂.

Thus accordingly, in certain preferred embodiments of the present invention the compound of formula (lab) is a compound of formula (Ibh):

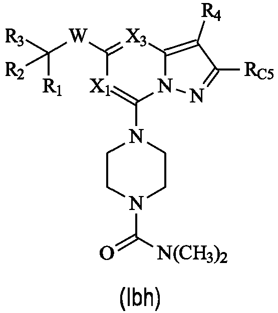

W, R₁, R₂, R₃, R₄, X₁, X₃ and R_{C5} in the compound of formula (Ibh) are as defined for the compound of formula (I) of the present invention.

Further accordingly, in certain preferred embodiments of the present invention the compound of formula (lac) is a compound of formula (Ibi):

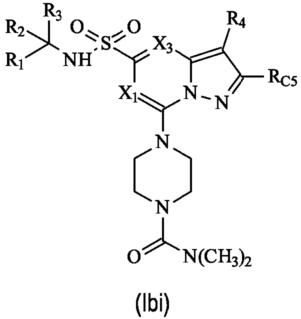

R₁, R₂, R₃, R₄, X₁, X₃ and R_{C5} in the compound of formula (Ibi) are as defined for the compound of formula (I) of the present invention.

Further accordingly, in certain preferred embodiments of the present invention the compound of formula (lad) is a compound of formula (Ibj):

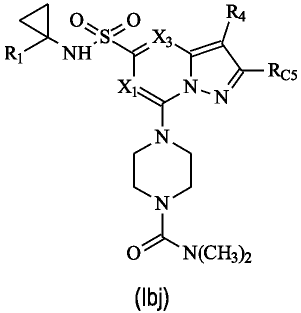

R₁, R₄, X₁, X₃ and R_{C5} in the compound of formula (Ibi) are as defined for the compound of formula (I) of the present invention.

Further accordingly, in certain preferred embodiments of the present invention the compound of formula (lae) is a compound of formula (Ibk):

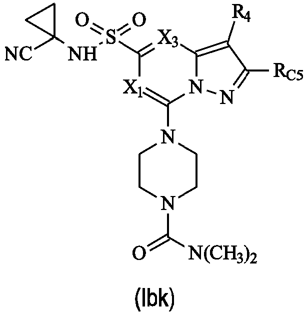

R₄, X₁, X₃ and R_{C5} in the compound of formula (Ibi) are as defined for the compound of formula (I) of the present invention.

Further preferred within the scope of the present invention are embodiments wherein X₁ and X₃ are each CH. Thus, in certain preferred embodiments, the compound of formula (laf) is a compound of formula (IbL):

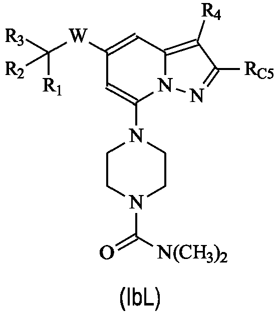

W, R₁, R₂, R₃, R₄, and R_{C5} in the compound of formula (IbL) are as defined for the compound of formula (I) of the present invention.

Further accordingly, in certain preferred embodiments of the present invention the compound of formula (lag) is a compound of formula (Ibm):

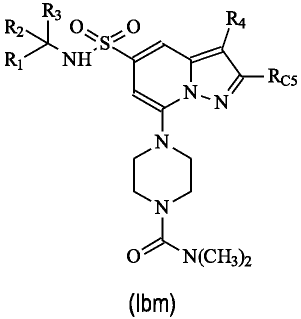

R₁, R₂, R₃, R₄, and R_{C5} in the compound of formula (Ibm) are as defined for the compound of formula (I) of the present invention.

Further accordingly, in certain preferred embodiments of the present invention the compound of formula (lah) is a compound of formula (Ibn):

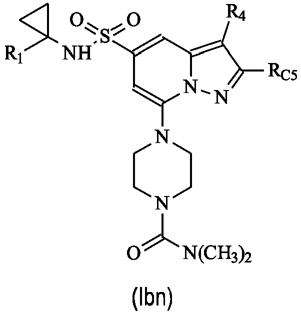

R₁, R₄, and R_{C5} in the compound of formula (Ibn) are as defined for the compound of formula (I) of the present invention.

Further accordingly, in certain preferred embodiments of the present invention the compound of formula (lai) is a compound of formula (Ibo):

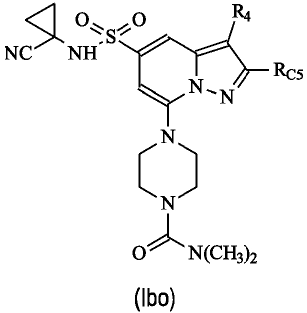

R₄, and R_{C5} in the compound of formula (Ibn) are as defined for the compound of formula (I) of the present invention.

In certain embodiments of the present invention, X₁ is CF and X₃ is CH. Thus, the compound of formula (I) is a compound of formula (Ibp):

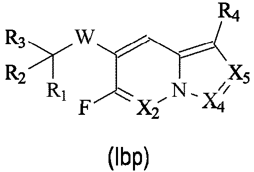

W, R₁, R₂, R₃, R₄, X₂, X₄, and X₅ in the compound of formula (Ibp) are as defined hereinabove for the compound of formula (I).

Further accordingly, as preferably W is -NHS(O)₂-, in certain preferred embodiments of the present invention, the compound of formula (Ibp) of the present invention is a compound of formula (Ibq):

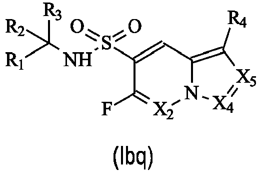

R₁, R₂, R₃, R₄, X₂, X₄, and X₅ in the compound of formula (Ibq) are as defined hereinabove for the compound of formula (I).

Further accordingly, as preferably R₂ and R₃ together with the carbon atom to which they are attached form cyclopropyl, in certain preferred embodiments of the present invention, the compound of formula (Ibq) of the present invention is a compound of formula (Ibr):

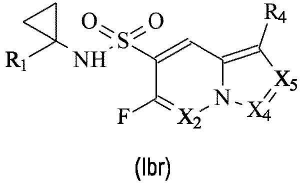

R₁, R₄, X₂, X₄, and X₅ in the compound of formula (Ibr) are as defined hereinabove for the compound of formula (I).

Further accordingly, as preferably R₁ is cyano, in certain preferred embodiments of the present invention, the compound of formula (Ibr) of the present invention is a compound of formula (Ibs):

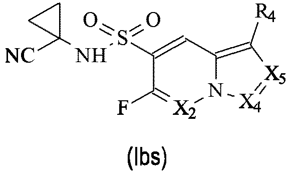

R₄, X₂, X₄, and X₅ in the compound of formula (Ibs) are as defined hereinabove for the compound of formula (I).

It is however noted that the compounds of formula (Ibp), (Ibq) or (Ibr) wherein R₁ is methyl or fluoromethyl are also encompassed within the scope of the present invention.

In certain embodiments of the present invention, preferably X₂ is C-Y_{C2}-R_{C2}. Most preferably, - Y_{C2}-R_{C2} is piperazinyl (preferably N-piperazinyl) substituted (preferably N-substituted, preferably at a different N-atom than that attached to the ring system as shown in formula (I)), with -CON(C₁₋₅ alkyl)(C₁₋₅ alkyl), preferably with -CON(CH₃)₂.

Thus accordingly, in certain embodiments of the present invention the compound of formula (Ibp) is a compound of formula (Ibt):

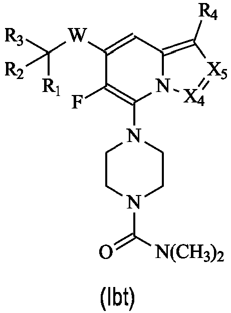

W, R₁, R₂, R₃, R₄, X₄, and X₅ in the compound of formula (Ibt) are as defined hereinabove for the compound of formula (I).

Further accordingly, in certain embodiments of the present invention the compound of formula (Ibq) is a compound of formula (Ibu):

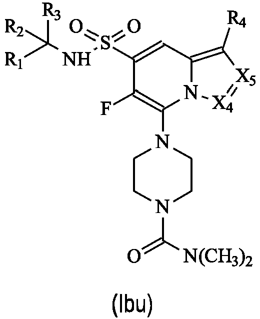

R₁, R₂, R₃, R₄, X₄, and X₅ in the compound of formula (Ibu) are as defined hereinabove for the compound of formula (I).

Further accordingly, in certain embodiments of the present invention the compound of formula (Ibr) is a compound of formula (Ibv).

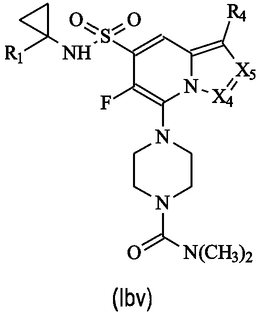

R₁, R₄, X₄, and X₅ in the compound of formula (Ibv) are as defined hereinabove for the compound of formula (I).

Further accordingly, in certain embodiments of the present invention the compound of formula (Ibs) is a compound of formula (Ibw).

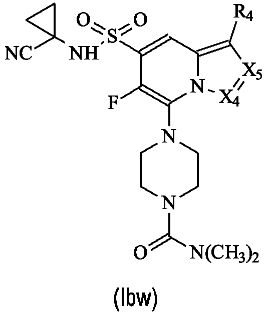

R₄, X₄, and X₅ in the compound of formula (Ibt) are as defined hereinabove for the compound of formula (I).

It is however noted that the compounds of formula (Ibt), (Ibu) or (Ibv) wherein R₁ is methyl or fluoromethyl are also encompassed within the scope of the present invention.

In certain preferred embodiments, X₄ is C-R_{C4} and X₅ is C-Rcs. Thus, the compound of formula (I) is in certain embodiments a compound of formula (Ibx):

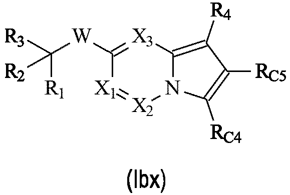

W, R₁, R₂, R₃, R₄, R_{C4}, R_{C5}, X₁, X₂, and X₃ in the compound of formula (Ibx) are as defined hereinabove for the compound of formula (I).

Further accordingly, as preferably W is -NHS(O)₂-, in certain preferred embodiments of the present invention, the compound of formula (Ibx) of the present invention is a compound of formula (Iby):

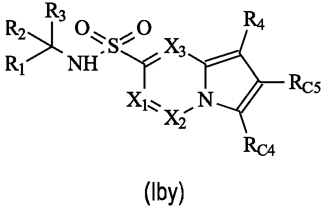

R₁, R₂, R₃, R₄, R_{C4}, R_{C5}, X₁, X₂, and X₃ in the compound of formula (Iby) are as defined hereinabove for the compound of formula (I).

Further accordingly, as preferably R₂ and R₃ together with the carbon atom to which they are attached form cyclopropyl, in certain preferred embodiments of the present invention, the compound of formula (Iby) of the present invention is a compound of formula (Ibz):

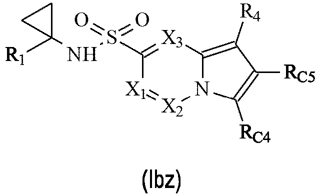

R₁, R₄, R_{C4}, R_{C5}, X₁, X₂, and X₃ in the compound of formula (Ibz) are as defined hereinabove for the compound of formula (I).

Further accordingly, as preferably R₁ is cyano, in certain preferred embodiments of the present invention, the compound of formula (Ibz) of the present invention is a compound of formula (Ica):

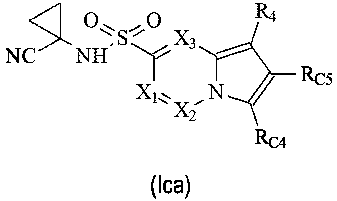

R₄, R_{C4}, R_{C5}, X₁, X₂, and X₃ in the compound of formula (Ica) are as defined hereinabove for the compound of formula (I).

It is however noted that the compounds of formula (Ibx), (Iby) or (Ibz) wherein R₁ is methyl or fluoromethyl are also encompassed within the scope of the present invention.

In certain preferred embodiments X₁ is CH and X₃ is CH.

Thus accordingly, in certain embodiments of the present invention the compound of formula (Ibx) is a compound of formula (Icb):

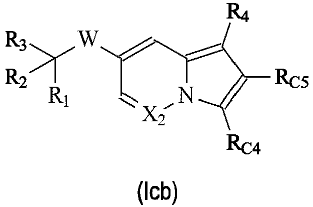

W, R₁, R₂, R₃, R₄, R_{C4}, R_{C5}, and X₂ in the compound of formula (Icb) are as defined hereinabove for the compound of formula (I).

Further accordingly, in certain embodiments of the present invention the compound of formula (Iby) is a compound of formula (Icc):

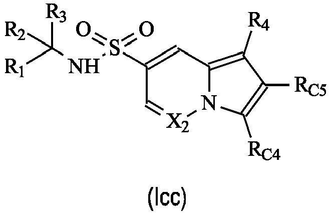

R₁, R₂, R₃, R₄, R_{C4}, R_{C5}, and X₂ in the compound of formula (Icc) are as defined hereinabove for the compound of formula (I).

Further accordingly, in certain embodiments of the present invention the compound of formula (Ibz) is a compound of formula (Icd):

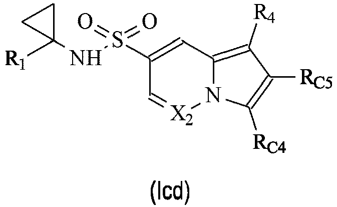

R₁, R₄, R_{C4}, R_{C5}, and X₂ in the compound of formula (Icd) are as defined hereinabove for the compound of formula (I).

Further accordingly, in certain embodiments of the present invention the compound of formula (Ica) is a compound of formula (Ice):

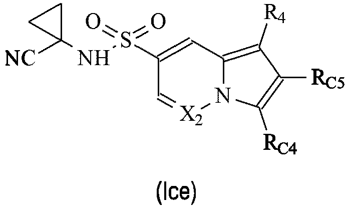

R₄, R_{C4}, R_{C5}, and X₂ in the compound of formula (Ice) are as defined hereinabove for the compound of formula (I).

In certain preferred embodiments X₄ is CH and X₅ is CH.

Thus accordingly, in certain embodiments of the present invention the compound of formula (Icb) is a compound of formula (Icf):

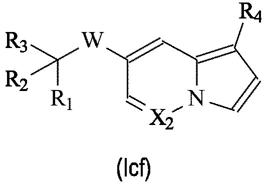

W, R₁, R₂, R₃, R₄, and X₂ in the compound of formula (Icf) are as defined hereinabove for the compound of formula (I).

Further accordingly, in certain embodiments of the present invention the compound of formula (Icc) is a compound of formula (Icg):

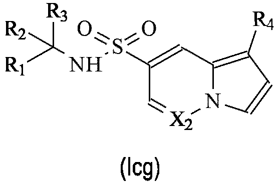

R₁, R₂, R₃, R₄, and X₂ in the compound of formula (Icg) are as defined hereinabove for the compound of formula (I).

Further accordingly, in certain embodiments of the present invention the compound of formula (Icd) is a compound of formula (Ich):

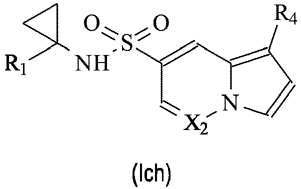

R₁, R₄, and X₂ in the compound of formula (Ich) are as defined hereinabove for the compound of formula (I).

Further accordingly, in certain embodiments of the present invention the compound of formula (Icd) is a compound of formula (Ici):

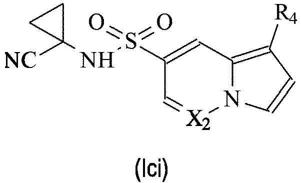

R₄ and X₂ in the compound of formula (Ici) are as defined hereinabove for the compound of formula (I).

Preferred compound of formula (I) are selected from the following compounds: 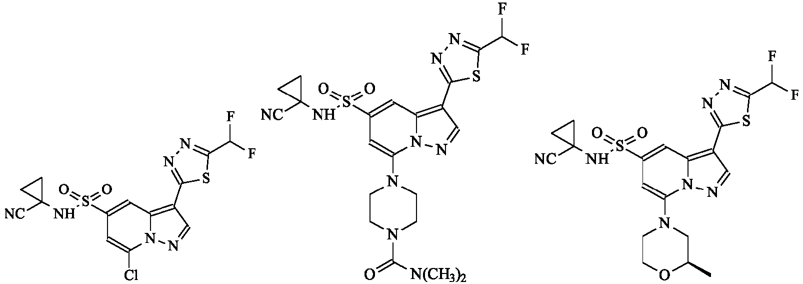 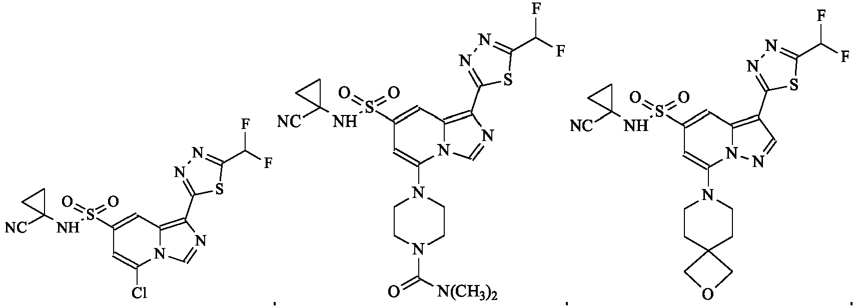 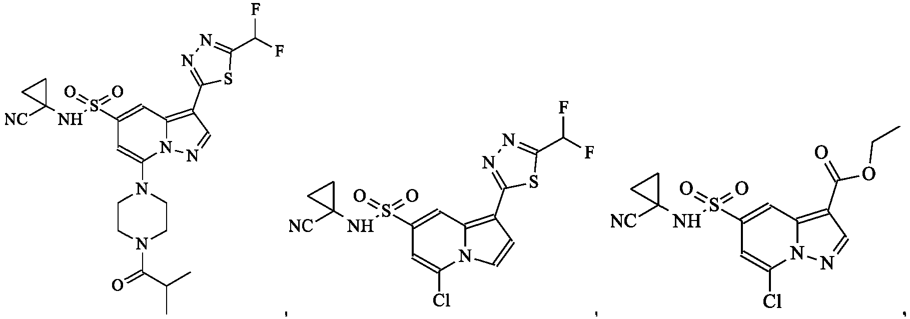 and 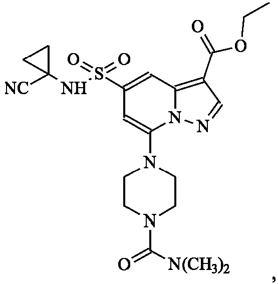 or an enantiomer, diastereoisomer, tautomer, pharmaceutically acceptable solvate, phramaceutically acceptable crystal form or pharmaceutically acceptable salt thereof.

Further preferred compounds of formula (I) are selected from: 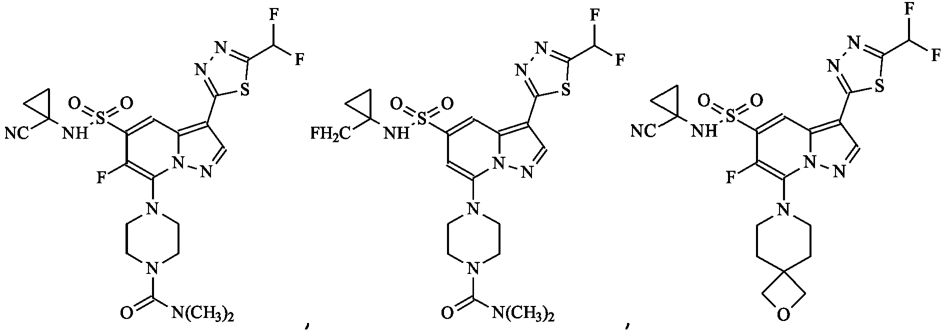 and 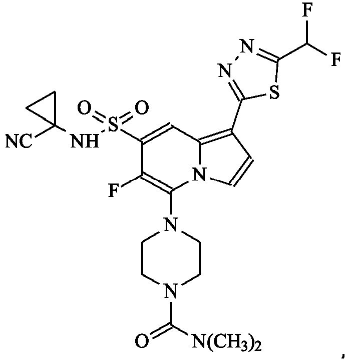 or an enantiomer, diastereoisomer, tautomer, pharmaceutically acceptable solvate, pharmaceutically acceptable crystal form, pharmaceutically acceptable salt thereof.

Further preferred compounds of formula (I) are selected from: 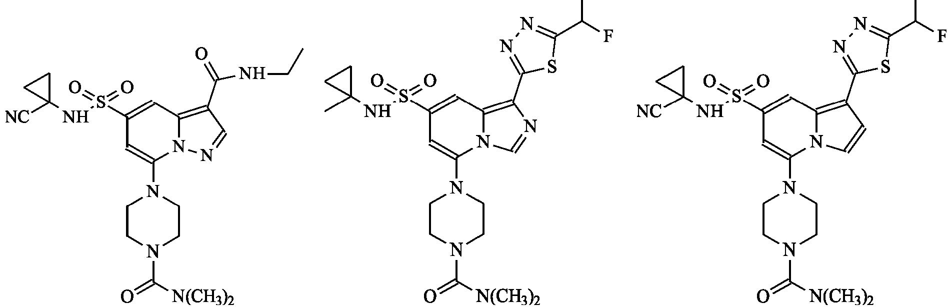 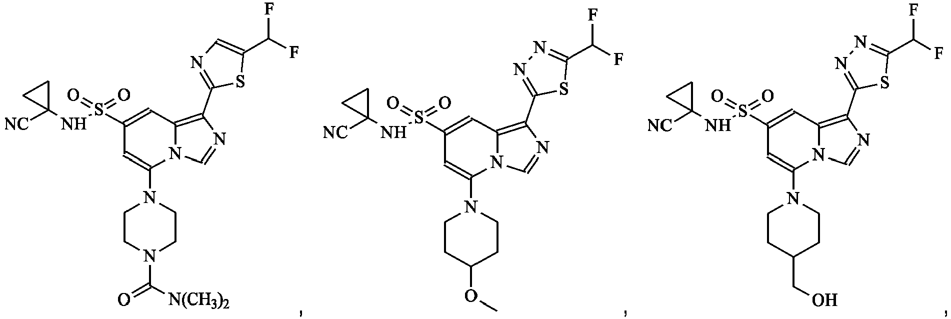 and 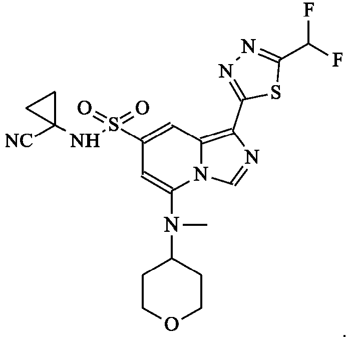 or an enantiomer. diastereoisomer. tautomer. pharmaceutically acceptable solvate, pharmaceutically acceptable crystal form, pharmaceutically acceptable salt thereof.

Particularly preferred are exemplified compounds, i.e. the compounds 1 to 12, as described hereinbelow.

Preferably, the compound of formula (I) is selected from: 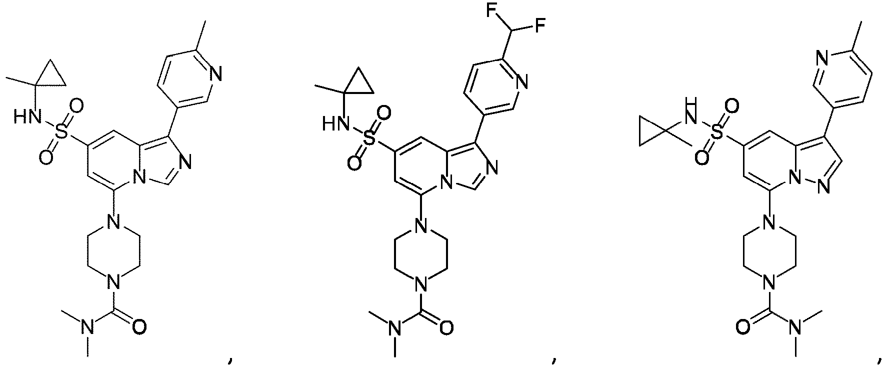 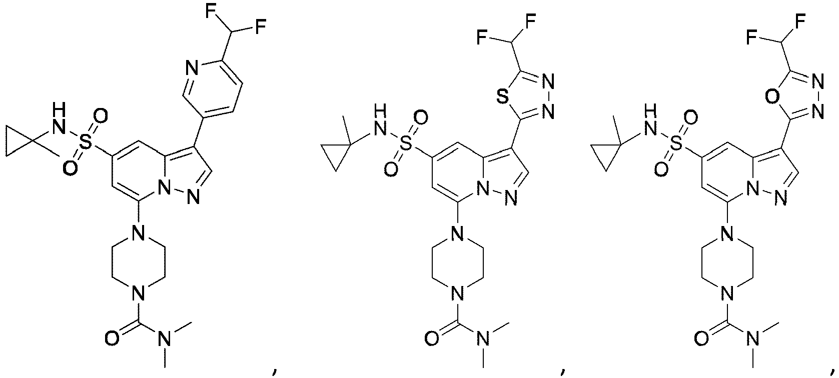 and 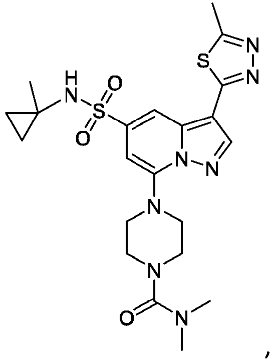 or a pharmaceutically acceptable salt, hydrate or solvate thereof.

The present invention also relates to each of the intermediates described further below in the examples section of this specification, including any one of these intermediates in non-salt form or in the form of a salt (e.g., a pharmaceutically acceptable salt) of the respective compound. Such intermediates can be used, in particular, in the synthesis of the compounds of formula (I).

The scope of the invention embraces all pharmaceutically acceptable salt forms of the compounds of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (I) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) is a hydrochloride salt. Accordingly, it is preferred that the compound of formula (I), including any one of the specific compounds of formula (I) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (I) is in the form of a hydrochloride salt.

The present invention also specifically relates to the compound of formula (I), including any one of the specific compounds of formula (I) described herein, in non-salt form.

Moreover, the scope of the invention embraces the compounds of formula (I) in any solvated form, including, e.g., solvates with water (i.e., as a hydrate) or solvates with organic solvents such as, e.g., methanol, ethanol, isopropanol, acetic acid, ethyl acetate, ethanolamine, DMSO, or acetonitrile. All physical forms, including any amorphous or crystalline forms (i.e., polymorphs), of the compounds of formula (I) are also encompassed within the scope of the invention. It is to be understood that such solvates and physical forms of pharmaceutically acceptable salts of the compounds of the formula (I) are likewise embraced by the invention.

Furthermore, the compounds of formula (I) may exist in the form of different isomers, in particular stereoisomers (including, e.g., geometric isomers (or cis/trans isomers), enantiomers and diastereomers) or tautomers (including, in particular, prototropic tautomers, such as keto/enol tautomers or thione/thiol tautomers). All such stereoisomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds of formula (I). It will be understood that some compounds may exhibit tautomerism. In such cases, the formulae provided herein expressly depict only one of the possible tautomeric forms. The formulae and chemical names as provided herein are intended to encompass any tautomeric form of the corresponding compound and not to be limited merely to the specific tautomeric form depicted by the drawing or identified by the name of the compound.

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., ²H; also referred to as "D"). Accordingly, the invention also embraces compounds of formula (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces compounds of formula (I), in which one or more atoms are replaced by a positron-emitting isotope of the corresponding atom, such as, e.g., ¹⁸F, ¹¹C, ¹³N, ¹⁵O, ⁷⁶Br, **⁷⁷Br,** ¹²⁰I and/or ¹²⁴I. Such compounds can be used as tracers, trackers or imaging probes in positron emission tomography (PET). The invention thus includes (i) compounds of formula (I), in which one or more fluorine atoms (or, e.g., all fluorine atoms) are replaced by ¹⁸F atoms, (ii) compounds of formula (I), in which one or more carbon atoms (or, e.g., all carbon atoms) are replaced by ¹¹C atoms, (iii) compounds of formula (I), in which one or more nitrogen atoms (or, e.g., all nitrogen atoms) are replaced by ¹³N atoms, (iv) compounds of formula (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula (I), in which one or more bromine atoms (or, e.g., all bromine atoms) are replaced by ⁷⁷Br atoms, (vii) compounds of formula (I), in which one or more iodine atoms (or, e.g., all iodine atoms) are replaced by ¹²⁰I atoms, and (viii) compounds of formula (I), in which one or more iodine atoms (or, e.g., all iodine atoms) are replaced by ¹²⁴I atoms. In general, it is preferred that none of the atoms in the compounds of formula (I) are replaced by specific isotopes.

The present invention further embraces the prodrugs of the compounds of formula (I). As preferably understood herein, the term "prodrug" of the compound of formula (I) refers to a derivative of the compounds of formula (I) that upon administration to a subject becomes metabolized to the said compound of formula (I). Said prodrugs of the compound of formula (I) may include modifications of -OH, -NH₂, or -COOH group if present in the compound of formula (I), which preferably can be hydrolyzed to - OH, -NH₂, or -COOH groups, respectively, e.g. upon administration to the subject. For example, as known to the skilled person, such prodrugs may preferably include for the compounds of formula (I) which comprise -OH moiety derivatives wherein said -OH moiety is turned into an -ORₓ moiety, wherein Rₓ preferably comprises a moiety selected from -CO-, -CH₂-O-CO, -CH₂-O-CO-O-, and -CH(CH₃)-O-COO-, more preferably wherein Rₓ is selected from -CO-R_{y}, -CH₂-O-CO-R_{y}, -CH₂-O-CO-O-R_{y}, and -CH(CH₃)-O-COO-R_{y}, wherein R_{y} is preferably carbocyclyl, heterocyclyl, C₁₋₅ alkyl, -NH-(C₁₋₅ alkyl) or -S-(C₁₋₅ alkyl), wherein the said alkyl is optionally substituted with a group selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein the said carbocyclyl and heterocyclyl are each optionally substituted with a group selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl). Furthermore, for example, as known to the skilled person, such prodrugs may preferably include for the compounds of formula (I) which comprise -NH₂ moiety derivatives wherein said -NH₂ moiety is turned into -NHCOO-R_{y} moiety, wherein R_{y} is as defined hereinabove. Furthermore, for examples, as known to the skilled person, such prodrugs may preferably include for the compounds of formula (I) which comprise -COOH moiety derivatives wherein said -COOH group is turned into -COOR_{y} moiety, wherein R_{y} is as defined hereinabove. Further examples of groups that can be derivatized to yield prodrugs are known to the skilled person.

### Pharmaceutical compositions

The compounds provided herein may be administered as compounds *per se* or may be formulated as medicaments. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor^{®} HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

The pharmaceutical compositions may also comprise one or more preservatives, particularly one or more antimicrobial preservatives, such as, e.g., benzyl alcohol, chlorobutanol, 2-ethoxyethanol, m-cresol, chlorocresol (e.g., 2-chloro-3-methyl-phenol or 4-chloro-3-methyl-phenol), benzalkonium chloride, benzethonium chloride, benzoic acid (or a pharmaceutically acceptable salt thereof), sorbic acid (or a pharmaceutically acceptable salt thereof), chlorhexidine, thimerosal, or any combination thereof.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) or the above described pharmaceutical compositions comprising a compound of formula (I) may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, or vaginal administration.

If said compounds or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

For oral administration, the compounds or pharmaceutical compositions are preferably administered by oral ingestion, particularly by swallowing. The compounds or pharmaceutical compositions can thus be administered to pass through the mouth into the gastrointestinal tract, which can also be referred to as "oral-gastrointestinal" administration.

Alternatively, said compounds or pharmaceutical compositions can be administered in the form of a suppository or pessary, or may be applied topically in the form of a gel, hydrogel, lotion, solution, cream, ointment or dusting powder. The compounds of the present invention may also be dermally or transdermally administered, for example, by the use of a skin patch.

Said compounds or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides, copolymers of L-glutamic acid and gamma-ethyl-L-glutamate, poly(2-hydroxyethyl methacrylate), ethylene vinyl acetate, or poly-D-(-)-3-hydroxybutyric acid. Sustained-release pharmaceutical compositions also include liposomally entrapped compounds. The present invention thus also relates to liposomes containing a compound of the invention.

Said compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to an emulsification/spray drying process.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the compounds or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. Preferred routes of administration are oral administration or parenteral administration. For each of the compounds or pharmaceutical compositions provided herein, it is particularly preferred that the respective compound or pharmaceutical composition is to be administered orally (particularly by oral ingestion).

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of the compounds according to the invention for oral administration to a human (of approximately 70 kg body weight) may be 0.05 to 2000 mg, preferably 0.1 mg to 1000 mg, of the active ingredient per unit dose. The unit dose may be administered, e.g., 1 to 3 times per day. The unit dose may also be administered 1 to 7 times per week, e.g., with not more than one administration per day. It will be appreciated that it may be necessary to make routine variations to the dosage depending on the age and weight of the patient/subject as well as the severity of the condition to be treated. The precise dose and also the route of administration will ultimately be at the discretion of the attendant physician or veterinarian.

### Therapeutic use

In one embodiment, the present invention relates to the compound of formula (I), or a pharmaceutically acceptable salt, hydrate or solvate thereof, or a pharmaceutical composition as defined herein for use in therapy.

The present invention provides compounds that function as inhibitors of PARG. Thus, the present invention provides a method of inhibiting PARG enzyme activity in vitro or in vivo, said method comprising contacting a cell with an effective amount of the compound of formula (I), or a pharmaceutically acceptable salt, hydrate or solvate thereof, as defined herein.

The present invention also provides a method of selectively inhibiting PARG enzyme activity over PARP1 or ARH3 enzyme activity in vitro or in vivo. The said method comprises the steps of contacting a cell with an effective amount of a compound, or a pharmaceutically acceptable salt, hydrate or solvate thereof, as defined herein.

In a further embodiment, the present invention relates to the compound of formula (I), as disclosed herein, for use in a method of treating a disease or disorder in which PARG activity is implicated in a subject or patient in need of such treatment. Said method of treatment comprises administering to said subject/patient a therapeutically effective amount of a compound of formula (I), or a pharmaceutically acceptable salt, hydrate or solvate thereof, or a pharmaceutical composition as defined herein. In other words, in one embodiment the present invention relates to the compound of formula (I), as disclosed herein, for use in treating a disease or disorder in which PARG activity is implicated.

In a further embodiment, the present invention relates to a method of inhibiting cell proliferation, in vitro or in vivo, said method comprising contacting a cell with an effective amount of the compound of formula (I), or a pharmaceutically acceptable salt, hydrate or solvate thereof, as defined herein. Thus, the present invention relates to the compound of formula (I) or a pharmaceutically acceptable salt thereof for use in of inhibiting cell proliferation, in vitro or in vivo.

Thus, in a further embodiment, the present invention relates to a method of treating a proliferative disorder in a subject or patient in need of such treatment. The said method of treating a proliferative disorder in a subject or patient in need thereof comprises administering to said subject/patient a therapeutically effective amount of the compound of formula (I), or a pharmaceutically acceptable salt, hydrate or solvate thereof, or a pharmaceutical composition as defined herein. Preferably as disclosed herein, the proliferative disorder is cancer. Thus, the present invention relates to a method of treating cancer in a subject or patient in need thereof. The said method of treating cancer in a subject or patient in need thereof comprises administering to said subject/patient a therapeutically effective amount of the compound of formula (I), or a pharmaceutically acceptable salt, hydrate or solvate thereof, or a pharmaceutical composition as defined herein. In a particular embodiment, the cancer is human cancer.

In one embodiment, the present invention relates to the compound of formula (I) or a pharmaceutically acceptable salt, hydrate or solvate thereof, for use in treating a proliferative disorder. Preferably as disclosed herein, the proliferative disorder is cancer. Therefore, the present invention relates to the compound of formula (I) or a pharmaceutically acceptable salt, hydrate or solvate thereof for use in treating cancer. In a particular embodiment, the cancer is human cancer.

In a further embodiment, the present invention relates to the compound of formula (I), or a pharmaceutically acceptable salt, hydrate or solvate thereof, as defined herein, for use in the manufacture of a medicament for the treatment of a proliferative condition. In a preferred embodiment, the proliferative condition is cancer, more preferably a human cancer. Thus, preferably the present invention relates to the compound of formula (I), or a pharmaceutically acceptable salt, hydrate or solvate thereof, as defined herein, for use in the manufacture of a medicament for the treatment of cancer, preferably for the treatment of human cancer.

In a further embodiment, the present invention relates to the compound of formula (I), or a pharmaceutically acceptable salt, hydrate or solvate thereof, as defined herein, for use in the manufacture of a medicament for the inhibition of PARG enzyme activity. Preferably, the inhibition of PARG enzyme activity is selective inhibition of PARG enzyme activity over PARP1 or ARH3 enzyme activity. Thus, the present invention relates to the compound of formula (I), or a pharmaceutically acceptable salt, hydrate or solvate thereof, as defined herein, for use in the manufacture of a medicament for the selective inhibition of PARG enzyme activity over PARP1 or ARH3 enzyme activity.

The present invention further provides the compound of formula (I), or a pharmaceutically acceptable salt, hydrate or solvate thereof, as defined herein for use in the manufacture of a medicament for the treatment of a disease or disorder in which PARG activity is implicated, as defined herein.

As understood herein, the term "proliferative disorder" are used interchangeably herein and pertain to an unwanted or uncontrolled cellular proliferation of excessive or abnormal cells which is undesired, such as, neoplastic or hyperplastic growth, whether in vitro or in vivo. Examples of proliferative conditions include, but are not limited to, pre-malignant and malignant cellular proliferation, including but not limited to, malignant neoplasms and tumours, cancers, leukemias, psoriasis, bone diseases, fibroproliferative disorders (e.g., of connective tissues), and atherosclerosis. Any type of cell may be treated, including but not limited to, lung, colon, breast, ovarian, prostate, liver, pancreas, brain, and skin.

The anti-proliferative effects of the compound of formula (I) of the present invention have particular application in the treatment of human cancers (by virtue of their inhibition of PARG enzyme activity). The anti-cancer effect may arise through one or more mechanisms, including but not limited to, the regulation of cell proliferation, the inhibition of angiogenesis (the formation of new blood vessels), the inhibition of metastasis (the spread of a tumour from its origin), the inhibition of invasion (the spread of tumour cells into neighbouring normal structures), or the promotion of apoptosis (programmed cell death).

The anti proliferative treatment with the compound of formula (I) or a pharmaceutically acceptable salt, hydrate or solvate thereof, as defined hereinbefore, may be applied as a sole therapy or may involve, in addition to the compound of the invention, conventional surgery or radiotherapy or chemotherapy. Such chemotherapy may include one or more of the following categories of anti-tumour agents:-
(i) other antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5oc-reductase such as finasteride;
(iii) anti-invasion agents [for example c-Src kinase family inhibitors like 4-(6-chloro-2,3-methylenedioxyanilino)-7-[2-(4-methylpiperazin-1 -yl)ethoxy]-5-tetrahydropyran-4- yloxyquinazoline (AZD0530; International Patent Application WO 01/94341 ), N-(2-chloro-6- methylphenyl)-2-(6-[4-(2-hydroxyethyl)piperazin-1 -yl]-2-methylpyrimidin-4-ylamino}thiazole- 5-carboxamide (dasatinib, BMS-354825; J. Med. Chem., 2004, 47, 6658-6661 ) and bosutinib (SKI-606), and metalloproteinase inhibitors like marimastat, inhibitors of urokinase plasminogen activator receptor function or antibodies to Heparanase];
(iv) inhibitors of growth factor function: for example such inhibitors include growth factor antibodies and growth factor receptor antibodies (for example the anti-erbB2 antibody trastuzumab [Herceptin^{™}], the anti-EGFR antibody panitumumab, the anti-erbB1 antibody cetuximab [Erbitux, C225] and any growth factor or growth factor receptor antibodies disclosed by Stern et al. (Critical reviews in oncology/haematology, 2005, Vol. 54, pp1 1 -29); such inhibitors also include tyrosine kinase inhibitors, for example inhibitors of the epidermal growth factor family (for example EGFR family tyrosine kinase inhibitors such as N-(3-chloro- 4-fluorophenyl)-7-methoxy-6-(3-morpholinopropoxy)quinazolin-4-amine (gefitinib, ZD1839), N-(3-ethynylphenyl)-6,7-bis(2-methoxyethoxy)quinazolin-4-amine (erlotinib, OSI-774) and 6- acrylamido-N-(3-chloro-4-fluorophenyl)-7-(3-morpholinopropoxy)-quinazolin-4-amine (CI 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib (BAY 43-9006), tipifarnib (R1 15777) and lonafarnib (SCH66336)), inhibitors of cell signalling through MEK and/or AKT kinases, c-kit inhibitors, abl kinase inhibitors, PI3 kinase inhibitors, PIt3 kinase inhibitors, CSF-1 R kinase inhibitors, IGF receptor (insulin-like growth factor) kinase inhibitors; aurora kinase inhibitors (for example AZD1 152, PH739358, VX-680, MLN8054, R763, MP235, MP529, VX-528 AND AX39459) and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin^{™}) and for example, a VEGF receptor tyrosine kinase inhibitor such as vandetanib (ZD6474), vatalanib (PTK787), sunitinib (SU1 1248), axitinib (AG-013736), pazopanib (GW 786034) and 4-(4-fluoro-2-methylindol-5-yloxy)-6-methoxy-7-(3-pyrrolidin-1 - ylpropoxy)quinazoline (AZD2171 ; Example 240 within WO 00/47212), compounds such as those disclosed in International Patent Applications WO97/22596, WO 97/30035, WO 97/32856 and WO 98/13354 and compounds that work by other mechanisms (for example linomide, inhibitors of integrin αvβ3 function and angiostatin)];
(vi) vascular damaging agents such as Combretastatin A4 and compounds disclosed in International Patent Applications WO 99/02166, WO 00/40529, WO 00/41669, WO 01 /92224, WO 02/04434 and WO 02/08213; (vii) an endothelin receptor antagonist, for example zibotentan (ZD4054) or atrasentan;
(viii) antisense therapies, for example those which are directed to the targets listed above, such as ISIS 2503, an anti-ras antisense;
(ix) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multidrug resistance gene therapy; and
(x) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In a particular embodiment, the antiproliferative treatment defined hereinbefore may involve, in addition to the compound of formula (I) of the invention, conventional surgery or radiotherapy or chemotherapy.Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect the present invention further relates to the compound of formula (I) or a pharmaceutically acceptable salt, hydrate or solvate thereof, as defined herein, for use in the treatment of a cancer (for example a cancer involving a solid tumour) in combination with another anti-tumour agent. The anti-tumour agent is preferably selected from the anti-tumour agents as listed hereinabove.

As understood herein, the term "combination" refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

### Examples

The following examples are merely illustrative of the present invention.

### Synthesis of the compounds of formula (I)

The syntheses of embodiments **A, B** and **C** of the compounds of formula (I) according to the present invention are preferably carried out according to the general synthetic sequences as shown in Schemes 1-3.

In addition to said routes described below, also other routes may be used to synthesize the target compounds, in accordance with common general knowledge of a person skilled in the art of organic synthesis. The order of transformations exemplified in the following Schemes is therefore not intended to be limiting, and suitable synthesis steps from various schemes can be combined to form additional synthesis sequences. In addition, modification of any of the substituents can be achieved before and/or after the exemplified transformations. These modifications can be such as the introduction of protective groups, cleavage of protective groups, reduction or oxidation of functional groups, halogenation, metallation, metal-catalyzed coupling reactions, substitution or other reactions known to a person skilled in the art. These transformations include those which introduce a functionality allowing for further interconversion of substituents. Appropriate protective groups and their introduction and cleavage are well-known to a person skilled in the art (see for example: Greene's Protective Groups in Organic Synthesis; Editor: P.G.M. Wuts, 5th edition, Wiley 2014). Specific examples are described in the subsequent paragraphs. Further, it is possible that two or more successive steps may be performed without work-up being performed between said steps, e.g. a "one-pot" reaction, as it is well-known to a person skilled in the art.

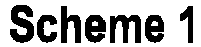

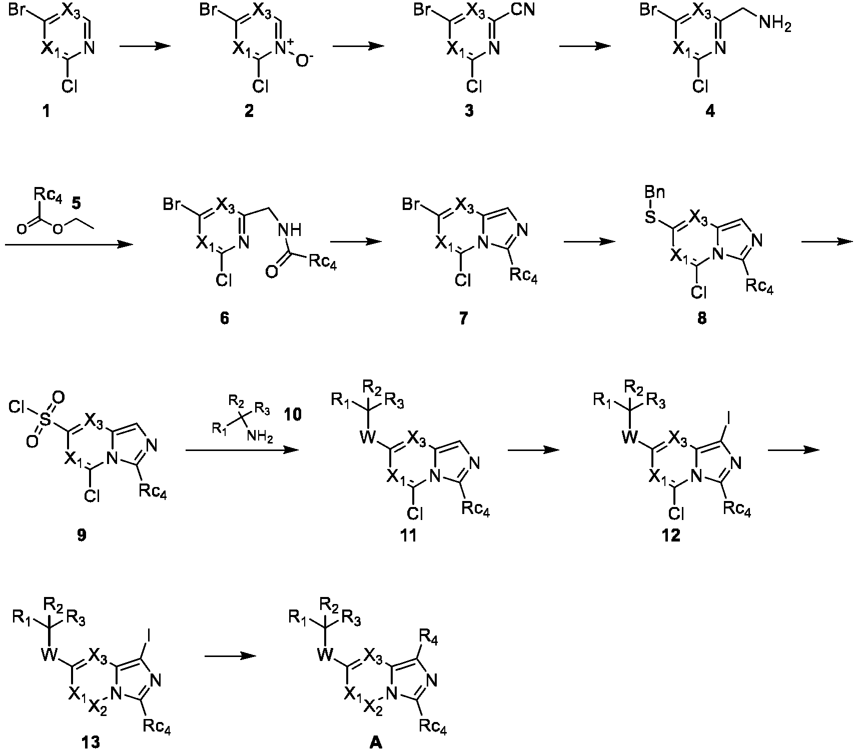

Scheme 1 illustrates a preferred synthetic approach to the compounds of the general formula A.

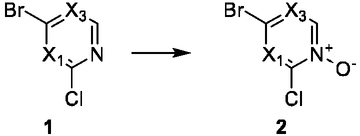

In the first step, a compound of formula **1,** in which X₁ and X₃ are as defined for the compound of formula (I) is oxidized to a compound of formula **2.** The oxidation is preferably carried out in a solvent like THF, DCM, MeCN, chloroform, tert-butyl alcohol, acetone or a mixture of AcOH and H₂O, in the presence of an oxidant like mCPBA (meta-chloroperoxybenzoic acid), urea hydrogen peroxide under neutral or acidic conditions (see for examples: Rao et al, J. Heterocyclic Chem., 2004, 41, 13). The reaction is performed at temperatures ranging from 0-50°C. The reaction is preferably run for 3-36 hours.

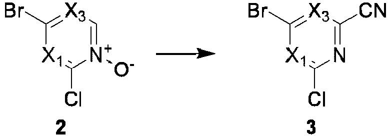

In the second step, a compound of formula **2** in which X₁ and X₃ are as defined for the compound of formula (I) is converted to compound of formula **3.** The cyanation is preferably carried out with diethyl cyanophosphonate or trimethylsilyl cyanide in a solvent like MeCN, THF, DCM or 1,2-dichloroethane. Adding N,N-dimethylcarbamoyl chloride can improve the yield. (see for example: Clements et al, US2009/239876). The reaction is performed at temperatures ranging from room temperature to the boiling point. The reaction can be performed at temperatures above the boiling point using pressure tubes and a microwave oven. The reaction is preferably completed after 1-225 hours.

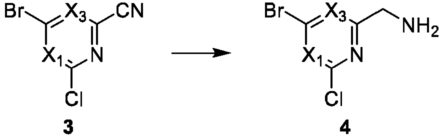

In the third step, a compound of formula **3** in which X₁ and X₃ are as defined for the compound of formula (I) is reduced to give a compound of formula **4.** The reaction is preferably carried out in THF in the presence of a reducing agent like BH₃.THF, BH₃.Me₂S, PtO₂/H₂, sodium tetrahydroborate ect., (see for example: Long et al, WO2018/71535). The reaction is performed at temperatures ranging from room temperature to 40°C. The reaction is preferably completed after 0.5-24 hours.

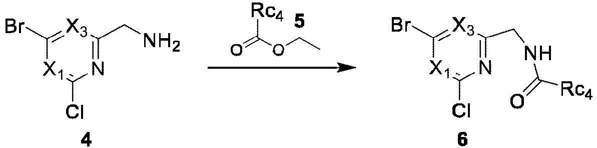

In the fourth step, a compound of formula **4** in which X₁ and X₃ are as defined for the compound of formula (I) is reacted with a compound **5** in which R_{C4} is as defined for the compound of formula (I) to give a compound of formula **6.** The formylation can alternatively be carried out by condensation with formic acid, or acylation with formyl chloride, corresponding anhydride or ester, in a solvent like DCM, dioxane or THF, in the presence of a base like trimethylamine, N-ethyl-N-isopropylpropan-2-amine (see for example: Foote et al, WO2009/56886). The reaction is performed at temperatures ranging from -10°C to the boiling point of the respective solvent. The reaction is preferably completed after 0.5-48 hours.

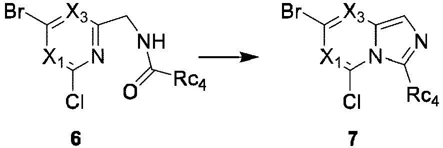

In the fifth step, a compound of formula **6** in which X₁, X₃ and R_{C4} are as defined for the compound of formula (I) is convert to a compound of formula **7** under acidic conditions. The cyclization is preferably carried out in a solvent like toluene, DCM, 1,2-dichloro-ethane or THF in the presence of trichlorophosphate or trifluoroacetic anhydride (see for example: Brown et al, WO2017/007700). The reaction is performed at temperatures ranging from room temperature to 120°C. The reaction is preferably completed after 1-24 hours.

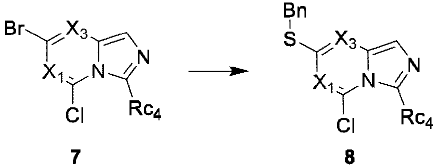

In the sixth step, a compound of formula **7** in which X₁, X₃ and R_{C4} are as defined for the compound of formula (I) is reacted with benzyl mercaptan to give a compound of formula **8.** The coupling reaction can be carried out by a palladium catalyzed C-S cross-coupling reaction (see for example: Jiang, Buchwald in 'Metal-Catalyzed Cross-Coupling Reactions', 2nd edition.: de Meijere, Diederich, Eds.: Wiley-VCH: Weinheim, Germany, 2004). Preferred is the herein described use of tris(dibenzylideneacetone) dipalladium(0), (9,9-dimethyl-9H-xanthene-4,5-diyl)bis(diphenylphosphane) and N-ethyl-N-isopropylpropan-2-amine in dioxane. The reactions are preferably run under an atmosphere of argon for 1-48 hours at 80-100°C in a microwave oven or in an oil bath.

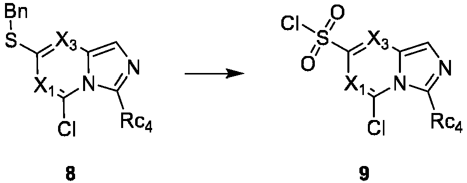

In the seventh step, a compound of formula 8 in which X₁, X₃ and R_{C4} are as defined for the compound of formula (I) is reacted with chlorination reagent to give a sulfonyl chloride of formula 9. This sulfonyl chloride formation can be carried out by treating with NCS, sulfonyl chloride, DCDMH, Cl₂ etc., in MeCN with equivalent acetic acid and water. (see for example: Sutton et al, WO 2021/055744). Preferred is the herein described use of DCDMH in MeCN with acetic acid and water. The reactions are preferably run under an atmosphere of argon for 0.5-5 hours at 0°C to room temperature.

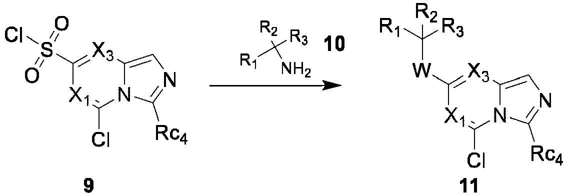

In the eighth step, a compound of formula 9 in which X₁, X₃ and R_{C4} are as defined for the compound of formula (I) is reacted with an amine of formula 10 in which R₁, R₂ and R₃ are as defined for the compound of formula (I) give a compound of formula 11. This reaction can be carried out under basic condition (see for example: Sutton et al, WO 2021/055744). Preferred is the herein described use of trimethylamine, pyridine etc., in DCM, THF or DMF. The reactions are preferably run under an atmosphere of argon for 0.5-24 hours at 0°C to room temperature.

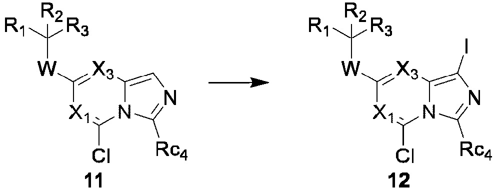

In the ninth step, a compound of formula 11 in which X₁, X₃, R_{C4}, W, R₁, R₂, and R₃ are as defined for the compound of formula (I) is converted to a compound of formula 12. This iodization can be carried out by treatment with NIS, I₂ etc., in MeCN, THF, dioxane, DMF etc. (see for example: Bentley et al, WO2011/138266). Preferred is the herein described use of NIS in MeCN. The reactions are preferably run under an atmosphere of argon for 0.5-5 hours at 0°C to room temperature. The iodine can also be replaced by chlorine or bromine which also could be used for following chemistry.

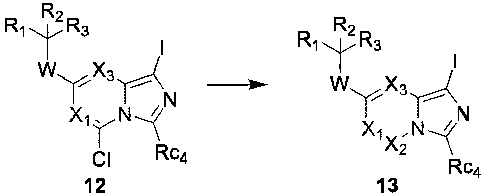

In the tenth step, a compound of formula 12 in which X₁, X₃, R_{C4}, W, R₁, R₂, and R₃ are as defined for the compound of formula (I) is reacted with various amines to give a compound of formula 13, in which X₂ is defined as for the compound of formula (I). This coupling reaction can be carried out under basic conditions (see for example: Brown et al, WO2017/007700). Preferred is the herein described use of trimethylamine, N-ethyl-N,N-diisopropylamine or pyridine etc. in MeCN, NMP, DMF etc. The reactions are preferably run for 1-24 hours at 70-150°C in a microwave oven or in an oil bath.

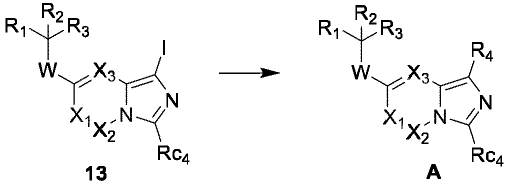

In the final step, a compound of formula **13** in which X₁, X₂, X₃, R_{C4}, W, R₁, R₂, and R₃ are as defined for the compound of formula (I) is reacted with various boronic acids or boronic esters to give a compound of formula **A.** The coupling reaction is catalyzed by palladium catalysts, e.g. by Pd(0) catalysts like tetrakis(triphenylphosphine) palladium(0) [Pd(PPh₃)₄], tris(dibenzylideneacetone) di-palladium(0) [Pd₂(dba)₃], or by Pd(ll) catalysts like dichlorobis(triphenylphosphine)-palladium(II) [Pd(PPh₃)₂C1₂], palladium(II) acetate and triphenylphosphine or by [I,I'-bis(diphenylphosphino)ferrocene]palladium dichloride. The reaction is preferably carried out in a solvent like 1,2-dimethoxyethane, dioxane, DMF, DME, THF, or isopropanol with water and in the presence of a base like potassium carbonate, sodium carbonate, sodium bicarbonate or potassium phosphate. (see for example: Hall, Boronic Acids, 2005 Wiley VCH Verlag GmbH & Co. KGaA, Weinheim, ISBN 3-527- 30991-8 and references cited therein). The reaction is performed at temperatures ranging from room temperature to the boiling point of the respective solvent. Further on, the reaction can be performed at temperatures above the boiling point using pressure tubes and a microwave oven. The reaction is preferably completed after 1 to 36 hours.

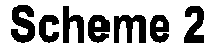

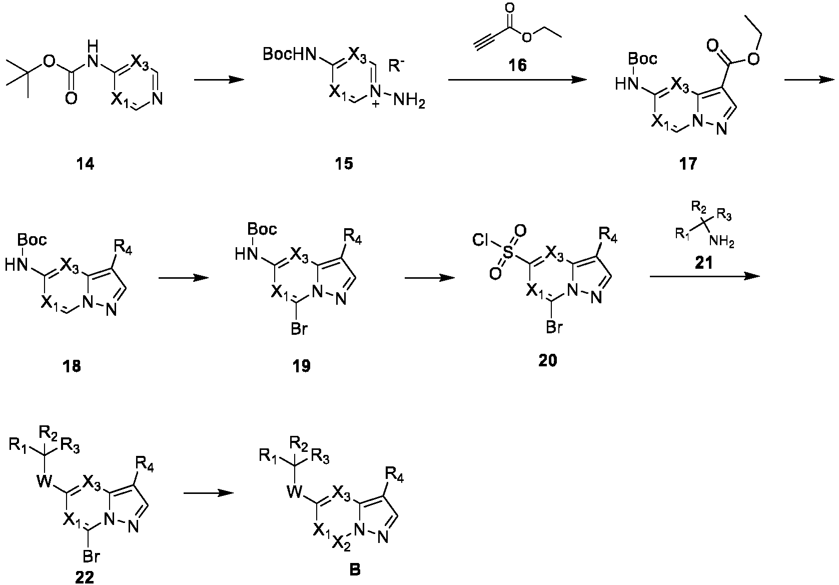

Scheme 2 illustrates a preferred synthetic approach to the compounds of the general formula **B.** As it is to be understandable to the skilled person, the scheme can also be extended to the compounds of formula (I) wherein X₄ is N and X₅ is C-R_{C5}, for example upon metalation and functionalization of X₅ is C-H position of e.g. compound B to C-Rcs (see for example: Balkenhohl et al; Org. Lett., 2018, 20, 3114).

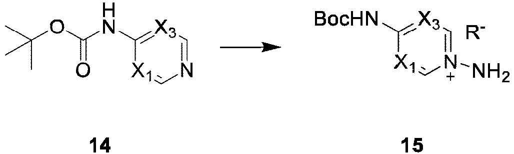

In the first step a compound of formula 14 in which X₁ and X₃ are as defined for the compound of formula (I) is converted to a compound of formula 15, in which the anion R- comes from the corresponding amination reagent. The pyridine amination can be carried out with an amination reagent, such as (2,4-dinitro-phenyl)-hydrazine , mesitylenesulfonyl hydroxylamine etc. in a solvent like THF, DCM, MeCN etc. (see for example: Hu et al, US2020/369676). The reaction is performed at temperatures ranging from 0-80°C. The reaction is preferably completed after 3 to 36 hours.

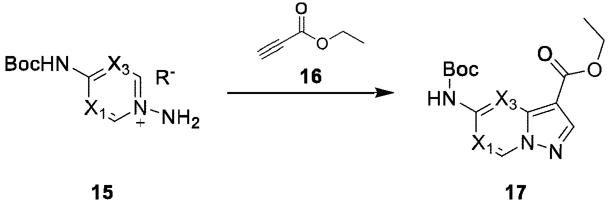

In the second step, a compound of formula **15** in which X₁ and X₃ are as defined for the compound of formula (I) is reacted with ethyl propiolate **16** to give a compound of formula 17. The cyclization is preferably carried out in a solvent like DMF or a mixture of DCM and H₂O, in the presence of a base like potassium carbonate or sodium hydroxide (see for example: Balkenhohl et al, Org. Lett., 2018, 20, 3114). The reaction is performed at temperatures ranging from 0°C to room temperature. The reaction is preferably completed after 5 to 48 hours.

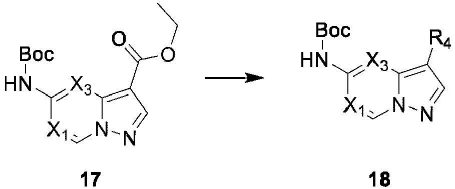

In the third step, a compound of formula **17** in which X₁ and X₃ are as defined for the compound of formula (I) is converted to a compound of formula **18** by several steps. If R₄ is 2-(difluoromethyl)-1,3,4-thiadiazole, a compound of formula **17** is reacted with hydrazine hydrate to produce a hydrazide. This hydrazide formation can be carried out under neutral conditions (see for example: Dong et al, J. Med. Chem. 2020, 63, 3028). The hydrazide formation is preferably performed in EtOH and the reactions are preferably run for 1-24 hours at 50-100°C with heating or microwave conditions. The hydrazide is then reacted with ethyl 2,2-difluoroacetate to produce a di-acyl hydrazine. This reaction can be carried out under basic conditions, preferred is the herein described use of DBU in EtOH, THF, or DMF. The reactions are preferably run for 0.5-24 hours at room temperature to 100°C in a microwave oven or in an oil bath. Finally, the di-acyl hydrazine is cyclized by treatment with oxygen/sulfur exchange reagents to a compound of formula **18,** in which R₄ is 2-(difluoromethyl)-1,3,4-thiadiazole group (see for example: Brunet et al, WO2020/127974). Preferred is the herein described use of Lawessons reagent in toluene or THF. The reactions are preferably run for 0.5-24 hours at 50-130°C.

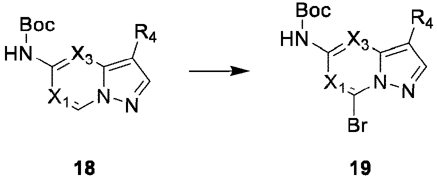

In the fourth step, a compound of formula **18** in which X₁, X₃ and R₄ are as defined for the compound of formula (I) is converted a compound of formula **19.** This reaction is preferably performed by treatment with TMPMgCl·LiCl or TMPZnCl·LiCl (see for example: Balkenhohl et al; Org. Lett., 2018, 20, 3114) first in THF at -78°C for 0.2-0.5 hours. The mixture is then treated with 1,2-dibromo-1,1,2,2-tetrachloroethane. This reaction is preferably run under an atmosphere of argon for 0.5-5 hours at 0°C to room temperature (see for example: Balkenhohl et al; Org. Lett., 2018, 20, 3114). The bromine also can be replaced by chlorine or iodine, which can also be used for following chemistry.

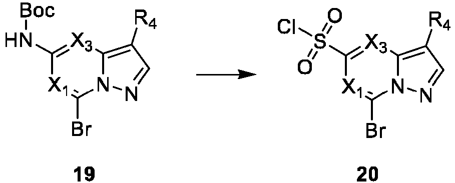

In the fifth step, a compound of formula **19** in which X₁, X₃ and R₄ are as defined for the compound of formula (I) is converted a compound of formula **20.** The BocNH₂ group is deprotected under acidic conditions, then the resulting NH₂ group is converted to sulfonyl chloride group by a Sandmeyer reaction to give a compound of formula 20. The Sandmeyer reaction is preferably performed by treatment with hydrogen chloride, acetic acid, sodium nitrite in water at -15°C to -5°C for 0.2-1 hours first. Then sulfur dioxide, acetic acid, copper (I) chloride is added. This step is preferably run for 0.08-2 hours at 0-10°C (see for example: Chatterjee et al, WO2014/78802).

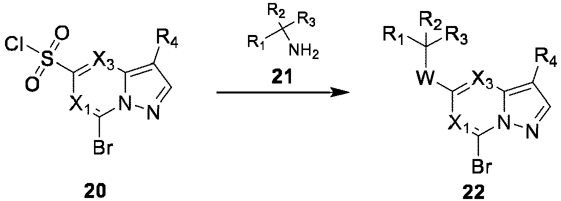

In the sixth step, a compound of formula 20 in which X₁, X₃ and R₄ are as defined for the compound of formula (I) is reacted is reacted with an amine of formula 21 in which R₁, R₂ and R₃ are as defined for the compound of formula (I) to give a compound of formula 22. This reaction can be carried out under basic condition (see for example: Sutton et al, WO 2021/055744). Preferred is the herein described use of trimethylamine, pyridine etc., in DCM, THF or DMF. The reactions are preferably run under an atmosphere of argon for 0.5-24 hours at 0°C to room temperature.

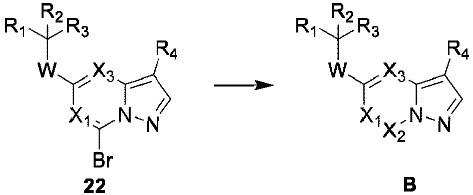

In the final step, a compound of formula **22** in which X₁, X₃, W, R₁, R₂, R₃ and R₄ are as defined for the compound of formula (I) is coupled with various amines to give a compound of formula **B,** in which X₂ is defined as for the compound of formula (I). This coupling reaction can be carried out by a palladium-catalyzed C-N cross-coupling reaction (see for example: a) Jiang, Buchwald in 'Metal-Catalyzed Cross-Coupling Reactions', 2nd edition.: de Meijere, Diederich, Eds.: Wiley-VCH: Weinheim, Germany, 2004; b) Sutton et al, WO 2021/055744). Preferred is the herein described use of cesium carbonate and Pd-PEPPSI-IHept Cl in dioxane. The reactions are preferably run under an atmosphere of argon for 1-48 hours at 80-120°C in a microwave oven or in an oil bath. Preferred is also the herein described use of cesium carbonate RuPhos-Pd-G3, Ruphos in dioxane or palladium acetate, Ruphos, tert-butyl alcohol sodium in THF. The reactions are preferably run under an atmosphere of argon for 1-24 hours at 70-130°C in a microwave oven or in an oil bath.

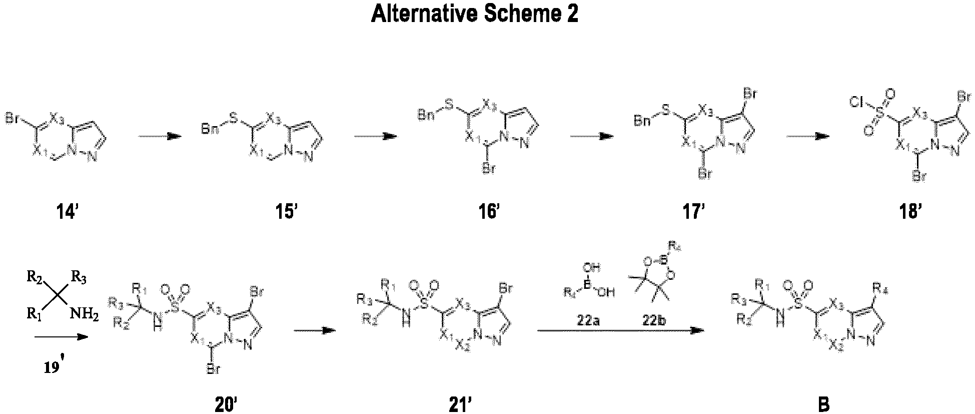

Alternative Scheme 2 illustrates an alternative preferred synthetic approach to the compounds of the general formula B. As it is to be understandable to the skilled person, the scheme can also be extended to the compounds of formula (I) wherein X₄ = N and X₅ = C-R_{C5}, for example upon metalation and functionalization of X₅ = C-H position of e.g. compound B to X₅ = C-R_{C5} (see for example: Balkenhohl et al; Org. Lett., 2018, 20, 3114).

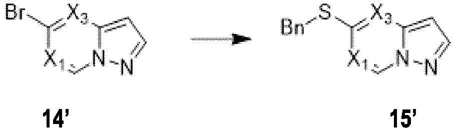

In the first step, a compound of formula 14' in which X₁ and X₃ are as defined for the compound of formula (I) is converted to a compound of formula 15'. The coupling reaction can be carried out by a palladium catalyzed C-S cross-coupling reaction (see for example: Jiang, Buchwald in 'Metal-Catalyzed Cross-Coupling Reactions', 2nd edition.: de Meijere, Diederich, Eds.: Wiley-VCH: Weinheim, Germany, 2004). Preferred is the herein described use of tris(dibenzylideneacetone) dipalladium(0), (9,9-dimethyl-9H-xanthene-4,5-diyl)bis(diphenylphosphane) and N-ethyl-Nisopropylpropan-2-amine in dioxane. The reactions are preferably run under an atmosphere of argon for 1-48 hours at 80-100°C in a microwave oven or in an oil bath.

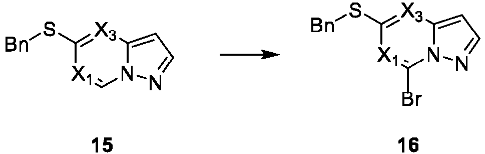

In the second step, a compound of formula 15' in which X₁ and X₃ are as defined for the compound of formula (I) is converted a compound of formula 16'. This reaction is preferably performed by treatment with TMPMgCl·LiCl or TMPZnCl·LiCl (see for example: Balkenhohl et al; Org. Lett., 2018, 20, 3114) in THF at -78°C for 0.2-0.5 hours, followed by treatment with 1,2-dibromo-1,1,2,2-tetrachloroethane. This reaction is preferably run under an atmosphere of argon for 0.5-5 hours at 0°C to room temperature (see for example: Balkenhohl et al; Org. Lett., 2018, 20, 3114). The bromine also can be replaced by chlorine or iodine, which can also be used for following chemistry.

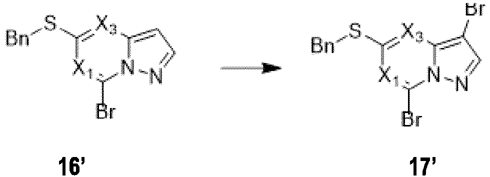

In the third step, a compound of formula 16' in which X₁ and X₃ are as defined for the compound of formula (I) is converted to a compound of formula 17 by bromination. This bromination can be carried out by treatment with NBS, Br₂ etc., in MeCN, THF, dioxane, DMF etc. (see for example: a) Bentley et al; WO2011/138266). Preferred is the herein described use of NBS in MeCN. The reactions are preferably run under an atmosphere of argon for 0.5-5 hours at 0°C to room temperature (i.e. approx. 20°C).

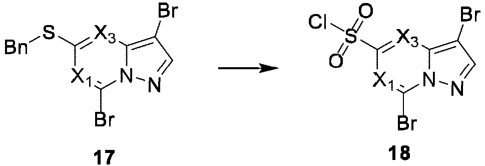

In the fourth step, a compound of formula 17' in which X₁, X₃ are as defined for the compound of formula (I) is converted a compound of formula 18'. This sulfonyl chloride formation can be carried out by treatment with NCS, sulfonyl chloride, DCDMH, Cl₂ etc., in MeCN with equivalent acetic acid and water (see for example: a) Sutton et al, WO 2021/055744). Preferred is the herein described use of NCS in acetic acid and water. The reactions are preferably run under an atmosphere of argon for 0.5-5 hours at 0°C to room temperature (i.e. approx. 20°C).

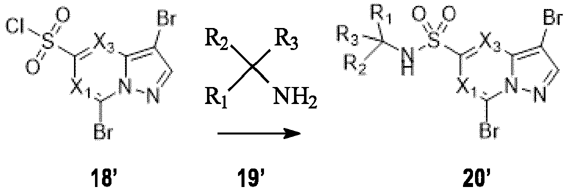

In the fifth step, a compound of formula 18' in which X₁, X₃ are as defined for the compound of formula (I) is reacted with an amine of formula 19' in which R₁, R₂ and R₃ are as defined for the compound of formula (I) to give a compound of formula 20'. This sulfonamide formation can be carried out under basic condition (see for example: a) Sutton et al, WO 2021/055744). Preferred is the herein described use of trimethylamine in DCM. The reactions are preferably run under an atmosphere of argon for 0.5-24 hours at 0°C to room temperature (i.e. approx. 20°C).

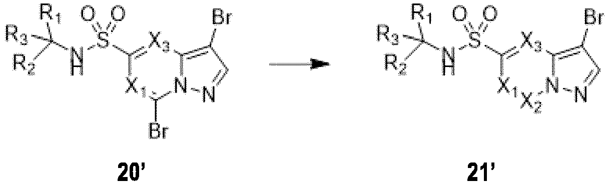

In the sixth step, a compound of formula 20' in which X₁, X₃, W, R₁, R₂ and R₃ are as defined for the compound of formula (I) is reacted with various amines to give a compound of formula 21', in which X₂ is defined as for the compound of formula (I). This S_{NR} reaction can be carried out without base or under basic condition (see for example: Schmitz et al, WO2009/023179). Preferred is the herein described use of trimethylamine, DIPEA, pyridine etc., in DMSO or DMF. The reactions are preferably run under an atmosphere of argon for 0.5-24 hours at 80°C to 150 °C.

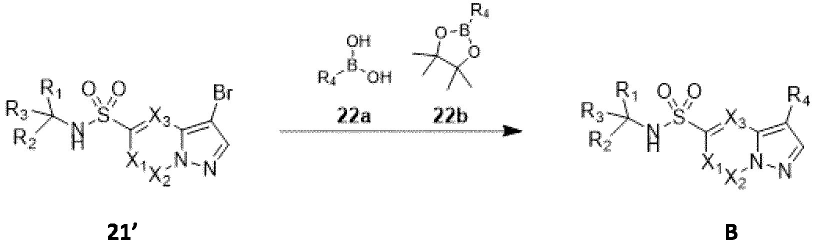

In the final step, a compound of formula 21' in which X₁,X₂, X₃, W, R₁, R₂ and R₃ are as defined for the compound of formula (I) is coupled with various amines, in which X₂ is defined as for the compound of formula (I), to give a compound of formula B. The coupling reaction is catalyzed by palladium catalysts, e.g. by Pd(0) catalysts like tetrakis(triphenylphosphine) palladium(0) [Pd(PPh₃)₄], tris(dibenzylideneacetone) di-palladium(0) [Pd₂(dba)₃], or by Pd(II) catalysts like dichlorobis(triphenylphosphine)-palladium(ll) [Pd(PPh₃)₂Cl₂], palladium(II) acetate and triphenylphosphine or by [I,I'-bis(diphenylphosphino)ferrocene]palladium dichloride. The reaction is preferably carried out in a solvent like 1,2-dimethoxyethane, dioxane, DMF, DME, THF, or isopropanol with water and in the presence of a base like potassium carbonate, sodium carbonate, sodium bicarbonate or potassium phosphate. (see for example: Hall, Boronic Acids, 2005 Wiley VCH Verlag GmbH & Co. KGaA, Weinheim, ISBN 3-527- 30991-8 and references cited therein). The reaction is performed at temperatures ranging from room temperature to the boiling point of the respective solvent. Further on, the reaction can be performed at temperatures above the boiling point using pressure tubes and a microwave oven. The reaction is preferably completed after 1 to 36 hours.

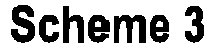

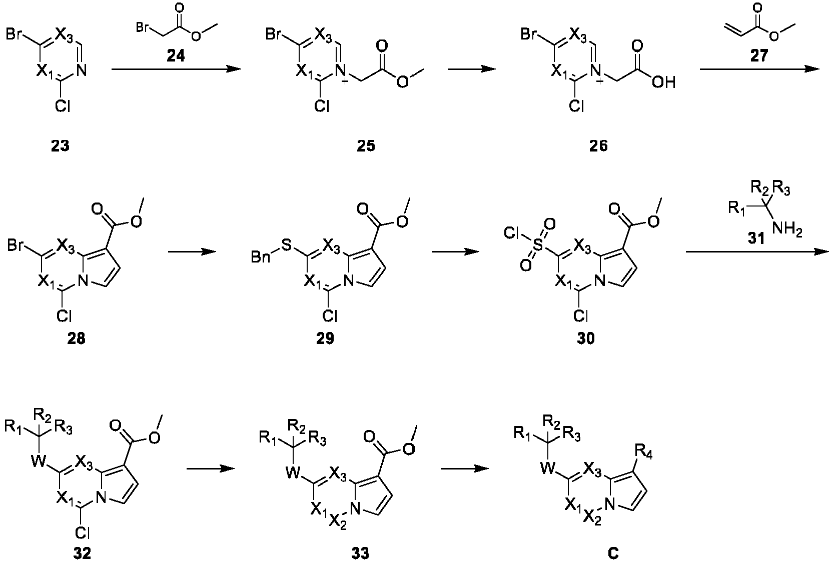

Scheme 3 illustrates a preferred synthetic approach to the compounds of the general formula C. As it is to be understandable to the skilled person, the compounds of formula (I) wherein X₄ is C-R_{C4} and Xs is CH are obtainable through functionalization of the X₄ is CH position of compounds of formula (I) wherein Xs is CH through bromination (see for example: Kim et al, KR2016041674) or iodization (see for example: Biagetti et al, US20150361100) of the X₄ is CH position followed by palladium-catalyzed cross-coupling reactions. As it is to be understandable to the skilled person, the compounds of formula (I) wherein Xs is C-R_{C5} and X₄ is CH are obtainable through functionalization of the Xs is CH position of compounds of formula (I) wherein X₄ is CH through bromination (see for example: Jimenez et al, WO2015004304) of the Xs is CH position followed by palladium-catalyzed cross-coupling reactions. As it is to be understandable to the skilled person, the compounds of formula (I) wherein X₄ is C-R_{C4} and Xs is C-R_{C4} are obtainable through functionalization of the Xs is CH position of compounds of formula (I) wherein X₄ is C-R_{C4} through bromination (see for example: Yao et al, Org. Lett. 2020, 22, 4511) of the X₅ is C-H position followed by palladium-catalyzed cross-coupling reactions.

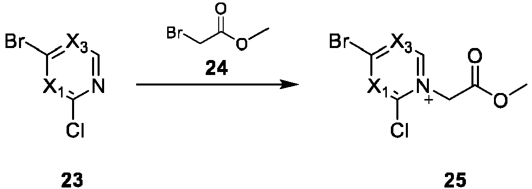

In the first step, a compound of formula **23** in which X₁ and X₃ are as defined for the compound of formula (I) is reacted with methyl 2-bromoacetate **24** to give a compound of formula 25. The reaction is preferably carried out without solvent or in a solvent like MeCN, EtOH, toluene, butyl alcohol, preferably in the presence of sodium iodide. (see for example: Khoroshilov et al, Tetrahedron, 2013, 69, 4353). The reaction is performed at temperatures ranging from 50-170°C. The reaction is preferably completed after 0.5 to 36 hours.

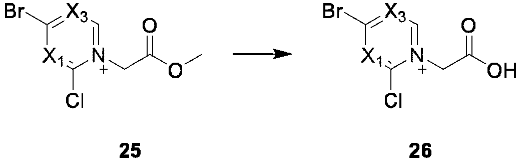

In the second step, a compound of formula **25** in which X₁ and X₃ are as defined for the compound of formula (I) is converted to a compound of formula 26. The reaction is preferably carried out in pyridine in the presence of lithium iodide. (see for example: Liang et al, US2010/216806). The reaction is performed at temperatures ranging from 80-150°C. The reaction is preferably completed after 5 to 24 hours.

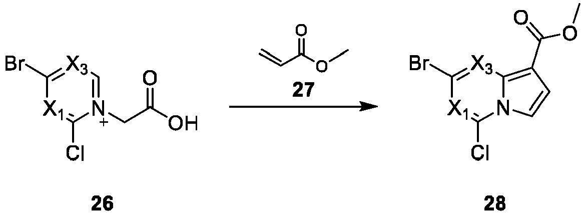

In the third step, a compound of formula 26 in which X₁ and X₃ are as defined for the compound of formula (I) is reacted with methyl acrylate 27 to give a compound of formula 28. The reaction is preferably carried out without solvent or in a solvent like toluene, 1,2-dichloroethane, preferably in the presence of manganese(IV) oxide and triethylamine. (see for example: Le Diguarher et al, US2015/31648). The reaction is performed at temperatures ranging from 80-140°C. The reaction is preferably completed after 8-36 hours.

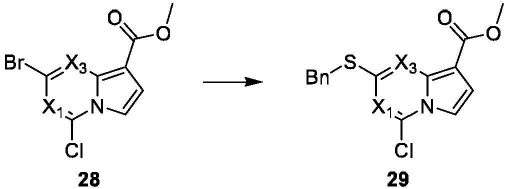

In the fourth step, a compound of formula **28** in which X₁ and X₃ are as defined for the compound of formula (I) is reacted with benzyl mercaptan to give a compound of formula **29.** The coupling reaction can be carried out by a palladium catalyzed C-S cross-coupling reaction (see for example: Jiang, Buchwald in 'Metal-Catalyzed Cross-Coupling Reactions', 2nd edition.: de Meijere, Diederich, Eds.: Wiley-VCH: Weinheim, Germany, 2004). Preferred is the herein described use of tris(dibenzylideneacetone) dipalladium(0), (9,9-dimethyl-9H-xanthene-4,5-diyl)bis(diphenylphosphane) and N-ethyl-N-isopropylpropan-2-amine in dioxane. The reactions are preferably run under an atmosphere of argon for 1-48 hours at 80-100°C in a microwave oven or in an oil bath.

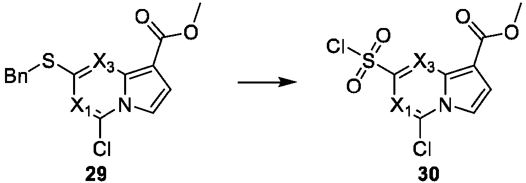

In the fifth step, a compound of formula 29 in which X₁ and X₃ are as defined for the compound of formula (I) is reacted with chlorination reagent to give a sulfonyl chloride of formula 30. This sulfonyl chloride formation can be carried out by treating with NCS, sulfonyl chloride, DCDMH, Cl₂ etc., in MeCN with equivalent acetic acid and water. (see for example: Sutton et al, WO 2021/055744). Preferred is the herein described use of DCDMH in MeCN with equivalent acetic acid and water. The reactions are preferably run under an atmosphere of argon for 0.5-5 hours at 0°C to room temperature.

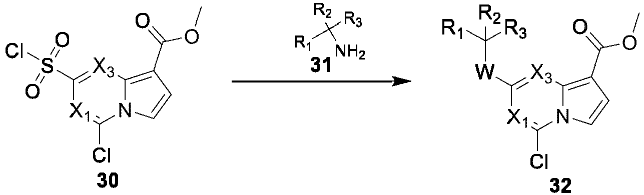

In the sixth step, a compound of formula 30 in which X₁ and X₃ are as defined for the compound of formula (I) is reacted with an amine of formula 31 in which R₁, R₂ and R₃ are as defined for the compound of formula (I) give a compound of formula 32. This reaction can be carried out under basic condition (see for example: Sutton et al, WO 2021/055744). Preferred is the herein described use of trimethylamine, pyridine etc., in DCM, THF or DMF. The reactions are preferably run under an atmosphere of argon for 0.5-24 hours at 0°C to room temperature.

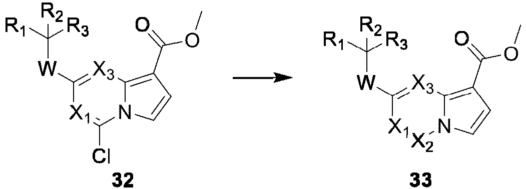

In the seventh step, a compound of formula **32** in which X₁, X₃, W, R₁, R₂ and R₃ are as defined for the compound of formula (I) is coupled with various amines to give a compound of formula **33,** in which X₂ is defined as for the compound of formula (I). This coupling reaction can be carried out by a palladium-catalyzed C-N cross-coupling reaction (see for example: a) Jiang, Buchwald in 'Metal-Catalyzed Cross-Coupling Reactions', 2nd edition.: de Meijere, Diederich, Eds.: Wiley-VCH: Weinheim, Germany, 2004; b) Sutton et al, WO 2021/055744). Preferred is the herein described use of cesium carbonate and Pd-PEPPSI-IHept Cl in dioxane. The reactions are preferably run under an atmosphere of argon for 1-48 hours at 80-120°C in a microwave oven or in an oil bath. Preferred is also the herein described use of cesium carbonate RuPhos-Pd-G3, Ruphos in dioxane or palladium acetate, Ruphos, tert-butyl alcohol sodium in THF. The reactions are preferably run under an atmosphere of argon for 1-24 hours at 70-130°C in a microwave oven or in an oil bath.

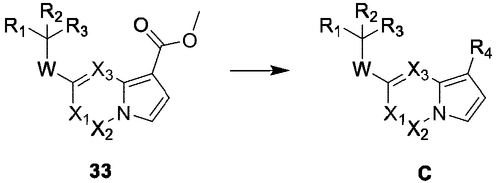

In the final step, a compound of formula 33 in which X₁, X₂, X₃, W, R₁, R₂, R₃ and R₄ are as defined for the compound of formula (I) is converted to a compound of formula C by several steps. If R₄ is 2-(difluoromethyl)-1,3,4-thiadiazole, a compound of formula 33 is reacted with hydrazine hydrate to produce a hydrazide. This hydrazide formation can be carried out under neutral conditions (see for example: Dong et al, J. Med. Chem. 2020, 63, 3028). The hydrazide formation is preferably performed in EtOH and the reactions are preferably run for 1-24 hours at 50-100°C with heating or microwave conditions. The hydrazide is then reacted with ethyl 2,2-difluoroacetate to produce a di-acyl hydrazine. This reaction can be carried out under basic conditions, preferred is the herein described use of DBU in EtOH, THF, or DMF. The reactions are preferably run for 0.5-24 hours at room temperature to 100°C in a microwave oven or in an oil bath. Finally, the di-acyl hydrazine is cyclized by treatment with oxygen/sulfur exchange reagents to a compound of formula **C,** in which R₄ is 2-(difluoromethyl)-1,3,4-thiadiazole group (see for example: Brunet et al, WO2020/127974). Preferred is the herein described use of Lawessons reagent in toluene or THF. The reactions are preferably run for 0.5-24 hours at 50-130°C.

### Preparative examples

### General considerations

Abbreviations used in the descriptions that follow are: AcOH (acetic acid); br. (broad, ¹H NMR signal); t-BuOH (Tert-butyl alcohol); cataCXium A-Pd-G3 (Mesylate[(di(1-adamantyl)-n-butylphosphine)-2-(2'-amino-1,1'-biphenyl)]palladium(II); [(Di(1-adamantyl)-butylphosphine)-2-(2'-amino-1,1'-biphenyl)]palladium(II) methanesulfonate); CDCl₃ (deuterated chloroform); cHex (cyclohexane); Cs₂CO₃ (cesium carbonate); DCE (dichloroethane); d (doublet, ¹H NMR signal); DCM (dichloromethane); DIPEA (di-iso-propylethylamine); DMAP (4- N-N-dimethylaminopyridine), DME (1,2-dimethoxyethane), DMF (N-N-dimethylformamide); DMSO (dimethyl sulfoxide); ES (electrospray); EtOAc (ethyl acetate); EtOH (ethanol); h (hour(s)); ¹H NMR (proton nuclear magnetic resonance spectroscopy); H₂O (water); HPLC (High Performance Liquid Chromatography), iPrOH (iso-propanol); K₂CO₃ (potassium carbonate); m (multiplet, ¹H NMR signal); K₃PO₄ (tripotassium phosphate); mCPBA (meta-chloroperoxybenzoic acid), MeCN (acetonitrile), MeOH (methanol); min (minute(s)); MS (mass spectrometry); MTBE (methyl *tert-*butyl ether); NaHCO₃ (sodium hydrogenocarbonate); NCS (N-chlorosuccinimide); NIS (N-lodosuccinimide); NMR (nuclear magnetic resonance); Pd/C (palladium on charcoal); Pd-PEPPSI-IPent Cl (Dichloro[1,3-bis(2,6-Di-3-pentylphenyl)imidazol-2-ylidene](3-chloropyridyl)palladium(II)); q (quartet, ¹H NMR signal); quin (quintet, 1H NMR signal); rac (racemic); RT (retention time); s (singlet, ¹H NMR signal); t (triplet, ¹H NMR signal); TEA (triethylamine); TFA (trifluoroacetic acid); TFAA (trifluoroacetic anhydride), THF (tetrahydrofuran); UPLC (Ultra-High Performance Liquid Chromatography), UV (ultraviolet), wt-% (percent by weight).

General Procedure: All starting materials and solvents were obtained either from commercial sources or prepared according to literature references. Commercially available reagents and anhydrous solvents were used as supplied, without further purification. Unless otherwise stated all reactions were stirred. Organic solutions were routinely dried over anhydrous sodium sulfate. Column chromatography was performed on pre-packed silica (100-1000 mesh, 40-63 µm) cartridges using the amount indicated. All air- and moisture-sensitive reactions were carried out in oven-dried (at 120 °C) glassware under an inert atmosphere of nitrogen or argon. Compound names were generated using ChemDraw Prime (Perkin Elmer). In some cases generally accepted names of commercially available reagents were used in place of ChemDraw generated names.

### Reversed Phase HPLC conditions for LCMS Analysis of final compounds:

Method 1: SHIMADZU LCMS-2020 Kinetex EVO C18 2.1X30mm,5um at 50°C; Mobile Phase: A: 0.0375% TFA in water (v/v); B: 0.01875% TFA in MeCN (v/v); flow rate held at 1.5 mL/min; eluted with the mobile phase over 1.55 min employing UV detection at 220 nm and 254 nm. Gradient information: 0-0.80 min, ramped from 95% A-5% B to 5% A-95% B; 0.80-1.20 min, held at 5% A-95% B; 1.20-1.21 min, returned to 95% A-5% B, 1.21-1.55 min, held at 95% A-5% B.
Method 2: SHIMADZU LCMS-2020 Kinetex EVO C18 2.1X30mm,5um at 40°C ; Mobile Phase : A: 0.025% NH₃·H₂O in water (v/v) , B: MeCN; flow rate held at 1.5 mL/min; eluted with the mobile phase over 1.55 min employing UV detection at 220 nm and 254 nm. Gradient information: 0-0.80 min, ramped from 95% A-5% B to 5% A-95% B; 0.80-1.20 min, held at 5% A-95% B; 1.20-1.21 min, returned to 95% A-5% B, 1.21-1.55 min, held at 95% A-5% B.
Method 3: SHIMADZU LCMS-2020 Kinetex^{®} EVO C18 2.1X30 mm 5 um at 50°C; Mobile Phase: A: 0.0375% TFA in water (v/v); B: 0.01875% TFA in MeCN (v/v); flow rate held at 1.5 mL/min; eluted with the mobile phase over 1.00 min employing UV detection at 220 nm and 254 nm. Gradient information: 0.01-0.80 min, ramped from 95% A-5% B to 5% A-95% B; 0.80-0.95 min, held at 5% A-95% B; 0.95-0.96 min, returned to 95% A-5% B, 0.96-1.00 min, held at 95% A-5% B.
Method 4: SHIMADZU LCMS-2020 Kinetex^{®} EVO C18 2.1X20 mm 2.6 um at 50°C; Mobile Phase: A: 0.0375% TFA in water (v/v); B: 0.01875% TFA in MeCN (v/v); flow rate held at 2.0 mL/min; eluted with the mobile phase over 1.00 min employing UV detection at 220 nm and 254 nm. Gradient information: 0.01-0.60 min, ramped from 95% A-5% B to 5% A-95% B; 0.61-0.78 min, held at 5% A-95% B; 0.78-0.79 min, returned to 95% A-5% B, 0.79-0.80 min, held at 95% A-5% B.

### ¹H NMR Spectroscopy:

¹H NMR spectra were acquired on a Bruker Avance III spectrometer at 400 MHz using residual undeuterated solvent as reference. ¹H NMR signals are specified with their multiplicity / combined multiplicities as apparent from the spectrum; possible higher-order effects are not considered. Chemical shifts of the signals (δ) are specified as ppm (parts per million).

### Salt stoichiometry:

In the present text, in particular in the experimental section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown. Unless specified otherwise, suffixes to chemical names or structural formulae such as "hydrochloride", "trifluoroacetate", "sodium salt", or "x HO", "x CF3COOH", "x Na+", for example, are to be understood as not a stoichiometric specification, but solely as a salt form. This applies analogously to cases in which synthesis intermediates or example compounds or salts thereof have been obtained, by the preparation and/or purification processes described, as solvates, such as hydrates with (if defined) unknown stoichiometric composition.

### Preparation of Intermediate 1.1

### 4-bromo-2-chloropyridine 1-oxide

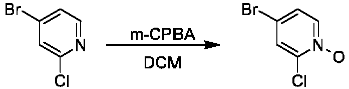

To a solution of 4-bromo-2-chloropyridine (80.0 g, 415.72 mmol) in DCM (600 mL) at 0 °C was added m-CPBA (126.60 g, 623.56 mmol, 85% purity) slowly before the mixture was stirred at 35 °C for 16 h. The reaction mixture was poured into ice water (1000 mL) and the pH was adjusted to 9 by adding Na₂CO₃ (aq., sat., 500 mL). The resulting mixture was extracted with DCM (500 mL, 3x), the organic phases were separated, dried over anhydrous Na₂SO₄, and concentrated in vacuum to give a residue. The residue was purified by silica gel column (DCM/EtOAc=1/0 to 3/1) to give the product 4-bromo-2-chloropyridine 1-oxide (46.0 g, 220.69 mmol, 53.09% yield) as a yellow solid.

RT 0.251 min (method 2); m/z 209.8 (M+H)⁺ (ESI⁺) **¹H NMR** (400 MHz, DMSO-*d₆*): 8.35 (d, *J = 7.2* Hz, 1H), 8.17 (d, *J* = 2.8 Hz, 1H), 7.65 (dd, J = 2.8, 7.2 Hz, 1H).

### Preparation of Intermediate 1.2

### 4-bromo-6-chloropicolinonitrile

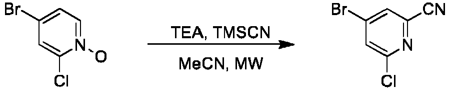

9 batches were run in parallel and combined for work-up: To a solution of 4-bromo-2-chloropyridine 1-oxide (1.5 g, 7.20 mmol) in MeCN (8 mL) was added TMSCN (2.50 g, 25.19 mmol) and TEA (2.18 g, 21.59 mmol) before the mixture was stirred at 100 °C for 4 h in the microwave. The 9 batches were combined, and the mixture was concentrated in vacuum to give a brown oil. The brown oil was purified by silica gel column (Petroleum ether/EtOAc=20/1 to 1/1) to give the product 4-bromo-6-chloropicolinonitrile (7.8 g, 35.87 mmol, 55.35% yield) as a yellow solid.

RT 0.808 min (method 1); m/z 216.9 (M+H)⁺ (ESI⁺) **¹H** NMR (400 MHz, DMSO-*d₆*): 8.51 (d, *J =* 1.6 Hz, 1H), 8.35 (d, *J* = 1.6 Hz, 1H).

### Preparation of Intermediate 1.3

### (4-bromo-6-chloropyridin-2-yl)methanamine

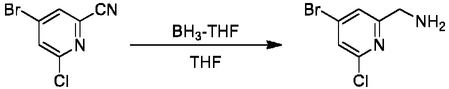

To a solution of 4-bromo-6-chloropicolinonitrile (7.8 g, 35.87 mmol) in THF (40 mL) at 0 °C was slowly added BH₃-THF (1 M, 89.68 mL). The mixture was slowly warmed to 20 °C and stirred for 16 h at this temperature. The reaction was quenched by adding MeOH (40 mL) and an aqueous solution of HCl (2M, 20 mL) before stirring for 0.5 h. The mixture was concentrated in vacuum to give the crude product (4-bromo-6-chloropyridin-2-yl)methanamine (15 g, crude) as a yellow oil, which was used in the next step without further purification.

RT 0.311 min (method 1); m/z 221.0 (M+H)⁺ (ESI⁺) **¹H NMR** (400 MHz, DMSO-*d₆*): 8.60-8.50 (m, 2H), 7.95 (s, 1H), 7.90 (s, 1H), 4.22-4.15 (m, 2H).

### Preparation of Intermediate 1.4

### Tert-butyl ((4-bromo-6-chloropyridin-2-yl)methyl)carbamate

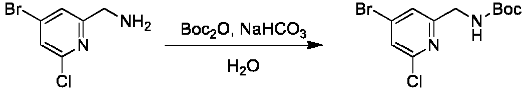

To a solution of (4-bromo-6-chloropyridin-2-yl)methanamine (15.0 g, 67.73 mmol) in H₂O (100 mL) was added NaHCO₃ (28.45 g, 338.63 mmol) and Boc₂O (29.56 g, 135.45 mmol) before the reaction mixture was stirred at 20 °C for 16 h. The resulting mixture was poured into water (100 mL) and extracted with EtOAc (100 mL, 2x). The combined organic layers were washed with brine (100 mL), dried over anhydrous Na₂SO₄, filtered and concentrated to give a residue. The residue was purified by silica gel column (Petroleum ether/EtOAc= 3/1 to 1/1) to give the product tert-butyl ((4-bromo-6-chloropyridin-2-yl)methyl)carbamate (4.8 g, 14.93 mmol, 22.04% yield) as a yellow solid.

**RT** 0.311 min **(method** 1); **m/z** 265.0 (M-56+H)⁺ (ESI⁺) **¹H NMR** (DMSO-*d*₆, 400 MHz): 7.79 (s, 1H), 7.51 (t, *J* = 6.0 Hz, 1H), 7.47 (s, 1H), 4.18 (d, *J* = 6.0 Hz, 2H), 1.40 (s, 9H).

### Preparation of Intermediate 1.5

### (4-bromo-6-chloropyridin-2-yl)methanamine

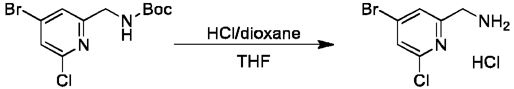

To a solution of tert-butyl ((4-bromo-6-chloropyridin-2-yl)methyl)carbamate (4.8 g, 14.93 mmol,) in THF (15 mL) was added a solution of HCl in dioxane (4 M, 37.31 mL) before the mixture was stirred at 25 °C for 2 h. The mixture was concentrated to give the product (4-bromo-6-chloropyridin-2-yl)methanamine (4.1 g, crude, HCl salt) as a yellow solid.

**RT** 0.300 min **(method 1);** m/z 221.0 (M+H)⁺ (ESI⁺) **¹H NMR** (DMSO-*d*₆, 400 MHz): 8.68-8.59 (br, 3H), 7.95 (s, 1H), 7.92 (s, 1H), 4.22-4.12 (m, 2H).

### Preparation of Intermediate 1.6

### N-((4-bromo-6-chloropyridin-2-yl)methyl)formamide

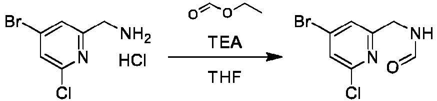

To a mixture of (4-bromo-6-chloropyridin-2-yl)methanamine (4.1 g, 13.99 mmol, HCl salt) and TEA (4.25 g, 41.96 mmol) in THF (10 mL) was added ethyl formate (20.72 g, 279.75 mmol) before the mixture was stirred at 30 °C for 16 h. The mixture was concentrated under reduced pressure to give a residue. The residue was purified by silica gel chromatography (petroleum ether/EtOAc=0/1) to give the product N-((4-bromo-6-chloropyridin-2-yl)methyl)formamide (1.9 g, 7.62 mmol, 54.44% yield) as a white solid.

**RT** 0.667 min **(method** 1); **m/z** 249.0 (M+H)⁺ (ESI⁺) **¹H NMR** (DMSO-*d*₆, 400 MHz): 8.66-8.59 (m, 1H), 8.17 (s, 1H), 7.81 (d, *J =* 1.6 Hz, 1H), 7.57 (d, *J* = 1.6 Hz, 1H), 4.39-4.35 (m, 2H).

### Preparation of Intermediate 1.7

### 7-bromo-5-chloroimidazo[1,5-a]pyridine

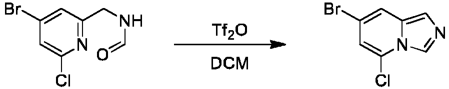

To a solution of N-((4-bromo-6-chloropyridin-2-yl)methyl)formamide (1.9 g, 7.62 mmol) in DCM (15 mL) was added Tf₂O (3.22 g, 11.42 mmol) at 0 °C before the mixture was stirred at 30 °C for 4 h. The mixture was added into ice and an aqueous, saturated solution of NaHCO₃ (100 mL) dropwise and extracted with DCM (60 mL, 3x). The organic layers were dried (Na₂SO₄), filtered and concentrated to give the product 7-bromo-5-chloroimidazo[1,5-a]pyridine (2 g, crude) as a yellow solid.

**RT** 0.642 min **(method 1); m/z** 230.9 (M+H)⁺ (ESI⁺) **¹H NMR** (DMSO-*d*₆, 400 MHz): 9.27 (s, 1H), 8.16 (s, 1H), 7.91 (s, 1H), 7.50 (s, 1H).

### Preparation of Intermediate 1.8

### 7-(benzylthio)-5-chloroimidazo[1,5-a]pyridine

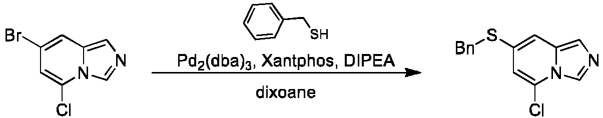

To a mixture of 7-bromo-5-chloroimidazo[1,5-a]pyridine (2.0 g, 8.64 mmol), Xantphos (499.94 mg, 864.02 µmol) and DIEA (2.23 g, 17.28 mmol) in dioxane (20 mL) was added phenylmethanethiol (1.61 g, 12.96 mmol) and Pd₂(dba)₃ (395.60 mg, 432.01 µmol) under N₂ before the mixture was stirred at 50 °C for 2 h. The reaction mixture was poured into water (200 mL) and extracted with EtOAc (100 mL, 2x). The combined organic layers were washed with brine (100 mL), dried (Na₂SO₄), filtered and concentrated to give a residue. The residue was purified by silica gel column (Petroleum ether /EtOAc=3/1 to 1/1) to give the product 7-(benzylthio)-5-chloroimidazo[1,5-a]pyridine (1.2 g, 4.37 mmol, 50.55% yield) as a yellow oil.

**RT** 0.644 min **(method 3); m/z** 274.9 (M+H)⁺ (ESI⁺) **¹H NMR** (DMSO-*d*₆, 400 MHz): 8.41 (s, 1H), 7.53 (s, 1H), 7.42 (s, 1H), 7.39-7.35 (m, 2H), 7.30 (t, *J* = 7.6 Hz, 2H), 7.26-7.20 (m, 1H), 7.00 (d, *J* = 1.2 Hz, 1H), 4.30 (s, 2H).

### Preparation of Intermediate 1.9

### 5-chloroimidazo[1,5-a]pyridine-7-sulfonyl chloride

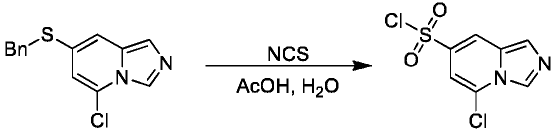

To a solution of 7-(benzylthio)-5-chloroimidazo[1,5-a]pyridine (300 mg, 1.09 mmol) in AcOH (9 mL) and H₂O (3 mL) was added NCS (437.39 mg, 3.28 mmol) at 0 °C before the mixture was stirred at 30 °C for 3 h. The reaction mixture was poured into water (100 mL) slowly and extracted with DCM (60 mL, 3x). The organic layers were dried (Na₂SO₄), filtered and concentrated to give crude product 5-chloroimidazo[1,5-a]pyridine-7-sulfonyl chloride (250 mg, crude) as a yellow oil which was used for next step without further purification.

**RT** 0.520 min **(method 3); m/z** 250.9 (M+H)⁺ (ESI⁺).

### Preparation of Intermediate 1.10

### 5-chloro-N-(1-methylcyclopropyl)imidazo[1,5-a]pyridine-7-sulfonamide

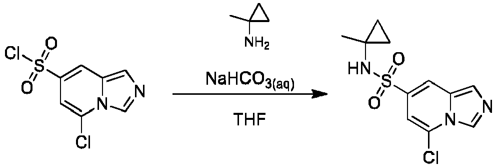

To a solution of 5-chloroimidazo[1,5-a]pyridine-7-sulfonyl chloride (250 mg, 995.66 µmol) and 1-methylcyclopropanamine (107 mg, 995.66 µmol) in THF (6 mL) was added an aqueous, saturated solution of NaHCO₃ (995.66 µmol, 3 mL) at 0 °C before the mixture was stirred at 0 °C for 0.5 h. The mixture was added into water (50 mL) and extracted with DCM (30 mL, 3x). The organic layers were dried (Na₂SO₄), filtered and concentrated to give a residue. The residue was purified by preparative TLC (Petroleum ether/EtOAc= 3/1) to give the product 5-chloro-N-(1-methylcyclopropyl)imidazo[1,5-a]pyridine-7-sulfonamide (110 mg, 384.95 µmol, 38.66% yield) as a white solid.

**RT** 0.459 min (method 3); m/z 285.9 (M+H)⁺ (ESI⁺) **¹H NMR** (DMSO-*d*₆, 400 MHz): 8.70 (s, 1H), 8.23-8.19 (m, 2H), 7.92 (s, 1H), 7.08 (s, 1H), 1.15 (s, 3H), 0.78-0.65 (m, 2H), 0.49-0.38 (m, 2H).

### Preparation of Intermediate 1.11

### 5-chloro-1-iodo-N-(1-methylcyclopropyl)imidazo[1,5-a]pyridine-7-sulfonamide

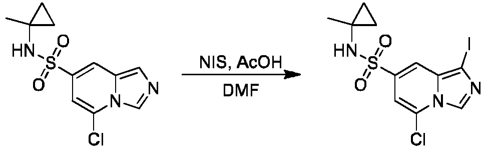

To a solution of 5-chloro-N-(1-methylcyclopropyl)imidazo[1,5-a]pyridine-7-sulfonamide (30 mg, 104.99 µmol) in DMF (0.3 mL) was added NIS (33.07 mg, 146.98 µmol) and AcOH (630.47 µg, 10.50 µmol) at 0 °C before reaction mixture was stirred at 20 °C for 3 h. The mixture was diluted with water (5 mL) and extracted with DCM (10 mL, 3x). The organic layers were dried (Na₂SO₄), filtered and concentrated to give a residue. The residue was purified by preparative TLC (Petroleum ether/EtOAc= 1/1) to give the product 5-chloro-1-iodo-N-(1-methylcyclopropyl)imidazo[1,5-a]pyridine-7-sulfonamide (30 mg, 72.88 µmol, 69.42% yield) as a yellow solid.

RT 0.552 min **(method** 3); **m/z** 411.9 (M+H)⁺ (ESI⁺) **¹H NMR** (DMSO-*d*₆, 400 MHz): 8.76 (s, 1H), 8.25 (br, 1H), 7.71 (s, 1H), 7.16 (s, 1H), 1.15 (s, 3H), 0.71-0.63 (m, 2H), 0.48-0.39 (m, 2H).

### Preparation of Intermediate 1.12

### 4-(1-iodo-7-(N-(1-methylcyclopropyl)sulfamoyl)imidazo[1,5-a]pyridin-5-yl)-N,N-dimethylpiperazine-1-carboxamide

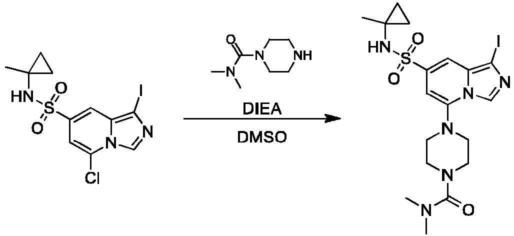

To a solution of 5-chloro-1-iodo-N-(1-methylcyclopropyl)imidazo[1,5-a]pyridine-7-sulfonamide (14.11 mg, 72.88 µmol) in DMSO (0.3 mL) was added DIEA (12.56 mg, 97.17 µmol) and 5-chloro-1-iodo-N-(1-methylcyclopropyl)imidazo[1,5-a]pyridine-7-sulfonamide (20 mg, 48.59 µmol) before the reaction mixture was stirred at 110 °C for 3 h. The mixture was diluted with water (5 mL) and extracted with DCM (3 mL, 3x). The organic layers were dried (Na₂SO₄), filtered and concentrated to give a residue. The residue was purified by preparative HPLC (column: Unisil 3-100 C18 Ultra 150*50 mm*3 µm; mobile phase: A: 0.225% formic acid in water, B: MeCN; B%: 36%- 66%, 7 min) to give the product 4-(1-iodo-7-(N-(1-methylcyclopropyl)sulfamoyl)imidazo[1,5-a]pyridin-5-yl)-N,N-dimethylpiperazine-1-carboxamide (6.82 mg, 12.43 µmol, 25.59% yield, 97.04% purity) as a yellow gum.

**RT** 0.557 min **(method** 3); **m/z** 533.1 (M+H)⁺ (ESI⁺); **¹H NMR** (DMSO-*d*₆, 400 MHz): 8.53 (s, 1H), 7.47 (s, 1H), 6.48 (s, 1H), 3.40-3.35 (m, 4H), 3.13-3.07 (m, 4H), 2.78 (s, 6H), 1.09 (s, 3H), 0.69-0.58 (m, 2H), 0.48-0.38 (m, 2H).

### Preparation of Example 1

### N,N-dimethyl-4-(7-(N-(1-methylcyclopropyl)sulfamoyl)-1-(6-methylpyridin-3-yl)imidazo[1,5-a]pyridin-5-yl)piperazine-1-carboxamide

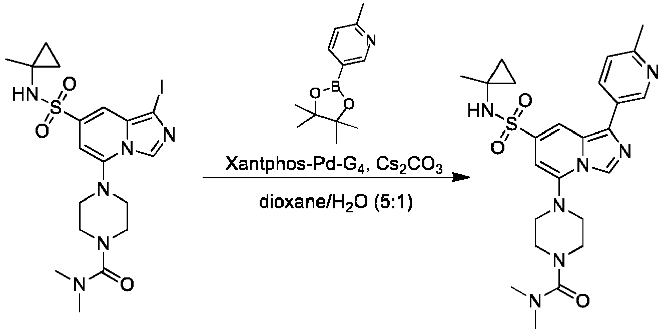

To a mixture of 2-methyl-5-(4,4,5,5-tetramethyl-1,3,2-dioxaborolan-2-yl)pyridine (9.98 mg, 72.88 µmol) and 4-(1-iodo-7-(N-(1-methylcyclopropyl)sulfamoyl)imidazo[1,5-a]pyridin-5-yl)-N,N-dimethyl piperazine-1-carboxamide (15.00 mg, 28.17 µmol) in dioxane (0.5 mL) and H₂O (0.1 mL) was added Cs₂CO₃ (23.75 mg, 72.88 µmol) and Xantphos Pd G₄ (3.51 mg, 3.64 µmol) under N₂ before the mixture was stirred at 80 °C for 2 h. The mixture was diluted with water (5 mL) and extracted with DCM (3 mL, 2x). The combined organic layers were dried (Na₂SO₄), filtered and concentrated to give a residue. The residue was purified by preparative HPLC (column: Phenomenex luna C18 150*40 mm* 15 µm; mobile phase: A: 0.225% formic acid in water, B: MeCN; B%: 10%- 40%, 9 min) to give the product N,N-dimethyl-4-(7-(N-(1-methylcyclopropyl)sulfamoyl)-1-(6-methylpyridin-3-yl)imidazo[1,5-a]pyridin-5-yl)piperazine-1-carboxamide (2.35 mg, 4.40 µmol, 12.07% yield, 99.9% purity) as a yellow gum.

**RT** 0.496 min **(method 3); m/z** 498.3 (M+H)⁺ (ESI⁺); **¹H NMR** (400 MHz, DMSO+D₂O): 8.90 (d, J = 1.6 Hz, 1H), 8.60 (s, 1H), 8.11 (dd, J = 2.0, 8.0 Hz, 1H), 7.95 (s, 1H), 7.42 (d, J = 8.0 Hz, 1H), 6.51 (s, 1H), 3.42-3.38 (m, 4H), 3.18-3.12 (m, 4H), 2.80 (s, 6H), 2.52 (s, 3H), 1.09 (s, 3H), 0.70-0.65 (m, 2H), 0.44-0.39 (m, 2H).

### Preparation of Example 3

### 4-(1-(6-(difluoromethyl)pyridin-3-yl)-7-(N-(1-methylcyclopropyl)sulfamoyl)imidazo[1,5-a]pyridin-5-yl)-N,N-dimethylpiperazine-1-carboxamide

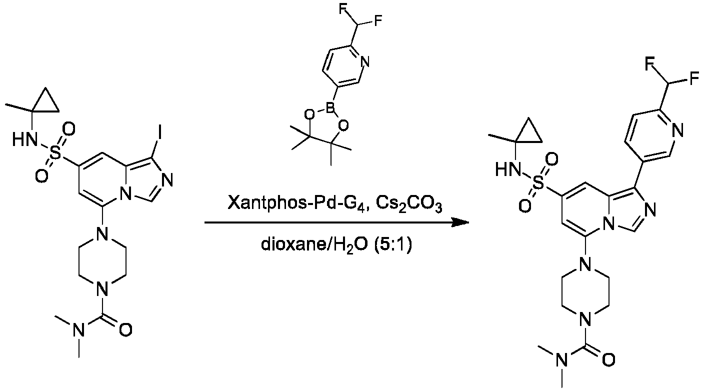

To a mixture of 2-(difluoromethyl)-5-(4,4,5,5-tetramethyl-1,3,2-dioxaborolan-2-yl) pyridine (12.60 mg, 72.88 µmol) and 4-(1-iodo-7-(N-(1-methylcyclopropyl)sulfamoyl)imidazo[1,5-a]pyridin-5-yl)-N,N-dimethylpiperazine-1-carboxamide (15 mg, 28.17 µmol) in dioxane (0.5 mL) and H₂O (0.1 mL)was added Cs₂CO₃ (23.75 mg, 72.88 µmol) and Xantphos Pd G₄ (3.51 mg, 3.64 µmol) under N₂ before the reaction mixture was stirred at 80 °C for 2 h. The mixture was poured into water (5 mL) and extracted with DCM (3 mL, 2x). The combined organic layers were dried (Na₂SO₄), filtered and concentrated to give a residue. The residue was purified by preparative HPLC (column: Waters Xbridge 150*25mm*5 µm; mobile phase: A: 0.225% ammonia hydroxide in water, B: MeCN; B%: 31%- 61%, 9 min) to give the product 4-(1-(6-(difluoromethyl)pyridin-3-yl)-7-(N-(1-methylcyclopropyl)sulfamoyl) imidazo[1,5-a]pyridin-5-yl)-N,N-dimethylpiperazine-1-carboxamide (3.68 mg, 6.89 µmol, 18.91% yield, 99.9% purity) as a yellow solid.

**RT** 0.582 min **(method 3); m/z** 534.3 (M+H)⁺ (ESI⁺); **¹H NMR** (400 MHz, DMSO): 9.20 (d, J = 1.6 Hz, 1H), 8.70 (s, 1H), 8.44 (dd, J = 2.0, 8.0 Hz, 1H), 8.42-8.11 (m, 1H), 8.07 (s, 1H), 7.84 (d, J = 8.4 Hz, 1H), 7.21-6.84 (m, 1H), 6.60 (s, 1H), 3.43-3.39 (m, 4H), 3.19-3.15 (m, 4H), 2.81 (s, 6H), 1.11 (s, 3H), 0.74-0.67 (m, 2H), 0.46-0.37 (m, 2H).

### Preparation of Intermediate 5.1

### 5-(benzylthio)pyrazolo[1,5-a]pyridine

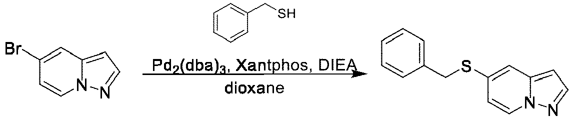

A mixture of 5-bromopyrazolo[1,5-a]pyridine (4.50 g, 22.84 mmol), phenylmethanethiol (3.12 g, 25.12 mmol, 2.94 mL), Xantphos (2.64 g, 4.57 mmol), Pd₂(dba)₃ (2.09 g, 2.28 mmol) and DIEA (5.90 g, 45.68 mmol, 7.96 mL) in dioxane (50 mL) was degassed and purged with N₂ (3x) before the mixture was stirred at 100 °C for 16 h under N₂ atmosphere. The mixture was filtered and the mother solution was concentrated in vacuum. The residue was purified by flash silica gel chromatography (ISCO^{®}; 40 g SepaFlash^{®} Silica Flash Column, Eluent of 0~50% Ethyl acetate/Petroleum @ 50 mL/min) to give the product 5-benzylsulfanylpyrazolo[1,5-a]pyridine (5 g, 20.81 mmol, 91.10% yield) as a yellow solid.

**RT** 0.490 min (method 4); **m/z** 240.8 (M+H)⁺ (ESI⁺); **¹H NMR** (DMSO-*d*₆, 400 MHz): 8.56 (d, *J* = 7.6 Hz, 1H), 7.94 (d, *J = 2.4* Hz, 1H), 7.56 (d, *J =* 1.6 Hz, 1H), 7.41 (d, J = 7.2 Hz, 2H), 7.31 (t, *J =* 6.8 Hz, 2H), 7.27-7.21 (m, 1H), 6.80 (dd, J = 2.0, 7.2 Hz, 1H), 6.45 (d, *J* = 1.6 Hz, 1H), 4.34 (s, 2H).

### Preparation of Intermediate 5.2

### 5-(benzylthio)-7-bromopyrazolo[1,5-a]pyridine

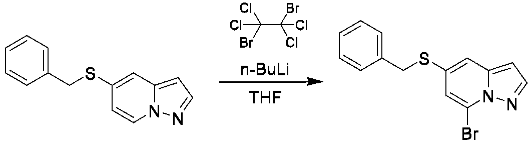

To a solution of 5-benzylsulfanylpyrazolo[1,5-a]pyridine (3.9 g, 16.23 mmol) in THF (40 mL) was added n-BuLi (2.5 M, 7.14 mL) dropwise at -78 °C, and the mixture was stirred at -78 °C for 10 min. 1,2-dibromo-1,1,2,2-tetrachloro-ethane (10.57 g, 32.46 mmol, 3.90 mL) was added dropwise at -78 °C. The mixture was allowed to warm to 20 °C and stirred for 30 min. The mixture was poured into aqueous, saturated NH₄Cl solution (200 mL) and extracted with EtOAc (50 mL, 2x). The combined organic phase was washed with brine (100 mL), dried (Na₂SO₄), filtered and concentrated to give a residue. The residue was purified by flash silica gel chromatography (ISCO^{®}; 80 g SepaFlash^{®} Silica Flash Column, Eluent of 0-20% EtOAc/Petroleum ethergradient @ 80 mL/min) to give the product 5-(benzylthio)-7-bromopyrazolo[1,5-a]pyridine (3.94 g, 12.34 mmol, 76.06% yield) as a gray solid.

**RT** 0.482 min **(method** 4); **m/z** 318.7 (M+H)⁺ (ESI⁺); **¹H NMR** (DMSO-*d*₆, 400 MHz): 8.05 (d, *J* = 2.0 Hz, 1H), 7.65 (d, *J* = 2.0 Hz, 1H), 7.41 (d, *J* = 7.2 Hz, 2H), 7.34-7.30 (m, 3H), 7.25 (d, *J =* 7.2 Hz, 1H), 6.67 (d, *J = 2.4* Hz, 1H), 4.37 (s, 2H).

### Preparation of Intermediate 5.3

### 5-(benzylthio)-3,7-dibromopyrazolo[1,5-a]pyridine

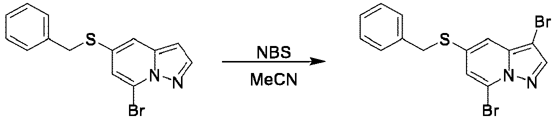

To a solution of 5-benzylsulfanyl-7-bromo-pyrazolo[1,5-a]pyridine (500 mg, 1.57 mmol) in MeCN (5 mL) was added NBS (292.72 mg, 1.64 mmol). The mixture was stirred at 20 °C for 1 h. LCMS showed that the starting material was consumed and mainly one peak with desired mass was detected. The mixture was concentrated to give the crude product 5-benzylsulfanyl-3,7-dibromo-pyrazolo[1,5-a]pyridine (623 mg) as a brown solid, which was used into next step without further purification.

RT 0.531 min (method 4); **m/z** 398.7 (M+H)⁺ (ESI⁺).

### Preparation of Intermediate 5.4

### 3,7-dibromopyrazolo[1,5-a]pyridine-5-sulfonyl chloride

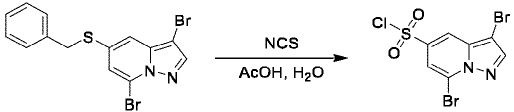

To a solution of 5-benzylsulfanyl-3,7-dibromo-pyrazolo[1,5-a]pyridine (623 mg, 1.56 mmol) in AcOH (6 mL) and water (3 mL) was added NCS (835.85 mg, 6.26 mmol) at 0 °C. The mixture was stirred at 20 °C for 2 h. The mixture was concentrated to give the crude product 3,7-dibromopyrazolo[1,5-a]pyridine-5-sulfonyl chloride (585 mg) as a brown solid which was directly used in the next step without further purification.

### Preparation of Intermediate 5.5

### 3,7-dibromo-N-(1-methylcyclopropyl)pyrazolo[1,5-a]pyridine-5-sulfonamide

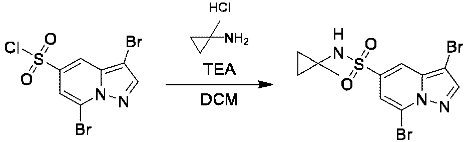

To a solution of 1-methylcyclopropanamine (252.12 mg, 2.34 mmol, HCl salt) and TEA (1.58 g, 15.62 mmol, 2.17 mL) in DCM (2 mL) was added a solution of 3,7-dibromopyrazolo[1,5-a]pyridine-5-sulfonyl chloride (585 mg, 1.56 mmol) in DCM (1 mL) dropwise at 0 °C. The mixture was stirred at 0 °C for 1 h. The mixture was concentrated to give a residue which was purified by flash silica gel chromatography (ISCO^{®}; 12 g SepaFlash^{®} Silica Flash Column, Eluent of 0~100% EtOAc/Petroleum ether gradient @ 20 mL/min) to give the product 3,7-dibromo-N-(1-methylcyclopropyl)pyrazolo[1,5-a]pyridine-5-sulfonamide (526 mg, 1.29 mmol, 82.30% yield) as a yellow solid.

**RT** 0.443 min (method 4); **m/z** 409.6 (M+H)⁺ (ESI⁺); **¹H NMR** (DMSO-*d*₆, 400 MHz): 8.52 (s, 1H), 8.47-8.43 (m, 1H), 7.96 (d, *J* = 2.0 Hz, 1H), 7.57 (d, *J* = 1.6 Hz, 1H), 1.13 (s, 3H), 0.71-0.64 (m, 2H), 0.49-0.44 (m, 2H).

### Preparation of Intermediate 5.6

### 4-(3-bromo-5-(N-(1-methylcyclopropyl)sulfamoyl)pyrazolo[1,5-a]pyridin-7-yl)-N,N-dimethylpiperazine-1-carboxamide

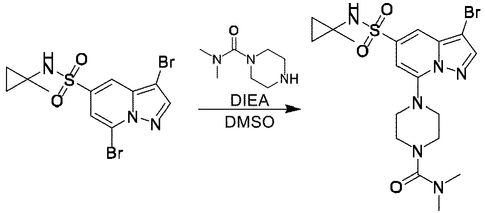

4-(1-(6-(difluoromethyl)pyridin-3-yl)-7-(N-(1-methylcyclopropyl)sulfamoyl)imidazo[1,5-a]pyridin-5-yl)-N,N-dimethylpiperazine-1-carboxamide was prepared using the conditions described in example 1. The product was isolated by preparative HPLC (column: Welch Ultimate C18 150*25 mm*5 µm; mobile phase: A: 0.225% formic acid in water, B: MeCN; 38%-68%, 10 min). The corresponding HPLC fractions were extracted with EtOAc (50 mL, 2x), and the combined organic phase was washed with brine (100 mL), dried (Na₂SO₄), filtered and concentrated to give the product 4-(3-bromo-5-(N-(1-methylcyclopropyl)sulfamoyl)pyrazolo[1,5-a]pyridin-7-yl)-N,N-dimethylpiperazine-1-carboxamide (480 mg, 988.88 µmol, 76.91% yield) as a brown solid.

**RT** 0.452 min **(method** 4); **m/z** 486.8 (M+H)⁺ (ESI⁺); **¹H NMR** (DMSO-*d*₆, 400 MHz): 8.38 (s, 1H), 8.33 (s, 1H), 7.63-7.55 (m, 1H), 6.65 (s, 1H), 3.48-3.40 (m, 4H), 3.38-3.34 (m, 4H), 2.80 (s, 6H), 1.10 (s, 3H), 0.70-0.65 (m, 2H), 0.46-0.41 (m, 2H).

### Preparation of Example 5

### N,N-dimethyl-4-(5-(N-(1-methylcyclopropyl)sulfamoyl)-3-(6-methylpyridin-3-yl)pyrazolo[1,5-a]pyridin-7-yl)piperazine-1-carboxamide

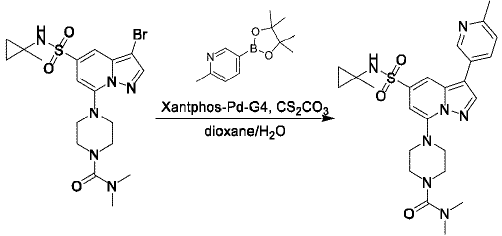

A solution of 4-(3-bromo-5-(N-(1-methylcyclopropyl)sulfamoyl)pyrazolo[1,5-a]pyridin-7-yl)-N,N-dimethylpiperazine-1-carboxamide (50.0 mg, 103.01 µmol), 2-methyl-5-(4,4,5,5-tetramethyl-1,3,2-dioxaborolan-2-yl)pyridine (33.85 mg, 154.51 µmol), Xantphos-Pd-G₄ (9.91 mg, 10.30 µmol) and Cs₂CO₃ (67.12 mg, 206.02 µmol) in dioxane (0.5 mL) and water (0.2 mL) was degassed and purged with N₂(3x) before the mixture was stirred at 80 °C for 2 h under N₂ atmosphere. The mixture was filtered and the filtrate was concentrated in vacuum. The residue was purified by preparative HPLC (column: Phenomenex luna C18 150*25 mm* 10 µm; mobile phase: A: 0.225% formic acid in water, B: MeCN; 7%-37%, 8 min) and lyophilized directly to give the product N,N-dimethyl-4-(5-(N-(1-methylcyclopropyl)sulfamoyl)-3-(6-methylpyridin-3-yl)pyrazolo[1,5-a]pyridin-7-yl)piperazine-1-carboxamide (13.47 mg, 26.66 µmol, 25.88% yield, 98.49% purity) as a white solid.

**RT** 0.322 min **(method** 4); **m/z** 498.2 (M+H)⁺ (ESI⁺); **¹H NMR** (CDCl₃, 400 MHz): 8.75 (d, *J = 1.6* Hz, 1H), 8.25 (s, 1H), 8.02 (d, *J* = 2.0 Hz, 1H), 7.79 (dd, J = 2.4, 8.0 Hz, 1H), 7.30 (d, *J* = 8.0 Hz, 1H), 6.57 (d, *J =* 1.6 Hz, 1H), 5.15 (s, 1H), 3.59-3.55 (m, 4H), 3.53 (br d, J = 4.9 Hz, 4H), 2.91 (s, 6H), 2.64 (s, 3H), 1.30 (s, 3H), 0.87-0.81 (m, 2H), 0.56-0.50 (m, 2H).

### Preparation of Example 7

### 4-(3-(6-(difluoromethyl)pyridin-3-yl)-5-(N-(1-methylcyclopropyl)sulfamoyl)pyrazolo[1,5-a]pyridin-7-yl)-N,N-dimethylpiperazine-1-carboxamide

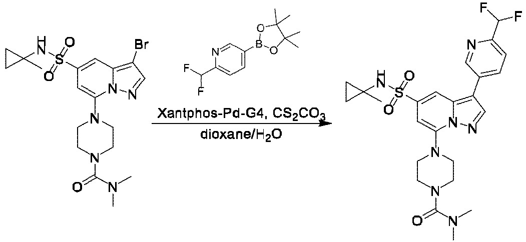

A mixture of N,N-dimethyl-4-(5-(N-(1-methylcyclopropyl)sulfamoyl)-3-(6-methylpyridin-3-yl)pyrazolo[1,5-a]pyridin-7-yl)piperazine-1-carboxamide (50.0 mg, 103.01 µmol), 2-(difluoromethyl)-5-(4,4,5,5-tetramethyl-1,3,2-dioxaborolan-2-yl)pyridine (39.41 mg, 154.51 µmol) , Xantphos-Pd-G4 (9.91 mg, 10.30 µmol) and Cs₂CO₃ (67.12 mg, 206.02 µmol) in dioxane (0.5 mL) and H₂O (0.2 mL) was degassed and purged with N₂ (3x) before the mixture was stirred at 80 °C for 2 h under N₂ atmosphere. The mixture was filtered and the filtrate was concentrated in vacuum. The residue was purified by preparative HPLC (column: Phenomenex luna C18 150*25 mm* 10 µm; mobile phase: A: 0.225% formic acid in water, B: MeCN; 32%-62%, 8 min) and lyophilized directly to give the product 4-(3-(6-(difluoromethyl)pyridin-3-yl)-5-(N-(1-methylcyclopropyl)sulfamoyl)pyrazolo[1,5-a]pyridin-7-yl)-N,N-dimethylpiperazine-1-carboxamide (5.71 mg, 10.48 µmol, 10.17% yield, 97.95% purity) as a white solid.

**RT** 0.447 min (method 4); **m/z** 534.2 (M+H)⁺ (ESI⁺); **¹H NMR** (CDCl₃, 400 MHz): 8.91 (d, *J = 1.6* Hz, 1H), 8.32 (s, 1H), 8.06-8.02 (m, 2H), 7.77 (d, *J* = 8.0 Hz, 1H), 6.86-6.58 (m, 2H), 5.05 (s, 1H), 3.56 (br d, *J* = 5.6 Hz, 8H), 2.92 (s, 6H), 1.32 (s, 3H), 0.86-0.82 (m, 2H), 0.58-0.53 (m, 2H).

### Preparation of Example 8

### Methyl 7-(4-(dimethylcarbamoyl)piperazin-1-yl)-5-(N-(1-methylcyclopropyl)sulfamoyl)pyrazolo[1,5-a]pyridine-3-carboxylate

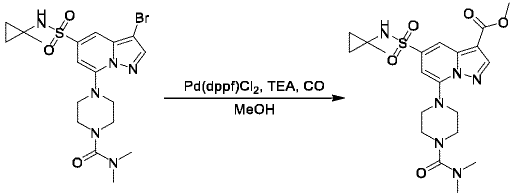

A mixture of N,N-dimethyl-4-(5-(N-(1-methylcyclopropyl)sulfamoyl)-3-(6-methylpyridin-3-yl)pyrazolo[1,5-a]pyridin-7-yl)piperazine-1-carboxamide (50.0 mg, 103.01 µmol), Pd(dppf)Cl₂·DCM (8.41 mg, 10.30 µmol) and TEA (31.27 mg, 309.03 µmol, 43.01 µL) in MeOH (1 mL) was degassed and purged with N₂ (3x) before the mixture was stirred at 80 °C for 2 h under CO atmosphere (50 psi). The mixture was filtered and the filtrate was concentrated to give a residue. The residue was purified by preparative HPLC (column: Phenomenex luna C18 150*25 mm* 10 µm; mobile phase: A: 0.225% formic acid in water, B: MeCN; 30%-60%, 10 min) and lyophilized directly to give the product methyl 7-(4-(dimethylcarbamoyl)piperazin-1-yl)-5-(N-(1-methylcyclopropyl)sulfamoyl)pyrazolo[1,5-a]pyridine-3-carboxylate (4.2 mg, 9.04 µmol, 4.39% yield, 100% purity) as a white solid.

**RT** 0.427 min (method 4); **m/z** 435.2 (M+H)⁺ (ESI⁺); **¹H NMR** (CDCl₃, 400 MHz): 8.51 (s, 1H), 8.37 (d, J = 2.0 Hz, 1H), 6.71 (d, J = 2.0 Hz, 1H), 5.04 (s, 1H), 3.96 (s, 3H), 3.54 (d, J = 4.0 Hz, 8H), 2.90 (s, 6H), 1.32 (s, 3H), 0.88-0.85 (m, 2H), 0.59-0.54 (m, 2H).

### Preparation of Intermediate 10.1

### 4-(3-(hydrazinecarbonyl)-5-(N-(1-methylcyclopropyl)sulfamoyl)pyrazolo[1,5-a]pyridin-7-yl)-N,N-dimethylpiperazine-1-carboxamide

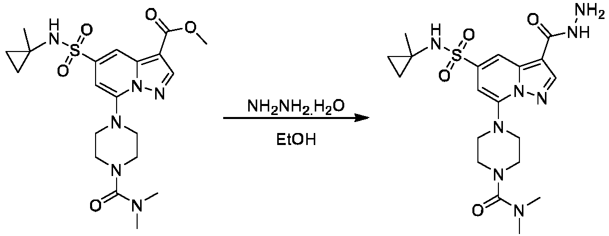

To a solution of methyl 7-(4-(dimethylcarbamoyl)piperazin-1-yl)-5-(N-(1-methylcyclopropyl)sulfamoyl)pyrazolo[1,5-a]pyridine-3-carboxylate (0.40 g, 0.861 mmol) in EtOH (10 mL) was added hydrazine hydrate (216 mg, 4.31 mmol). The mixture was stirred at 80°C for 16 h. The mixture was concentrated to give a residue which was dissolved in EtOAc (50 mL) and washed with HCl solution (1 N, 20 mL). The organic phase was dried (Na₂SO₄), filtered and concentrated to give the product 4-(3-(hydrazinecarbonyl)-5-(N-(1-methylcyclopropyl)sulfamoyl)pyrazolo[1,5-a]pyridin-7-yl)-N,N-dimethylpiperazine-1-carboxamide (260 mg, 0.560 mmol, 65.00% yield) as a yellow solid.

**RT** 0.890 min **(method** 3); **m/z** 465.1 (M+H)⁺ (ESI⁺).

### Preparation of Intermediate 10.2

### 4-(3-(2-(2,2-difluoroacetyl)hydrazine-1-carbonyl)-5-(N-(1-methylcyclopropyl)sulfamoyl)pyrazolo[1,5-a]pyridin-7-yl)-N,N-dimethylpiperazine-1-carboxamide

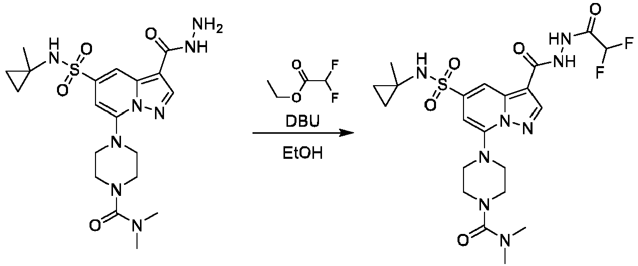

To a solution of 4-(3-(hydrazinecarbonyl)-5-(N-(1-methylcyclopropyl)sulfamoyl)pyrazolo[1,5-a]pyridin-7-yl)-N,N-dimethylpiperazine-1-carboxamide (100 mg, 0.215 mmol) and DBU (66 mg, 0.431 mmol) in EtOH (1 mL) was added ethyl 2,2-difluoroacetate (134 mg, 1.08 mmol). The mixture was stirred at 60 °C for 2h. The mixture was concentrated in vacuum and the residue was purified by reversed phase flash (12 g Flash Column Welch Ultimate XB_C18 20-40 µm; 120 A, Eluent of 5-95% ACN/ H₂O with 0.1% FA additive @ 10 mL/min). The fractions were adjusted to pH >7 with aqueous, saturated NaHCO₃ solution. The aqueous phase was extracted with EtOAc (40 mL, 2x). The combined organic phase was washed with brine (80 mL), dried (Na₂SO₄), filtered, and concentrated to give the product 4-(3-(2-(2,2-difluoroacetyl)hydrazine-1-carbonyl)-5-(N-(1-methylcyclopropyl)sulfamoyl)pyrazolo[1,5-a]pyridin-7-yl)-N,N-dimethylpiperazine-1-carboxamide (60 mg, 0.111 mmol, 51.37 % yield) as yellow oil.

**RT** 0.361 min **(method 4); m/z** 543.3 (M+H)⁺ (ESI⁺).

### Preparation of Examples 10 and 11

### 4-(3-(5-(difluoromethyl)-1,3,4-thiadiazol-2-yl)-5-(N-(1-methylcyclopropyl)sulfamoyl)pyrazolo[1,5-a]pyridin-7-yl)-N,N-dimethylpiperazine-1-carboxamide and 4-(3-(5-(difluoromethyl)-1,3,4-oxadiazol-2-yl)-5-(N-(1-methylcyclopropyl)sulfamoyl)pyrazolo[1,5-a]pyridin-7-yl)-N,N-dimethylpiperazine-1-carboxamide

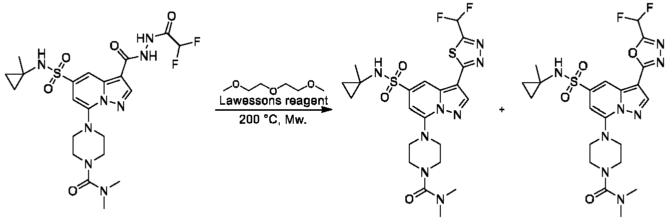

To a solution of 4-(3-(2-(2,2-difluoroacetyl)hydrazine-1-carbonyl)-5-(N-(1-methylcyclopropyl)sulfamoyl)pyrazolo[1,5-a]pyridin-7-yl)-N,N-dimethylpiperazine-1-carboxamide (25.0 mg, 0.0461 mmol) in THF (0.3 mL) was added Lawessons reagent (19 mg, 0.0461 mmol). The mixture was stirred at 100°C for 1 h in the microwave. TLC analysis showed that mainly starting material remained. The mixture was concentrated to remove THF and the residue was dissolved in 1-methoxy-2-(2-methoxyethoxy)ethane (0.3 mL). The solution was stirred at 200°C for another 1 h in the microwave. After cooling, the mixture was poured into water (10 mL). The aqueous phase was extracted with EtOAc (5 mL, 2x), the combined organic phase was washed with brine (10 mL, 2x), dried (Na₂SO₄), filtered and concentrated. The residue was purified by preparative TLC (SiO₂, Petroleum ether/ Ethyl acetate= 0/1) to give the crude products. The crude products were re-purified by preparative HPLC (column: Welch Xtimate C18 150*25 mm*5 um; mobile phase: A: 0.225% formic acid in water, B: MeCN; B%: 34%-64%, 10 min) to give the product 4-(3-(5-(difluoromethyl)-1,3,4-thiadiazol-2-yl)-5-(N-(1-methylcyclopropyl)sulfamoyl)pyrazolo[1,5-a]pyridin-7-yl)-N,N-dimethylpiperazine-1-carboxamide (1.2 mg, 0.00229 mmol, 4.98% yield) as a yellow gum:
**RT** 0.461 min **(method 4); m/z** 541.3 (M+H)+ (ESI+); **¹H NMR** (CDCl₃, 400 MHz): 8.61 (d, *J* = 2.0 Hz, 1H), 8.49 (s, 1H), 7.09 (t, *J* = 53.6 Hz, 1H), 6.79 (d, *J* = 2.0 Hz, 1H), 5.10 (s, 1H), 3.57 (s, 8H), 2.92 (s, 6H), 1.34 (s, 3H), 0.92-0.87 (m, 2H), 0.61-0.56 (m, 2H);
and the product 4-(3-(5-(difluoromethyl)-1,3,4-oxadiazol-2-yl)-5-(N-(1-methylcyclopropyl)sulfamoyl)pyrazolo[1,5-a]pyridin-7-yl)-N,N-dimethylpiperazine-1-carboxamide (2.1 mg, 0.00397 mmol, 8.61% yield) as a yellow solid.

**RT** 0.442 min **(method 4); m/z** 525.3 (M+H)+ (ESI+); **¹H NMR** (CDCl₃, 400 MHz): 8.61 (s, 1H), 8.47 (d, *J =* 1.6 Hz, 1H), 6.95 (t, *J* = 51.6 Hz, 1H), 6.81 (d, *J* = 2.0 Hz, 1H), 5.15 (s, 1H), 3.58 (s, 8H), 2.92 (s, 6H), 1.35 (s, 3H), 0.91-0.87 (m, 2H), 0.61-0.57 (m, 2H).

### Preparation of Intermediate 12.1

### tert-butyl ((3-bromo-7-(4-(dimethylcarbamoyl)piperazin-1-yl)pyrazolo[1,5-a]pyridin-5-yl)sulfonyl)(1-methylcyclopropyl)carbamate

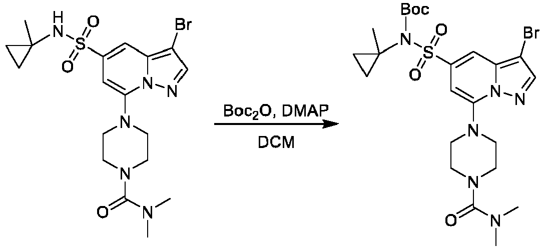

To a solution of 4-(3-bromo-5-(N-(1-methylcyclopropyl)sulfamoyl)pyrazolo[1,5-a]pyridin-7-yl)-N,N-dimethylpiperazine-1-carboxamide (175.0 mg, 0.361 mmol), DIPEA (0.19 mL, 1.08 mmol) and DMAP (4.4 mg, 0.0361 mmol) in DCM (1 mL) was added Boc₂O (118.0 mg, 0.541 mmol). The mixture was stirred at 20 °C for 1 h and was concentrated. The residue was purified by preparative TLC (SiO₂, Petroleum ether/ Ethyl acetate= 1/2) to give the product tert-butyl ((3-bromo-7-(4-(dimethylcarbamoyl)piperazin-1-yl)pyrazolo[1,5-a]pyridin-5-yl)sulfonyl)(1-methylcyclopropyl)carbamate (100 mg, 0.171 mmol, 47.37 % yield) as a white solid.

**RT** 0.546 min **(method 4); m/z** 585.2 (M+H)⁺ (ESI⁺).

### Preparation of Intermediate 12.2

### tert-butyl ((7-(4-(dimethylcarbamoyl)piperazin-1-yl)-3-(4,4,5,5-tetramethyl-1,3,2-dioxaborolan-2-yl)pyrazolo[1,5-a]pyridin-5-yl)sulfonyl)(1-methylcyclopropyl)carbamate

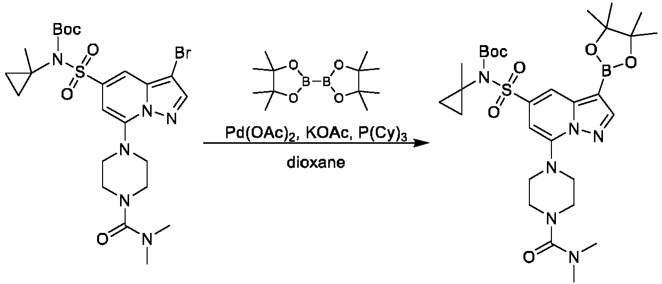

To a solution of tert-butyl ((3-bromo-7-(4-(dimethylcarbamoyl)piperazin-1-yl)pyrazolo[1,5-a]pyridin-5-yl)sulfonyl)(1-methylcyclopropyl)carbamate (50.0 mg, 0.0854 mmol), 4,4,5,5-tetramethyl-2-(4,4,5,5-tetramethyl-1,3,2-dioxaborolan-2-yl)-1,3,2-dioxaborolane (33 mg, 0.128 mmol) and AcOK (26 mg, 0.171 mmol) in 1,4-dioxane (0.5 mL) was added Pd(OAc)₂ (1.9 mg, 0.00854 mmol) and P(Cy)₃ (2.4 mg, 0.00854 mmol) under N₂ atmophere. The mixture was stirred at 80°C for 16 h. The reaction mixture was directly used in next step without further purification.

RT 0.573 min **(method** 4); **m/z** 633.5 (M+H)⁺ (ESI⁺).

### Preparation of Intermediate 12.3

### tert-butyl ((7-(4-(dimethylcarbamoyl)piperazin-1-yl)-3-(5-methyl-1,3,4-thiadiazol-2-yl)pyrazolo[1,5-a]pyridin-5-yl)sulfonyl)(1-methylcyclopropyl)carbamate

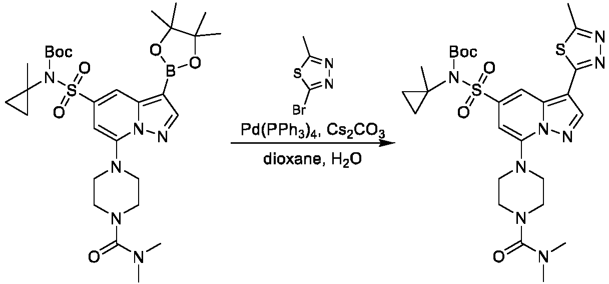

To a solution of tert-butyl ((7-(4-(dimethylcarbamoyl)piperazin-1-yl)-3-(4,4,5,5-tetramethyl-1,3,2-dioxaborolan-2-yl)pyrazolo[1,5-a]pyridin-5-yl)sulfonyl)(1-methylcyclopropyl)carbamate (80.0 mg, 0.126 mmol, crude), 2-bromo-5-methyl-1,3,4-thiadiazole (30 mg, 0.165 mmol) and Cs₂CO₃ (98 mg, 0.300 mmol) in 1,4-dioxane (0.1 mL) and water (0.02 mL) was added Pd(PPh₃)₄ (17 mg, 0.0150 mmol) under N₂. The mixture was stirred at 80°C for 2 h under N₂. After cooling the mixture was poured into water (10 mL). The aqueous phase was extracted with EtOAc (5 mL, 2x). The combined organic phase was washed with brine (10 mL), dried (Na₂SO₄), filtered and concentrated in vacuum. The residue was purified by preparative TLC (Petroleum ether/ Ethyl acetate= 1/1) to give the product tert-butyl ((7-(4-(dimethylcarbamoyl)piperazin-1-yl)-3-(5-methyl-1,3,4-thiadiazol-2-yl)pyrazolo[1,5-a]pyridin-5-yl)sulfonyl)(1-methylcyclopropyl)carbamate (30.0 mg, 0.0496 mmol, 30.016% yield) as a yellow oil.

**RT** 0.495 min **(method** 4); **m/z** 605.5 (M+H)⁺ (ESI⁺).

### Preparation of Example 12 (FoRx-06-375)

### N,N-dimethyl-4-(3-(5-methyl-1,3,4-thiadiazol-2-yl)-5-(N-(1-methylcyclopropyl)sulfamoyl)pyrazolo[1,5-a]pyridin-7-yl)piperazine-1-carboxamide

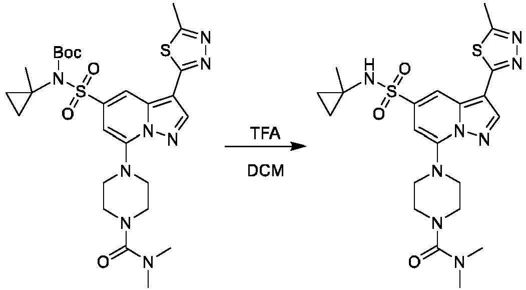

To a solution of tert-butyl ((7-(4-(dimethylcarbamoyl)piperazin-1-yl)-3-(5-methyl-1,3,4-thiadiazol-2-yl)pyrazolo[1,5-a]pyridin-5-yl)sulfonyl)(1-methylcyclopropyl)carbamate (30.0 mg, 0.0496 mmol) in DCM (0.5 mL) was added TFA (0.10 mL). The mixture was stirred at 20°C for 2h. The mixture was concentrated in vacuum. The residue was purified by preparative HPLC (column: Welch Xtimate C18 150*25 mm*5 um; mobile phase: A: 0.1% TFA in water, B: MeCN; B%: 24%-54%,10 min) and the corresponding HPLC fractions were lyophilized directly to give the product N,N-dimethyl-4-(3-(5-methyl-1,3,4-thiadiazol-2-yl)-5-(N-(1-methylcyclopropyl)sulfamoyl)pyrazolo[1,5-a]pyridin-7-yl)piperazine-1-carboxamide (4.1 mg, 0.00807 mmol, 16.26 % yield) as an off-white solid.

**RT** 0.431 min **(method 4); m/z** 505.3 (M+H)⁺ (ESI⁺); **¹H NMR** (CDCl₃, 400 MHz): 8.57 (s, 1H), 8.41 (s, 1H), 6.74 (s, 1H), 5.18 (br s, 1H), 3.56 (br s, 8H), 2.92 (s, 6H), 2.85 (s, 3H), 1.33 (s, 3H), 0.89 (s, 2H), 0.61-0.53 (m, 2H).

The following Table 1 provides an overview on the compounds described in the example section:

**Table 1**

| Example No. | Structure | Name of compound |
|---|---|---|
| 1 | 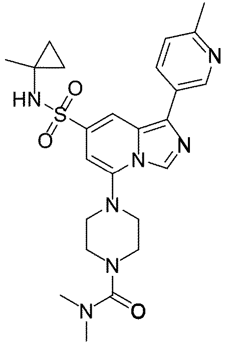 | N,N-dimethyl-4-(7-(N-(1-methylcyclopropyl)sulfamoyl)-1-(6-methylpyridin-3-yl)imidazo[1,5-a]pyridin-5-yl)piperazine-1-carboxamide |
| 3 | 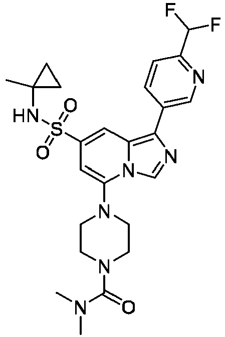 | 4-(1-(6-(difluoromethyl)pyridin-3-yl)-7-(N-(1-methylcyclopropyl)sulfamoyl)imidazo[1,5-a)pyridin-5-yl)-N,N-dimethylpiperazine-1-carboxamide |
| 5 | 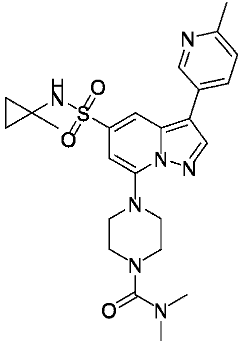 | N,N-dimethyl-4-(5-(N-(1-methylcyclopropyl)sulfamoyl)-3-(6-methylpyridin-3-yl)pyrazolo[1,5-a]pyridin-7-yl)piperazine-1-carboxamide |
| 7 | 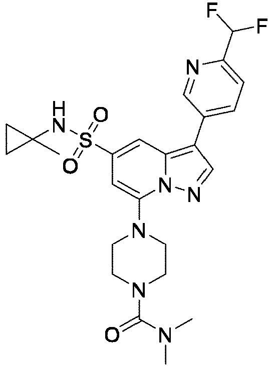 | 4-(3-(6-(difluoromethyl)pyridin-3-yl)-5-(N-(1-methylcyclopropyl)sulfamoyl)pyrazolo[1,5-a]pyridin-7-yl)-N,N-dimethylpiperazine-1-carboxamide |
| 8 | 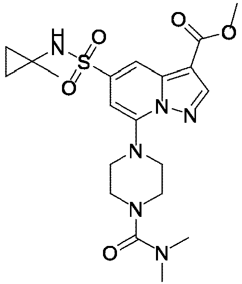 | methyl 7-(4-(dimethylcarbamoyl)piperazin-1-yl)-5-(N-(1-methylcyclopropyl)sulfamoyl)pyrazolo[1,5-a]pyridine-3-carboxylate |
| 10 | 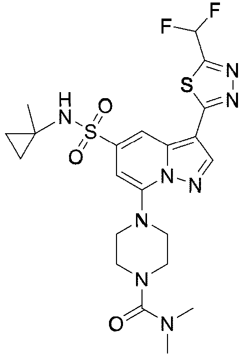 | 4-(3-(5-(difluoromethyl)-1,3,4-thiadiazol-2-yl)-5-(N-(1-methylcyclopropyl)sulfamoyl)pyrazolo[1,5-a]pyridin-7-yl)-N,N-dimethylpiperazine-1-carboxamide |
| 11 | 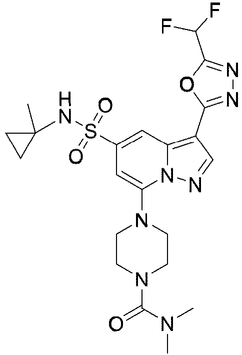 | 4-(3-(5-(difluoromethyl)-1,3,4-oxadiazol-2-yl)-5-(N-(1-methylcyclopropyl)sulfamoyl)pyrazolo[1,5-a]pyridin-7-yl)-N,N-dimethylpiperazine-1-carboxamide |
| 12 | 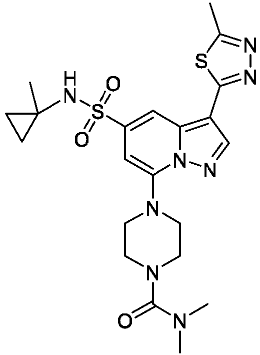 | N,N-dimethyl-4-(3-(5-methyl-1,3,4-thiadiazol-2-yl)-5-(N-(1-methylcyclopropyl)sulfamoyl)pyrazolo[1,5-a)pyridin-7-yl)piperazine-1-carboxamide |

### Biological evaluation of the exemplary compounds

Exemplary compounds of formula (I) were tested in selected biological and/or physicochemical assays one or more times. When tested more than once, data are reported as either average values or as median values, wherein the average value, also referred to as the arithmetic mean value, represents the sum of the values obtained divided by the number of times tested, and the median value represents the middle number of the group of values when ranked in ascending or descending order. If the number of values in the data set is odd, the median value is the middle value. If the number of values in the data set is even, the median is the arithmetic mean of the two middle values. The in vitro pharmacological, pharmacokinetic and physicochemical properties of the compounds can be determined according to the following assays and methods.

### PARG protein expression and purification

A codon optimized gene encoding human PARG (448-976 [H446G, L447S, L473S, N479S, S802A, R811K, M841I, S858P, I916T, T924D, D927K, C963S, A967T]) was synthesized by Genscript, and cloned into pET15b (Ncol/BamHI) with an N-terminal, Thrombin protease cleavable 6His-TwinStrep tag. Expression of the protein in E. *coli* BL21 (DE3) was induced by addition of 0.2 mM IPTG to a shake flask culture grown to OD600 = 0.8 at 37°C. Growth was allowed to continue at 30°C for a further 20 hours before harvesting by centrifugation and storage of the cell pellet at -80°C.

Protein was purified by IMAC and SEC: frozen cell pellets (typically 40 g wet weight) were resuspended by homogenization in 5 volumes buffer A (25 mM Tris/HCl pH 8.0, 200 mM NaCl, 2 mM DTT), supplemented with 1 mg of DNase I from bovine pancreas (Sigma-Aldrich) and protease inhibitors (Roche Complete^{™} EDTA-free protease inhibitor tablet), and lysed by passage through a Constant Systems BasicZ homogenizer. The lysate was clarified by centrifugation for 60 minutes at 25,000 g, 4°C, and the lysate supernatant was loaded onto 5 ml StrepTrap HP (Cytiva) pre-equilibrated with buffer A. The column was washed with buffer A (~10 CV), then buffer B containing 1M KCI (~5 CV), and then the protein was eluted with buffer A containing 2.5 mM d-Desthiobiotin. Pooled fractions containing 6HisTwinStrep-TEV-hPARG were incubated with TEV protease overnight at 4°C. hPARG was separated from uncleaved material and Thrombin protease through gel filtration with Superdex75 sizing column (GE Healthcare) pre-equilibrated with SEC buffer (15 mM Tris/HCl pH 8.5, 100 mM NaCl, 2 mM DTT). Pooled fractions containing pure hPARG were concentrated using a 10 k MWCO spin concentrator (VivaSpin) to 10 mg/mL, and then either used immediately for crystallisation or snap-frozen in liquid nitrogen for storage at -80°C.

### PARG enzymatic IC₅₀ assay

PARG enzyme as incubated with compound or vehicle (DMSO) for 15 minutes or 2 hours in a 384 well plate. After adding the PARG substrate ADP-ribose-pNP, the plate was read for absorbance intensity at 405 nm. The vehicle (DMSO) with high absorbance intensity represents no inhibition of enzymatic reaction while the low control (no enzyme) with low absorbance intensity represents full inhibition of enzymatic reaction.

### Materials:

hPARG: Peak Protein, 30 nM
Substrate: ADP-pNP, 800 µM, Jena Bioscience catalog # NU-955
Reaction time: 60 minutes
Assay buffer: 50 mM Tris-HCl pH 8.0, 100 mM NaCl, 2 mM DTT
Temperature: 30 °C
Total volume: 30 µL

### Controls:

- 0% inhibition control: DMSO
- 100% inhibition control: No enzyme

The protocol that was used for enzyme reaction and detection is as follows:
1. Transfer 100 nL of the final concentration of test compounds or vehicle (DMSO) to the appropriate wells of a microtiter plate.
2. Centrifuge the plate at 1000 rpm for 1 minute.
3. Transfer 14.6 µL of 2x final concentration of enzyme in assay buffer or assay buffer alone to the appropriate wells.
4. Centrifuge the plate at 1000 rpm for 1 minute.
5. Incubate the plate at room temperature for 15 minutes or 2 hours.
6. Transfer 15.4 µL of 2x substrate in assay buffer to all the test wells.
7. Centrifuge the plate at 1000 rpm for 1 minute.
8. Read the plate on a plate reader (e.g., Spark Tecan).

The Absorbance IC₅₀ value of compounds of Formula (I) in Examples 1 to 12 are provided in Table 2 below.

### Cellular PAR chain assay

The ability of compounds to inhibit PARG in response to DNA damage, was assessed with U2OS cells pretreated with the compounds for 1 hour, following a 1-hour treatment with or without the DNA alkylating agent temozolomide (TMZ). The cells were harvested and fixed in 70% ethanol, rehydrated with glucose and EDTA in PBS and subsequently blocked for 1 hour with PBS 1% BSA and 0.01% Tween-20 (PBT). The cells were incubated for 2 hours at room temperature with a mouse monoclonal antibody against poly (ADP) ribose (PAR) polymer. The cells were washed and incubated with an anti-mouse Alexa-488 conjugated secondary antibody for 1 hour at room temperature. Propidium iodide staining was used to determine DNA content in the cells (staining at 4°C overnight). The fluorescence intensity of the cells was assessed by flow cytometry (Cytoflex from Beckmann) and the percentage of PAR chain positive cells (gated in relation to TMZ+DMSO treated control) was determined. PAR chain positive cells % were fit against the concentration of the compound using a 4 parameter log-logistic function, generating PAR chain EC₅₀ values: $f \left(x\right) = c + \frac{d - c}{1 + {exp}^{b \left(log\left(x\right)-log\left(e\right)\right)}}$

The PAR chain EC₅₀ value for compounds of Formula (I) in Examples 1 to 25 are provided in Table 2 below.

### Cellular Viability Assay

NCIH-460 as a PARG-inhibition sensitive cell line and U2OS as PARG-inhibition insensitive cell line were plated at 1000 cells/well and 2000 cells/well, respectively, in 96-well white plates with clear flat bottom. After 24 hours, the compounds were added with the Tecan digital dispenser (D300e), in duplicates. The outer wells of the plate were excluded. After 96 hours of incubation, 150 µl of the growth medium were removed and 50 µl of Cell Titer-Glo (Promega) were added per well. Following an incubation of 10 minutes, luminescence was read using a plate reader (Tecan). Averaged values of the samples were normalized to DMSO treated control samples. Curves were fit as % of the control vs. log of the compound concentration using a 4 parameter log-logistic function: $f \left(x\right) = c + \frac{d - c}{1 + {exp}^{b \left(log\left(x\right)-log\left(e\right)\right)}}$

The PARGi (NCIH-460 and U2OS) cellular viability EC₅₀ values for compounds of Formula (I) in Examples 1 to 12 are provided in Table 2 below.
① Example number
② Structure
③ IC₅₀ in µM determined in PARG enzymatic assay (PARG protein and 15 mn incubation) described under PARG enzymatic IC₅₀ assay
④ IC₅₀ in µM determined in PARG enzymatic assay (PARG protein and 2 hours incubation) described under PARG enzymatic IC₅₀ assay
⑤. EC₅₀ in µM determined in cellular assay as described under Cellular PAR chain assay (conditions with treatment of TMZ).
⑥ EC₅₀ in µM determined in cellular assay as described under Cellular PAR chain assay (conditions without treatment of TMZ).
⑦ EC₅₀ in µM determined in NCIH-460 cells as described under Cellular viability assay.
⑧ EC₅₀ in µM determined in U2OS cells as described under Cellular viability assay.

**Table 2: Inhibition of PARG and cellular activity of compounds according to the present invention.**

| The IC₅₀ (inhibitory concentration at 50% of maximal effect) values are indicated in µM, empty space means that the corresponding compounds have not been tested in the respective assay. | | | | | | | |
|---|---|---|---|---|---|---|---|
| ① | ② | ③ | ④ | ⑤ | ⑥ | ⑦ | 8 |
| 1 | 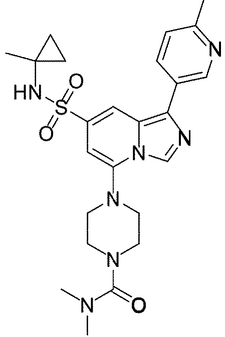 | | 0.064 | 1.096 | >10 | 13.358 | >16.841 |
| 3 | 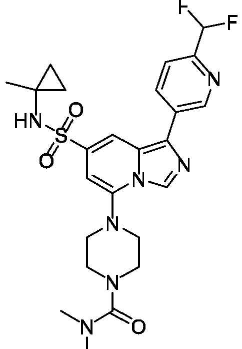 | | 0.066 | 0.218 | >10 | 4.468 | >20 |
| 5 | 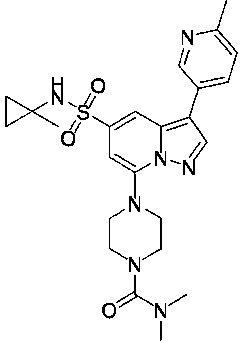 | | 0.054 | 0.223 | >10 | 3.222 | >20 |
| 7 | 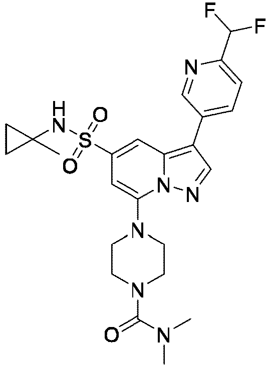 | | 0.028 | 0.328 | >10 | 3.943 | >20 |
| 8 | 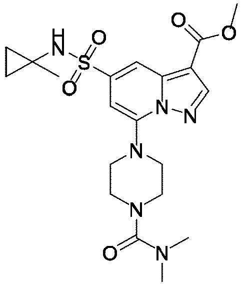 | | 0.043 | | | 7.22 | >20 |
| 10 | 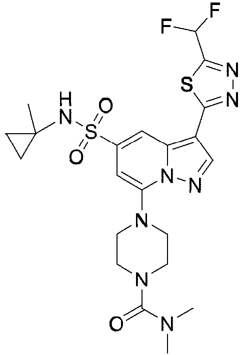 | | 0.042 | 0.266 | >10 | 1.303 | >20 |
| 11 | 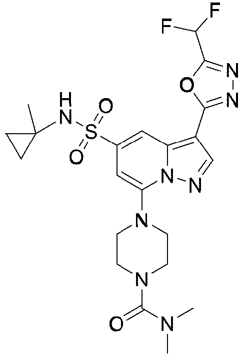 | | 0.098 | 0.739 | >10 | 4.578 | >20 |
| 12 | 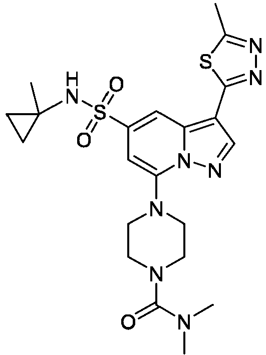 | | 0.038 | 1.487 | >10 | 6.052 | >20 |

### Further assays

### Kinetic solubility assay

The Kinetic solubility assay employs the shake flask method followed by HPLC-UV analysis. For exemplary compounds, the kinetic solubility was measured according to the following protocol:
1) Samples were weighed and dissolved in 100% DMSO to make a stock solution of 10 mM. About 100 µL of stock solution is needed to cover this assay.
2) Test compounds and controls (10 mM in DMSO, 10 µL/tube) were added into the buffer (490 µL/well) which placed in a Minni-Uniprep filter. The buffer was prepared as the customer's requirement.
3) Vortex the kinetic solubility samples for 2 minutes.
4) Incubate and shake the solubility solutions on an orbital shaker for 24 hr at room temperature
5) Transfer 200 µL each of solubility solution into 96-deep well for analysis when the samples were directly filtered by the syringeless filter device
6) Determine the test compound concentration of the filtrate using HPLC-UV.
7) Injected three UV standard solutions into HPLC from low to high concentration, followed by testing of the K.S. supernatant. Testing samples are injected in duplicate.

### Bidirectional permeability in Caco2

The bidirectional permeability in Caco-2 cells assay was performed for the exemplary compounds of formula (I) according to the following protocol:
1. Caco-2 cells purchased from ATCC were seeded onto polyethylene membranes (PET) in 96-well BD Insert plates at 1 x 105 cells/ cm2, and refreshed medium every 4~5 days until to the 21st to 28th day for confluent cell monolayer formation.
2. The integrity of the monolayer is verified by performing Lucifer yellow rejection assay.
3. The quality of the monolayer is verified by measuring the Unidirectional (A→B) permeability of fenoterol/nadolol (low permeability marker), propranolol/metopronolol (high permeability marker) and Bi-directional permeability of Digoxin (a P-glycoprotein substrate marker) in duplicate wells.
4. Standard assay conditions for test compounds:
   - Test concentration: 2 µM (DMSO≤1%);
   - Replicates: n=2;
   - Directions: bi-directional transport including A→B and B→A;
   - Incubation time: single time point, 2hours;
   - Transport buffer: HBSS containing 10 mM HEPES, pH7.40±0.05;
   - Incubation condition: 37±1°C, 5% CO2, relatively saturated humidity.
5. Spike dosing solution and mix with transport buffer and Stop Solution (containing an appropriate internal standard (IS)) as T0 sample.
6. At the end of incubation, sample solutions from both donor and receiver wells and mix with Stop Solution immediately.
7. All samples including T0 samples, donor samples and receiver samples are analyzed using LC/MS/MS. Concentrations of test compound are expressed as peak area ratio of analytes versus IS without a standard curve.

### Microsome metabolic stability (MMS) assay

The stability of the exemplary compounds was measured in the microsome metabolic stability assay as follows:
1) Test compounds will be incubated at 37°C with liver microsomes (pooled from multiple donors) at 1 µM in the presence of a NADPH regenerating system at 0.5 mg/ml microsomal protein.
2) Positive controls include Testosterone (3A4 substrate), Propafenone (2D6) and Diclofenac (2C9). They will be incubated with microsomes in the presence of a NADPH regenerating system.
3) Time samples (0, 5, 15, 30, 45 and 60 minutes) will be removed, immediately mixed with cold acetonitrile containing internal standard (IS). Test compound incubated with microsomes without NADPH regenerating system for 60min will be also included.
4) Single point for each test condition (n=1).
5) Samples will be analyzed by LC/MS/MS; disappearance of test compound will be assessed base on peak area ratios of analyte/IS(no standard curve).
6) An excel data summary, calculated intrinsic clearance and t½ values will be provided.
7) Using the following equation to calculate the microsome clearance:
   , int(mic) = 0.693/half life/mg microsome protein per mLwt: 40 g/kg, 30 g/kg, 32 g/kg, 20 g/kg and 88 g/kg for rat, monkey, dog, human and mouse.CLint(mic) to calculate the whole the liver clearance: microsomal protein / g liver weight: 45 mg/g for 5 speciesint(liver) = CLint(mic) * mg microsomal protein/g liver weight * g liver weight/kg body weight.

### In vitro metabolic stability of test compounds in CD-1 mouse, SD rat, beagle dog, cynomolgus monkey and human cryopreserved hepatocytes

1. Test compound (at 1 µM) is incubated with cryopreserved hepatocytes (0.5 × 106 cells per mL) in duplicates (n=2) at 37°C using 96-well plate format.
2) Time points are 0, 15, 30, 60 and 90 minutes in separate plates and medium control samples without cells at 0 and 90 minutes are also incubated. At each time point the reaction will be stopped by adding organic solution containing internal standard (IS).
3. Positive controls 7-ethoxycoumarin and 7-hydroxycoumarin are included in parallel.
4. Samples are analyzed by LC-MS/MS. Disappearance of test compound is assessed based on peak area ratios of analyte/IS (no standard curve).

Further embodiments of the present disclosure are disclosed in the following numbered items The scope of the claimed invention is defined by the claims. The following embodiments refer to the disclosure of the present description, and not necessarily to the claimed invention.
1. A compound of formula (I): 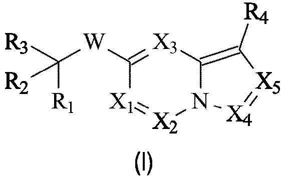 or an enantiomer, diastereoisomer, tautomer, pharmaceutically acceptable solvate, pharmaceutically acceptable crystal form or pharmaceutically acceptable salt thereof,
   wherein:
   R₁ is selected from the group consisting of hydrogen, chloro, fluoro, cyano, formyl, (C₁₋₂)alkyl, (C₂)alkenyl, (C₂)alkynyl (C₁₋₂)haloalkyl, -(C₁₋₂ alkylene)-OH and -(C₁₋₂ alkylene)-O-(C₁₋₂ alkyl), preferably wherein R₁ is selected from the group consisting of hydrogen, chloro, fluoro, cyano, formyl, (C₁₋₂)alkyl, (C₂)alkenyl, (C₂)alkynyl and (C₁₋₂)haloalkyl;
   R₂ and R₃ are independently each (C₁₋₂)alkyl or (C₁₋₂)haloalkyl, or R₂ and R₃ together with the carbon atom to which they are attached form cyclopropyl;
   W is selected from -NHS(O)_{y}-, -S(O)_{y}NH-, -NHS(O)(NH)-, -NHS(O)(NCH₃)-, -S(O)(NH)-NH-, - S(O)(NCH₃)-NH-, wherein y is 1 or 2;
   X₁ and X₃ are independently selected from the group consisting of N, CH, and CF;
   X₂ is N or C-Y_{C2}-R_{C2},
      wherein Y_{C2} is selected from a covalent bond, C₁₋₅ alkylene, C₂₋₅ alkenylene, C₂₋₅ alkynylene, cycloalkylne and heterocycloalkylene wherein said alkylene, said alkenylene and said alkynylene are each optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C_{1**-**5} alkyl)(C₁₋₅ alkyl), preferably selected from halogen, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more - CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-, and further wherein said cycloalkylene and said heterocycloalkylene are each optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), - (C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl),
      preferably wherein Y_{C2} is selected from a covalent bond, C₁₋₅ alkylene, C₂₋₅ alkenylene, and C₂₋₅ alkynylene, wherein said alkylene, said alkenylene and said alkynylene are each optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more - CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-, and
      wherein R_{C2} is selected from hydrogen, halo, -OH, -NH₂, -SH, -CN, C₁₋₁₂ alkyl, C₂₋₁₂ alkenyl, C₂₋₁₂ alkynyl, cycloalkyl, heterocycloalkyl, aryl, and heteroaryl,
         wherein said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), and
         wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), - (C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and - (C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl);
   X₄ is N or C-R_{C4},
      wherein R_{C4} is selected from hydrogen, halo, C₁₋₆ alkyl, C₂₋₆ alkynyl, -O(C₁₋₆ alkyl), -S(C₁₋₆ alkyl), -NH(C₁₋₆ alkyl), -N(C₁₋₆ alkyl)(C₁₋₆ alkyl), -CO(C₁₋₆ alkyl), C₁₋₆ haloalkyl, -O(C₁₋₆ haloalkyl), -S(C₁₋₆ haloalkyl), -NH(C₁₋₆ haloalkyl), -N(C₁₋₆ haloalkyl)₂, -CO-(C₁₋₆ haloalkyl), - (C₀₋₃ alkylene)-cycloalkyl, -O-(C₀₋₃ alkylene)-cycloalkyl, -CO-(C₀₋₃ alkylene)-cycloalkyl, -(C₀₋₃ alkylene)-heterocycloalkyl, -O-(C₀₋₃ alkylene)-heterocycloalkyl, -CO-(C₀₋₃ alkylene)-heterocycloalkyl, -(C₀₋₃ alkylene)-aryl, -O-(C₀₋₃ alkylene)-aryl, -CO-(C₀₋₃ alkylene)-aryl, -(C₀₋₃ alkylene)-heteroaryl, -O-(C₀₋₃ alkylene)-heteroaryl and -CO-(C₀₋₃ alkylene)-heteroaryl,
      wherein said alkyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N-*heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and
      wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl);
   X₅ is N or C-R_{C5},
      wherein R_{C5} is selected from hydrogen, halo, C₁₋₆ alkyl, -O(C₁₋₆ alkyl), -S(C₁₋₆ alkyl), -NH(C₁₋₆ alkyl), -N(C₁₋₆ alkyl)(C₁₋₆ alkyl) and C₁₋₆ haloalkyl;
   R₄ is Y_{R5}-R_{R5},
      wherein Y_{R5} is selected from a covalent bond, C₁₋₄ alkylene, C₂₋₄ alkenylene, and C₂₋₄ alkynylene, wherein said alkylene, said alkenylene and said alkynylene are each optionally substituted with one or more groups independently selected from halogen, -CN, -OH, -O(C₁₋₅ alkyl), -O(C₁₋₅ haloalkyl)-SH, -S(C₁₋₅ alkyl), -S(C₁₋₅ haloalkyl)-NH₂, -NH(C₁₋₅ alkyl), -NH(C₁₋₅ haloalkyl), -N(C₁₋₅ alkyl)(C₁₋₅ alkyl), and -N(C₁₋₅ haloalkyl)(C₁₋₅ alkyl) and further wherein one or more -CH₂- units comprised in said alkylene, said alkenylene or said alkynylene are each optionally replaced by a group independently selected from -O-, -NH-, -N(C₁₋₅ alkyl)-, -CO-, -COO-, -S-, -SO-, and -SO₂-, and
      wherein R_{R5} is selected from C₁₋₁₂ alkyl, C₂₋₁₂ alkenyl, C₂₋₁₂ alkynyl, cycloalkyl, heterocycloalkyl, aryl, and heteroaryl,
         wherein said alkyl, alkenyl, or alkynyl is optionally substituted with one or more groups independently selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
2. The compound of item 1, wherein R₁ is selected from the group consisting of cyano, (C₁₋₂)alkyl, and (C₁₋₂)haloalkyl.
3. The compound of item 1 or 2, wherein R₁ is selected from the group consisting of cyano, methyl and fluoromethyl.
4. The compound of any one of items1 to 3, wherein R₁ is cyano.
5. The compound of any one of items 1 to 4, wherein R₂ and R₃ together with the carbon atom to which they are attached form cyclopropyl.
6. The compound of any one of items 1 to 5, wherein W is -NHS(O)₂-.
7. The compound of any one of items 1 to 6, wherein X₁ and X₃ are each CH.
8. The compound of any one of items 1 to 7, wherein X₂ is C-Y_{C2}-R_{C2}.
9. The compound of item 8, wherein -Y_{C2}-R_{C2} is selected from -O-C₁₋₁₂ alkyl, -NH-C₁₋₁₂ alkyl, -N(C₁₋₅ alkyl)-C₁₋₁₂ alkyl, -O-C₂₋₁₂ alkenyl, -NH-C₂₋₁₂ alkenyl, -N(C₁₋₅ alkyl)-C₂₋₁₂ alkenyl, -O-C₂₋₁₂ alkynyl, - NH-C₂₋₁₂ alkynyl, -N(C₁₋₅ alkyl)-C₂₋₁₂ alkynyl, -(C₀₋₃ alkylene)-cycloalkyl, -CO-(C₀₋₃ alkylene)-cycloalkyl, -(C₀₋₃ alkylene)-CO-cycloalkyl, -CONH-(C₀₋₃ alkylene)-cycloalkyl, (C₀₋₃ alkylene)-CONH-cycloalkyl, -NHCO-(C_{0.3} alkylene)-cycloalkyl, -(C₀₋₃ alkylene)-NHCO-cycloalkyl, -NH-(C₀₋₃ alkylene)-cycloalkyl, -(C₀₋₃ alkylene)-NH-cycloalkyl, -O-(C₀₋₃ alkylene)-cycloalkyl, -(C₀₋₃ alkylene)-O-cycloalkyl, -SO₂-(C₀₋₃ alkylene)-cycloalkyl, -(C₀₋₃ alkylene)-SO₂-cycloalkyl, -CONH-cycloalkyl, - NHCO-cycloalkyl, -NH-cycloalkyl, -O-cycloalkyl, -CO-cycloalkyl, -SO₂-cycloalkyl, -(C₀₋₃ alkylene)-heterocycloalkyl, -CO-(C₀₋₃ alkylene)-heterocycloalkyl, -(C₀₋₃ alkylene)-CO-heterocycloalkyl, - CONH-(C₀₋₃ alkylene)-heterocycloalkyl, -(C₀₋₃ alkylene)-CONH-heterocycloalkyl, -NHCO-(C₀₋₃ alkylene)-heterocycloalkyl, -(C₀₋₃ alkylene)-NHCO-heterocycloalkyl, -NH-(C₀₋₃ alkylene)-heterocycloalkyl, -(C₀₋₃ alkylene)-NH-heterocycloalkyl, -O-(C₀₋₃ alkylene)-heterocycloalkyl, -(C₀₋₃ alkylene)-O-cycloalkyl, -SO₂-(C₀₋₃ alkylene)-heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)-aryl, -CO-(C₀₋₃ alkylene)-aryl, -(C₀₋₃ alkylene)-CO-aryl, -CONH-(C₀₋₃ alkylene)-aryl, -(C₀₋₃ alkylene)-CONH-aryl, - NHCO-(C₀₋₃ alkylene)-aryl, -(C₀₋₃ alkylene)-NHCO-aryl, -NH-(C₀₋₃ alkylene)-aryl, -(C₀₋₃ alkylene)-NH-aryl, -O-(C₀₋₃ alkylene)-aryl, -(C₀₋₃ alkylene)-O-aryl, -SO₂-(C₀₋₃ alkylene)-aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)-heteroaryl, -CO-(C₀₋₃ alkylene)-heteroaryl, -(C₀₋₃ alkylene)-CO-heteroaryl, -CONH-(C₀₋₃ alkylene)-heteroaryl, -(C₀₋₃ alkylene)-CONH-heteroaryl, -NHCO-(C₀₋₃ alkylene)-heteroaryl, -(C₀₋₃ alkylene)-NHCO-heteroaryl, -NH-(C₀₋₃ alkylene)-heteroaryl, -(C₀₋₃ alkylene)-NH-heteroaryl, -O-(C₀₋₃ alkylene)-heteroaryl, -(C₀₋₃ alkylene)-O-heteroaryl, -SO₂-(C₀₋₃ alkylene)-heteroaryl, -(C₀₋₃ alkylene)-SO₂-heteroaryl, -CONH-heteroaryl, -NHCO-heteroaryl, -NH-heteroaryl, -O-heteroaryl, - CO-heteroaryl and -SO₂-heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl), and -P(O)(O(C₁₋₅ alkyl))(C₁₋₅ alkyl), preferably selected from halogen, -CN, -OH, -O(C₁₋₅ alkyl), -O(C₁₋₅ haloalkyl), -C₁₋₅ haloalkyl, -SH, -S(C₁₋₅ alkyl), -S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl) and wherein said cycloalkyl, heterocycloalkyl, aryl or heteroaryl is optionally substituted with one or more groups independently selected from halogen, -CN, -OH, C₁₋₆ alkyl, C₁₋₆ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), - (C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), - (C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), - (C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₆ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
10. The compound of item 8 or 9, wherein -Y_{C2}-R_{C2} is selected from -(C₀₋₃ alkylene)-heterocycloalkyl, - CO-(C₀₋₃ alkylene)-heterocycloalkyl, -(C₀₋₃ alkylene)-CO-heterocycloalkyl, -CONH-(C₀₋₃ alkylene)-heterocycloalkyl, -(C₀₋₃ alkylene)-CONH-heterocycloalkyl, -NHCO-(C₀₋₃ alkylene)-heterocycloalkyl, -(C₀₋₃ alkylene)-NHCO-heterocycloalkyl, -NH-(C₀₋₃ alkylene)-heterocycloalkyl, -(C₀₋₃ alkylene)-NH-heterocycloalkyl, -O-(C₀₋₃ alkylene)-heterocycloalkyl, -(C₀₋₃ alkylene)-O-cycloalkyl, -SO₂-(C₀₋₃ alkylene)-heterocycloalkyl, -(C₀₋₃ alkylene)-SO₂-heterocycloalkyl, -CONH-heterocycloalkyl, - NHCO-heterocycloalkyl, -NH-heterocycloalkyl, -O-heterocycloalkyl, -CO-heterocycloalkyl, -SO₂-heterocycloalkyl, -(C₀₋₃ alkylene)-aryl, -CO-(C₀₋₃ alkylene)-aryl, -(C₀₋₃ alkylene)-CO-aryl, -CONH-(C₀₋₃ alkylene)-aryl, -(C₀₋₃ alkylene)-CONH-aryl, -NHCO-(C₀₋₃ alkylene)-aryl, -(C₀₋₃ alkylene)-NHCO-aryl, -NH-(C₀₋₃ alkylene)-aryl, -(C₀₋₃ alkylene)-NH-aryl, -O-(C₀₋₃ alkylene)-aryl, -(C₀₋₃ alkylene)-O-aryl, -SO₂-(C₀₋₃ alkylene)-aryl, -(C₀₋₃ alkylene)-SO₂-aryl, -CONH-aryl, -NHCO-aryl, - NH-aryl, -O-aryl, -CO-aryl, -SO₂-aryl, -(C₀₋₃ alkylene)-heteroaryl, -CO-(C₀₋₃ alkylene)-heteroaryl, - (C₀₋₃ alkylene)-CO-heteroaryl, -CONH-(C₀₋₃ alkylene)-heteroaryl, -(C₀₋₃ alkylene)-CONH-heteroaryl, -NHCO-(C₀₋₃ alkylene)-heteroaryl, -(C₀₋₃ alkylene)-NHCO-heteroaryl, -NH-(C₀₋₃ alkylene)-heteroaryl, -(C₀₋₃ alkylene)-NH-heteroaryl, -O-(C₀₋₃ alkylene)-heteroaryl, -(C₀₋₃ alkylene)-O-heteroaryl, -SO₂-(C₀₋₃ alkylene)-heteroaryl, -(C₀₋₃ alkylene)-SO₂-heteroaryl, -CONH-heteroaryl, - NHCO-heteroaryl, -NH-heteroaryl, -O-heteroaryl, -CO-heteroaryl and -SO₂-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), - (C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), - (C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₆ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), - S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), - (C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
11. The compound of any one of items 8 to 10, wherein -Y_{C2}-R_{C2} is selected from -(C₀₋₃ alkylene)-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)aryl, -CONH-aryl, - NHCO-aryl, -NH-aryl, -O-aryl, -CO-aryl, -SO₂-aryl, -(C₀₋₃ alkylene)-heteroaryl, -CONH-heteroaryl, - NHCO-heteroaryl, -NH-heteroaryl, -O-heteroaryl, -CO-heteroaryl and -SO₂-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), - (C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), - (C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₆ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), - S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₆ alkyl), -NH(C₁₋₆ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), - (C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₆ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
12. The compound of any one of claims 8 to 11, wherein -Y_{C2}-R_{C2} is selected from -(C₀₋₃ alkylene)-heterocycloalkyl, -(C₀₋₃ alkylene)-aryl, and -(C₀₋₃ alkylene)-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
13. The compound of any one of items 8 to 12, wherein -Y_{C2}-R_{C2} is selected from heterocycloalkyl, aryl, and heteroaryl, preferably heterocycloalkyl and heteroaryl, more preferably heterocycloalkyl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
14. The compound of any one of items 8 to 13, wherein -Y_{C2}-R_{C2} is optionally substituted aryl, preferably -Y_{C2}-R_{C2} is phenyl, optionally substituted with one or more groups independently selected from halogen, -CN, -OH, C₁₋₅ alkyl, **C₁₋₅** haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
15. The compound of any one of items 8 to 13, wherein -Y_{C2}-R_{C2} is an optionally substituted heteroaryl, preferably wherein -Y_{C2}-R_{C2} is imidazolyl, pyridazinyl, thiazolyl, pyridinyl, pyrimidinyl, pyrazinyl, or indazolyl, wherein heteroaryl is optionally substituted with one or more groups independently selected from halogen, -CN, -OH, C₁₋₅ alkyl, **C₁₋₅** haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
16. The compound of any one of items 8 to 13, wherein -Y_{C2}-R_{C2} is optionally substituted heterocycloalkyl, preferably wherein -Y_{C2}-R_{C2} is morpholinyl, 1,1-dioxothiomorpholinyl, azetinyl, pyrrolidinyl, piperidinyl, 6-oxo-1,6- dihydropyridinyl, or piperazinyl, wherein heterocycloalkyl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
17. The compound of item 16, wherein -Y_{C2}-R_{C2} is piperazinyl, optionally substituted with one or more groups independently selected from halogen, -CN, -OH, C₁₋₅ alkyl, C₁₋₅ haloalkyl, -O(C₁₋₅ alkyl), - O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), preferably wherein -Y_{C2}-R_{C2} is piperazinyl (preferably N-piperazinyl) optionally substituted (preferably N-substituted) with CO(C₁₋₅ alkyl), -CONH₂, -CONH(C₁₋₅ alkyl), and -CON(C₁₋₅ alkyl)(C₁₋₅ alkyl), more preferably wherein -Y_{C2}-R_{C2} is piperazinyl (preferably N-piperazinyl) substituted (preferably N-substituted, preferably at a different N-atom than that attached to the ring system as shown in formula (I)), with -CON(C₁₋₅ alkyl)(C₁₋₅ alkyl), preferably with -CON(CH₃)₂.
18. The compound of item 16, wherein -Y_{C2}-R_{C2} is 2-oxaspiro[3.5]non-6-en-7-yl, 2-oxaspiro[3.5]non-7-yl, 2-oxa-8-azaspiro[4.5]dec-8-yl, 9-oxa-3-azaspiro[5.5]undec-3-yl, 2-oxa-6-azaspiro[3.4]oct-6-yl, 1-oxa-7-azaspiro[3.5]non-7-yl, 1-oxa-8-azaspiro[4.5]dec-8-yl, 6-oxa-2-azaspiro[3.3]hept-2-yl, 2,8-diazaspiro[4.5]dec-8-yl, 7-oxa-3-azabicyclo[3.3.0]oct-3-yl, 8-oxa-3-azabicyclo[4.3.0]non-3-yl, 2-oxa-6-azaspiro[3.5]non-6-yl, 7-oxo-3,6,8-triazabicyclo[4.3.0]non-3-yl, 3-pyrrolino[3,4-c]pyrazol-2-yl, 3,6- diazabicyclo[3.1.1]hept-3-yl, or 2,7-diazaspiro[3.5]non-7-yl.
19. The compound of any one of items 1 to 16, wherein X₄ is C-R_{C4}, wherein R_{C4} is selected from hydrogen, halo, C₁₋₆ alkyl, C₂₋₆ alkynyl, -O-C₁₋₆ alkyl, -S-C₁₋₆ alkyl, -NH-C₁₋₆ alkyl, C₁₋₆ haloalkyl, - (C₀₋₃ alkylene)-cycloalkyl, -(C₀₋₃ alkylene)-heterocycloalkyl, -(C₀₋₃ alkylene)-aryl and -(C₀₋₃ alkylene)-heteroaryl, wherein said alkyl is optionally substituted with one or more groups independently selected from halogen, -CN, -OH, -O(C₁₋₅ alkyl), -O(C₁₋₅ haloalkyl), -SH, -S(C₁₋₅ alkyl), -O(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, **C₁₋₅** alkyl, **C₁₋₅** haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
20. The compound of item 19, wherein R_{C4} is selected from hydrogen, halo, C₁₋₆ alkyl, C₂₋₆ alkynyl, -O-C₁₋₆ alkyl, -S-C₁₋₆ alkyl, -NH-C₁₋₆ alkyl, and C₁₋₆ haloalkyl, preferably wherein R_{C4} is selected from hydrogen, halo, C₁₋₂ alkyl, and C₂₋₃ alkynyl, more preferably wherein R_{C4} is selected from hydrogen, halo, and C₁₋₂ alkyl, even more preferably wherein R_{C4} is hydrogen or halo.
21. The compound of item 19, wherein R_{C4} is selected from -(C₀₋₃ alkylene)-cycloalkyl, -(C₀₋₃ alkylene)-heterocycloalkyl, -(C₀₋₃ alkylene)-aryl and -(C₀₋₃ alkylene)-heteroaryl, wherein said cycloalkyl, heterocycloalkyl, aryl or heteroaryl is optionally substituted with one or more groups independently selected from halogen, -CN, -OH, C₁₋₅ alkyl, **C₁₋₅** haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
22. The compound of item 19 or 21, wherein R_{C4} is selected from -(C₀₋₃ alkylene)-cycloalkyl, -(C₀₋₃ alkylene)-heterocycloalkyl, and -(C₀₋₃ alkylene)-heteroaryl, preferably wherein R_{C4} is selected from cycloalkyl, heterocycloalkyl, and heteroaryl, wherein said cycloalkyl, heterocycloalkyl, aryl or heteroaryl is optionally substituted with one or more groups independently selected from halogen, -CN, -OH, C₁₋₅ alkyl, **C₁₋₅** haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -NH(C₁₋₅ haloalkyl)(C₁₋₅ alkyl), -CONH₂, -CONH(C₁₋₅ alkyl), and -CON(C₁₋₅ alkyl)(C₁₋₅ alkyl).
23. The compound of item 21, wherein R_{C4} is selected from heterocycloalkyl and heteroaryl, wherein said heterocycloalkyl or heteroaryl is optionally substituted with one or more groups independently selected from halogen, -CN, -OH, C₁₋₅ alkyl, **C₁₋₅** haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -NH(C₁₋₅ haloalkyl)(C₁₋₅ alkyl), -CONH₂, -CONH(C₁₋₅ alkyl), and -CON(C₁₋₅ alkyl)(C₁₋₅ alkyl).
24. The compound of item 19, wherein R_{C4} is selected from -CH₂-cycloalkyl, -CH₂-heterocycloalkyl, - CH₂-aryl and -CH₂-heteroaryl, more preferably wherein R_{C4} is selected from -CH₂-heterocycloalkyl, and -CH₂-heteroaryl, wherein said cycloalkyl, heterocycloalkyl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
25. The compound of item 23, wherein R_{C4} is heteroaryl, preferably wherein R_{C4} is imidazolyl, pyridazinyl, thiazolyl, pyridinyl, pyrimidinyl, pyrazinyl, or indazolyl, wherein said heteroaryl may be optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
26. The compound of item 23, wherein R_{C4} is heterocycloalkyl, preferably wherein R_{C4} is morpholinyl, 1,1-dioxothiomorpholinyl, azetinyl, pyrrolidinyl, piperidinyl, 6-oxo-1,6- dihydropyridinyl, or piperazinyl, wherein said heterocycloalkyl is optionally substituted with one or more groups independently selected from halogen, -CN, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CONH₂, -CONH(C₁₋₅ alkyl), and -CON(C₁₋₅ alkyl)(C₁₋₅ alkyl).
27. The compound of item 26, wherein R_{C4} is piperazinyl, preferably wherein R_{C4} is piperazinyl (preferably N-piperazinyl) substituted (preferably N-substituted, preferably at a different N-atom than that attached to the ring system shown in formula (I)), with -CON(C₁₋₅ alkyl)(C₁₋₅ alkyl), preferably with -CON(CH₃)₂.
28. The compound of item 23, wherein R_{C4} is heterocycloalkyl, preferably wherein R_{C4} is 2-oxaspiro[3.5]non-6-en-7-yl, 2-oxaspiro[3.5]non-7-yl, 2-oxa-8-azaspiro[4.5]dec-8-yl, 9-oxa-3-azaspiro[5.5]undec-3-yl, 2-oxa-6-azaspiro[3.4]oct-6-yl, 1-oxa-7-azaspiro[3.5]non-7-yl, 1-oxa-8-azaspiro[4.5]dec-8-yl, 6-oxa-2-azaspiro[3.3]hept-2-yl, 2,8-diazaspiro[4.5]dec-8-yl, 7-oxa-3-azabicyclo[3.3.0]oct-3-yl, 8-oxa-3-azabicyclo[4.3.0]non-3-yl, 2-oxa-6-azaspiro[3.5]non-6-yl, 7-oxo-3,6,8-triazabicyclo[4.3.0]non-3-yl, 3-pyrrolino[3,4-c]pyrazol-2-yl, 3,6-diazabicyclo[3.1.1]hept-3-yl, or 2,7-diazaspiro[3.5]non-7-yl.
29. The compound of any one of items 1 to 28, wherein if X₂ comprises cycloalkyl, heterocycloalkyl, aryl or heteroaryl, X₄ is C-R_{C4} wherein R_{C4} is selected from hydrogen, halo, C₁₋₆ alkyl, -O-C₁₋₆ alkyl, -S-C₁₋₆ alkyl, -NH-C₁₋₆ alkyl, and C₁₋₆ haloalkyl; preferably wherein R_{C4} is selected from hydrogen, and halo.
30. The compound of any one of items 1 to 28, wherein if X₄ comprises cycloalkyl, heterocycloalkyl, aryl or heteroaryl, X₂ does not comprise any of the groups selected from cycloalkyl, heterocycloalkyl, aryl and heteroaryl.
31. The compound of any one of items 1 to 28, wherein if X₂ comprises cycloalkyl, heterocycloalkyl, aryl or heteroaryl and X₄ comprises cycloalkyl, heterocycloalkyl, aryl or heteroaryl, then together R_{C4} and -Y_{C2}-R_{C2} include not more than 12 non-hydrogen atoms, preferably not more than 10 non-hydrogen atoms.
32. The compound of any one of items 1 to 31, wherein X₅ is C-R_{C5}, wherein R_{C5} is selected from hydrogen, halo, C₁₋₃ alkyl, -O-C₁₋₃ alkyl, -S-C₁₋₃ alkyl, -NH-C₁₋₃ alkyl, and C₁₋₃ haloalkyl, preferably, wherein R_{C5} is selected from hydrogen, halo, C₁₋₃ alkyl, and C₁₋₃ haloalkyl.
33. The compound of any one of items 1 to 32, wherein Y_{R5} is selected from a covalent bond, C₁₋₂ alkylene, -CO-(C₁₋₂ alkylene), -(C₁₋₂ alkylene)-CO-, -CONH-(C₁₋₂ alkylene)-, -(C₁₋₂ alkylene)-CONH-, -NHCO-(C₁₋₂ alkylene)-, -(C₁₋₂ alkylene)-NHCO-, -NH-(C₁₋₂ alkylene)-, -(C₁₋₂ alkylene)-NH-, -O-(C₁₋₂ alkylene)-, -(C₁₋₂ alkylene)-O-, -SO₂-(C₁₋₂ alkylene)-, -(C₁₋₂ alkylene)-SO₂-, -CONH-, -CON(C₁₋₅ alkyl)-, -NHCO-, -N(C₁₋₅ alkyl)CO-, -NH-, -O-, -CO-, -COO- and -SO₂-.
34. The compound of any one of items 1 to 33, wherein R_{R5} is selected from cycloalkyl, heterocycloalkyl, aryl, and heteroaryl, preferably wherein R_{R5} is selected from heterocycloalkyl, aryl, and heteroaryl, more preferably wherein R_{R5} is selected from aryl and heteroaryl, most preferably wherein R_{R5} is heteroaryl wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
35. The compound of any one of items 1 to 34, wherein Y_{R5} is selected from a covalent bond, C₁₋₂ alkylene, -CO-(C₁₋₂ alkylene)-, -(C₁₋₂ alkylene)-CO-, -CONH-(C₁₋₂ alkylene)-, -(C₁₋₂ alkylene)-CONH-, -NHCO-(C₁₋₂ alkylene), -(C₁₋₂ alkylene)-NHCO-, -NH-(C₁₋₂ alkylene), -(C₁₋₂ alkylene)-NH-, -O-(C₁₋₂ alkylene), -(C₁₋₂ alkylene)-O-, -SO₂-(C₁₋₂ alkylene)-, -(C₁₋₂ alkylene)-SO₂-, -CONH-, - NHCO-, -NH-, -O-, -CO- and -SO₂-.
36. The compound of item 34 or 35, wherein R₄ is selected from -(C₀₋₂ alkylene)-cycloalkyl, -CO-(C₀₋₂ alkylene)-cycloalkyl, -(C₀₋₂ alkylene)-CO-cycloalkyl, -CONH-(C₀₋₂ alkylene)-cycloalkyl, -(C₀₋₂ alkylene)-CONH-cycloalkyl, -NHCO-(C₀₋₂ alkylene)-cycloalkyl, -(C₀₋₂ alkylene)-NHCO-cycloalkyl, - NH-(C₀₋₂ alkylene)-cycloalkyl, -(C₀₋₂ alkylene)-NH-cycloalkyl, -O-(C₀₋₂ alkylene)-cycloalkyl, -(C₀₋₂ alkylene)-O-cycloalkyl, -SO₂-(C₀₋₂ alkylene)-cycloalkyl, -(C₀₋₂ alkylene)-SO₂-cycloalkyl, -CONH- cycloalkyl, -NHCO-cycloalkyl, -NH-cycloalkyl, -O-cycloalkyl, -CO-cycloalkyl, -SO₂-cycloalkyl, -(C₀₋₂ alkylene)-heterocycloalkyl, -CO-(C₀₋₂ alkylene)-heterocycloalkyl, -(C₀₋₂ alkylene)-CO-heterocycloalkyl, -CONH-(C₀₋₂ alkylene)-heterocycloalkyl, -(C₀₋₂ alkylene)-CONH-heterocycloalkyl, -NHCO-(C₀₋₂ alkylene)-heterocycloalkyl, -(C₀₋₂ alkylene)-NHCO-heterocycloalkyl, -NH-(C₀₋₂ alkylene)-heterocycloalkyl, -(C₀₋₂ alkylene)-NH-heterocycloalkyl, -O-(C₀₋₂ alkylene)-heterocycloalkyl, -(C₀₋₂ alkylene)-O-heterocycloalkyl, -SO₂-(C₀₋₂ alkylene)-heterocycloalkyl, -(C₀₋₂ alkylene)-SO₂-heterocycloalkyl, -CONH-heterocycloalkyl, -NHCO-heterocycloalkyl, -NH- heterocycloalkyl, -O-heterocycloalkyl, -CO-heterocycloalkyl, -SO₂-heterocycloalkyl, -(C₀₋₂ alkylene)-aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, -SO₂-(C₀₋₂ alkylene)-aryl, -(C₀₋₂ alkylene)-SO₂-aryl, -CONH-aryl, -NHCO-aryl, -NH-aryl, -O-aryl, -CO-aryl, -SO₂-aryl, - (C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, - (C₀₋₂ alkylene)-NHCO-heteroaryl, -NH-(C₀₋₂ alkylene)heteroaryl, -(C₀₋₂ alkylene)-NH-heteroaryl, -O-(C₀₋₂ alkylene)heteroaryl, -(C₀₋₂ alkylene)-O-heteroaryl, -SO₂-(C₀₋₂ alkylene)heteroaryl, -(C₀₋₂ alkylene)-SO₂-heteroaryl, -CONH-heteroaryl, -NHCO-heteroaryl, -NH-heteroaryl, -O-heteroaryl, - CO-heteroaryl, and -SO₂-heteroaryl, wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
37. The compound of item 36, wherein R₄ is selected from -(C₀₋₂ alkylene)aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, -SO₂-(C₀₋₂ alkylene)-aryl, -(C₀₋₂ alkylene)-SO₂-aryl, - CONH-aryl, -NHCO-aryl, -NH-aryl, -O-aryl, -CO-aryl, -SO₂-aryl, -(C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, - NH-(C₀₋₂ alkylene)-heteroaryl, -(C₀₋₂ alkylene)-NH-heteroaryl, -O-(C₀₋₂ alkylene)-heteroaryl, -(C₀₋₂ alkylene)-O-heteroaryl, -SO₂-(C₀₋₂ alkylene)-heteroaryl, -(C₀₋₂ alkylene)-SO₂-heteroaryl, -CONH-heteroaryl, -NHCO-heteroaryl, -NH-heteroaryl, -O-heteroaryl, -CO-heteroaryl, and -SO₂-heteroaryl, wherein said aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
38. The compound of any one of items 1 to 32, wherein R₄ is selected from C₁₋₁₂ alkyl, C₂₋₁₂ alkenyl, C₂₋₁₂ alkynyl, cycloalkyl, heterocycloalkyl, aryl, and heteroaryl, preferably wherein R₄ is selected from cycloalkyl, heterocycloalkyl, aryl, and heteroaryl, more preferably wherein R₄ is selected from aryl, and heteroaryl, even more preferably wherein R₄ is heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, C₁₋₅ alkyl, **C₁₋₅** haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
39. The compound of item 37 or 38, wherein R₄ is a five membered heteroaryl, optionally substituted with one or more groups independently selected from halogen, -CN, -OH, **C₁₋₅** alkyl, **C₁₋₅** haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
40. The compound of item 39, wherein the five membered heteroaryl is selected from imidazolyl, isoxazolyl, pyrazolyl, 1,2,3-triazolyl, 1,2,4-triazolyl, thiazolyl, 1,2,4-oxadiazolyl, 1,3,4-oxadiazolyl, 1,2,4-thiadiazolyl, or 1,3,4-thiadiazolyl, preferably wherein the five membered heteroaryl is 1,2,4-thiadiazolyl, optionally substituted with one or more groups independently selected from halogen, -CN, -OH, C₁₋₅ alkyl, C₁₋₅ haloalkyl, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CONH₂, -CONH(C₁₋₅ alkyl), and -CON(C₁₋₅ alkyl)(C₁₋₅ alkyl), preferably optionally substituted with C₁₋₅ alkyl, **C₁₋₅** haloalkyl, -O(C₁₋₅ alkyl), -SH, -S(C₁₋₅ alkyl), more preferably optionally substituted with C₁₋₅ alkyl, **C₁₋₅** haloalkyl, even more preferably optionally substituted with C₁₋₅ haloalkyl, preferably selected from -CH₂F, -CHF₂ and CF₃, most preferably optionally substituted with -CHF₂.
41. A pharmaceutical composition comprising the compound of any one of items 1 to 40 or a pharmaceutically acceptable salt, hydrate or solvate thereof, and a pharmaceutically acceptable carrier.
42. The compound of any one of items 1 to 40 or a pharmaceutically acceptable salt, hydrate or solvate thereof, or a pharmaceutical composition of item 41, for use in therapy.
43. The compound for use or the pharmaceutical composition for use of item 42, for use in a method of treating a disease or disorder in which PARG activity is implicated.
44. The compound for use or the pharmaceutical composition for use of item 42, for use in a method of treating a proliferative disorder.
45. The compound for use of the pharmaceutical composition for use of item 44, wherein the proliferative disorder is cancer, preferably a human cancer.

Further examples and/or embodiments of the present disclosure are disclosed in the following numbered paragraphs.

The scope of the claimed invention is defined by the claims. The following embodiments refer to the disclosure of the present description, and not necessarily to the claimed invention.
1. A compound of formula (I): 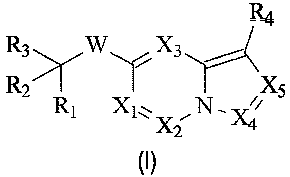 or an enantiomer, diastereoisomer, tautomer, pharmaceutically acceptable solvate, pharmaceutically acceptable crystal **form** or pharmaceutically acceptable salt thereof,
   wherein:
   R₁ is selected from the group consisting of hydrogen, chloro, fluoro, cyano, formyl, (C₁₋₂)alkyl, (C₂)alkenyl, (C₂)alkynyl and (C₁₋₂)haloalkyl;
   R₂ and R₃ are independently each (C₁₋₂)alkyl or (C₁₋₂)haloalkyl, or R₂ and R₃ together with the carbon atom to which they are attached form cyclopropyl;
   W is selected from -NHS(O)_{y}-, -S(O)_{y}NH-, -NHS(O)(NH)-, -NHS(O)(NCH₃)-, -S(O)(NH)-NH-, - S(O)(NCH₃)-NH-, wherein y is 1 or 2;
   X₁ and X₃ are independently selected from the group consisting of N, CH, and CF;
   X₂ is N or C-Y_{C2}-R_{C2},
      wherein Y_{C2} is selected from a covalent bond, C₁₋₅ alkylene, C₂₋₅ alkenylene, and C₂₋₅ alkynylene, wherein said alkylene, said alkenylene and said alkynylene are each optionally substituted with one or more groups independently selected from halogen, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more -CH₂- units comprised in said alkylene, said alkenylene or said alkynylene are each optionally replaced by a group independently selected from -O-, -NH- , -N(C₁₋₅ alkyl)-, -CO-, -S-, -SO-, and -SO₂-, and
      wherein R_{C2} is selected from hydrogen, halo, -OH, -NH₂, -SH, -CN, C₁₋₁₂ alkyl, C₂₋₁₂ alkenyl, C₂₋₁₂ alkynyl, cycloalkyl, heterocycloalkyl, aryl, and heteroaryl,
         wherein said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), and
         wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C_{1- 5} alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), - (C₁₋₅ alkylene)-CON(C₁₋₅ alkyl)(C₁₋₅ alkyl), -(C₁₋₅ alkylene)-CO-(N-heterocycloalkyl), - **(C₁₋₅** alkylene)-NHCO-(C₁₋₅ alkyl), -(C₁₋₅ alkylene)-N(C₁₋₅ **alkyl)-CO-(C₁₋₅** alkyl), -(C₁₋₅ alkylene)-NHCONH₂, -(C₁₋₅ alkylene)-NHCONH-(C₁₋₅ alkyl), **-(C₁₋₅** alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), **-CO(C₁₋₅** alkyl), -CONH₂, -CONH(C₁₋₅ alkyl), and -CON(C₁₋₅ alkyl)(C₁₋₅ alkyl);
   X₄ is N or C-R_{C4},
      wherein R_{C4} is selected from hydrogen, halo, C₁₋₆ alkyl, C₂₋₆ alkynyl, -O(C₁₋₆ alkyl), -S(C₁₋₆ alkyl), -NH(C₁₋₆ alkyl), -N(C₁₋₆ alkyl)(C₁₋₆ alkyl), -CO(C₁₋₆ alkyl), C₁₋₆ haloalkyl, -O(C₁₋₆ haloalkyl), -S(C₁₋₅ haloalkyl), -NH(C₁₋₆ haloalkyl), -N(C₁₋₆ haloalkyl)₂, -CO-(C₁₋₆ haloalkyl), - (C₀₋₃ alkylene)-cycloalkyl, -O-(C₀₋₃ alkylene)-cycloalkyl, -CO-(C₀₋₃ alkylene)-cycloalkyl, -(C₀₋₃ alkylene)-heterocycloalkyl, -O-(C₀₋₃ alkylene)-heterocycloalkyl, -CO-(C₀₋₃ alkylene)-heterocycloalkyl, -(C₀₋₃ alkylene)-aryl, -O-(C₀₋₃ alkylene)-aryl, -CO-(C₀₋₃ alkylene)-aryl, -(C₀₋₃ alkylene)-heteroaryl, -O-(C₀₋₃ alkylene)-heteroaryl and -CO-(C₀₋₃ alkylene)-heteroaryl,
      wherein said alkyl is optionally substituted with one or more groups independently selected from halogen, -CN, -OH, -O(C₁₋₅ alkyl), -O(C₁₋₅ haloalkyl), **C₁₋₅** haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), **-CO(C₁₋₅** alkyl), -CONH₂, -CONH(C₁-_{S} alkyl), -CON(C₁₋₅ alkyl)(C₁₋₅ alkyl), -CO-(N-heterocycloalkyl), -NHCO-(C₁₋₅ alkyl), -N(C₁₋₅ **alkyl)-CO-(C₁₋₅** alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁-₅ alkyl), and -CON(C₁₋₅ alkyl)(C₁₋₅ alkyl), and
      wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl);
   X₅ is N or C-R_{C5},
      wherein R_{C5} is selected from hydrogen, halo, C₁₋₆ alkyl, -O(C₁₋₆ alkyl), -S(C₁₋₆ alkyl), -NH(C₁₋₆ alkyl), -N(C₁₋₆ alkyl)(C₁₋₆ alkyl) and C₁₋₆ haloalkyl;
   R₄ is Y_{R5}-R_{R5},
      wherein Y_{R5} is selected from a covalent bond, C₁₋₄ alkylene, C₂₋₄ alkenylene, and C₂₋₄ alkynylene, wherein said alkylene, said alkenylene and said alkynylene are each optionally substituted with one or more groups independently selected from halogen, -CN, -OH, -O(C₁₋₅ alkyl), -O(C₁₋₅ haloalkyl)-SH, -S(C₁₋₅ alkyl), -S(C₁₋₅ haloalkyl)-NH₂, -NH(C₁₋₅ alkyl), -NH(C₁₋₅ haloalkyl), -N(C₁₋₅ alkyl)(C₁₋₅ alkyl), and -N(C₁₋₅ haloalkyl)(C₁₋₅ alkyl) and further wherein one or more -CH₂- units comprised in said alkylene, said alkenylene or said alkynylene are each optionally replaced by a group independently selected from -O-, -NH-, -N(C₁₋₅ alkyl)-, -CO-, -COO-, -S-, -SO-, and -SO₂-, and
      wherein R_{R5} is selected from C₁₋₁₂ alkyl, C₁₋₁₂ alkenyl, C₂₋₁₂ alkynyl, cycloalkyl, heterocycloalkyl, aryl, and heteroaryl,
         wherein said alkyl, alkenyl, or alkynyl is optionally substituted with one or more groups independently selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
2. The compound of paragraph 1, wherein R₁ is cyano.
3. The compound of paragraph 1 or 2, wherein R₂ and R₃ together with the carbon atom to which they are attached form cyclopropyl.
4. The compound of any one of paragraphs 1 to 3, wherein W is -NHS(O)₂-.
5. The compound of any one of paragraphs 1 to 4, wherein X₁ and X₃ are each CH.
6. The compound of any one of paragraphs 1 to 5, wherein X₂ is C-Y_{C2}-R_{C2} , wherein -Y_{C2}-R_{C2} is selected from -O-C₁₋₁₂ alkyl, -NH-C₁₋₁₂ alkyl, -N(C₁₋₅ alkyl)-C₁₋₁₂ alkyl, -O-C₂₋₁₂ alkenyl, -NH-C₂₋₁₂ alkenyl, -N(C₁₋₅ alkyl)-C₂₋₁₂ alkenyl, -O-C₂₋₁₂ alkynyl, -NH-C₂₋₁₂ alkynyl, -N(C₁₋₅ alkyl)-C₂₋₁₂ alkynyl, -(C₀₋₃ alkylene)-cycloalkyl, -CO-(C₀₋₃ alkylene)-cycloalkyl, -(C₀₋₃ alkylene)-CO-cycloalkyl, -CONH-(C₀₋₃ alkylene)-cycloalkyl, (C₀₋₃ alkylene)-CONH-cycloalkyl, -NHCO-(C₀₋₃ alkylene)-cycloalkyl, -(C₀-₃ alkylene)-NHCO-cycloalkyl, -NH-(C₀₋₃ alkylene)-cycloalkyl, -(C₀₋₃ alkylene)-NH-cycloalkyl, -O-(C₀₋₃ alkylene)-cycloalkyl, -(C₀₋₃ alkylene)-O-cycloalkyl, -SO₂-(C₀₋₃ alkylene)-cycloalkyl, -(C₀₋₃ alkylene)-SO₂-cycloalkyl, -CONH-cycloalkyl, -NHCO-cycloalkyl, -NH-cycloalkyl, -O-cycloalkyl, - CO-cycloalkyl, -SO₂-cycloalkyl, -(C₀₋₃ alkylene)-heterocycloalkyl, -CO-(C₀₋₃ alkylene)-heterocycloalkyl, -(C₀₋₃ alkylene)-CO-heterocycloalkyl, -CONH-(C₀₋₃ alkylene)-heterocycloalkyl, - (C₀₋₃ alkylene)-CONH-heterocycloalkyl, -NHCO-(C₀₋₃ alkylene)-heterocycloalkyl, -(C₀₋₃ alkylene)-NHCO-heterocycloalkyl, -NH-(C₀₋₃ alkylene)-heterocycloalkyl, -(C₀₋₃ alkylene)-NH-heterocycloalkyl, -O-(C₀₋₃ alkylene)-heterocycloalkyl, -(C₀₋₃ alkylene)-O-cycloalkyl, -SO₂-(C₀₋₃ alkylene)-heterocycloalkyl, -(C₀₋₃ alkylene)-SO₂-heterocycloalkyl, -CONH-heterocycloalkyl, - NHCO-heterocycloalkyl, -NH-heterocycloalkyl, -O-heterocycloalkyl, -CO-heterocycloalkyl, -SO₂-heterocycloalkyl, -(C₀₋₃ alkylene)-aryl, -CO-(C₀₋₃ alkylene)-aryl, -(C₀₋₃ alkylene)-CO-aryl, -CONH-(C₀₋₃ alkylene)-aryl, -(C₀₋₃ alkylene)-CONH-aryl, -NHCO-(C₀₋₃ alkylene)-aryl, -(C₀₋₃ alkylene)-NHCO-aryl, -NH-(C₀₋₃ alkylene)-aryl, -(C₀₋₃ alkylene)-NH-aryl, -O-(C₀₋₃ alkylene)-aryl, -(C₀₋₃ alkylene)-O-aryl, -SO₂-(C₀₋₃ alkylene)-aryl, -(C₀₋₃ alkylene)-SO₂-aryl, -CONH-aryl, -NHCO-aryl, - NH-aryl, -O-aryl, -CO-aryl, -SO₂-aryl, -(C₀₋₃ alkylene)-heteroaryl, -CO-(C₀₋₃ alkylene)-heteroaryl, - (C₀₋₃ alkylene)-CO-heteroaryl, -CONH-(C₀₋₃ alkylene)-heteroaryl, -(C₀₋₃ alkylene)-CONH-heteroaryl, -NHCO-(C₀₋₃ alkylene)-heteroaryl, -(C₀₋₃ alkylene)-NHCO-heteroaryl, -NH-(C₀₋₃ alkylene)-heteroaryl, -(C₀₋₃ alkylene)-NH-heteroaryl, -O-(C₀₋₃ alkylene)-heteroaryl, -(C₀₋₃ alkylene)-O-heteroaryl, -SO₂-(C₀₋₃ alkylene)-heteroaryl, -(C₀₋₃ alkylene)-SO₂-heteroaryl, -CONH-heteroaryl, - NHCO-heteroaryl, -NH-heteroaryl, -O-heteroaryl, -CO-heteroaryl and -SO₂-heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), -C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl) and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
   preferably wherein -Y_{C2}-R_{C2} is selected from -(C₀₋₃ alkylene)-heterocycloalkyl, -CO-(C₀₋₃ alkylene)-heterocycloalkyl, -(C₀₋₃ alkylene)-CO-heterocycloalkyl, -CONH-(C₀₋₃ alkylene)-heterocycloalkyl, - (C₀₋₃ alkylene)-CONH-heterocycloalkyl, -NHCO-(C₀₋₃ alkylene)-heterocycloalkyl, -(C₀₋₃ alkylene)-NHCO-heterocycloalkyl, -NH-(C₀₋₃ alkylene)-heterocycloalkyl, -(C₀₋₃ alkylene)-NH-heterocycloalkyl, -O-(C₀₋₃ alkylene)-heterocycloalkyl, -(C₀₋₃ alkylene)-O-cycloalkyl, -SO₂-(C₀₋₃ alkylene)-heterocycloalkyl, -(C₀₋₃ alkylene)-SO₂-heterocycloalkyl, -CONH-heterocycloalkyl, - NHCO-heterocycloalkyl, -NH-heterocycloalkyl, -O-heterocycloalkyl, -CO-heterocycloalkyl, -SO₂-heterocycloalkyl, -(C₀₋₃ alkylene)-aryl, -CO-(C₀₋₃ alkylene)-aryl, -(C₀₋₃ alkylene)-CO-aryl, -CONH-(C₀₋₃ alkylene)-aryl, -(C₀₋₃ alkylene)-CONH-aryl, -NHCO-(C₀₋₃ alkylene)-aryl, -(C₀₋₃ alkylene)-NHCO-aryl, -NH-(C₀₋₃ alkylene)-aryl, -(C₀₋₃ alkylene)-NH-aryl, -O-(C₀₋₃ alkylene)-aryl, -(C₀₋₃ alkylene)-O-aryl, -SO₂-(C₀₋₃ alkylene)-aryl, -(C₀₋₃ alkylene)-SO₂-aryl, -CONH-aryl, -NHCO-aryl, - NH-aryl, -O-aryl, -CO-aryl, -SO₂-aryl, -(C₀₋₃ alkylene)-heteroaryl, -CO-(C₀₋₃ alkylene)-heteroaryl, - (C₀₋₃ alkylene)-CO-heteroaryl, -CONH-(C₀₋₃ alkylene)-heteroaryl, -(C₀₋₃ alkylene)-CONH-heteroaryl, -NHCO-(C₀₋₃ alkylene)-heteroaryl, -(C₀₋₃ alkylene)-NHCO-heteroaryl, -NH-(C₀₋₃ alkylene)-heteroaryl, -(C₀₋₃ alkylene)-NH-heteroaryl, -O-(C₀₋₃ alkylene)-heteroaryl, -(C₀₋₃ alkylene)-O-heteroaryl, -SO₂-(C₀₋₃ alkylene)-heteroaryl, -(C₀₋₃ alkylene)-SO₂-heteroaryl, -CONH-heteroaryl, - NHCO-heteroaryl, -NH-heteroaryl, -O-heteroaryl, -CO-heteroaryl and -SO₂-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
   more preferably wherein -Y_{C2}-R_{C2} is selected from -(C₀₋₃ alkylene)-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)aryl, -CONH-aryl, -NHCO-aryl, -NH-aryl, - O-aryl, -CO-aryl, -SO₂-aryl, -(C₀₋₃ alkylene)-heteroaryl, -CONH-heteroaryl, -NHCO-heteroaryl, -NH-heteroaryl, -O-heteroaryl, -CO-heteroaryl and -SO₂-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
   even more preferably wherein -Y_{C2}-R_{C2} is selected from -(C₀₋₃ alkylene)-heterocycloalkyl, -(C₀₋₃ alkylene)-aryl, and -(C₀₋₃ alkylene)-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
   even more preferably wherein -Y_{C2}-R_{C2} is selected from heterocycloalkyl, aryl, and heteroaryl, preferably heterocycloalkyl and heteroaryl, more preferably heterocycloalkyl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
7. The compound of paragraph 6, wherein -Y_{C2}-R_{C2} is optionally substituted aryl, preferably -Y_{C2}-R_{C2} is phenyl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁-₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), or wherein -Y_{C2}-R_{C2} is an optionally substituted heteroaryl, preferably wherein -Y_{C2}-R_{C2} is imidazolyl, pyridazinyl, thiazolyl, pyridinyl, pyrimidinyl, pyrazinyl, or indazolyl, wherein heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), or wherein - Y_{C2}-R_{C2} is optionally substituted heterocycloalkyl, preferably wherein -Y_{C2}-R_{C2} is morpholinyl, 1,1-dioxothiomorpholinyl, azetinyl, pyrrolidinyl, piperidinyl, 6-oxo-1,6- dihydropyridinyl, or piperazinyl, wherein heterocycloalkyl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).
8. The compound of paragraph 6 or 7, wherein -Y_{C2}-R_{C2} is piperazinyl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), preferably wherein -Y_{C2}-R_{C2} is piperazinyl (preferably N-piperazinyl) optionally substituted (preferably N-substituted) with CO(C₁₋₅ alkyl), -CONH₂, -CONH(C₁₋₅ alkyl), and -CON(C₁₋₅ alkyl)(C₁₋₅ alkyl), more preferably wherein -Y_{C2}-R_{C2} is piperazinyl (preferably N-piperazinyl) substituted (preferably N-substituted, preferably at a different N-atom than that attached to the ring system as shown in formula (I)), with -CON(C₁₋₅ alkyl)(C₁₋₅ alkyl), preferably with -CON(CH₃)₂.
9. The compound of any one of paragraphs 1 to 8, wherein X₄ is C-R_{C4}, wherein R_{C4} is selected from hydrogen, halo, C₁₋₆ alkyl, C₂₋₆ alkynyl, -O-C₁₋₆ alkyl, -S-C₁₋₆ alkyl, -NH-C₁₋₆ alkyl, C₁₋₆ haloalkyl, - (C₀₋₃ alkylene)-cycloalkyl, -(C₀₋₃ alkylene)-heterocycloalkyl, -(C₀₋₃ alkylene)-aryl and -(C₀₋₃ alkylene)-heteroaryl, wherein said alkyl is optionally substituted with one or more groups independently selected from halogen, -CN, -OH, -O(C₁₋₅ alkyl), -O(C₁₋₅ haloalkyl), -SH, -S(C₁₋₅ alkyl), -O(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).
10. The compound of paragraph 9, wherein R_{C4} is selected from hydrogen, halo, C₁₋₆ alkyl, C₂₋₆ alkynyl, -O-C₁₋₆ alkyl, -S-C₁₋₆ alkyl, -NH-C₁₋₆ alkyl, and C₁₋₆ haloalkyl,
   preferably wherein R_{C4} is selected from hydrogen, halo, C₁₋₂ alkyl, and C₂₋₃ alkynyl,
   more preferably wherein R_{C4} is selected from hydrogen, halo, and C₁₋₂ alkyl,
   even more preferably wherein R_{C4} is hydrogen or halo.
11. The compound of any one of paragraphs 1 to 10, wherein X₅ is C-Rcs, wherein R_{C5} is selected from hydrogen, halo, C₁₋₃ alkyl, -O-C₁₋₃ alkyl, -S-C₁₋₃ alkyl, -NH-C₁₋₃ alkyl, and C₁₋₃ haloalkyl, preferably, wherein R_{C5} is selected from hydrogen, halo, C₁₋₃ alkyl, and C₁₋₃ haloalkyl.
12. The compound of any one of paragraphs 1 to 11, wherein R₄ is selected from -(C₀₋₂ alkylene)-cycloalkyl, -CO-(C₀₋₂ alkylene)-cycloalkyl, -(C₀₋₂ alkylene)-CO-cycloalkyl, -CONH-(C₀₋₂ alkylene)-cycloalkyl, -(C₀₋₂ alkylene)-CONH-cycloalkyl, -NHCO-(C₀₋₂ alkylene)-cycloalkyl, -(C₀₋₂ alkylene)-NHCO-cycloalkyl, -NH-(C₀₋₂ alkylene)-cycloalkyl, -(C₀₋₂ alkylene)-NH-cycloalkyl, -O-(C₀₋₂ alkylene)-cycloalkyl, -(C₀₋₂ alkylene)-O-cycloalkyl, -SO₂-(C₀₋₂ alkylene)-cycloalkyl, -(C₀₋₂ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, -(C₀₋₂ alkylene)-heterocycloalkyl, -CO-(C₀₋₂ alkylene)-heterocycloalkyl, -(C₀₋₂ alkylene)-CO-heterocycloalkyl, -CONH-(C₀₋₂ alkylene)-heterocycloalkyl, -(C₀₋₂ alkylene)-CONH-heterocycloalkyl, -NHCO-(C₀₋₂ alkylene)-heterocycloalkyl, -(C₀₋₂ alkylene)-NHCO-heterocycloalkyl, -NH-(C₀₋₂ alkylene)-heterocycloalkyl, -(C₀₋₂ alkylene)-NH-heterocycloalkyl, -O-(C₀₋₂ alkylene)-heterocycloalkyl, -(C₀₋₂ alkylene)-O-heterocycloalkyl, -SO₂-(C₀₋₂ alkylene)-heterocycloalkyl, -(C₀₋₂ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₂ alkylene)-aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, -SO₂-(C₀₋₂ alkylene)-aryl, -(C₀₋₂ alkylene)-SO₂-aryl, -CONH-aryl, -NHCO-aryl, - NH-aryl, -O-aryl, -CO-aryl, -SO₂-aryl, -(C₀₋₂ alkylene)-heteroaryl, -CO-(C₀₋₂ alkylene)-heteroaryl, - (C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)heteroaryl, -(C₀₋₂ alkylene)-NH-heteroaryl, -O-(C₀₋₂ alkylene)heteroaryl, -(C₀₋₂ alkylene)-O-heteroaryl, -SO₂-(C₀₋₂ alkylene)heteroaryl, -(C₀₋₂ alkylene)-SO₂-heteroaryl, -CONH-heteroaryl, - NHCO-heteroaryl, -NH-heteroaryl, -O-heteroaryl, -CO-heteroaryl, and -SO₂-heteroaryl, wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
   preferably wherein R₄ is selected from -(C₀₋₂ alkylene)aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, - (C₀₋₂ alkylene)-O-aryl, -SO₂-(C₀₋₂ alkylene)-aryl, -(C₀₋₂ alkylene)-SO₂-aryl, -CONH-aryl, -NHCO-aryl, -NH-aryl, -O-aryl, -CO-aryl, -SO₂-aryl, -(C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)-heteroaryl, -(C₀₋₂ alkylene)-NH-heteroaryl, -O-(C₀₋₂ alkylene)-heteroaryl, -(C₀₋₂ alkylene)-O-heteroaryl, -SO₂-(C₀₋₂ alkylene)-heteroaryl, -(C₀₋₂ alkylene)-SO₂-heteroaryl, -CONH-heteroaryl, - NHCO-heteroaryl, -NH-heteroaryl, -O-heteroaryl, -CO-heteroaryl, and -SO₂-heteroaryl, wherein said aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
   or wherein R₄ is selected from C₁₋₁₂ alkyl, C₂₋₁₂ alkenyl, C₂₋₁₂ alkynyl, cycloalkyl, heterocycloalkyl, aryl, and heteroaryl, preferably wherein R₄ is selected from cycloalkyl, heterocycloalkyl, aryl, and heteroaryl, more preferably wherein R₄ is selected from aryl, and heteroaryl, even more preferably wherein R₄ is heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
   preferably wherein R₄ is a five membered heteroaryl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
   preferably wherein the five membered heteroaryl is selected from imidazolyl, isoxazolyl, pyrazolyl, 1,2,3-triazolyl, 1,2,4-triazolyl, thiazolyl, 1,2,4-oxadiazolyl, 1,3,4-oxadiazolyl, 1,2,4-thiadiazolyl, or 1,3,4-thiadiazolyl, preferably wherein the five membered heteroaryl is 1,2,4-thiadiazolyl, optionally substituted with one or more groups independently selected from halogen, -CN, -OH, C₁₋₅ alkyl, C₁₋₅ haloalkyl, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CONH₂, -CONH(C₁₋₅ alkyl), and -CON(C₁₋₅ alkyl)(C₁₋₅ alkyl), preferably optionally substituted with C₁₋₅ alkyl, C₁₋₅ haloalkyl, -O(C₁₋₅ alkyl), -SH, -S(C₁₋₅ alkyl), more preferably optionally substituted with C₁₋₅ alkyl, C₁₋₅ haloalkyl, even more preferably optionally substituted with C₁₋₅ haloalkyl, preferably selected from -CH₂F, -CHF₂ and CF₃, most preferably optionally substituted with -CHF₂.
13. A pharmaceutical composition comprising the compound of any one of paragraphs 1 to 12 or a pharmaceutically acceptable salt, hydrate or solvate thereof, and a pharmaceutically acceptable carrier.
14. The compound of any one of paragraphs 1 to 12 or a pharmaceutically acceptable salt, hydrate or solvate thereof, or a pharmaceutical composition of paragraph 13, for use in therapy.
15. The compound for use or the pharmaceutical composition for use of paragraph 14, for use in a method of treating a disease or disorder in which PARG activity is implicated,
   or for use in a method of treating a proliferative disorder, preferably wherein the proliferative disorder is cancer, preferably a human cancer.

## Claims

1. A compound of formula (I): 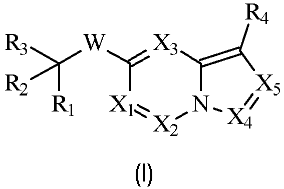 or an enantiomer, diastereoisomer, tautomer, pharmaceutically acceptable solvate, pharmaceutically acceptable crystal form, or pharmaceutically acceptable salt thereof,
wherein:
R₁ is selected from the group consisting of hydrogen, chloro, fluoro, cyano, formyl, (C₁₋₂)alkyl, (C₂)alkenyl, (C₂)alkynyl, (C₁₋₂)haloalkyl, -(C₁₋₂ alkylene)-OH and -(C₁₋₂ alkylene)-O-(C₁₋₂ alkyl);
R₂ and R₃ together with the carbon atom to which they are attached form cyclopropyl;
W is -NHS(O)₂-, wherein the left side of W as defined herein is attached to the carbon atom that carries R₁, R₂ and R₃, and the right side of W as defined herein is attached to the ring system shown in formula (I);
X₁ is CF or CH and X₃ is CH;
X₂ is C-Y_{C2}-R_{C2},
wherein Y_{C2} is selected from a covalent bond, C₁₋₅ alkylene, C₂₋₅ alkenylene, C₂₋₅ alkynylene, cycloalkylene, cycloalkenylene, heterocycloalkylene and heterocycloalkenylene, wherein said alkylene, said alkenylene and said alkynylene are each optionally substituted with one or more groups independently selected from halogen, CN, OH, O(C₁₋₅ alkyl), -O(C₁₋₅ haloalkyl), C₁₋₅ haloalkyl, SH, S(C₁₅ alkyl), -S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(*N*-heterocycloalkyl), -CO(C₁₋₅ alkyl), CONH₂, CONH(C₁₋₅ alkyl), CON(C₁₋₅ alkyl)(C₁₋₅ alkyl), -CO-(*N*-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), and N(C₁₋₅ alkyl)CON(C₁₋₅ alkyl)(C₁₋₅ alkyl), preferably selected from halogen, CN, OH, O(C₁₋₅ alkyl), SH, S(C₁₅ alkyl), NH₂, NH(C₁₋₅ alkyl), and N(C₁₋₅ alkyl)(C₁₋₅ alkyl), and further wherein one or more -CH₂- units comprised in said alkylene, said alkenylene or said alkynylene are each optionally replaced by a group independently selected from -O-, NH-, N(C₁₋₅ alkyl)-, CO-, S-, -SO-, and SO₂-, and further wherein said cycloalkylene, said cycloalkenylene, said heterocycloalkylene and said heterocycloalkenylene are each optionally substituted with one or more groups independently selected from halogen, CN, OH, C₁₋₅ alkyl, C₁₋₅ haloalkyl, O(C₁₋₅ alkyl), -O(C₁₋₅ haloalkyl), SH, S(C₁₅ alkyl), -S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(*N*-heterocycloalkyl), - CO(C₁₋₅ alkyl), CONH₂, CONH(C₁₋₅ alkyl), CON(C₁₋₅ alkyl)(C₁₋₅ alkyl), -CO-(*N*-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), and N(C₁₋₅ alkyl)CON(C₁₋₅ alkyl)(C₁₋₅ alkyl), -(C₁₋₅ alkylene)-CN, -(C₁₋₅ alkylene)OH, -(C₁₋₅ alkylene)O(C₁₋₅ alkyl), -(C₁₋₅ alkylene)-O(C₁₋₅ haloalkyl), -(C₁₋₅ alkylene)SH, -(C₁₋₅ alkylene)S(C₁₅ alkyl), -(C₁₋₅ alkylene)-S(C₁₋₅ haloalkyl), -(C₁₋₅ alkylene)NH₂, -(C₁₋₅ alkylene)NH(C₁₋₅ alkyl), -(C₁₋₅ alkylene)-NH(C₁₋₅ haloalkyl), - (C₁₋₅ alkylene)N(C₁₋₅ alkyl)(C₁₋₅ alkyl), -(C₁₋₅ alkylene)N(C₁₋₅ alkyl)(C₁₋₅ haloalkyl), -(C₁₋₅ alkylene)(*N*-heterocycloalkyl), -(C₁₋₅ alkylene)N(C₁₋₅ haloalkyl)(C₁₋₅ alkyl), -(C₁₋₅ alkylene)-CO(C₁₋₅ alkyl), -(C₁₋₅ alkylene)CONH₂, -(C₁₋₅ alkylene)CONH(C₁₋₅ alkyl), -(C₁₋₅ alkylene)CON(C₁₋₅ alkyl)(C₁₋₅ alkyl), -(C₁₋₅ alkylene)CO-(*N*-heterocycloalkyl), -(C₁₋₅ alkylene)NHCO-(C₁₋₅ alkyl), -(C₁₋₅ alkylene)N(C₁₋₅ alkyl)-CO-(C₁₋₅ alkyl), -(C₁₋₅ alkylene)NHCONH₂, -(C₁₋₅ alkylene)NHCONH-(C₁₋₅ alkyl), -(C₁₋₅ alkylene)NHCON(C₁₋₅ alkyl)(C₁₋₅ alkyl), -(C₁₋₅ alkylene)N(C₁₋₅ alkyl)CONH₂, -(C₁₋₅ alkylene)N(C₁₋₅ alkyl)CONH-(C₁₋₅ alkyl), and -(C₁₋₅ alkylene)N(C₁₋₅ alkyl)CON(C₁₋₅ alkyl)(C₁₋₅ alkyl), preferably selected from halogen, CN, OH, C₁₋₅ alkyl, O(C₁₋₅ alkyl), SH, S(C₁₅ alkyl), NH₂, NH(C₁₋₅ alkyl), and N(C₁₋₅ alkyl)(C₁₋₅ alkyl), and
R_{C2} is selected from hydrogen, halo, -OH, -NH₂, -SH, -CN, C₁₋₁₂ alkyl, C₂₋₁₂ alkenyl, C₂₋₁₂ alkynyl, cycloalkyl, cycloalkenyl, heterocycloalkyl, heterocycloalkenyl, aryl, and heteroaryl,
wherein said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl)-,-O(C₁₋₅ haloalkyl)-, C₁₋₅ haloalkyl, -SH, -S(C₁₋₅ alkyl), -S(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), preferably selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), and
wherein said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N*-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), - (C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -COO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N*-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), - (C₁₋₅ alkylene)-CON(C₁₋₅ alkyl)(C₁₋₅ alkyl), -(C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), - (C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), - (C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), -S(C₁₋₅ haloalkyl), -(C₁₋₅ alkylene)-SH, -(C₁₋₅ alkylene)-S(C₁₋₅ alkyl), - (C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl);
X₄ is N or C-R_{C4},
wherein R_{C4} is selected from hydrogen, halo, C₁₋₆ alkyl, C₂₋₆ alkynyl, -O(C₁₋₆ alkyl), -S(C₁₋₆ alkyl), - NH(C₁₋₆ alkyl), -N(C₁₋₆ alkyl)(C₁₋₆ alkyl), -CO(C₁₋₆ alkyl), C₁₋₆ haloalkyl, -O(C₁₋₆ haloalkyl), -S(C₁₋₆ haloalkyl), -NH(C₁₋₆ haloalkyl), -N(C₁₋₆ haloalkyl)₂, -CO(C₁₋₆ haloalkyl), -(C₀₋₃ alkylene)cycloalkyl, - O-(C₀₋₃ alkylene)-cycloalkyl, -CO-(C₀₋₃ alkylene)-cycloalkyl, -(C₀₋₃ alkylene)cycloalkenyl, -O-(C₀₋₃ alkylene)-cycloalkenyl, -CO-(C₀₋₃ alkylene)-cycloalkenyl, -(C₀₋₃ alkylene)-heterocycloalkyl, -O-(C₀₋₃ alkylene)-heterocycloalkyl, -CO-(C₀₋₃ alkylene)-heterocycloalkyl, -(C₀₋₃ alkylene)-heterocycloalkenyl, -O-(C₀₋₃ alkylene)-heterocycloalkenyl, -CO-(C₀₋₃ alkylene)-heterocycloalkenyl, -(C₀₋₃ alkylene)-aryl, -O-(C₀₋₃ alkylene)-aryl, -CO-(C₀₋₃ alkylene)-aryl, -(C₀₋₃ alkylene)-heteroaryl, - O-(C₀₋₃ alkylene)-heteroaryl and -CO-(C₀₋₃ alkylene)-heteroaryl,
wherein said alkyl or said alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and
wherein said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(*N-*heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), - (C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), (C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl);
X₅ is N or C-R_{C5},
wherein R_{C5} is selected from hydrogen, halo, C₁₋₆ alkyl, -O(C₁₋₆ alkyl), -S(C₁₋₆ alkyl), -NH(C₁₋₆ alkyl), -N(C₁₋₆ alkyl)(C₁₋₆ alkyl) and C₁₋₆ haloalkyl;
R₄ is Y_{R5}-R_{R5},
wherein Y_{R5} is selected from a covalent bond, C₁₋₄ alkylene, C₂₋₄ alkenylene, and C₂₋₄ alkynylene, wherein said alkylene, said alkenylene and said alkynylene are each optionally substituted with one or more groups independently selected from halogen, -CN, -OH, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), -SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), and -N(C₁₋₅ haloalkyl)(C₁₋₅ alkyl), and further wherein one or more -CH₂- units comprised in said alkylene, said alkenylene or said alkynylene are each optionally replaced by a group independently selected from -O-, NH-, N(C₁₋₅ alkyl)-, CO-, -COO-, S-, -SO-, and SO₂-, and
wherein R_{R5} is selected from C₁₋₁₂ alkyl, C₂₋₁₂ alkenyl, C₂₋₁₂ alkynyl, cycloalkyl, cycloalkenyl, heterocycloalkyl, heterocycloalkenyl, aryl, and heteroaryl,
wherein said alkyl, alkenyl, or alkynyl is optionally substituted with one or more groups independently selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, cycloalkenyl, heterocycloalkyl, heterocycloalkenyl, aryl or heteroaryl is optionally substituted with one or more groups independently selected from halogen, -CN, -OH, -C₁₋₅ alkyl, - C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), -SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and - CON(C₁₋₅ alkyl)(C₁₋₅ alkyl).

2. The compound of claim 1, wherein R₁ is selected from the group consisting of chloro, fluoro, cyano, formyl, (C₁₋₂)alkyl, (C₂)alkenyl, (C₂)alkynyl and (C₁₋₂)haloalkyl,
preferably wherein R₁ is selected from the group consisting of cyano, (C₁₋₂)alkyl, and (C₁₋₂)haloalkyl, more preferably wherein R₁ is selected from the group consisting of cyano, methyl and fluoromethyl, even more preferably
wherein R₁ is cyano, wherein R₁ is methyl, or
wherein R₁ is fluoromethyl.

3. The compound of claim 1 or 2, wherein Y_{C2} is selected from a covalent bond, C₁₋₅ alkylene, C₂₋₅ alkenylene, C₂₋₅ alkynylene, cycloalkylene and heterocycloalkylene wherein said alkylene, said alkenylene and said alkynylene are each optionally substituted with one or more groups independently selected from halogen, CN, OH, O(C₁₋₅ alkyl), -O(C₁₋₅ haloalkyl), C₁₋₅ haloalkyl, SH, S(C₁₅ alkyl), -S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(*N*-heterocycloalkyl), -CO(C₁₋₅ alkyl), CONH₂, CONH(C₁₋₅ alkyl), CON(C₁₋₅ alkyl)(C₁₋₅ alkyl), -CO-(*N*-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), and N(C₁₋₅ alkyl)CON(C₁₋₅ alkyl)(C₁₋₅ alkyl), preferably selected from halogen, CN, OH, O(C₁₋₅ alkyl), SH, S(C₁₅ alkyl), NH₂, NH(C₁₋₅ alkyl), and N(C₁₋₅ alkyl)(C₁₋₅ alkyl), and further wherein one or more -CH₂- units comprised in said alkylene, said alkenylene or said alkynylene are each optionally replaced by a group independently selected from -O-, NH-, N(C₁₋₅ alkyl)-, CO-, S-, -SO-, and SO₂-, and further wherein said cycloalkylene and said heterocycloalkylene are each optionally substituted with one or more groups independently selected from halogen, CN, OH, C₁₋₅ alkyl, C₁₋₅ haloalkyl, O(C₁₋₅ alkyl), -O(C₁₋₅ haloalkyl), SH, S(C₁₅ alkyl), -S(C₁₋₅ haloalkyl), NH₂, NH(C₁₋₅ alkyl), -NH(C₁₋₅ haloalkyl), N(C₁₋₅ alkyl)(C₁₋₅ alkyl), N(C₁₋₅ haloalkyl)(C₁₋₅ alkyl), -(*N*-heterocycloalkyl), -CO(C₁₋₅ alkyl), CONH₂, CONH(C₁₋₅ alkyl), CON(C₁₋₅ alkyl)(C₁₋₅ alkyl), -CO-(*N*-heterocycloalkyl), NHCO-(C₁₋₅ alkyl), N(C₁₋₅ alkyl)-CO-(C₁₋₅ alkyl), NHCONH₂, NHCONH-(C₁₋₅ alkyl), NHCON(C₁₋₅ alkyl)(C₁₋₅ alkyl), N(C₁₋₅ alkyl)CONH₂, N(C₁₋₅ alkyl)CONH-(C₁₋₅ alkyl), and N(C₁₋₅ alkyl)CON(C₁₋₅ alkyl)(C₁₋₅ alkyl), -(C₁₋₅ alkylene)-CN, -(C₁₋₅ alkylene)OH, -(C₁₋₅ alkylene)O(C₁₋₅ alkyl), -(C₁₋₅ alkylene)-O(C₁₋₅ haloalkyl), -(C₁₋₅ alkylene)SH, - (C₁₋₅ alkylene)S(C₁₋₅ alkyl), -(C₁₋₅ alkylene)-S(C₁₋₅ haloalkyl), -(C₁₋₅ alkylene)NH₂, -(C₁₋₅ alkylene)NH(C₁₋₅ alkyl), -(C₁₋₅ alkylene)-NH(C₁₋₅ haloalkyl), -(C₁₋₅ alkylene)N(C₁₋₅ alkyl)(C₁₋₅ alkyl), -(C₁₋₅ alkylene)N(C₁₋₅ alkyl)(C₁₋₅ haloalkyl), -(C₁₋₅ alkylene)(*N*-heterocycloalkyl), -(C₁₋₅ alkylene)N(C₁₋₅ haloalkyl)(C₁₋₅ alkyl), -(C₁₋₅ alkylene)-CO(C₁₋₅ alkyl), -(C₁₋₅ alkylene)CONH₂, -(C₁₋₅ alkylene)CONH(C₁₋₅ alkyl), -(C₁₋₅ alkylene)CON(C₁₋₅ alkyl)(C₁₋₅ alkyl), -(C₁₋₅ alkylene)CO-(N-heterocycloalkyl), -(C₁₋₅ alkylene)NHCO-(C₁₋₅ alkyl), -(C₁₋₅ alkylene)N(C₁₋₅ alkyl)-CO-(C₁₋₅ alkyl), - (C₁₋₅ alkylene)NHCONH₂, -(C₁₋₅ alkylene)NHCONH-(C₁₋₅ alkyl), -(C₁₋₅ alkylene)NHCON(C₁₋₅ alkyl)(C₁₋₅ alkyl), -(C₁₋₅ alkylene)N(C₁₋₅ alkyl)CONH₂, -(C₁₋₅ alkylene)N(C₁₋₅ alkyl)CONH-(C₁₋₅ alkyl), and -(C₁₋₅ alkylene)N(C₁₋₅ alkyl)CON(C₁₋₅ alkyl)(C₁₋₅ alkyl), preferably selected from halogen, CN, OH, C₁₋₅ alkyl, O(C₁₋₅ alkyl), SH, S(C₁₅ alkyl), NH₂, NH(C₁₋₅ alkyl), and N(C₁₋₅ alkyl)(C₁₋₅ alkyl), or
wherein -Y_{C2}-R_{C2} is selected from -O-C₁₋₁₂ alkyl, -NH-C₁₋₁₂ alkyl, -N(C₁₋₅ alkyl)-C₁₋₁₂ alkyl, -O-C₂₋₁₂ alkenyl, -NH-C₂₋₁₂ alkenyl, -N(C₁₋₅ alkyl)-C₂₋₁₂ alkenyl, -O-C₂₋₁₂ alkynyl, -NH-C₂₋₁₂ alkynyl, -N(C₁₋₅ alkyl)-C₂₋₁₂ alkynyl, (C₀₋₃ alkylene)-cycloalkyl, -CO-(C₀₋₃ alkylene)cycloalkyl, (C₀₋₃ alkylene)-CO-cycloalkyl, -CONH-(C₀₋₃ alkylene)cycloalkyl, -(C₀₋₃ alkylene)-CONH-cycloalkyl, -NHCO-(C₀₋₃ alkylene)cycloalkyl, (C₀₋₃ alkylene)-NHCO-cycloalkyl, -NH-(C₀₋₃ alkylene)cycloalkyl, -(C₀₋₃ alkylene)-NH-cycloalkyl, -O-(C₀₋₃ alkylene)cycloalkyl, -(C₀₋₃ alkylene)-O-cycloalkyl, -SO₂-(C₀₋₃ alkylene)cycloalkyl, -(C₀₋₃ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, (C₀₋₃ alkylene)-cycloalkenyl, -CO-(C₀₋₃ alkylene)cycloalkenyl, (C₀₋₃ alkylene)-CO-cycloalkenyl, -CONH-(C₀₋₃ alkylene)cycloalkenyl, -(C₀₋₃ alkylene)-CONH-cycloalkenyl, -NHCO-(C₀₋₃ alkylene)cycloalkenyl, (C₀₋₃ alkylene)-NHCO-cycloalkenyl, -NH-(C₀₋₃ alkylene)cycloalkenyl, -(C₀₋₃ alkylene)-NH-cycloalkenyl, -O-(C₀₋₃ alkylene)cycloalkenyl, -(C₀₋₃ alkylene)-O-cycloalkenyl, -SO₂-(C₀₋₃ alkylene)cycloalkenyl, -(C₀₋₃ alkylene)-SO₂-cycloalkenyl, -CONH-cycloalkenyl, -NHCO-cycloalkenyl, -NH-cycloalkenyl, -O-cycloalkenyl, -CO-cycloalkenyl, -SO₂-cycloalkenyl, -(C₀₋₃ alkylene)-heterocycloalkyl, -CO-(C₀₋₃ alkylene)heterocycloalkyl, -(C₀₋₃ alkylene)-CO-heterocycloalkyl, -CONH-(C₀₋₃ alkylene)heterocycloalkyl, -(C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, -(C₀₋₃ alkylene)-NH-heterocycloalkyl, -O-(C₀₋₃ alkylene) heterocycloalkyl, -(C₀₋₃ alkylene)-O-heterocycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)-heterocycloalkenyl, -CO-(C₀₋₃ alkylene)heterocycloalkenyl, -(C₀₋₃ alkylene)-CO-heterocycloalkenyl, -CONH-(C₀₋₃ alkylene)heterocycloalkenyl, -(C₀₋₃ alkylene)-CONH-heterocycloalkenyl, -NHCO-(C₀₋₃ alkylene)heterocycloalkenyl, -(C₀₋₃ alkylene)-NHCO-heterocycloalkenyl, -NH-(C₀₋₃ alkylene)heterocycloalkenyl, -(C₀₋₃ alkylene)-NH-heterocycloalkenyl, -O-(C₀₋₃ alkylene) heterocycloalkenyl, -(C₀₋₃ alkylene)-O-heterocycloalkenyl, -SO₂-(C₀₋₃ alkylene)heterocycloalkenyl, -(C₀₋₃ alkylene)-SO₂-heterocycloalkenyl, -CONH-heterocycloalkenyl, -NHCO-heterocycloalkenyl, - NH-heterocycloalkenyl, -O-heterocycloalkenyl, -CO-heterocycloalkenyl, -SO₂-heterocycloalkenyl, (C₀₋₃ alkylene)aryl, -CO-(C₀₋₃ alkylene)aryl, -(C₀₋₃ alkylene)-CO-aryl, -CONH-(C₀₋₃ alkylene)aryl, - (C₀₋₃ alkylene)-CONH-aryl, -NHCO-(C₀₋₃ alkylene)aryl, -(C₀₋₃ alkylene)-NHCO-aryl, -NH-(C₀₋₃ alkylene)aryl, -(C₀₋₃ alkylene)-NH-aryl, -O-(C₀₋₃ alkylene)aryl, -(C₀₋₃ alkylene)-O-aryl, -SO₂-(C₀₋₃ alkylene)aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)heteroaryl, -CO-(C₀₋₃ alkylene)heteroaryl, -(C₀₋₃ alkylene)-CO-heteroaryl, - CONH-(C₀₋₃ alkylene)heteroaryl, -(C₀₋₃ alkylene)-CONH-heteroaryl, -NHCO-(C₀₋₃ alkylene)heteroaryl, -(C₀₋₃ alkylene)-NHCO-heteroaryl, -NH-(C₀₋₃ alkylene)heteroaryl, (C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, -CO-heteroaryl and -SO₂-heteroaryl, preferably -Y_{C2}-R_{C2} is selected from -O-C₁₋₁₂ alkyl, -NH-C₁₋₁₂ alkyl, -N(C₁₋₅ alkyl)-C₁₋₁₂ alkyl, -O-C₂₋₁₂ alkenyl, -NH-C₂₋₁₂ alkenyl, -N(C₁₋₅ alkyl)-C₂₋₁₂ alkenyl, -O-C₂₋₁₂ alkynyl, -NH-C₂₋₁₂ alkynyl, -N(C₁₋₅ alkyl)-C₂₋₁₂ alkynyl, (C₀₋₃ alkylene)-cycloalkyl, -CO-(C₀₋₃ alkylene)cycloalkyl, (C₀₋₃ alkylene)-CO-cycloalkyl, -CONH-(C₀₋₃ alkylene)cycloalkyl, -(C₀₋₃ alkylene)-CONH-cycloalkyl, -NHCO-(C₀₋₃ alkylene)cycloalkyl, (C₀₋₃ alkylene)-NHCO-cycloalkyl, -NH-(C₀₋₃ alkylene)cycloalkyl, -(C₀₋₃ alkylene)-NH-cycloalkyl, -O-(C₀₋₃ alkylene)cycloalkyl, -(C₀₋₃ alkylene)-O-cycloalkyl, -SO₂-(C₀₋₃ alkylene)cycloalkyl, -(C₀₋₃ alkylene)-SO₂-cycloalkyl, -CONH-cycloalkyl, -NHCO-cycloalkyl, -NH-cycloalkyl, -O-cycloalkyl, - CO-cycloalkyl, -SO₂-cycloalkyl, -(C₀₋₃ alkylene)-heterocycloalkyl, -CO-(C₀₋₃ alkylene)heterocycloalkyl, -(C₀₋₃ alkylene)-CO-heterocycloalkyl, -CONH-(C₀₋₃ alkylene)heterocycloalkyl, -(C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, -(C₀₋₃ alkylene)-NH-heterocycloalkyl, -O-(C₀₋₃ alkylene) heterocycloalkyl, -(C₀₋₃ alkylene)-O-cycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, (C₀₋₃ alkylene)aryl, -CO-(C₀₋₃ alkylene)aryl, -(C₀₋₃ alkylene)-CO-aryl, -CONH-(C₀₋₃ alkylene)aryl, -(C₀₋₃ alkylene)-CONH-aryl, - NHCO-(C₀₋₃ alkylene)aryl, -(C₀₋₃ alkylene)-NHCO-aryl, -NH-(C₀₋₃ alkylene)aryl, -(C₀₋₃ alkylene)-NH-aryl, -O-(C₀₋₃ alkylene)aryl, -(C₀₋₃ alkylene)-O-aryl, -SO₂-(C₀₋₃ alkylene)aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)heteroaryl, - CO-(C₀₋₃ alkylene)heteroaryl, -(C₀₋₃ alkylene)-CO-heteroaryl, -CONH-(C₀₋₃ alkylene)heteroaryl, - (C₀₋₃ alkylene)-CONH-heteroaryl, -NHCO-(C₀₋₃ alkylene)heteroaryl, -(C₀₋₃ alkylene)-NHCO-heteroaryl, -NH-(C₀₋₃ alkylene)heteroaryl, (C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, - CO-heteroaryl and -SO₂-heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl), -O(C₁₋₅ haloalkyl), -C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl) and wherein said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), - CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), - (C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl),
or
wherein -Y_{C2}-R_{C2} is selected from -(C₀₋₃ alkylene)-heterocycloalkyl, -CO-(C₀₋₃ alkylene)heterocycloalkyl, -(C₀₋₃ alkylene)-CO-heterocycloalkyl, -CONH-(C₀₋₃ alkylene)heterocycloalkyl, -(C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, -(C₀₋₃ alkylene)-NH-heterocycloalkyl, -O-(C₀₋₃ alkylene) heterocycloalkyl, (C₀₋₃ alkylene)-O-cycloalkyl, (C₀₋₃ alkylene)-O-heterocycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)-heterocycloalkenyl, -CO-(C₀₋₃ alkylene)heterocycloalkenyl, -(C₀₋₃ alkylene)-CO-heterocycloalkenyl, -CONH-(C₀₋₃ alkylene)heterocycloalkenyl, -(C₀₋₃ alkylene)-CONH-heterocycloalkenyl, -NHCO-(C₀₋₃ alkylene)heterocycloalkenyl, -(C₀₋₃ alkylene)-NHCO-heterocycloalkenyl, -NH-(C₀₋₃ alkylene)heterocycloalkenyl, -(C₀₋₃ alkylene)-NH-heterocycloalkenyl, -O-(C₀₋₃ alkylene) heterocycloalkenyl, (C₀₋₃ alkylene)-O-heterocycloalkenyl, -SO₂-(C₀₋₃ alkylene)heterocycloalkenyl, -(C₀₋₃ alkylene)-SO₂-heterocycloalkenyl, -CONH-heterocycloalkenyl, - NHCO-heterocycloalkenyl, -NH-heterocycloalkenyl, -O-heterocycloalkenyl, -CO-heterocycloalkenyl, -SO₂-heterocycloalkenyl, -(C₀₋₃ alkylene)aryl, -CO-(C₀₋₃ alkylene)aryl, -(C₀₋₃ alkylene)-CO-aryl, -CONH-(C₀₋₃ alkylene)aryl, -(C₀₋₃ alkylene)-CONH-aryl, -NHCO-(C₀₋₃ alkylene)aryl, -(C₀₋₃ alkylene)-NHCO-aryl, -NH-(C₀₋₃ alkylene)aryl, -(C₀₋₃ alkylene)-NH-aryl, -O-(C₀₋₃ alkylene)aryl, -(C₀₋₃ alkylene)-O-aryl, -SO₂-(C₀₋₃ alkylene)aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)heteroaryl, -CO-(C₀₋₃ alkylene)heteroaryl, -(C₀₋₃ alkylene)-CO-heteroaryl, -CONH-(C₀₋₃ alkylene)heteroaryl, -(C₀₋₃ alkylene)-CONH-heteroaryl, -NHCO-(C₀₋₃ alkylene)heteroaryl, -(C₀₋₃ alkylene)-NHCO-heteroaryl, - NH-(C₀₋₃ alkylene)heteroaryl, -(C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, -CO-heteroaryl and -SO₂-heteroaryl, preferably -Y_{C2}-R_{C2} is selected from -(C₀₋₃ alkylene)-heterocycloalkyl, -CO-(C₀₋₃ alkylene)heterocycloalkyl, -(C₀₋₃ alkylene)-CO-heterocycloalkyl, -CONH-(C₀₋₃ alkylene)heterocycloalkyl, -(C₀₋₃ alkylene)-CONH-heterocycloalkyl, -NHCO-(C₀₋₃ alkylene)heterocycloalkyl, -(C₀₋₃ alkylene)-NHCO-heterocycloalkyl, -NH-(C₀₋₃ alkylene)heterocycloalkyl, -(C₀₋₃ alkylene)-NH-heterocycloalkyl, -O-(C₀₋₃ alkylene) heterocycloalkyl, (C₀₋₃ alkylene)-O-cycloalkyl, -SO₂-(C₀₋₃ alkylene)heterocycloalkyl, -(C₀₋₃ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)aryl, -CO-(C₀₋₃ alkylene)aryl, -(C₀₋₃ alkylene)-CO-aryl, -CONH-(C₀₋₃ alkylene)aryl, -(C₀₋₃ alkylene)-CONH-aryl, - NHCO-(C₀₋₃ alkylene)aryl, -(C₀₋₃ alkylene)-NHCO-aryl, -NH-(C₀₋₃ alkylene)aryl, -(C₀₋₃ alkylene)-NH-aryl, -O-(C₀₋₃ alkylene)aryl, -(C₀₋₃ alkylene)-O-aryl, -SO₂-(C₀₋₃ alkylene)aryl, -(C₀₋₃ alkylene)-SO₂-aryl, -CONH-aryl, -NHCO-aryl, -NH-aryl, -O-aryl, -CO-aryl, -SO₂-aryl, -(C₀₋₃ alkylene)heteroaryl, - CO-(C₀₋₃ alkylene)heteroaryl, -(C₀₋₃ alkylene)-CO-heteroaryl, -CONH-(C₀₋₃ alkylene)heteroaryl, - (C₀₋₃ alkylene)-CONH-heteroaryl, -NHCO-(C₀₋₃ alkylene)heteroaryl, -(C₀₋₃ alkylene)-NHCO-heteroaryl, -NH-(C₀₋₃ alkylene)heteroaryl, -(C₀₋₃ alkylene)-NH-heteroaryl, -O-(C₀₋₃ alkylene)heteroaryl, -(C₀₋₃ alkylene)-O-heteroaryl, -SO₂-(C₀₋₃ alkylene)heteroaryl, -(C₀₋₃ alkylene)-SO₂-heteroaryl, -CONH-heteroaryl, -NHCO-heteroaryl, -NH-heteroaryl, -O-heteroaryl, - CO-heteroaryl and -SO₂-heteroaryl, wherein said heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, - (C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl),
or
wherein -Y_{C2}-R_{C2} is selected from -(C₀₋₃ alkylene)-heterocycloalkyl, -CONH-heterocycloalkyl, - NHCO-heterocycloalkyl, -NH-heterocycloalkyl, -O-heterocycloalkyl, -CO-heterocycloalkyl, -SO₂-heterocycloalkyl, -(C₀₋₃ alkylene)-heterocycloalkenyl, -CONH-heterocycloalkenyl, -NHCO-heterocycloalkenyl, -NH-heterocycloalkenyl, -O-heterocycloalkenyl, -CO-heterocycloalkenyl, -SO₂-heterocycloalkenyl, -(C₀₋₃ alkylene)aryl, -CONH-aryl, -NHCO-aryl, -NH-aryl, -O-aryl, -CO-aryl, -SO₂-aryl, -(C₀₋₃ alkylene)heteroaryl, -CONH-heteroaryl, -NHCO-heteroaryl, -NH-heteroaryl, - O-heteroaryl, -CO-heteroaryl and -SO₂-heteroaryl, preferably -Y_{C2}-R_{C2} is selected from -(C₀₋₃ alkylene)-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)aryl, -CONH-aryl, -NHCO-aryl, -NH-aryl, -O-aryl, -CO-aryl, -SO₂-aryl, -(C₀₋₃ alkylene)heteroaryl, -CONH-heteroaryl, -NHCO-heteroaryl, -NH-heteroaryl, -O-heteroaryl, -CO-heteroaryl and -SO₂-heteroaryl, wherein said heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, - (C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), -(C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl),
or
wherein -Y_{C2}-R_{C2} is selected from -(C₀₋₃ alkylene)-heterocycloalkyl, -(C₀₋₃ alkylene)-heterocycloalkenyl, -(C₀₋₃ alkylene)-aryl, and -(C₀₋₃ alkylene)-heteroaryl, preferably -Y_{C2}-R_{C2} is selected from -(C₀₋₃ alkylene)-heterocycloalkyl, -(C₀₋₃ alkylene)-aryl, and -(C₀₋₃ alkylene)-heteroaryl, wherein said heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CO(C₁₋₅ haloalkyl), -CO-cycloalkyl, -COO(C₁₋₅ alkyl), -COO(C₁₋₅ haloalkyl), -COO-cycloalkyl, -CONH₂, -CONH(C₁₋₅ alkyl), -CON(C₁₋₅ alkyl)(C₁₋₅ alkyl), - CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -OCONH₂, -OCONH-(C₁₋₅ alkyl), -OCON(C₁₋₅ alkyl)(C₁₋₅ alkyl), -NHCOO(C₁₋₅ alkyl), -N(C₁₋₅ alkyl)COO-(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), -(C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CO(C₁₋₅ haloalkyl), -(C₁₋₅ alkylene)-CO-cycloalkyl, -(C₁₋₅ alkylene)-CONH₂, -(C₁₋₅ alkylene)-CONH(C₁₋₅ alkyl), -(C₁₋₅ alkylene)-CON(C₁₋₅ alkyl)(C₁₋₅ alkyl), -(C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), -(C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), -(C₁₋₅ alkylene)-N(C₁₋₅ alkyl)CON(C₁₋₅ alkyl)(C₁₋₅ alkyl), - (C₁₋₅ alkylene)-OCONH₂, -(C₁₋₅ alkylene)-OCONH-(C₁₋₅ alkyl), -(C₁₋₅ alkylene)-OCON(C₁₋₅ alkyl)(C₁₋₅ alkyl), -(C₁₋₅ alkylene)-NHCOO(C₁₋₅ alkyl), and -(C₁₋₅ alkylene)-N(C₁₋₅ alkyl)COO-(C₁₋₅ alkyl).

4. The compound of claim 1 or 2,
wherein Y_{C2} is selected from a covalent bond, C₁₋₅ alkylene, C₂₋₅ alkenylene, and C₂₋₅ alkynylene, wherein said alkylene, said alkenylene and said alkynylene are each optionally substituted with one or more groups independently selected from halogen, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more -CH₂-units comprised in said alkylene, said alkenylene or said alkynylene are each optionally replaced by a group independently selected from -O-, -NH-, -N(C₁₋₅ alkyl)-, -CO-, -S-, -SO-, and -SO₂-, and wherein R_{C2} is selected from hydrogen, halo, -OH, -NH₂, -SH, -CN, C₁₋₁₂ alkyl, C₂₋₁₂ alkenyl, C₂₋₁₂ alkynyl, cycloalkyl, heterocycloalkyl, aryl, and heteroaryl,
wherein said alkyl, alkenyl, or alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), and -P(O)(O(C₁₋₅ alkyl))(C₁₋₅ alkyl), preferably selected from halogen, -CN, -OH, -O(C₁₋₅ alkyl), - O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), and
wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(O)(C₁₋₅ alkyl), -S(O)₂(C₁₋₅ alkyl), -S(O)(NH)(C₁₋₅ alkyl), -S(O)(N(C₁₋₅ alkyl))(C₁₋₅ alkyl), -N=S(O)(C₁₋₅ alkyl)(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(N-heterocycloalkyl), -CO(C₁₋₅ alkyl), -CONH₂, -CONH(C₁₋₅ alkyl), -CON(C₁₋₅ alkyl)(C₁₋₅ alkyl), -CO-(N-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), -N(C₁₋₅ alkyl)CON(C₁₋₅ alkyl)(C₁₋₅ alkyl), -P(O)(C₁₋₅ alkyl)(C₁₋₅ alkyl), -P(O)(O(C₁₋₅ alkyl))(O(C₁₋₅ alkyl)), -P(O)(O(C₁₋₅ alkyl))(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-S(O)(C₁₋₅ alkyl), -(C₁₋₅ alkylene)-S(O)₂(C₁₋₅ alkyl), -(C₁₋₅ alkylene)-S(O)(NH)(C₁₋₅ alkyl), - (C₁₋₅ alkylene)-S(O)(N(C₁₋₅ alkyl))(C₁₋₅ alkyl), -(C₁₋₅ alkylene)-P(O)(C₁₋₅ alkyl)(C₁₋₅ alkyl), -(C₁₋₅ alkylene)-P(O)(O(C₁₋₅ alkyl))(O(C₁₋₅ alkyl)), -(C₁₋₅ alkylene)-P(O)(O(C₁₋₅ alkyl))(C₁₋₅ alkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, (C₁₋₅ alkylene)-CONH(C₁₋₅ alkyl), -(C₁₋₅ alkylene)-CON(C₁₋₅ alkyl)(C₁₋₅ alkyl), - (C₁₋₅ alkylene)-CO-(N-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), (C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -(C₁₋₅ alkylene)-OH, -(C₁₋₅ alkylene)-O(C₁₋₅ alkyl), -(C₁₋₅ alkylene)-O(C₁₋₅ haloalkyl), -SH, -S(C₁₋₅ alkyl), - S(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(C₁₋₅ alkylene)-NH₂, -(C₁₅ alkylene)-NH(C₁₋₅ alkyl), -(C₁₋₅ alkylene)-NH(C₁₋₅ haloalkyl), - (C₁₋₅ alkylene)-N(C₁₋₅ alkyl)(C₁₋₅ alkyl), -(C₁₋₅ alkylene)-N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), more preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), - S(C₁-₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), preferably wherein -Y_{C2}-R_{C2} is selected from -O-C₁₋₁₂ alkyl, -NH-C₁₋₁₂ alkyl, -N(C₁₋₅ alkyl)-C₁₋₁₂ alkyl, -O-C₂₋₁₂ alkenyl, -NH-C₂₋₁₂ alkenyl, -N(C₁₋₅ alkyl)-C₂₋₁₂ alkenyl, -O-C₂₋₁₂ alkynyl, -NH-C₂₋₁₂ alkynyl, -N(C₁₋₅ alkyl)-C₂₋₁₂ alkynyl, -(C₀₋₃ alkylene)-cycloalkyl, -CO-(C₀₋₃ alkylene)-cycloalkyl, -(C₀-₃ alkylene)-CO-cycloalkyl, -CONH-(C₀₋₃ alkylene)-cycloalkyl, (C₀₋₃ alkylene)-CONH-cycloalkyl, - NHCO-(C₀₋₃ alkylene)-cycloalkyl, -(C₀₋₃ alkylene)-NHCO-cycloalkyl, -NH-(C₀₋₃ alkylene)-cycloalkyl, -(C₀₋₃ alkylene)-NH-cycloalkyl, -0-(C_{O}-₃ alkylene)-cycloalkyl, -(C₀₋₃ alkylene)-O-cycloalkyl, -SO₂-(C₀₋₃ alkylene)-cycloalkyl, -(C₀₋₃ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, -(C₀₋₃ alkylene)-heterocycloalkyl, -CO-(C₀₋₃ alkylene)-heterocycloalkyl, -(C₀₋₃ alkylene)-CO-heterocycloalkyl, -CONH-(C₀₋₃ alkylene)-heterocycloalkyl, -(C₀₋₃ alkylene)-CONH-heterocycloalkyl, -NHCO-(C₀₋₃ alkylene)-heterocycloalkyl, -(C₀₋₃ alkylene)-NHCO-heterocycloalkyl, -NH-(C₀₋₃ alkylene)-heterocycloalkyl, -(C₀₋₃ alkylene)-NH-heterocycloalkyl, -O-(C₀₋₃ alkylene)-heterocycloalkyl, -(C₀₋₃ alkylene)-O-cycloalkyl, -SO₂-(C₀₋₃ alkylene)-heterocycloalkyl, -(C₀₋₃ alkylene)-SO₂-heterocycloalkyl, -CONH-heterocycloalkyl, - NHCO-heterocycloalkyl, -NH-heterocycloalkyl, -O-heterocycloalkyl, -CO-heterocycloalkyl, -SO₂-heterocycloalkyl, -(C₀₋₃ alkylene)-aryl, -CO-(C₀₋₃ alkylene)-aryl, -(C₀₋₃ alkylene)-CO-aryl, -CONH-(C₀₋₃ alkylene)-aryl, -(C₀₋₃ alkylene)-CONH-aryl, -NHCO-(C₀₋₃ alkylene)-aryl, -(C₀₋₃ alkylene)-NHCO-aryl, -NH-(C₀₋₃ alkylene)-aryl, -(C₀₋₃ alkylene)-NH-aryl, -O-(C₀₋₃ alkylene)-aryl, -(C₀₋₃ alkylene)-O-aryl, -SO₂-(C₀₋₃ alkylene)-aryl, -(C₀₋₃ alkylene)-SO₂-aryl, -CONH-aryl, -NHCO-aryl, - NH-aryl, -O-aryl, -CO-aryl, -SO₂-aryl, -(C₀₋₃ alkylene)-heteroaryl, -CO-(C₀₋₃ alkylene)-heteroaryl, - (C₀₋₃ alkylene)-CO-heteroaryl, -CONH-(C₀₋₃ alkylene)-heteroaryl, -(C₀₋₃ alkylene)-CONH-heteroaryl, -NHCO-(C₀₋₃ alkylene)-heteroaryl, -(C₀₋₃ alkylene)-NHCO-heteroaryl, -NH-(C₀₋₃ alkylene)-heteroaryl, -(C₀₋₃ alkylene)-NH-heteroaryl, -O-(C₀₋₃ alkylene)-heteroaryl, -(C₀₋₃ alkylene)-O-heteroaryl, -SO₂-(C₀₋₃ alkylene)-heteroaryl, -(C₀₋₃ alkylene)-SO₂-heteroaryl, -CONH-heteroaryl, - NHCO-heteroaryl, -NH-heteroaryl, -O-heteroaryl, -CO-heteroaryl and -SO₂-heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), -C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁ alkyl), -CO(C₁₋₅ alkyl), -CONH₂, -CONH(C₁₋₅ alkyl), and -CON(C₁₋₅ alkyl)(C₁₋₅ alkyl) and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
more preferably wherein -Y_{C2}-R_{C2} is selected from -(C₀₋₃ alkylene)-heterocycloalkyl, -CO-(C₀₋₃ alkylene)-heterocycloalkyl, -(C₀₋₃ alkylene)-CO-heterocycloalkyl, -CONH-(C₀₋₃ alkylene)-heterocycloalkyl, -(C₀₋₃ alkylene)-CONH-heterocycloalkyl, -NHCO-(C₀₋₃ alkylene)-heterocycloalkyl, -(C₀₋₃ alkylene)-NHCO-heterocycloalkyl, -NH-(C₀₋₃ alkylene)-heterocycloalkyl, -(C₀₋₃ alkylene)-NH-heterocycloalkyl, -O-(C₀₋₃ alkylene)-heterocycloalkyl, -(C₀₋₃ alkylene)-O-cycloalkyl, -SO₂-(C₀₋₃ alkylene)-heterocycloalkyl, -(C₀₋₃ alkylene)-SO₂-heterocycloalkyl, -CONH-heterocycloalkyl, - NHCO-heterocycloalkyl, -NH-heterocycloalkyl, -O-heterocycloalkyl, -CO-heterocycloalkyl, -SO₂-heterocycloalkyl, -(C₀₋₃ alkylene)-aryl, -CO-(C₀₋₃ alkylene)-aryl, -(C₀₋₃ alkylene)-CO-aryl, -CONH-(C₀₋₃ alkylene)-aryl, -(C₀₋₃ alkylene)-CONH-aryl, -NHCO-(C₀₋₃ alkylene)-aryl, -(C₀₋₃ alkylene)-NHCO-aryl, -NH-(C₀₋₃ alkylene)-aryl, -(C₀₋₃ alkylene)-NH-aryl, -O-(C₀₋₃ alkylene)-aryl, -(C₀₋₃ alkylene)-O-aryl, -SO₂-(C₀₋₃ alkylene)-aryl, -(C₀₋₃ alkylene)-SO₂-aryl, -CONH-aryl, -NHCO-aryl, - NH-aryl, -O-aryl, -CO-aryl, -SO₂-aryl, -(C₀₋₃ alkylene)-heteroaryl, -CO-(C₀₋₃ alkylene)-heteroaryl, - (C₀₋₃ alkylene)-CO-heteroaryl, -CONH-(C₀₋₃ alkylene)-heteroaryl, -(C₀₋₃ alkylene)-CONH-heteroaryl, -NHCO-(C₀₋₃ alkylene)-heteroaryl, -(C₀₋₃ alkylene)-NHCO-heteroaryl, -NH-(C₀₋₃ alkylene)-heteroaryl, -(C₀₋₃ alkylene)-NH-heteroaryl, -O-(C₀₋₃ alkylene)-heteroaryl, -(C₀₋₃ alkylene)-O-heteroaryl, -SO₂-(C₀₋₃ alkylene)-heteroaryl, -(C₀₋₃ alkylene)-SO₂-heteroaryl, -CONH-heteroaryl, - NHCO-heteroaryl, -NH-heteroaryl, -O-heteroaryl, -CO-heteroaryl and -SO₂-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
even more preferably wherein -Y_{C2}-R_{C2} is selected from -(C₀₋₃ alkylene)-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₃ alkylene)aryl, -CONH-aryl, -NHCO-aryl, -NH-aryl, - O-aryl, -CO-aryl, -SO₂-aryl, -(C₀₋₃ alkylene)-heteroaryl, -CONH-heteroaryl, -NHCO-heteroaryl, -NH-heteroaryl, -O-heteroaryl, -CO-heteroaryl and -SO₂-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -NH₂, -NH(C₁₋₅ alkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
again more preferably wherein -Y_{C2}-R_{C2} is selected from -(C₀₋₃ alkylene)-heterocycloalkyl, -(C₀₋₃ alkylene)-aryl, and -(C₀₋₃ alkylene)-heteroaryl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
still more preferably wherein -Y_{C2}-R_{C2} is selected from heterocycloalkyl, aryl, and heteroaryl, preferably heterocycloalkyl and heteroaryl, more preferably heterocycloalkyl, wherein said heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl).

5. The compound of claims 3 or 4, wherein -Y_{C2}-R_{C2} is optionally substituted aryl, preferably -Y_{C2}-R_{C2} is phenyl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), or wherein -Y_{C2}-R_{C2} is an optionally substituted heteroaryl, preferably wherein -Y_{C2}-R_{C2} is imidazolyl, pyridazinyl, thiazolyl, pyridinyl, pyrimidinyl, pyrazinyl, or indazolyl, wherein heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
or wherein -Y_{C2}-R_{C2} is optionally substituted heterocycloalkyl, preferably wherein -Y_{C2}-R_{C2} is morpholinyl, 1,1-dioxothiomorpholinyl, azetinyl, pyrrolidinyl, piperidinyl, 6-oxo-1,6- dihydropyridinyl, or piperazinyl, wherein heterocycloalkyl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl),
preferably wherein -Y_{C2}-R_{C2} is piperazinyl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CO(C₁₋₅ alkyl), -CONH₂, -CONH(C₁₋₅ alkyl), and -CON(C₁₋₅ alkyl)(C₁₋₅ alkyl), preferably wherein -Y_{C2}-R_{C2} is piperazinyl (preferably N-piperazinyl) optionally substituted (preferably N-substituted) with CO(C₁₋₅ alkyl), -CONH₂, -CONH(C₁₋₅ alkyl), and -CON(C₁₋₅ alkyl)(C₁₋₅ alkyl), more preferably wherein -Y_{C2}-R_{C2} is piperazinyl (preferably N-piperazinyl) substituted (preferably N-substituted, preferably at a different N-atom than that attached to the ring system as shown in formula (I)), with -CON(C₁₋₅ alkyl)(C₁ alkyl), preferably with -CON(CH₃)₂.

6. The compound of any one of claims 1 to 5, wherein X₄ is C-R_{C4}, wherein R_{C4} is selected from hydrogen, halo, C₁₋₆ alkyl, C₂₋₆ alkynyl, -O(C₁₋₆ alkyl), -S(C₁₋₆ alkyl), -NH(C₁₋₆ alkyl), -N(C₁₋₆ alkyl)(C₁₋₆ alkyl), -CO(C₁₋₆ alkyl), C₁₋₆ haloalkyl, -O(C₁₋₆ haloalkyl), -S(C₁₋₆ haloalkyl), -NH(C₁₋₆ haloalkyl), - N(C₁₋₆ haloalkyl)₂, -CO(C₁₋₆ haloalkyl), -(C₀₋₃ alkylene)cycloalkyl, -O-(C₀₋₃ alkylene)-cycloalkyl, -CO-(C₀₋₃ alkylene)-cycloalkyl, -(C₀₋₃ alkylene)-heterocycloalkyl, -0-(C_{O}-₃ alkylene)-heterocycloalkyl, - CO-(C₀₋₃ alkylene)-heterocycloalkyl, -(C₀₋₃ alkylene)-aryl, -O-(C₀₋₃ alkylene)-aryl, -CO-(C₀₋₃ alkylene)-aryl, -(C₀₋₃ alkylene)-heteroaryl, -O(C₀₋₃ alkylene)-heteroaryl and -CO-(C₀₋₃ alkylene)-heteroaryl;
wherein said alkyl or said alkynyl is optionally substituted with one or more groups independently selected from halogen, -CN, -OH, -O(C₁₋₅ alkyl), -O(C₁₋₅ haloalkyl), C₁₋₅ haloalkyl, -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁₋₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), preferably selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and
wherein said cycloalkyl, cycloalkenyl, heterocycloalkyl, heterocycloalkenyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -(*N*-heterocycloalkyl), -CO(C₁₋₅ alkyl), -CONH₂, -CONH(C₁₋₅ alkyl), -CON(C₁₋₅ alkyl)(C₁₋₅ alkyl), -CO-(*N*-heterocycloalkyl), -NHCO-(C₁₋₅ alkyl), -N(C₁₋₅ alkyl)-CO-(C₁₋₅ alkyl), -NHCONH₂, -NHCONH-(C₁-₅ alkyl), -NHCON(C₁₋₅ alkyl)(C₁₋₅ alkyl), -N(C₁₋₅ alkyl)CONH₂, -N(C₁₋₅ alkyl)CONH-(C₁₋₅ alkyl), and -N(C₁₋₅ alkyl)CON(C₁₋₅ alkyl)(C₁₋₅ alkyl), -(C₁₋₅ alkylene)-CN, -(C₁₋₅ alkylene)-OH, -(C₁₋₅ alkylene)-O(C₁₋₅ alkyl), -(C₁₋₅ alkylene)-O(C₁₋₅ haloalkyl), -(C₁₋₅ alkylene)-SH, -(C₁₋₅ alkylene)-S(C₁₋₅ alkyl), -(C₁₋₅ alkylene)-S(C₁₋₅ haloalkyl), -(C₁₋₅ alkylene)-NH₂, -(C₁₋₅ alkylene)-NH(C₁₋₅ alkyl), -(C₁₋₅ alkylene)-NH(C₁₋₅ haloalkyl), -(C₁₋₅ alkylene)-N(C₁₋₅ alkyl)(C₁₋₅ alkyl), -(C₁₋₅ alkylene)-N(C₁₋₅ alkyl)(C₁₋₅ haloalkyl), -(C₁₋₅ alkylene)-(N-heterocycloalkyl), -(C₁₋₅ alkylene)-N(C₁₋₅ haloalkyl)(C₁₋₅ alkyl), -(C₁₋₅ alkylene)-CO(C₁₋₅ alkyl), -(C₁₋₅ alkylene)-CONH₂, (C₁₋₅ alkylene)-CONH(C₁₋₅ alkyl), -(C₁₋₅ alkylene)-CON(C₁₋₅ alkyl)(C₁₋₅ alkyl), - (C₁₋₅ alkylene)-CO-(*N*-heterocycloalkyl), -(C₁₋₅ alkylene)-NHCO-(C₁₋₅ alkyl), -(C₁₋₅ alkylene)-N(C₁₋₅ alkyl)-CO-(C₁₋₅ alkyl), (C₁₋₅ alkylene)-NHCONH₂, -(C₁₋₅ alkylene)-NHCONH-(C₁₋₅ alkyl), -(C₁₋₅ alkylene)-NHCON(C₁₋₅ alkyl)(C₁₋₅ alkyl), -(C₁₋₅ alkylene)-N(C₁₋₅ alkyl)CONH₂, -(C₁₋₅ alkylene)-N(C₁₋₅ alkyl)CONH-(C₁₋₅ alkyl), and -(C₁₋₅ alkylene)-N(C₁₋₅ alkyl)CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
or
wherein X₄ is C-R_{C4}, wherein R_{C4} is selected from hydrogen, halo, C₁₋₆ alkyl, C₂₋₆ alkynyl, -O-C₁₋₆ alkyl, -S-C₁₋₆ alkyl, -NH-C₁₋₆ alkyl, C₁₋₆ haloalkyl, -(C₀₋₃ alkylene)-cycloalkyl, -(C₀₋₃ alkylene)-heterocycloalkyl, -(C₀₋₃ alkylene)-aryl and -(C₀₋₃ alkylene)-heteroaryl, wherein said alkyl is optionally substituted with one or more groups independently selected from halogen, -CN, -OH, -O(C₁₋₅ alkyl), -O(C₁₋₅ haloalkyl), -SH, -S(C₁₋₅ alkyl), -O(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ haloalkyl), -S(C₁₋₅ alkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably wherein R_{C4} is selected from hydrogen, halo, C₁₋₆ alkyl, C₂₋₆ alkynyl, -O-C₁-₆ alkyl, -S-C₁₋₆ alkyl, -NH-C₁₋₆ alkyl, and C₁-₆ haloalkyl,
more preferably wherein R_{C4} is selected from hydrogen, halo, C₁₋₂ alkyl, and C₂₋₃ alkynyl,
even more preferably wherein R_{C4} is selected from hydrogen, halo, and C₁₋₂ alkyl,
again more preferably wherein R_{C4} is hydrogen or halo.

7. The compound of any one of claims 1 to 6, wherein X₅ is C-R_{C5}, wherein R_{C5} is selected from hydrogen, halo, C₁₋₃ alkyl, -O-C₁₋₃ alkyl, -S-C₁₋₃ alkyl, -NH-C₁₋₃ alkyl, and C₁₋₃ haloalkyl, preferably, wherein R_{C5} is selected from hydrogen, halo, C₁₋₃ alkyl, and C₁₋₃ haloalkyl.

8. The compound of any one of claims 1 to 7, wherein R_{R5} is selected from C₁₋₁₂ alkyl, C₂₋₁₂ alkenyl, C₂₋₁₂ alkynyl, cycloalkyl, heterocycloalkyl, aryl, and heteroaryl,
wherein said alkyl, alkenyl, or alkynyl is optionally substituted with one or more groups independently selected from halogen, -CN, -OH, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, -C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), -SO₂(C₁₋₅ alkyl), -S(C₁₋₅ haloalkyl), -SO(C₁₋₅ haloalkyl), -SO₂(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

9. The compound of any one of claims 1 to 7, wherein R₄ is selected from -(C₀₋₂ alkylene)-cycloalkyl, -CO-(C₀₋₂ alkylene)-cycloalkyl, -(C₀₋₂ alkylene)-CO-cycloalkyl, -CONH-(C₀₋₂ alkylene)-cycloalkyl, - (C₀₋₂ alkylene)-CONH-cycloalkyl, -NHCO-(C₀₋₂ alkylene)-cycloalkyl, -(C₀₋₂ alkylene)-NHCO-cycloalkyl, -NH-(C₀₋₂ alkylene)-cycloalkyl, -(C₀₋₂ alkylene)-NH-cycloalkyl, -O-(C₀₋₂ alkylene)-cycloalkyl, -(C₀₋₂ alkylene)-O-cycloalkyl, -SO₂-(C₀₋₂ alkylene)-cycloalkyl, -(C₀₋₂ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, -(C₀₋₂ alkylene)-cycloalkenyl, -CO-(C₀₋₂ alkylene)-cycloalkenyl, -(C₀₋₂ alkylene)-CO-cycloalkenyl, -CONH-(C₀₋₂ alkylene)-cycloalkenyl, -(C₀₋₂ alkylene)-CONH-cycloalkenyl, -NHCO-(C₀₋₂ alkylene)-cycloalkenyl, -(C₀₋₂ alkylene)-NHCO-cycloalkenyl, -NH-(C₀₋₂ alkylene)-cycloalkenyl, -(C₀₋₂ alkylene)-NH-cycloalkenyl, -O-(C₀₋₂ alkylene)-cycloalkenyl, -(C₀₋₂ alkylene)-O-cycloalkenyl, -SO₂-(C₀₋₂ alkylene)-cycloalkenyl, -(C₀₋₂ alkylene)-SO₂-cycloalkenyl, - CONH-cycloalkenyl, -NHCO-cycloalkenyl, -NH-cycloalkenyl, -O-cycloalkenyl, -CO-cycloalkenyl, -SO₂-cycloalkenyl, -(C₀₋₂ alkylene)-heterocycloalkyl, -CO-(C₀₋₂ alkylene)-heterocycloalkyl, -(C₀₋₂ alkylene)-CO-heterocycloalkyl, -CONH-(C₀₋₂ alkylene)-heterocycloalkyl, - (C₀₋₂ alkylene)-CONH-heterocycloalkyl, -NHCO-(C₀₋₂ alkylene)-heterocycloalkyl, -(C₀₋₂ alkylene)-NHCO-heterocycloalkyl, -NH-(C₀₋₂ alkylene)-heterocycloalkyl, -(C₀₋₂ alkylene)-NH-heterocycloalkyl, -O-(C₀₋₂ alkylene)-heterocycloalkyl, -(C₀₋₂ alkylene)-O-heterocycloalkyl, -SO₂-(C₀₋₂ alkylene)-heterocycloalkyl, -(C₀₋₂ alkylene)-SO₂-heterocycloalkyl, -CONH-heterocycloalkyl, - NHCO-heterocycloalkyl, -NH-heterocycloalkyl, -O-heterocycloalkyl, -CO-heterocycloalkyl, -SO₂-heterocycloalkyl, -(C₀₋₂ alkylene)-heterocycloalkenyl, -CO-(C₀₋₂ alkylene)-heterocycloalkenyl, -(C₀₋₂ alkylene)-CO-heterocycloalkenyl, -CONH-(C₀₋₂ alkylene)-heterocycloalkenyl, -(C₀₋₂ alkylene)-CONH-heterocycloalkenyl, -NHCO-(C₀₋₂ alkylene)-heterocycloalkenyl, -(C₀₋₂ alkylene)-NHCO-heterocycloalkenyl, -NH-(C₀₋₂ alkylene)-heterocycloalkenyl, -(C₀₋₂ alkylene)-NH-heterocycloalkenyl, -O-(C₀₋₂ alkylene)-heterocycloalkenyl, -(C₀₋₂ alkylene)-O-heterocycloalkenyl, -SO₂-(C₀₋₂ alkylene)-heterocycloalkenyl, -(C₀₋₂ alkylene)-SO₂-heterocycloalkenyl, -CONH-heterocycloalkenyl, -NHCO-heterocycloalkenyl, -NH-heterocycloalkenyl, -O-heterocycloalkenyl, -CO-heterocycloalkenyl, -SO₂-heterocycloalkenyl, -(C₀₋₂ alkylene)-aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, -SO₂-(C₀₋₂ alkylene)-aryl, -(C₀₋₂ alkylene)-SO₂-aryl, -CONH-aryl, -NHCO-aryl, -NH-aryl, -O-aryl, -CO-aryl, -SO₂-aryl, -(C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)-heteroaryl, -(C₀₋₂ alkylene)-NH-heteroaryl, -O-(C₀₋₂ alkylene)-heteroaryl, -(C₀₋₂ alkylene)-O-heteroaryl, -SO₂-(C₀₋₂ alkylene)-heteroaryl, -(C₀₋₂ alkylene)-SO₂-heteroaryl, -CONH-heteroaryl, -NHCO-heteroaryl, -NH-heteroaryl, -O-heteroaryl, -CO-heteroaryl, and -SO₂-heteroaryl,
wherein said cycloalkyl, cycloalkenyl, heterocycloalkyl, heterocycloalkenyl, aryl or heteroaryl is optionally substituted with one or more groups independently selected from halogen, -CN, -OH, - C₁₋₅ alkyl, -C₁₋₅ haloalkyl, -O(C₁₋₅ alkyl), -O(C₁₋₅ haloalkyl), -SH, -S(C₁₋₅ alkyl), -SO(C₁₋₅ alkyl), - SO₂(C₁₋₅ alkyl), -S(C₁₋₅ haloalkyl), -SO(C₁₋₅ haloalkyl), -SO₂(C₁₋₅ haloalkyl), -NH₂, -NH(C₁₋₅ alkyl), - NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), preferably selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl).

10. The compound of any one of claims 1 to 9, wherein R₄ is selected from -(C₀₋₂ alkylene)-cycloalkyl, -CO-(C₀₋₂ alkylene)-cycloalkyl, -(C₀₋₂ alkylene)-CO-cycloalkyl, -CONH-(C₀₋₂ alkylene)-cycloalkyl, - (C₀₋₂ alkylene)-CONH-cycloalkyl, -NHCO-(C₀₋₂ alkylene)-cycloalkyl, -(C₀₋₂ alkylene)-NHCO-cycloalkyl, -NH-(C₀₋₂ alkylene)-cycloalkyl, -(C₀₋₂ alkylene)-NH-cycloalkyl, -O-(C₀₋₂ alkylene)-cycloalkyl, -(C₀₋₂ alkylene)-O-cycloalkyl, -SO₂-(C₀₋₂ alkylene)-cycloalkyl, -(C₀₋₂ alkylene)-SO₂-cycloalkyl, -CONH-cycloalkyl, -NHCO-cycloalkyl, -NH-cycloalkyl, -O-cycloalkyl, -CO-cycloalkyl, -SO₂-cycloalkyl, -(C₀₋₂ alkylene)-heterocycloalkyl, -CO-(C₀₋₂ alkylene)-heterocycloalkyl, -(C₀₋₂ alkylene)-CO-heterocycloalkyl, -CONH-(C₀₋₂ alkylene)-heterocycloalkyl, -(C₀₋₂ alkylene)-CONH-heterocycloalkyl, -NHCO-(C₀₋₂ alkylene)-heterocycloalkyl, -(C₀₋₂ alkylene)-NHCO-heterocycloalkyl, -NH-(C₀₋₂ alkylene)-heterocycloalkyl, -(C₀₋₂ alkylene)-NH-heterocycloalkyl, -O-(C₀₋₂ alkylene)-heterocycloalkyl, -(C₀₋₂ alkylene)-O-heterocycloalkyl, -SO₂-(C₀₋₂ alkylene)-heterocycloalkyl, -(C₀₋₂ alkylene)-SO₂-heterocycloalkyl, -CONH-heterocycloalkyl, -NHCO-heterocycloalkyl, -NH-heterocycloalkyl, -O-heterocycloalkyl, -CO-heterocycloalkyl, -SO₂-heterocycloalkyl, -(C₀₋₂ alkylene)-aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, -(C₀₋₂ alkylene)-O-aryl, -SO₂-(C₀₋₂ alkylene)-aryl, -(C₀₋₂ alkylene)-SO₂-aryl, -CONH-aryl, -NHCO-aryl, - NH-aryl, -O-aryl, -CO-aryl, -SO₂-aryl, -(C₀₋₂ alkylene)-heteroaryl, -CO-(C₀₋₂ alkylene)-heteroaryl, - (C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)heteroaryl, -(C₀₋₂ alkylene)-NH-heteroaryl, -O-(C₀₋₂ alkylene)heteroaryl, -(C₀₋₂ alkylene)-O-heteroaryl, -SO₂-(C₀₋₂ alkylene)heteroaryl, -(C₀₋₂ alkylene)-SO₂-heteroaryl, -CONH-heteroaryl, - NHCO-heteroaryl, -NH-heteroaryl, -O-heteroaryl, -CO-heteroaryl, and -SO₂-heteroaryl, wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
or
wherein R₄ is selected from -(C₀₋₂ alkylene)-aryl, -CO-(C₀₋₂ alkylene)-aryl, -(C₀₋₂ alkylene)-CO-aryl, -CONH-(C₀₋₂ alkylene)-aryl, -(C₀₋₂ alkylene)-CONH-aryl, -NHCO-(C₀₋₂ alkylene)-aryl, -(C₀₋₂ alkylene)-NHCO-aryl, -NH-(C₀₋₂ alkylene)-aryl, -(C₀₋₂ alkylene)-NH-aryl, -O-(C₀₋₂ alkylene)-aryl, - (C₀₋₂ alkylene)-O-aryl, -SO₂-(C₀₋₂ alkylene)-aryl, -(C₀₋₂ alkylene)-SO₂-aryl, -CONH-aryl, -NHCO-aryl, -NH-aryl, -O-aryl, -CO-aryl, -SO₂-aryl, -(C₀₋₂ alkylene)-heteroaryl, -CO-(C₀₋₂ alkylene)-heteroaryl, -(C₀₋₂ alkylene)-CO-heteroaryl, -CONH-(C₀₋₂ alkylene)-heteroaryl, -(C₀₋₂ alkylene)-CONH-heteroaryl, -NHCO-(C₀₋₂ alkylene)-heteroaryl, -(C₀₋₂ alkylene)-NHCO-heteroaryl, -NH-(C₀₋₂ alkylene)-heteroaryl, -(C₀₋₂ alkylene)-NH-heteroaryl, -O-(C₀₋₂ alkylene)-heteroaryl, -(C₀₋₂ alkylene)-O-heteroaryl, -SO₂-(C₀₋₂ alkylene)-heteroaryl, -(C₀₋₂ alkylene)-SO₂-heteroaryl, -CONH-heteroaryl, - NHCO-heteroaryl, -NH-heteroaryl, -O-heteroaryl, -CO-heteroaryl, and -SO₂-heteroaryl, wherein said aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
or
wherein R₄ is selected from C₁₋₁₂ alkyl, C₂₋₁₂ alkenyl, C₂₋₁₂ alkynyl, cycloalkyl, heterocycloalkyl, aryl, and heteroaryl, preferably wherein R₄ is selected from cycloalkyl, heterocycloalkyl, aryl, and heteroaryl, more preferably wherein R₄ is selected from aryl, and heteroaryl, even more preferably wherein R₄ is heteroaryl, wherein said alkyl, alkenyl, or alkynyl is optionally substituted with one or more groups independently selected from halogen, -CN, -OH, -O(C₁₋₅ alkyl), -O(C₁₋₅ haloalkyl), SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl), and wherein said cycloalkyl, heterocycloalkyl, aryl or heteroaryl is optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁₋₅ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
or
wherein R₄ is a five membered heteroaryl, optionally substituted with one or more groups independently selected from halogen, -CN, -OH, C₁₋₅ alkyl, C₁₋₅ haloalkyl, -O(C₁₋₅ alkyl), -O(C₁₋₅ haloalkyl), -SH, -S(C₁₋₅ alkyl), -S(C₁₋₅ haloalkyl), -NH₂, -NH(C₁₋₅ alkyl), -NH(C₁₋₅ haloalkyl), -N(C₁₋₅ alkyl)(C₁ alkyl), -N(C₁₋₅ haloalkyl)(C₁₋₅ alkyl), -CONH₂, -CONH(C₁₋₅ alkyl), and -CON(C₁₋₅ alkyl)(C₁₋₅ alkyl),
preferably wherein the five membered heteroaryl is selected from imidazolyl, isoxazolyl, pyrazolyl, 1,2,3-triazolyl, 1,2,4-triazolyl, thiazolyl, 1,2,4-oxadiazolyl, 1,3,4-oxadiazolyl, 1,2,4-thiadiazolyl, or 1,3,4-thiadiazolyl, preferably wherein the five membered heteroaryl is 1,2,4-thiadiazolyl, optionally substituted with one or more groups independently selected from halogen, -CN, -OH, C₁₋₅ alkyl, C₁₋₅ haloalkyl, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CONH₂, -CONH(C₁₋₅ alkyl), and -CON(C₁₋₅ alkyl)(C₁₋₅ alkyl), preferably optionally substituted with C₁₋₅ alkyl, C₁₋₅ haloalkyl, -O(C₁₋₅ alkyl), -SH, -S(C₁₋₅ alkyl), more preferably optionally substituted with C₁₋₅ alkyl, C₁₋₅ haloalkyl, even more preferably optionally substituted with C₁₋₅ haloalkyl, preferably selected from -CH₂F, -CHF₂ and CF₃, most preferably optionally substituted with -CHF₂.

11. The compound of claim 1, selected from: 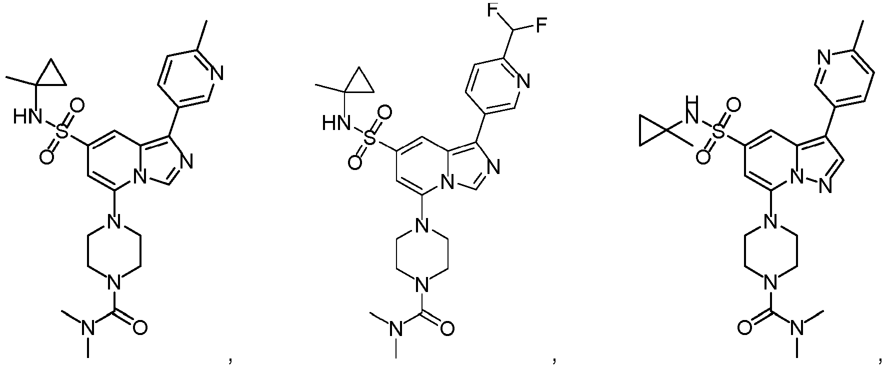 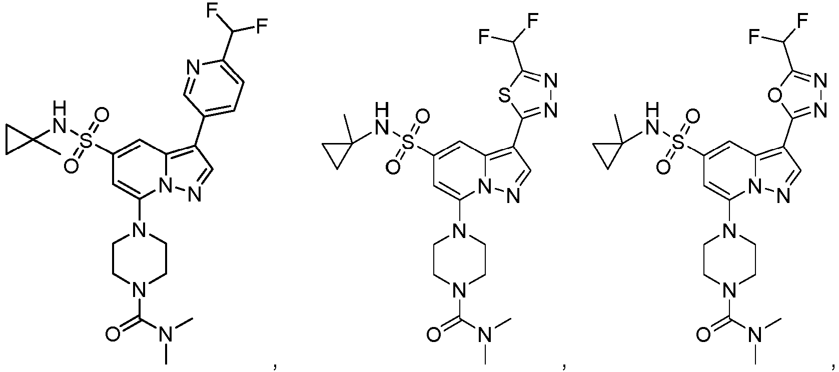 and 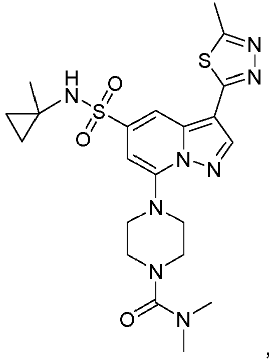 or a pharmaceutically acceptable salt, hydrate or solvate thereof.

12. The compound of claim 1, wherein the compound is a compound according to formula (lap): 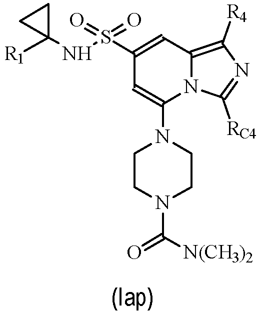 or a compound according to formula (Ibn): 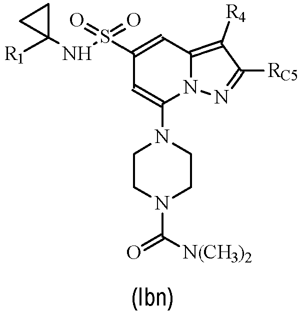
or an enantiomer, diastereoisomer, tautomer, pharmaceutically acceptable solvate, pharmaceutically acceptable crystal form, or pharmaceutically acceptable salt thereof,
wherein R₁, R₄, R_{C4} and R_{C5} are each defined as in any one of claims 1 to 10.

13. A pharmaceutical composition comprising the compound of any one of claims 1 to 12 or a pharmaceutically acceptable salt, hydrate or solvate thereof, and a pharmaceutically acceptable carrier.

14. The compound of any one of claims 1 to 12 or a pharmaceutically acceptable salt, hydrate or solvate thereof, or a pharmaceutical composition of claim 13, for use in therapy.

15. The compound for use or the pharmaceutical composition for use of claim 14, for use in a method of treating a proliferative disorder, preferably wherein the proliferative disorder is cancer, preferably a human cancer, more preferably wherein the cancer is selected from lung, colon, breast, ovarian, prostate, liver, pancreas, brain, and skin cancer.

## Patentansprüche

1. Verbindung der Formel (I): 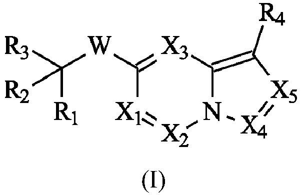 oder ein Enantiomer, Diastereomer, Tautomer, pharmazeutisch akzeptables Solvat, eine pharmazeutisch akzeptable Kristallform oder ein pharmazeutisch akzeptables Salz davon, wobei:
R₁ ausgewählt ist aus der Gruppe bestehend aus Wasserstoff, Chlor, Fluor, Cyano, Formyl, (C₁₋₂)-Alkyl, (C₂)-Alkenyl, (C₂)-Alkinyl, (C₁₋₂)-Halogenalkyl, -(C₁₋₂-Alkylen)-OH und -(C₁₋₂-Alkylen)-O-(C₁₋₂-Alkyl);
R₂ und R₃ zusammen mit dem Kohlenstoffatom, an das sie gebunden sind, Cyclopropyl bilden; W -NHS(O)₂- ist, wobei die hierin definierte linke Seite von W an das Kohlenstoffatom gebunden ist, das R₁, R₂ und R₃ trägt, und die hierin definierte rechte Seite von W an das in Formel (I) gezeigte Ringsystem gebunden ist;
X₁ CF ist oder CH und X₃ CH ist;
X₂ C-Y_{C2}-R_{C2} ist,
wobei Y_{C2} ausgewählt ist aus einer kovalenten Bindung, C₁₋₅-Alkylen, C₂₋₅-Alkenylen, C₂₋₅-Alkinylen, Cycloalkylen, Cycloalkenylen, Heterocycloalkylen und Heterocycloalkenylen, wobei das Alkylen, das Alkenylen und das Alkinylen jeweils optional mit einer oder mehreren Gruppen substituiert sind, die unabhängig voneinander ausgewählt sind aus Halogen, CN, OH, O(C₁₋₅-Alkyl), -O(C₁₋₅-Halogenalkyl), C₁₋₅ -Halogenalkyl, SH, S(C₁₋₅-Alkyl), -S(C₁₋₅-Halogenalkyl), NH₂, NH(C₁₋₅-Alkyl), -NH(C₁₋₅-Halogenalkyl), N(C₁₋₅-Alkyl)(C₁₋₅-Alkyl), N(C₁₋₅-Halogenalkyl)(C₁₋₅-Alkyl), -(*N*-Heterocycloalkyl), -CO(C₁₋₅-Alkyl), CONH₂, CONH(C₁₋₅-Alkyl), CON(C₁₋₅-Alkyl)(C₁₋₅-Alkyl), -CO-(N-Heterocycloalkyl), NHCO-(C₁₋₅-Alkyl), N(C₁₋₅-Alkyl)-CO-(C₁₋₅-Alkyl), NHCONH₂, NHCONH-(C₁₋₅-Alkyl), NHCON(C₁₋₅-Alkyl)(C₁₋₅-Alkyl), N(C₁₋₅-Alkyl) CONH₂, N(C₁₋₅-Alkyl)CONH-(C₁₋₅-Alkyl) und N(C₁₋₅-Alkyl)CON(C₁₋₅-Alkyl)(C₁₋₅-Alkyl), bevorzugt ausgewählt aus Halogen, CN, OH, O(C₁₋₅-Alkyl), SH, S(C₁₋₅-Alkyl), NH₂, NH(C₁₋₅-Alkyl) und N(C₁₋₅-Alkyl)(C₁₋₅-Alkyl), und ferner, wobei eine oder mehrere -CH₂-Einheiten, die in dem Alkylen, dem Alkenylen oder dem Alkinylen umfasst sind, jeweils optional durch eine Gruppe ersetzt sind, die unabhängig ausgewählt ist aus -O-, NH-, N(C₁₋₅-Alkyl)-, CO-, S-, -SO- und SO₂-, und ferner, wobei das Cycloalkylen, das Cycloalkenylen, das Heterocycloalkylen und das Heterocycloalkenylen jeweils optional mit einer oder mehreren Gruppen substituiert sind, die unabhängig ausgewählt sind aus Halogen, CN, OH, C₁₋₅-Alkyl, C₁₋₅-Halogenalkyl, O(C₁₋₅-Alkyl), -O(C₁₋₅-Halogenalkyl), SH, S(C₁₅-Alkyl), -S(C₁₋₅-Halogenalkyl), NH₂, NH(C₁₋₅-Alkyl), -NH(C₁₋₅-Halogenalkyl), N(C₁₋₅-Alkyl)(C₁₋₅-Alkyl), N(C₁₋₅-Halogenalkyl)(C₁₋₅-Alkyl), -(*N-*Heterocycloalkyl), -CO(C₁₋₅-Alkyl), CONH₂, CONH(C₁₋₅-Alkyl), CON(C₁₋₅-Alkyl)(C₁₋₅-Alkyl), -CO-(*N*-Heterocycloalkyl), NHCO-(C₁₋₅-Alkyl), N(C₁₋₅-Alkyl)-CO-(C₁₋₅-Alkyl), NHCONH₂, NHCONH-(C₁₋₅-Alkyl), NHCON(C₁₋₅-Alkyl)(C₁₋₅-Alkyl), N(C₁₋₅-Alkyl)CONH₂, N(C₁₋₅-Alkyl)CONH-(C₁₋₅-Alkyl) und N(C₁₋₅-Alkyl)CON(C₁₋₅-Alkyl)(C₁₋₅-Alkyl), -(C₁₋₅-Alkylen)-CN, -(C₁₋₅-Alkylen)OH, -(C₁₋₅-Alkylen)O(C₁₋₅-Alkyl), -(C₁₋₅-Alkylen)-O(C₁₋₅-Halogenalkyl), (C₁₋₅-Alkylen)SH, -(C₁₋₅-Alkylen)S(C₁₅-Alkyl), -(C₁₋₅-Alkylen)-S(C₁₋₅-Halogenalkyl), -(C₁₋₅-Alkylen)NH₂, -(C₁₋₅-Alkylen)NH(C₁₋₅-Alkyl), -(C₁₋₅-Alkylen)-NH(C₁₋₅-Halogenalkyl), -(C₁₋₅-Alkylen)N(C₁₋₅-Alkyl)(C₁₋₅-Alkyl), -(C₁₋₅-Alkylen)N(C₁₋₅-Alkyl)(C₁₋₅-Halogenalkyl), -(C₁₋₅-Alkylen)(N-Heterocycloalkyl), -(C₁₋₅-Alkylen)N(C₁₋₅-Halogenalkyl)(C₁₋₅-Alkyl), -(C₁₋₅-Alkylen)-CO(C₁₋₅-Alkyl), -(C₁₋₅-Alkylen)CONH₂, -(C₁₋₅-Alkylen)CONH(C₁₋₅-Alkyl), -(C₁₋₅-Alkylen)CON(C₁₋₅-Alkyl)C₁₋₅-Alkyl), -(C₁₋₅-Alkylen)CO-(N-Heterocycloalkyl), -(C₁₋₅-Alkylen)NHCO-(C₁₋₅-Alkyl), -(C₁₋₅-Alkylen)N(C₁₋₅-Alkyl)-CO-(C₁₋₅-Alkyl), -(C₁₋₅-Alkylen)NHCONH₂, -(C₁₋₅-Alkylen) NHCONH-(C₁₋₅-Alkyl), -(C₁₋₅-Alkylen)NHCON(C₁₋₅-Alkyl)(C₁₋₅-Alkyl), -(C₁₋₅-Alkylen)N(C₁₋₅-Alkyl)CONH₂, -(C₁₋₅-Alkylen)N(C₁₋₅-Alkyl) CONH-(C₁₋₅-Alkyl) und -(C₁₋₅-Alkylen)N(C₁₋₅-Alkyl)CON(C₁₋₅-Alkyl)(C₁₋₅-Alkyl), bevorzugt ausgewählt aus Halogen, CN, OH, C₁₋₅-Alkyl, O(C₁₋₅-Alkyl), SH, S(C₁₅-Alkyl), NH₂, NH(C₁₋₅-Alkyl) und N(C₁₋₅-Alkyl)(C₁₋₅-Alkyl), und
R_{C2} ausgewählt ist aus Wasserstoff, Halogen, -OH, -NH₂, -SH, -CN, C₁₋₁₂-Alkyl, C₂₋₁₂-Alkenyl, C₂₋₁₂-Alkinyl, Cycloalkyl, Cycloalkenyl, Heterocycloalkyl, Heterocycloalkenyl, Aryl und Heteroaryl,
wobei das Alkyl, Alkenyl oder Alkinyl optional mit einer oder mehreren Gruppen substituiert ist, die unabhängig voneinander ausgewählt sind aus Halogen, -CN, -OH, -O(C₁₋₅-Alkyl), - O(C₁₋₅-Halogenalkyl), C₁₋₅-Halogenalkyl, -SH, -S(C₁₋₅-Alkyl), -S(C₁₋₅-Halogenalkyl), -NH₂, - NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(*N*-Heterocycloalkyl), -NHCO-(C₁₋₅-Alkyl), - N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -P(O)(C₁₋₅-Alkyl)(C₁₋₅-Alkyl), -P(O)(O(C₁₋₅-Alkyl))(O(C₁₋₅-Alkyl)) und -P(O)(O(C₁₋₅-Alkyl))(C₁₋₅-Alkyl), bevorzugt ausgewählt aus Halogen, -CN, -OH, -O(C₁₋₅-Alkyl)-, -O(C₁₋₅-Halogenalkyl)-, C₁₋₅-Halogenalkyl, -SH, -S(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), - CO(C₁₋₅-Alkyl), -CONH₂, -CONH(C₁₋₅-Alkyl) und -CON(C₁₋₅-Alkyl)(C₁₋₅-Alkyl), bevorzugt ausgewählt aus Halogen, -CN, -OH, -O(C₁₋₅-Alkyl), -O(C₁₋₅-Halogenalkyl), C₁₋₅-Halogenalkyl, -SH, -S(C₁₋₅-Alkyl), -S(C₁₋₅-Halogenalkyl), -NH₂, -NH(C₁₋₅-Alkyl), -NH(C₁₋₅-Halogenalkyl), - N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(N-Heterocycloalkyl), -NHCO-(C₁₋₅-Alkyl), -N(C₁₋₅-Alkyl)-CO-(C₁₋₅-Alkyl), -NHCONH₂, - NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -S(O)(C₁₋₅-Alkyl), - S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -P(O)(C₁₋₅-Alkyl)(C₁₋₅-Alkyl), -P(O)(O(C₁₋₅-Alkyl))(O(C₁₋₅-Alkyl)) und - P(O)(O(C₁₋₅-Alkyl))(C₁₋₅-Alkyl), bevorzugt ausgewählt aus Halogen, -CN, -OH, -O(C₁₋₅-Alkyl), -O(C₁₋₅-Halogenalkyl), C₁₋₅-Halogenalkyl, -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, -CONH(C₁₋₅-Alkyl) und -CON(C₁₋₅-Alkyl)(C₁₋₅-Alkyl), und
wobei das Cycloalkyl, Cycloalkenyl, Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(O)(C₁₋₅-Alkyl), -S(O)₂(C₁₋₅-Alkyl), - S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), - S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CO(C₁₋₅-Halogenalkyl), -CO-Cycloalkyl, -COO(C₁₋₅-Alkyl), -COO(C₁₋₅-Halogenalkyl), -COO-Cycloalkyl, -CONH₂, -CONH(C₁₋₅-Alkyl), -CON(C₁₋₅-Alkyl)(C₁₋₅-Alkyl), -CO-(N-Heterocycloalkyl), -NHCO-(C₁₋₅-Alkyl), -N(C₁₋₅-Alkyl)-CO-(C₁₋₅-Alkyl), -NHCONH₂, - NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -OCONH₂, - OCONH-(C₁₋₅-Alkyl), -OCON(C₁₋₅-Alkyl)(C₁₋₅-Alkyl), -NHCOO(C₁₋₅-Alkyl), -N(C₁₋₅-Alkyl)COO-(C₁₋₅-Alkyl), -P(O)(C₁₋₅-Alkyl)(C₁₋₅-Alkyl), -P(O)(O(C₁₋₅-Alkyl))(O(C₁₋₅-Alkyl)), -P(O)(O(C₁₋₅-Alkyl))(C₁₋₅-Alkyl), -(C₁₋₅-Alkylen)-CN, -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), -(C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(*N*-Heterocycloalkyl), -(C₁₋₅-Alkylen)-N(C₁₋₅-Halogenalkyl)(C₁₅-Alkyl), -(C₁₋₅-Alkylen)-CO(C₁₋₅-Alkyl), -(C₁₋₅-Alkylen)-CO(C₁₋₅-Halogenalkyl), -(C₁₋₅-Alkylen)-CO-Cycloalkyl, -(C₁₋₅-Alkylen)-CONH₂, -(C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(N-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl), -(C₁₋₅-Alkylen)-N(C₁₋₅-Alkyl)CON(C₁₋₅-Alkyl)(C₁₋₅-Alkyl), -(C₁₋₅-Alkylen)-OCONH₂, -(C₁₋₅-Alkylen)-OCONH-(C₁₋₅-Alkyl), -(C₁₋₅-Alkylen)-OCON(C₁₋₅-Alkyl)(C₁₋₅-Alkyl), -(C₁₋₅-Alkylen)-NHCOO(C₁₋₅-Alkyl) und -(C₁₋₅-Alkylen)-N(C₁₋₅-Alkyl)COO-(C₁₋₅-Alkyl), bevorzugt ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), - S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N-*Heterocycloalkyl), -CO(C₁₋₅-Alkyl), -COO(C₁₋₅-Alkyl), -CONH₂, -CONH(C₁₋₅-Alkyl), - CON(C₁₋₅-Alkyl)(C₁₋₅-Alkyl), -CO-(*N*-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -OCONH₂, -OCONH-(C₁₋₅-Alkyl), -OCON(C₁₋₅-Alkyl)(C₁₋₅-Alkyl), - NHCOO(C₁₋₅-Alkyl), -N(C₁₋₅-Alkyl)COO-(C₁₋₅-Alkyl), -P(O)(C₁₋₅-Alkyl)(C₁₋₅-Alkyl), - P(O)(O(C₁₋₅-Alkyl))(O(C₁₋₅-Alkyl)), -P(O)(O(C₁₋₅-Alkyl))(C₁₋₅-Alkyl), -(C₁₋₅-Alkylen)-CN, - (C₁₋₅-Alkylen)-OH, -(C₁₋₅-Alkylen)-O(C₁₋₅-Alkyl), -(C₁₋₅-Alkylen)-O(C₁₋₅-Halogenalkyl), - (C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), -(C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), - (C₁₋₅-Alkylen)-N(C₁₋₅-Alkyl)(C₁₋₅-Halogenalkyl), -(C₁₋₅-Alkylen)-(N-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CONH₂, -(C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(*N*-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl), -(C₁₋₅-Alkylen)-N(C₁₋₅-Alkyl)CON(C₁₋₅-Alkyl)(C₁₋₅-Alkyl), -(C₁₋₅-Alkylen)-OCONH₂, -(C₁₋₅-Alkylen)-OCONH-(C₁₋₅-Alkyl), -(C₁₋₅-Alkylen)-OCON(C₁₋₅-Alkyl)(C₁₋₅-Alkyl), -(C₁₋₅-Alkylen)-NHCOO(C₁₋₅-Alkyl) und -(C₁₋₅-Alkylen)-N(C₁₋₅-Alkyl)COO-(C₁₋₅-Alkyl), noch bevorzugter ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -(*N*-Heterocycloalkyl), -CO(C₁₋₅-Alkyl), -CONH₂, - CONH(C₁₋₅-Alkyl), -CON(C₁₋₅-Alkyl)(C₁₋₅-Alkyl), -CO-(N-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -P(O)(C₁₋₅-Alkyl)(C₁₋₅-Alkyl), -P(O)(O(C₁₋₅-Alkyl))(O(C₁₋₅-Alkyl)), -P(O)(O(C₁₋₅-Alkyl))(C₁₋₅-Alkyl), -(C₁₋₅-Alkylen) - CN, -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), -(C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(*N*-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CONH₂, -(C₁₋₅-Alkylen)-CONH(C₁₋₅-Alkyl), -(C₁₋₅-Alkylen)-CON(C₁₋₅-Alkyl)(C₁₋₅-Alkyl), - (C₁₋₅-Alkylen)-CO-(*N*-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl) und -(C₁₋₅-Alkylen)-N(C₁₋₅-Alkyl)CON(C₁₋₅-Alkyl)(C₁₋₅-Alkyl), noch bevorzugter ausgewählt aus Halogen, -CN, - OH, C₁₋₅-Alkyl, C₁₋₅-Halogenalkyl, -O(C₁₋₅-Alkyl), -O(C₁₋₅-Halogenalkyl), -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -SH, -S(C₁₋₅-Alkyl), -S(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Halogenalkyl)(C₁₋₅-Alkyl), -CO(C₁₋₅-Alkyl), -CONH₂, - CONH(C₁₋₅-Alkyl) und -CON(C₁₋₅-Alkyl)(C₁₋₅-Alkyl), noch bevorzugter ausgewählt aus Halogen, -CN, -OH, C₁₋₅-Alkyl, C₁₋₅-Halogenalkyl, -O(C₁₋₅-Alkyl), -O(C₁₋₅-Halogenalkyl), - SH, -S(C₁₋₅-Alkyl), -S(C₁₋₅-Halogenalkyl), -NH₂, -NH(C₁₋₅-Alkyl), -NH(C₁₋₅-Halogenalkyl), - N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, - CONH(C₁₋₅-Alkyl) und -CON(C₁₋₅-Alkyl)(C₁₋₅-Alkyl);
X₄ N oder C-R_{C4} ist, wobei R_{C4} ausgewählt ist aus Wasserstoff, Halogen, C₁₋₆-Alkyl, C₂₋₆-Alkinyl, -O(C₁₋₆-Alkyl), -S(C₁₋₆-Alkyl), -NH(C₁₋₆-Alkyl), -N(C₁₋₆-Alkyl)(C₁₋₆-Alkyl), -CO(C₁₋₆-Alkyl), C₁₋₆-Halogenalkyl, -O(C₁₋₆-Halogenalkyl), -S(C₁₋₆-Halogenalkyl), -NH(C₁₋₆-Halogenalkyl), -N(C₁₋₆-Halogenalkyl)₂, -CO(C₁₋₆-Halogenalkyl), -(C₀₋₃-Alkylen)cycloalkyl, - O-(C₀₋₃-Alkylen)-Cycloalkyl, -CO-(C₀₋₃-Alkylen)-Cycloalkyl, -(C₀₋₃-Alkylen)cycloalkenyl, - O-(C₀₋₃-Alkylen)-Cycloalkenyl, -CO-(C₀₋₃-Alkylen)-Cycloalkenyl, -(C₀₋₃-Alkylen)-Heterocycloalkyl, -O-(C₀₋₃-Alkylen)-Heterocycloalkyl, -CO-(C₀₋₃-Alkylen)-Heterocycloalkyl, -(C₀₋₃-Alkylen)-Heterocycloalkenyl, -O-(C₀₋₃-Alkylen)-Heterocycloalkenyl, -CO-(C₀₋₃-Alkylen)-Heterocycloalkenyl, -(C₀₋₃-Alkylen)-Aryl, -O-(C₀₋₃-Alkylen)-Aryl, -CO-(C₀₋₃-Alkylen)-Aryl, -(C₀₋₃-Alkylen)-Heteroaryl, -O-(C₀₋₃-Alkylen)-Heteroaryl und -CO-(C₀₋₃-Alkylen)-Heteroaryl,
wobei das Alkyl oder das Alkinyl optional mit einer oder mehreren Gruppen substituiert ist, die unabhängig voneinander ausgewählt sind aus Halogen, -CN, -OH, -O(C₁₋₅-Alkyl), -O(C₁₋₅-Halogenalkyl), C₁₋₅-Halogenalkyl, -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(*N*-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl) und -N(C₁₋₅-Alkyl)CON(C₁₋₅-Alkyl)(C₁₋₅-Alkyl), bevorzugt ausgewählt aus Halogen, -CN, -OH, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), - N(C₁₋₅-Alkyl)(C₁₋₅-Alkyl), -N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl), und
wobei das Cycloalkyl, Cycloalkenyl, Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(N-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl) und -N(C₁₋₅-Alkyl)CON(C₁₋₅-Alkyl)(C₁₋₅-Alkyl), -(C₁₋₅-Alkylen)-CN, -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(*N*-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CONH₂, - (C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(*N*-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl) und -(C₁₋₅-Alkylen)-N(C₁₋₅-Alkyl)CON(C₁₋₅-Alkyl)(C₁₋₅-Alkyl), bevorzugt ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl);
X₅ N oder C-R_{C5} ist,
wobei R_{C5} ausgewählt ist aus Wasserstoff, Halogen, C₁₋₆-Alkyl, -O(C₁₋₆-Alkyl), -S(C₁₋₆-Alkyl), -NH(C₁₋₆-Alkyl), -N(C₁₋₆-Alkyl)(C₁₋₆-Alkyl) und C₁₋₆-Halogenalkyl;
R₄ Y_{R5}-R_{R5} ist,
wobei Y_{R5} ausgewählt ist aus einer kovalenten Bindung, C₁₋₄-Alkylen, C₂₋₄-Alkenylen und C₂₋₄-Alkinylen, wobei das Alkylen, das Alkenylen und das Alkinylen jeweils optional mit einer oder mehreren Gruppen substituiert sind, die unabhängig voneinander ausgewählt sind aus Halogen, -CN, -OH, -O(C₁₋₅-Alkyl), -O(C₁₋₅-Halogenalkyl), -SH, -S(C₁₋₅-Alkyl), -SO(C₁₋₅-Alkyl), -SO₂(C₁₋₅-Alkyl), -S(C₁₋₅-Halogenalkyl), -SO(C₁₋₅-Halogenalkyl), -SO₂(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl) und -N(C₁₋₅-Halogenalkyl)(C₁₋₅-Alkyl), und ferner, wobei eine oder mehrere -CH₂-Einheiten, die in dem Alkylen, dem Alkenylen oder dem Alkinylen umfasst sind, jeweils optional durch eine Gruppe ersetzt sind, die unabhängig voneinander ausgewählt ist aus -O-; NH-; N(C₁₋₅-Alkyl)-; CO-; -COO-; S-; -SO- und SO₂-, und
wobei R_{R5} ausgewählt ist aus C₁₋₁₂-Alkyl, C₂₋₁₂-Alkenyl, C₂₋₁₂-Alkinyl, Cycloalkyl, Cycloalkenyl, Heterocycloalkyl, Heterocycloalkenyl, Aryl und Heteroaryl,
wobei das Alkyl, Alkenyl oder Alkinyl optional mit einer oder mehreren Gruppen substituiert ist, die unabhängig voneinander ausgewählt sind aus Halogen, -CN, -OH, -O(C₁₋₅-Alkyl), - O(C₁₋₅-Halogenalkyl), -SH, -S(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), - CONH₂, -CONH(C₁₋₅-Alkyl) und -CON(C₁₋₅-Alkyl)(C₁₋₅-Alkyl), und wobei das Cycloalkyl, Cycloalkenyl, Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, -C₁₋₅-Alkyl, -C₁₋₅-Halogenalkyl, -O(C₁₋₅-Alkyl), -O(C₁₋₅-Halogenalkyl), - SH, -S(C₁₋₅-Alkyl), -SO(C₁₋₅-Alkyl), -SO₂(C₁₋₅-Alkyl), -S(C₁₋₅-Halogenalkyl), -SO(C₁₋₅-Halogenalkyl), -SO₂(C₁₋₅-Halogenalkyl), -NH₂, -NH(C₁₋₅-Alkyl), -NH(C₁₋₅-Halogenalkyl), - N(C₁₋₅-Alkyl)(C₁₋₅-Alkyl), -N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl).

2. Verbindung nach Anspruch 1, wobei R₁ ausgewählt ist aus der Gruppe bestehend aus Chlor, Fluor, Cyano, Formyl, (C₁₋₂)-Alkyl, (C₂)-Alkenyl, (C₂)-Alkinyl und (C₁₋₂)-Halogenalkyl, bevorzugt wobei R₁ ausgewählt ist aus der Gruppe besthend aus Cyano, (C₁₋₂)-Alkyl und (C₁₋₂)-Halogenalkyl noch bevorzugter, wobei R₁ ausgewählt ist aus der Gruppe bestehend aus Cyano, Methyl und Fluormethyl, sogar noch bevorzugter
wobei R₁ Cyano ist, wobei R₁ Methyl ist, oder
wobei R₁ Fluormethyl ist.

3. Verbindung nach Anspruch 1 oder 2, wobei Y_{C2} ausgewählt ist aus einer kovalenten Bindung, C₁₋₅-Alkylen, C₂₋₅-Alkenylen, C₂₋₅-Alkinylen, Cycloalkylen und Heterocycloalkylen, wobei das Alkylen, das Alkenylen und das Alkinylen jeweils optional mit einer oder mehreren Gruppen substituiert sind, die unabhängig voneinander ausgewählt sind aus Halogen, CN, OH, O(C₁₋₅-Alkyl), -O(C₁₋₅-Halogenalkyl), C₁₋₅-Halogenalkyl, SH, S(C₁₅-Alkyl), -S(C₁₋₅-Halogenalkyl), NH₂, NH(C₁₋₅-Alkyl), -NH(C₁₋₅-Halogenalkyl), N(C₁₋₅-Alkyl)(C₁₋₅-Alkyl), N(C₁₋₅-Halogenalkyl)(C₁₋₅-Alkyl), -(*N*-Heterocycloalkyl), -CO(C₁₋₅-Alkyl), CONH₂, CONH(C₁₋₅-Alkyl), CON(C₁₋₅-Alkyl)(C₁₋₅-Alkyl), -CO-(N-Heterocycloalkyl), NHCO-(C₁₋₅-Alkyl), N(C₁₋₅-Alkyl)-CO-(C₁₋₅-Alkyl), NHCONH₂, NHCONH-(C₁₋₅-Alkyl), NHCON(C₁₋₅-Alkyl)(C₁₋₅-Alkyl), N(C₁₋₅-Alkyl) CONH₂, N(C₁₋₅-Alkyl)CONH-(C₁₋₅-Alkyl) und N(C₁₋₅-Alkyl)CON(C₁₋₅-Alkyl)(C₁₋₅-Alkyl), bevorzugt ausgewählt aus Halogen, CN, OH, O(C₁₋₅-Alkyl), SH, S(C₁₅-Alkyl), NH₂, NH(C₁₋₅-Alkyl) und N(C₁₋₅-Alkyl)(C₁₋₅-Alkyl), und ferner, wobei eine oder mehrere -CH₂-Einheiten, die in dem Alkylen, dem Alkenylen oder dem Alkinylen umfasst sind, jeweils optional durch eine Gruppe ersetzt sind, die unabhängig ausgewählt ist aus -O-, NH-, N(C₁₋₅-Alkyl)-, CO-, S-, -SO- und SO₂-, und wobei das Cycloalkylen und das Heterocycloalkylen jeweils optional mit einer oder mehreren Gruppen substituiert sind, die unabhängig voneinander ausgewählt sind aus Halogen, CN, OH, C₁₋₅-Alkyl, C₁₋₅-Halogenalkyl, O(C₁₋₅-Alkyl), -O(C₁₋₅-Halogenalkyl), SH, S(C₁₅-Alkyl), -S(C₁₋₅-Halogenalkyl), NH₂, NH(C₁₋₅-Alkyl), -NH(C₁₋₅-Halogenalkyl), N(C₁₋₅-Alkyl)(C₁₋₅-Alkyl), N(C₁₋₅-Halogenalkyl)(C₁₋₅-Alkyl), -(*N*-Heterocycloalkyl), -CO(C₁₋₅-Alkyl), CONH₂, CONH(C₁₋₅-Alkyl), CON(C₁₋₅-Alkyl)(C₁₋₅-Alkyl), -CO-(*N*-Heterocycloalkyl), NHCO-(C₁₋₅-Alkyl), N(C₁₋₅-Alkyl)-CO-(C₁₋₅-Alkyl), NHCONH₂, NHCONH-(C₁₋₅-Alkyl), NHCON(C₁₋₅-Alkyl)(C₁₋₅-Alkyl), N(C₁₋₅-Alkyl)CONH₂, N(C₁₋₅-Alkyl)CONH-(C₁₋₅-Alkyl) und N(C₁₋₅-Alkyl)CON(C₁₋₅-Alkyl)(C₁₋₅-Alkyl), -(C₁₋₅-Alkylen)-CN, -(C₁₋₅-Alkylen)OH, -(C₁₋₅-Alkylen)O(C₁₋₅-Alkyl), -(C₁₋₅-Alkylen)-O(C₁₋₅-Halogenalkyl), -(C₁₋₅-Alkylen)SH, -(C₁₋₅-Alkylen)S(C₁₋₅-Alkyl), -(C₁₋₅-Alkylen)-S(C₁₋₅-Halogenalkyl), -(C₁₋₅-Alkylen)NH₂, -(C₁₋₅-Alkylen)NH(C₁₋₅-Alkyl), -(C₁₋₅-Alkylen)-NH(C₁₋₅-Halogenalkyl), -(C₁₋₅-Alkylen)N(C₁₋₅-Alkyl)(C₁₋₅-Alkyl), -(C₁₋₅-Alkylen)N(C₁₋₅-Alkyl)(C₁₋₅-Halogenalkyl), -(C₁₋₅-Alkylen)(*N-*Heterocycloalkyl), -(C₁₋₅-Alkylen)N(C₁₋₅-Halogenalkyl)(C₁₋₅-Alkyl), -(C₁₋₅-Alkylen)-CO(C₁₋₅-Alkyl), -(C₁₋₅-Alkylen)CONH₂, -(C₁₋₅-Alkylen)CONH(C₁₋₅-Alkyl), -(C₁₋₅-Alkylen)CON(C₁₋₅-Alkyl)(C₁₋₅-Alkyl), -(C₁₋₅-Alkylen)CO-(*N*-Heterocycloalkyl), -(C₁₋₅-Alkylen)NHCO-(C₁₋₅-Alkyl), -(C₁₋₅-Alkylen)N(C₁₋₅-Alkyl)-CO-(C₁₋₅-Alkyl), -(C₁₋₅-Alkylen)NHCONH₂, -(C₁₋₅-Alkylen)NHCONH-(C₁₋₅-Alkyl), -(C₁₋₅-Alkylen)NHCON(C₁₋₅-Alkyl)(C₁₋₅-Alkyl), -(C₁₋₅-Alkylen)N(C₁₋₅-Alkyl)CONH₂, -(C₁₋₅-Alkylen)N(C₁₋₅-Alkyl)CONH-(C₁₋₅-Alkyl) und -(C₁₋₅-Alkylen)N(C₁₋₅-Alkyl) CON(C₁₋₅-Alkyl)(C₁₋₅-Alkyl), bevorzugt ausgewählt aus Halogen, CN, OH, C₁₋₅-Alkyl, O(C₁₋₅-Alkyl), SH, S(C₁₅-Alkyl), NH₂, NH(C₁₋₅-Alkyl) und N(C₁₋₅-Alkyl)(C₁₋₅-Alkyl), oder
wobei -Y_{C2}-R_{C2} ausgewählt ist aus -O-C₁₋₁₂-Alkyl, -NH-C₁₋₁₂-Alkyl, -N(C₁₋₅-Alkyl)-C₁₋₁₂-Alkyl, -O-C₂₋₁₂-Alkenyl, -NH-C₂₋₁₂-Alkenyl, -N(C₁₋₅-Alkyl)-C₂₋₁₂-Alkenyl, -O-C₂₋₁₂-Alkinyl, - NH-C₂₋₁₂-Alkinyl, -N(C₁₋₅-Alkyl)-C₂₋₁₂-Alkinyl, (C₀₋₃-Alkylen)-Cycloalkyl, -CO-(C₀₋₃-Alkylen)cycloalkyl, (C₀₋₃-Alkylen)-CO-Cycloalkyl, -CONH-(C₀₋₃-Alkylen)cycloalkyl, -(C₀₋₃-Alkylen)-CONH-Cycloalkyl, -NHCO-(C₀₋₃-Alkylen)cycloalkyl, (C₀₋₃-Alkylen)-NHCO-Cycloalkyl, -NH-(C₀₋₃-Alkylen)cycloalkyl, -(C₀₋₃-Alkylen)-NH-Cycloalkyl, -O-(C₀₋₃-Alkylen)cycloalkyl, -(C₀₋₃-Alkylen)-O-Cycloalkyl, -SO₂-(C₀₋₃-Alkylen)cycloalkyl, -(C₀₋₃-Alkylen)-SO₂-Cycloalkyl, -CONH-Cycloalkyl, -NHCO-Cycloalkyl, -NH-Cycloalkyl, -O-Cycloalkyl, -CO-Cycloalkyl, -SO₂-Cycloalkyl, (C₀₋₃-Alkylen)-Cycloalkenyl, -CO-(C₀₋₃-Alkylen)cycloalkenyl, (C₀₋₃-Alkylen)-CO-Cycloalkenyl, -CONH-(C₀₋₃-Alkylen)cycloalkenyl, -(C₀₋₃-Alkylen)-CONH-Cycloalkenyl, -NHCO-(C₀₋₃-Alkylen)cycloalkenyl, (C₀₋₃-Alkylen)-NHCO-Cycloalkenyl, -NH-(C₀₋₃-Alkylen)cycloalkenyl, -(C₀₋₃-Alkylen)-NH-Cycloalkenyl, -O-(C₀₋₃-Alkylen)cycloalkenyl, -(C₀₋₃-Alkylen)-O-Cycloalkenyl, -SO₂-(C₀₋₃-Alkylen)cycloalkenyl, -(C₀₋₃-Alkylen)-SO₂-Cycloalkenyl, -CONH-Cycloalkenyl, -NHCO-Cycloalkenyl, -NH-Cycloalkenyl, -O-Cycloalkenyl, -CO-Cycloalkenyl, -SO₂-Cycloalkenyl, - (C₀₋₃-Alkylen)-Heterocycloalkyl, -CO-(C₀₋₃-Alkylen)-Heterocycloalkyl, -(C₀₋₃-Alkylen)-CO-Heterocycloalkyl, -CONH-(C₀₋₃-Alkylen)-Heterocycloalkyl, -(C₀₋₃-Alkylen)-CONH-Heterocycloalkyl, -NHCO-(C₀₋₃-Alkylen)heterocycloalkyl, -(C₀₋₃-Alkylen)-NHCO-Heterocycloalkyl, -NH-(C₀₋₃-Alkylen)heterocycloalkyl, -(C₀₋₃-Alkylen)-NH-Heterocycloalkyl, -O-(C₀₋₃-Alkylen)heterocycloalkyl, -(C₀₋₃-Alkylen)-O-Heterocycloalkyl, -SO₂-(C₀₋₃-Alkylen)heterocycloalkyl, -(C₀₋₃-Alkylen)-SO₂-Heterocycloalkyl, -CONH-Heterocycloalkyl, - NHCO-Heterocycloalkyl, -NH-Heterocycloalkyl, -O-Heterocycloalkyl, -CO-Heterocycloalkyl, -SO₂-Heterocycloalkyl, -(C₀₋₃-Alkylen)-Heterocycloalkenyl, -CO-(C₀₋₃-Alkylen)-Heterocycloalkenyl, -(C₀₋₃-Alkylen)-CO-Heterocycloalkenyl, -CONH-(C₀₋₃-Alkylen)heterocycloalkenyl, -(C₀₋₃-Alkylen)-CONH-Heterocycloalkenyl, -NHCO-(C₀₋₃-Alkylen)heterocycloalkenyl, -(C₀₋₃-Alkylen)-NHCO-Heterocycloalkenyl, -NH-(C₀₋₃-Alkylen)heterocycloalkenyl, -(C₀₋₃-Alkylen)-NH-Heterocycloalkenyl, -O-(C₀₋₃-Alkylen)heterocycloalkenyl, -(C₀₋₃-Alkylen)-O-Heterocycloalkenyl, -SO₂-(C₀₋₃-Alkylen)heterocycloalkenyl, -(C₀₋₃-Alkylen)-SO₂-Heterocycloalkenyl, -CONH-Heterocycloalkenyl, -NHCO-Heterocycloalkenyl, -NH-Heterocycloalkenyl, -O-Heterocycloalkenyl, -CO-Heterocycloalkenyl, -SO₂-Heterocycloalkenyl, (C₀₋₃-Alkylen)aryl, - CO-(C₀₋₃-Alkylen)aryl, -(C₀₋₃-Alkylen)-CO-Aryl, -CONH-(C₀₋₃-Alkylen)aryl, -(C₀₋₃-Alkylen)-CONH-Aryl, -NHCO-(C₀₋₃-Alkylen)aryl, -(C₀₋₃-Alkylen)-NHCO-Aryl, -NH-(C₀₋₃-Alkylen)aryl, -(C₀₋₃-Alkylen)-NH-Aryl, -O-(C₀₋₃-Alkylen)aryl, -(C₀₋₃-Alkylen)-O-Aryl, -SO₂-(C₀₋₃-Alkylen)aryl, -(C₀₋₃-Alkylen)-SO₂-Aryl, -CONH-Aryl, -NHCO-Aryl, -NH-Aryl, -O-Aryl, -CO-Aryl, -SO₂-Aryl, -(C₀₋₃-Alkylen)heteroaryl, -CO-(C₀₋₃-Alkylen)heteroaryl, -(C₀₋₃-Alkylen)-CO-Heteroaryl, -CONH-(C₀₋₃-Alkylen)heteroaryl, -(C₀₋₃-Alkylen)-CONH-Heteroaryl, -NHCO-(C₀₋₃-Alkylen)heteroaryl, -(C₀₋₃-Alkylen)-NHCO-Heteroaryl, -NH-(C₀₋₃-Alkylen)heteroaryl, (C₀₋₃-Alkylen)-NH-Heteroaryl, -O-(C₀₋₃-Alkylen)heteroaryl, -(C₀₋₃-Alkylen)-O-Heteroaryl, -SO₂-(C₀₋₃-Alkylen)heteroaryl, -(C₀₋₃-Alkylen)-SO₂-Heteroaryl, - CONH-Heteroaryl, -NHCO-Heteroaryl, -NH-Heteroaryl, -O-Heteroaryl, -CO-Heteroaryl und - SO₂-Heteroaryl, wobei bevorzugt -Y_{C2}-R_{C2} ausgewählt ist aus -O-C₁₋₁₂-Alkyl, -NH-C₁₋₁₂-Alkyl, -N(C₁₋₅-Alkyl)-C₁₋₁₂-Alkyl, -O-C₂₋₁₂-Alkenyl, -NH-C₂₋₁₂-Alkenyl, -N(C₁₋₅-Alkyl)-C₂-₁₂-Alkenyl, -O-C₂₋₁₂-Alkinyl, -NH-C₂₋₁₂-Alkinyl, -N(C₁₋₅-Alkyl)-C₂₋₁₂-Alkinyl, (C₀₋₃-Alkylen)-Cycloalkyl, -CO-(C₀₋₃-Alkylen)cycloalkyl, (C₀₋₃-Alkylen)-CO-Cycloalkyl, -CONH-(C₀₋₃-Alkylen)cycloalkyl, -(C₀₋₃-Alkylen)-CONH-Cycloalkyl, -NHCO-(C₀₋₃-Alkylen)cycloalkyl, (C₀₋₃-Alkylen)-NHCO-Cycloalkyl, -NH-(C₀₋₃-Alkylen)cycloalkyl, -(C₀₋₃-Alkylen)-NH-Cycloalkyl, -O-(C₀₋₃-Alkylen)cycloalkyl, -(C₀₋₃-Alkylen)-O-Cycloalkyl, -SO₂-(C₀₋₃-Alkylen)cycloalkyl, -(C₀₋₃-Alkylen)-SO₂-Cycloalkyl, -CONH-Cycloalkyl, -NHCO-Cycloalkyl, -NH-Cycloalkyl, -O-Cycloalkyl, -CO-Cycloalkyl, -SO₂-Cycloalkyl, -(C₀₋₃-Alkylen)-Heterocycloalkyl, -CO-(C₀₋₃-Alkylen)heterocycloalkyl, -(C₀₋₃-Alkylen)-CO-Heterocycloalkyl, -CONH-(C₀₋₃-Alkylen)heterocycloalkyl, -(C₀₋₃-Alkylen)-CONH-Heterocycloalkyl, -NHCO-(C₀₋₃-Alkylen)heterocycloalkyl, -(C₀₋₃-Alkylen)-NHCO-Heterocycloalkyl, -NH-(C₀₋₃-Alkylen)heterocycloalkyl, -(C₀₋₃-Alkylen)-NH-Heterocycloalkyl, -O-(C₀₋₃-Alkylen)heterocycloalkyl, -(C₀₋₃-Alkylen)-O-Cycloalkyl, -SO₂-(C₀₋₃-Alkylen)heterocycloalkyl, -(C₀₋₃-Alkylen)-SO₂-Heterocycloalkyl, -CONH-Heterocycloalkyl, - NHCO-Heterocycloalkyl, -NH-Heterocycloalkyl, -O-Heterocycloalkyl, -CO-Heterocycloalkyl, -SO₂-Heterocycloalkyl, (C₀₋₃-Alkylen)aryl, -CO-(C₀₋₃-Alkylen)aryl, -(C₀₋₃-Alkylen)-CO-Aryl, -CONH-(C₀₋₃-Alkylen)aryl, -(C₀₋₃-Alkylen)-CONH-Aryl, -NHCO-(C₀₋₃-Alkylen)aryl, -(C₀₋₃-Alkylen)-NHCO-Aryl, -NH-(C₀₋₃-Alkylen)aryl, -(C₀₋₃-Alkylen)-NH-Aryl, -O-(C₀₋₃-Alkylen)aryl, -(C₀₋₃-Alkylen)-O-Aryl, -SO₂-(C₀₋₃-Alkylen)aryl, -(C₀₋₃-Alkylen)-SO₂-Aryl, -CONH-Aryl, -NHCO-Aryl, -NH-Aryl, -O-Aryl, -CO-Aryl, -SO₂-Aryl, -(C₀₋₃-Alkylen)heteroaryl, -CO-(C₀₋₃-Alkylen)heteroaryl, -(C₀₋₃-Alkylen)-CO-Heteroaryl, -CONH-(C₀₋₃-Alkylen)heteroaryl, -(C₀₋₃-Alkylen)-CONH-Heteroaryl, -NHCO-(C₀₋₃-Alkylen)heteroaryl, -(C₀₋₃-Alkylen)-NHCO-Heteroaryl, -NH-(C₀₋₃-Alkylen)heteroaryl, (C₀₋₃-Alkylen)-NH-Heteroaryl, -O-(C₀₋₃-Alkylen)heteroaryl, -(C₀₋₃-Alkylen)-O-Heteroaryl, -SO₂-(C₀₋₃-Alkylen)heteroaryl, -(C₀₋₃-Alkylen)-SO₂-Heteroaryl, -CONH-Heteroaryl, -NHCO-Heteroaryl, -NH-Heteroaryl, -O-Heteroaryl, -CO-Heteroaryl und -SO₂-Heteroaryl, wobei das Alkyl, Alkenyl oder Alkinyl optional mit einer oder mehreren Gruppen substituiert ist, die unabhängig voneinander ausgewählt sind aus Halogen, -CN, -OH, -O(C₁₋₅-Alkyl), -O(C₁₋₅-Halogenalkyl), C₁₋₅-Halogenalkyl, -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(*N*-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -P(O)(C₁₋₅-Alkyl)(C₁₋₅-Alkyl), - P(O)(O(C₁₋₅-Alkyl))(O(C₁₋₅-Alkyl)) und -P(O)(O(C₁₋₅-Alkyl))(C₁₋₅-Alkyl), bevorzugt ausgewählt aus Halogen, -CN, -OH, -O(C₁₋₅-Alkyl), -O(C₁₋₅-Halogenalkyl), -C₁₋₅-Halogenalkyl, -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, -CONH(C₁₋₅-Alkyl) und -CON(C₁₋₅-Alkyl)(C₁₋₅-Alkyl) und wobei das Cycloalkyl, Cycloalkenyl, Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -(*N*-Heterocycloalkyl), -CO(C₁₋₅-Alkyl), -CO(C₁₋₅-Halogenalkyl), -CO-Cycloalkyl, -COO(C₁₋₅-Alkyl), -COO(C₁₋₅-Halogenalkyl), -COO-Cycloalkyl, -CONH₂, -CONH(C₁₋₅-Alkyl), -CON(C₁₋₅-Alkyl)(C₁₋₅-Alkyl), -CO-(*N-*Heterocycloalkyl), -NHCO-(C₁₋₅-Alkyl), -N(C₁₋₅-Alkyl)-CO-(C₁₋₅-Alkyl), -NHCONH₂, - NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -OCONH₂, - OCONH-(C₁₋₅-Alkyl), -OCON(C₁₋₅-Alkyl)(C₁₋₅-Alkyl), -NHCOO(C₁₋₅-Alkyl), -N(C₁₋₅-Alkyl)COO-(C₁₋₅-Alkyl), -P(O)(C₁₋₅-Alkyl)(C₁₋₅-Alkyl), -P(O)(O(C₁₋₅-Alkyl))(O(C₁₋₅-Alkyl)), -P(O)(O(C₁₋₅-Alkyl))(C₁₋₅-Alkyl), -(C₁₋₅-Alkylen)-CN, -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), -(C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(N-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CO(C₁₋₅-Halogenalkyl), -(C₁₋₅-Alkylen)-CO-Cycloalkyl, -(C₁₋₅-Alkylen)-CONH₂, -(C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(N-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl), -(C₁₋₅-Alkylen)-N(C₁₋₅-Alkyl)CON(C₁₋₅-Alkyl)(C₁₋₅-Alkyl), -(C₁₋₅-Alkylen)-OCONH₂, -(C₁₋₅-Alkylen)-OCONH-(C₁₋₅-Alkyl), -(C₁₋₅-Alkylen)-OCON(C₁₋₅-Alkyl)(C₁₋₅-Alkyl), -(C₁₋₅-Alkylen)-NHCOO(C₁₋₅-Alkyl) und -(C₁₋₅-Alkylen)-N(C₁₋₅-Alkyl)COO-(C₁₋₅-Alkyl),
oder
wobei -Y_{C2}-R_{C2} ausgewählt ist aus -(C₀₋₃-Alkylen)-Heterocycloalkyl, -CO-(C₀₋₃-Alkylen)heterocycloalkyl, -(C₀₋₃-Alkylen)-CO-Heterocycloalkyl, -CONH-(C₀₋₃ -Alkylen)heterocycloalkyl, -(C₀₋₃-Alkylen)-CONH-Heterocycloalkyl, -NHCO-(C₀₋₃-Alkylen)heterocycloalkyl, -(C₀₋₃-Alkylen)-NHCO-Heterocycloalkyl, -NH-(C₀₋₃-Alkylen)heterocycloalkyl, -(C₀₋₃-Alkylen)-NH-Heterocycloalkyl, -O-(C₀₋₃-Alkylen)heterocycloalkyl, (C₀₋₃-Alkylen)-O-Cycloalkyl, (C₀₋₃-Alkylen)-O-Heterocycloalkyl, - SO₂-(C₀₋₃-Alkylen)heterocycloalkyl, -(C₀₋₃-Alkylen)-SO₂-Heterocycloalkyl, -CONH-Heterocycloalkyl, -NHCO-Heterocycloalkyl, -NH-Heterocycloalkyl, -O-Heterocycloalkyl, - CO-Heterocycloalkyl, -SO₂-Heterocycloalkyl, -(C₀₋₃-Alkylen)-Heterocycloalkenyl, -CO-(C₀₋₃-Alkylen)heterocycloalkenyl, -(C₀₋₃-Alkylen)-CO-Heterocycloalkenyl, -CONH-(C₀₋₃-Alkylen)heterocycloalkenyl, -(C₀₋₃-Alkylen)-CONH-Heterocycloalkenyl, -NHCO-(C₀₋₃-Alkylen)heterocycloalkenyl, -(C₀₋₃-Alkylen)-NHCO-Heterocycloalkenyl, -NH-(C₀₋₃-Alkylen)heterocycloalkenyl, -(C₀₋₃-Alkylen)-NH-Heterocycloalkenyl, -O-(C₀₋₃-Alkylen)heterocycloalkenyl, (C₀₋₃-Alkylen)-O-heterocycloalkenyl, -SO₂-(C₀₋₃-Alkylen)heterocycloalkenyl, -(C₀₋₃-Alkylen)-SO₂-Heterocycloalkenyl, -CONH-Heterocycloalkenyl, -NHCO-Heterocycloalkenyl, -NH-Heterocycloalkenyl, -O-Heterocycloalkenyl, -CO-Heterocycloalkenyl, -SO₂-Heterocycloalkenyl, -(C₀₋₃-Alkylen)aryl, - CO-(C₀₋₃-Alkylen)aryl, -(C₀₋₃-Alkylen)-CO-Aryl, -CONH-(C₀₋₃-Alkylen)aryl, -(C₀₋₃-Alkylen)-CONH-Aryl, -NHCO-(C₀₋₃-Alkylen)aryl, -(C₀₋₃-Alkylen)-NHCO-Aryl, -NH-(C₀₋₃-Alkylen)aryl, -(C₀₋₃-Alkylen)-NH-Aryl, -O-(C₀₋₃-Alkylen)aryl, -(C₀₋₃-Alkylen)-O-Aryl, -SO₂-(C₀₋₃-Alkylen)aryl, -(C₀₋₃-Alkylen)-SO₂-Aryl, -CONH-Aryl, -NHCO-Aryl, -NH-Aryl, -O-Aryl, -CO-Aryl, -SO₂-Aryl, -(C₀₋₃-Alkylen)heteroaryl, -CO-(C₀₋₃-Alkylen)heteroaryl, -(C₀₋₃-Alkylen)-CO-Heteroaryl, -CONH-(C₀₋₃-Alkylen)heteroaryl, -(C₀₋₃-Alkylen)-CONH-Heteroaryl, -NHCO-(C₀₋₃-Alkylen)heteroaryl, -(C₀₋₃-Alkylen)-NHCO-Heteroaryl, -NH-(C₀₋₃-Alkylen)heteroaryl, -(C₀₋₃-Alkylen)-NH-Heteroaryl, -O-(C₀₋₃-Alkylen)heteroaryl, -(C₀₋₃-Alkylen)-O-Heteroaryl, -SO₂-(C₀₋₃-Alkylen)heteroaryl, -(C₀₋₃-Alkylen)-SO₂-Heteroaryl, - CONH-Heteroaryl, -NHCO-Heteroaryl, -NH-Heteroaryl, -O-Heteroaryl, -CO-Heteroaryl und - SO₂-Heteroaryl, wobei bevorzugt -Y_{C2}-R_{C2} ausgewählt ist aus -(C₀₋₃-Alkylen)-Heterocycloalkyl, -CO-(C₀₋₃-Alkylen)heterocycloalkyl, -(C₀₋₃-Alkylen)-CO-Heterocycloalkyl, -CONH-(C₀₋₃-Alkylen)heterocycloalkyl, -(C₀₋₃-Alkylen)-CONH-Heterocycloalkyl, -NHCO-(C₀₋₃-Alkylen)heterocycloalkyl, -(C₀₋₃-Alkylen)-NHCO-Heterocycloalkyl, -NH-(C₀₋₃ -Alkylen)heterocycloalkyl, -(C₀₋₃-Alkylen)-NH-Heterocycloalkyl, -O-(C₀₋₃-Alkylen)heterocycloalkyl, (C₀₋₃-Alkylen)-O-Cycloalkyl, -SO₂-(C₀₋₃-Alkylen)heterocycloalkyl, -(C₀₋₃-Alkylen)-SO₂-Heterocycloalkyl, -CONH-Heterocycloalkyl, -NHCO-Heterocycloalkyl, - NH-Heterocycloalkyl, -O-Heterocycloalkyl, -CO-Heterocycloalkyl, -SO₂-Heterocycloalkyl, - (C₀₋₃-Alkylen)aryl, -CO-(C₀₋₃-Alkylen)aryl, -(C₀₋₃-Alkylen)-CO-Aryl, -CONH-(C₀₋₃-Alkylen)aryl, -(C₀₋₃-Alkylen)-CONH-Aryl, -NHCO-(C₀₋₃-Alkylen)aryl, -(C₀₋₃-Alkylen)-NHCO-Aryl, -NH-(C₀₋₃-Alkylen)aryl, -(C₀₋₃-Alkylen)-NH-Aryl, -O-(C₀₋₃-Alkylen)aryl, -(C₀₋₃-Alkylen)-O-Aryl, -SO₂-(C₀₋₃-Alkylen)aryl, -(C₀₋₃-Alkylen)-SO₂-Aryl, -CONH-Aryl, -NHCO-Aryl, -NH-Aryl, -O-Aryl, -CO-Aryl, -SO₂-Aryl, -(C₀₋₃-Alkylen)heteroaryl, -CO-(C₀₋₃-Alkylen)heteroaryl, -(C₀₋₃-Alkylen)-CO-Heteroaryl, -CONH-(C₀₋₃-Alkylen)heteroaryl, - (C₀₋₃-Alkylen)-CONH-Heteroaryl, -NHCO-(C₀₋₃-Alkylen)heteroaryl, -(C₀₋₃-Alkylen)-NHCO-Heteroaryl, -NH-(C₀₋₃-Alkylen)heteroaryl, -(C₀₋₃-Alkylen)-NH-Heteroaryl, -O-(C₀₋₃-Alkylen)heteroaryl, -(C₀₋₃-Alkylen)-O-Heteroaryl, -SO₂-(C₀₋₃-Alkylen)heteroaryl, -(C₀₋₃-Alkylen)-SO₂-Heteroaryl, -CONH-Heteroaryl, -NHCO-Heteroaryl, -NH-Heteroaryl, -O-Heteroaryl, -CO-Heteroaryl und -SO₂-Heteroaryl, wobei das Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), - S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N-*Heterocycloalkyl), -CO(C₁₋₅-Alkyl), -CO(C₁₋₅-Halogenalkyl), -CO-Cycloalkyl, -COO(C₁₋₅-Alkyl), -COO(C₁₋₅-Halogenalkyl), -COO-Cycloalkyl, -CONH₂, -CONH(C₁₋₅-Alkyl), - CON(C₁₋₅-Alkyl)(C₁₋₅-Alkyl), -CO-(*N*-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -OCONH₂, -OCONH-(C₁₋₅-Alkyl), -OCON(C₁₋₅-Alkyl)(C₁₋₅-Alkyl), - NHCOO(C₁₋₅-Alkyl), -N(C₁₋₅-Alkyl)COO-(C₁₋₅-Alkyl), -P(O)(C₁₋₅-Alkyl)(C₁₋₅-Alkyl), - P(O)(O)C₁₋₅-Alkyl))(O(C₁₋₅-Alkyl)), -P(O)(O(C₁₋₅-Alkyl))(C₁₋₅-Alkyl), -(C₁₋₅-Alkylen)-CN, - (C₁₋₅-Alkylen)-OH, - (C₁₋₅-Alkylen)-O(C₁₋₅-Alkyl), -(C₁₋₅-Alkylen)-O(C₁₋₅-Halogenalkyl), - (C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), -(C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), - (C₁₋₅-Alkylen)-N(C₁₋₅-Alkyl)(C₁₋₅-Halogenalkyl), -(C₁₋₅-Alkylen)-(*N*-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CO(C₁₋₅-Halogenalkyl), -(C₁₋₅-Alkylen)-CO-Cycloalkyl, -(C₁₋₅-Alkylen)-CONH₂, - (C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(*N*-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl), -(C₁₋₅-Alkylen)-N(C₁₋₅-Alkyl)CON(C₁₋₅-Alkyl)(C₁₋₅-Alkyl), -(C₁₋₅-Alkylen)-OCONH₂, -(C₁₋₅-Alkylen)-OCONH-(C₁₋₅-Alkyl), -(C₁₋₅-Alkylen)-OCON(C₁₋₅-Alkyl)(C₁₋₅-Alkyl), -(C₁₋₅-Alkylen)-NHCOO(C₁₋₅-Alkyl) und -(C₁₋₅-Alkylen)-N(C₁₋₅-Alkyl)COO-(C₁₋₅-Alkyl),
oder
wobei -Y_{C2}-R_{C2} ausgewählt ist aus -(C₀₋₃-Alkylen)-Heterocycloalkyl, -CONH-Heterocycloalkyl, -NHCO-Heterocycloalkyl, -NH-Heterocycloalkyl, -O-Heterocycloalkyl, - CO-Heterocycloalkyl, -SO₂-Heterocycloalkyl, -(C₀₋₃-Alkylen)-Heterocycloalkenyl, -CONH-Heterocycloalkenyl, -NHCO-Heterocycloalkenyl, -NH-Heterocycloalkenyl, -O-Heterocycloalkenyl, -CO-Heterocycloalkenyl, -SO₂-Heterocycloalkenyl, -(C₀₋₃-Alkylen)aryl, - CONH-Aryl, -NHCO-Aryl, -NH-Aryl, -O-Aryl, -CO-Aryl, -SO₂-Aryl, -(C₀₋₃-Alkylen)heteroaryl, -CONH-Heteroaryl, -NHCO-Heteroaryl, -NH-Heteroaryl, -O-Heteroaryl, -CO-Heteroaryl und -SO₂-Heteroaryl, wobei bevorzugt -Y_{C2}-R_{C2} ausgewählt ist aus -(C₀₋₃-Alkylen)-Heterocycloalkyl, -CONH-Heterocycloalkyl, -NHCO-Heterocycloalkyl, -NH-Heterocycloalkyl, -O-Heterocycloalkyl, -CO-Heterocycloalkyl, -SO₂-Heterocycloalkyl, -(C₀₋₃-Alkylen)aryl, -CONH-Aryl, -NHCO-Aryl, -NH-Aryl, -O-Aryl, -CO-Aryl, -SO₂-Aryl, -(C₀₋₃-Alkylen)heteroaryl, -CONH-Heteroaryl, -NHCO-heteroaryl, -NH-heteroaryl, -O-Heteroaryl, - CO-Heteroaryl und -SO₂-Heteroaryl, wobei das Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(O)(C₁₋₅-Alkyl), -S(O)₂(C₁₋₅-Alkyl), - S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), - S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CO(C₁₋₅-Halogenalkyl), -CO-Cycloalkyl, -COO(C₁₋₅-Alkyl), -COO(C₁₋₅-Halogenalkyl), -COO-Cycloalkyl, -CONH₂, -CONH(C₁₋₅-Alkyl), -CON(C₁₋₅-Alkyl)(C₁₋₅-Alkyl), -CO-(*N-*Heterocycloalkyl), -NHCO-(C₁₋₅-Alkyl), -N(C₁₋₅-Alkyl)-CO-(C₁₋₅-Alkyl), -NHCONH₂, - NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -OCONH₂, - OCONH-(C₁₋₅-Alkyl), -OCON(C₁₋₅-Alkyl)(C₁₋₅-Alkyl), -NHCOO(C₁₋₅-Alkyl), -N(C₁₋₅-Alkyl)COO-(C₁₋₅-Alkyl), -P(O)(C₁₋₅-Alkyl)(C₁₋₅-Alkyl), -P(O)(O(C₁₋₅-Alkyl))(O(C₁₋₅-Alkyl)), -P(O)(O(C₁₋₅-Alkyl))(C₁₋₅-Alkyl), -(C₁₋₅-Alkylen)-CN, -(C₁₋₅-Alkylen)-OH, - (C₁₋₅-Alkylen)-O(C₁₋₅-Alkyl), -(C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), -(C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(N-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CO(C₁₋₅-Halogenalkyl), -(C₁₋₅-Alkylen)-CO-Cycloalkyl, -(C₁₋₅-Alkylen)-CONH₂, -(C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(*N-*Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁- ₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl), -(C₁₋₅-Alkylen)-N(C₁₋₅-Alkyl)CON(C₁₋₅-Alkyl)(C₁₋₅-Alkyl), -(C₁₋₅-Alkylen)-OCONH₂, -(C₁₋₅-Alkylen)-OCONH-(C₁₋₅-Alkyl), -(C₁₋₅-Alkylen)-OCON(C₁₋₅-Alkyl)(C₁₋₅-Alkyl), -(C₁₋₅-Alkylen)-NHCOO(C₁₋₅-Alkyl) und -(C₁₋₅-Alkylen)-N(C₁₋₅-Alkyl)COO-(C₁₋₅-Alkyl),
oder
wobei -Y_{C2}-R_{C2} ausgewählt ist aus -(C₀₋₃-Alkylen)-Heterocycloalkyl, -(C₀₋₃-Alkylen)-Heterocycloalkenyl, -(C₀₋₃-Alkylen)-Aryl und -(C₀₋₃-Alkylen)-Heteroaryl, bevorzugt, wobei - Y_{C2}-R_{C2} ausgewählt ist aus -(C₀₋₃-Alkylen)-Heterocycloalkyl, -(C₀₋₃-Alkylen)-Aryl und -(C₀₋₃-Alkylen)-Heteroaryl, wobei das Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -(*N*-Heterocycloalkyl), -CO(C₁₋₅-Alkyl), -CO(C₁₋₅-Halogenalkyl), -CO-Cycloalkyl, -COO(C₁₋₅-Alkyl), -COO(C₁₋₅-Halogenalkyl), -COO-Cycloalkyl, -CONH₂, -CONH(C₁₋₅-Alkyl), -CON(C₁₋₅-Alkyl)(C₁₋₅-Alkyl), -CO-(N-Heterocycloalkyl), -NHCO-(C₁₋₅-Alkyl), -N(C₁₋₅-Alkyl)-CO-(C₁₋₅-Alkyl), -NHCONH₂, - NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -OCONH₂, - OCONH-(C₁₋₅-Alkyl), -OCON(C₁₋₅-Alkyl)(C₁₋₅-Alkyl), -NHCOO(C₁₋₅-Alkyl), -N(C₁₋₅-Alkyl)COO-(C₁₋₅-Alkyl), -P(O)(C₁₋₅-Alkyl)(C₁₋₅-Alkyl), -P(O)(O(C₁₋₅-Alkyl))(O(C₁₋₅-Alkyl)), -P(O)(O(C₁₋₅-Alkyl))(C₁₋₅-Alkyl), -(C₁₋₅-Alkylen)-CN, -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), -(C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(*N*-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CO(C₁₋₅-Halogenalkyl), -(C₁₋₅-Alkylen)-CO-Cycloalkyl, -(C₁₋₅-Alkylen)-CONH₂, -(C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(N-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl), -(C₁₋₅-Alkylen)-N(C₁₋₅-Alkyl)CON(C₁₋₅-Alkyl)(C₁₋₅-Alkyl), - (C₁₋₅-Alkylen)-OCONH₂, -(C₁₋₅-Alkylen)-OCONH-(C₁₋₅-Alkyl), -(C₁₋₅-Alkylen)-OCON(C₁₋₅-Alkyl)(C₁₋₅-Alkyl), -(C₁₋₅-Alkylen)-NHCOO(C₁₋₅-Alkyl) und -(C₁₋₅-Alkylen)-N(C₁₋₅-Alkyl)COO-(C₁₋₅-Alkyl).

4. Verbindung nach Anspruch 1 oder 2,
wobei Y_{C2} ausgewählt ist aus einer kovalenten Bindung, C₁₋₅-Alkylen, C₂₋₅-Alkenylen und C₂₋₅-Alkinylen, wobei das Alkylen, das Alkenylen und das Alkinylen jeweils optional mit einer oder mehreren Gruppen substituiert sind, die unabhängig voneinander ausgewählt sind aus Halogen, -CN, -OH, -O(C₁₋₅-Alkyl), -SH, -S(C₁₋₅-Alkyl), -NH₂, -NH(C₁₋₅-Alkyl) und -N(C₁₋₅-Alkyl)(C₁₋₅-Alkyl) und ferner wobei eine oder mehrere der -CH₂-Einheiten, die in dem Alkylen, dem Alkenylen oder dem Alkinylen umfasst sind, jeweils optional durch eine Gruppe ersetzt sind, die unabhängig ausgewählt ist aus -O-, -NH-, -N(C₁₋₅-Alkyl)-, -CO-, -S-, -SO- und -SO₂-, und wobei R_{C2} ausgewählt ist aus Wasserstoff, Halogen, -OH, -NH₂, -SH, -CN, C₁₋₁₂-Alkyl, C₂₋₁₂-Alkenyl, C₂₋₁₂-Alkinyl, Cycloalkyl, Heterocycloalkyl, Aryl und Heteroaryl,
wobei das Alkyl, Alkenyl oder Alkinyl optional mit einer oder mehreren Gruppen substituiert ist, die unabhängig voneinander ausgewählt sind aus Halogen, -CN, -OH, -O(C₁₋₅-Alkyl), - O(C₁₋₅-Halogenalkyl) und C₁₋₅-Halogenalkyl, -SH, -S(C₁₋₅-Alkyl), -S(C₁₋₅-Halogenalkyl), - NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(*N*-Heterocycloalkyl), -NHCO-(C₁₋₅-Alkyl), - N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -P(O)(C₁₋₅-Alkyl)(C₁₋₅-Alkyl), -P(O)(O(C₁₋₅-Alkyl))(O(C₁₋₅-Alkyl)) und -P(O)(O(C₁₋₅-Alkyl))(C₁₋₅-Alkyl), bevorzugt ausgewählt aus Halogen, -CN, -OH, -O(C₁₋₅-Alkyl), -O(C₁₋₅-Halogenalkyl), C₁₋₅-Halogenalkyl, -SH, -S(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), - CO(C₁₋₅-Alkyl), -CONH₂, -CONH(C₁₋₅-Alkyl) und -CON(C₁₋₅-Alkyl)(C₁₋₅-Alkyl), und
wobei das Cycloalkyl, Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), - S(O)(C₁₋₅-Alkyl), -S(O)₂(C₁₋₅-Alkyl), -S(O)(NH)(C₁₋₅-Alkyl), -S(O)(N(C₁₋₅-Alkyl))(C₁₋₅-Alkyl), -N=S(O)(C₁₋₅-Alkyl)(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N-*Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(N-Heterocycloalkyl), -NHCO-(C₁₋₅-Alkyl), -N(C₁₋₅-Alkyl)-CO-(C₁₋₅-Alkyl), - NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl), -N(C₁₋₅-Alkyl)CON(C₁₋₅-Alkyl)(C₁₋₅-Alkyl), -P(O)(C₁₋₅-Alkyl)(C₁₋₅-Alkyl), -P(O)(O(C₁₋₅-Alkyl))(O(C₁₋₅-Alkyl)), -P(O)(O(C₁₋₅-Alkyl))(C₁₋₅-Alkyl), -(C₁₋₅-Alkylen)-CN, -(C₁₋₅-Alkylen)-OH, -(C₁₋₅-Alkylen)-O(C₁₋₅-Alkyl), - (C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-S(O)(C₁₋₅-Alkyl), -(C₁₋₅-Alkylen)-S(O)₂(C₁₋₅-Alkyl), -(C₁₋₅-Alkylen)-S(O)(NH)(C₁₋₅-Alkyl), - (C₁₋₅-Alkylen)-S(O)(N(C₁₋₅-Alkyl))(C₁₋₅-Alkyl), -(C₁₋₅-Alkylen)-P(O)(C₁₋₅-Alkyl)(C₁₋₅-Alkyl), -(C₁₋₅-Alkylen)-P(O)(O(C₁₋₅-Alkyl))(O(C₁₋₅-Alkyl)), -(C₁₋₅-Alkylen)-P(O)(O(C₁₋₅-Alkyl))(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(*N*-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CONH₂, -(C₁₋₅-Alkylen)-CONH(C₁₋₅-Alkyl), -(C₁₋₅-Alkylen)-CON(C₁₋₅-Alkyl)(C₁₋₅-Alkyl), - (C₁₋₅-Alkylen)-CO-(N-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl) und -(C₁₋₅-Alkylen)-N(C₁₋₅-Alkyl)CON(C₁₋₅-Alkyl)(C₁₋₅-Alkyl), bevorzugt ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -SH, -S(C₁₋₅-Alkyl), -S(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), - (C₁₋₅-Alkylen)-N(C₁₋₅-Alkyl)(C₁₋₅-Alkyl), -(C₁₋₅-Alkylen)-N(C₁₋₅-Halogenalkyl)(C₁₋₅-Alkyl), - CO(C₁₋₅-Alkyl), -CONH₂, -CONH(C₁₋₅-Alkyl) und -CON(C₁₋₅-Alkyl)(C₁₋₅-Alkyl), noch bevorzugter ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, -CONH(C₁₋₅-Alkyl) und -CON(C₁₋₅-Alkyl)(C₁₋₅-Alkyl), bevorzugt, wobei -Y_{C2}-R_{C2} ausgewählt ist aus -O-C₁₋₁₂-Alkyl, -NH-C₁₋₁₂-Alkyl, -N(C₁₋₅-Alkyl)-C₁₋₁₂-Alkyl, -O-C₂₋₁₂-Alkenyl, -NH-C₂₋₁₂-Alkenyl, -N(C₁₋₅-Alkyl)-C₂₋₁₂-Alkenyl, -O-C₂₋₁₂-Alkinyl, -NH-C₂₋₁₂-Alkinyl, -N(C₁₋₅-Alkyl)-C₂₋₁₂-Alkinyl, -(C₀₋₃-Alkylen)-Cycloalkyl, - CO-(C₀₋₃-Alkylen)-Cycloalkyl, -(C₀₋₃-Alkylen)-CO-Cycloalkyl, -CONH-(C₀₋₃-Alkylen)-Cycloalkyl, (C₀₋₃-Alkylen)-CONH-Cycloalkyl, -NHCO-(C₀₋₃-Alkylen)-Cycloalkyl, -(C₀₋₃-Alkylen)-NHCO-Cycloalkyl, -NH-(C₀₋₃-Alkylen)-Cycloalkyl, -(C₀₋₃-Alkylen)-NH-Cycloalkyl, -O-(C₀₋₃-Alkylen)-Cycloalkyl, -(C₀₋₃-Alkylen)-O-Cycloalkyl, -SO₂-(C₀₋₃-Alkylen)-Cycloalkyl, -(C₀₋₃-Alkylen)-SO₂-Cycloalkyl, -CONH-Cycloalkyl, -NHCO-Cycloalkyl, -NH-Cycloalkyl, - O-Cycloalkyl, -CO-Cycloalkyl, -SO₂-Cycloalkyl, -(C₀₋₃-Alkylen)-Heterocycloalkyl, -CO-(C₀₋₃-Alkylen)-Heterocycloalkyl, -(C₀₋₃-Alkylen)-CO-Heterocycloalkyl, -CONH-(C₀₋₃-Alkylen)-Heterocycloalkyl, -(C₀₋₃-Alkylen)-CONH-Heterocycloalkyl, -NHCO-(C₀₋₃-Alkylen)-Heterocycloalkyl, -(C₀₋₃-Alkylen)-NHCO-Heterocycloalkyl, -NH-(C₀₋₃-Alkylen)-Heterocycloalkyl, -(C₀₋₃-Alkylen)-NH-Heterocycloalkyl, -O-(C₀₋₃-Alkylen)-Heterocycloalkyl, -(C₀₋₃-Alkylen)-O-Cycloalkyl, -SO₂-(C₀₋₃-Alkylen)-Heterocycloalkyl, -(C₀₋₃-Alkylen)-SO₂-Heterocycloalkyl, -CONH-Heterocycloalkyl, -NHCO-Heterocycloalkyl, -NH-Heterocycloalkyl, -O-Heterocycloalkyl, -CO-Heterocycloalkyl, -SO₂-Heterocycloalkyl, -(C₀₋₃-Alkylen)-Aryl, -CO-(C₀₋₃-Alkylen)-Aryl, -(C₀₋₃-Alkylen)-CO-Aryl, -CONH-(C₀₋₃-Alkylen)-Aryl, -(C₀₋₃-Alkylen)-CONH-Aryl, -NHCO-(C₀₋₃-Alkylen)-Aryl, -(C₀₋₃-Alkylen) -NHCO-Aryl, -NH-(C₀₋₃-Alkylen)-Aryl, -(C₀₋₃-Alkylen)-NH-Aryl, -O-(C₀₋₃-Alkylen)-Aryl, -(C₀₋₃-Alkylen)-O-Aryl, -SO₂-(C₀₋₃-Alkylen)-Aryl, -(C₀₋₃-Alkylen)-SO₂-Aryl, -CONH-Aryl, - NHCO-Aryl, -NH-Aryl, -O-Aryl, -CO-Aryl, -SO₂-Aryl, -(C₀₋₃-Alkylen)-Heteroaryl, -CO-(C₀₋₃-Alkylen)-Heteroaryl, -(C₀₋₃-Alkylen)-CO-Heteroaryl, -CONH-(C₀₋₃-Alkylen)-Heteroaryl, - (C₀₋₃-Alkylen)-CONH-Heteroaryl, -NHCO-(C₀₋₃-Alkylen)-Heteroaryl, -(C₀₋₃-Alkylen)-NHCO-heteroaryl, -NH-(C₀₋₃-Alkylen)-Heteroaryl, -(C₀₋₃-Alkylen)-NH-Heteroaryl, -O-(C₀₋₃-Alkylen)-Heteroaryl, -(C₀₋₃-Alkylen)-O-Heteroaryl, -SO₂-(C₀₋₃-Alkylen)-Heteroaryl, -(C₀₋₃-Alkylen)-SO₂-Heteroaryl, -CONH-Heteroaryl, -NHCO-Heteroaryl, -NH-Heteroaryl, -O-Heteroaryl, -CO-Heteroaryl und -SO₂-Heteroaryl, wobei das Alkyl, Alkenyl oder Alkinyl optional mit einer oder mehreren Gruppen substituiert ist, die unabhängig voneinander ausgewählt sind aus Halogen, -CN, -OH, -O(C₁₋₅-Alkyl), -O(C₁₋₅-Halogenalkyl), -C₁₋₅-Halogenalkyl, -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, -CONH(C₁₋₅-Alkyl) und -CON(C₁₋₅-Alkyl)(C₁₋₅-Alkyl) und wobei das Cycloalkyl, Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CO(C₁₋₅-Alkyl), -CONH₂, -CONH(C₁₋₅-Alkyl) und - CON(C₁₋₅-Alkyl)(C₁₋₅-Alkyl),
noch bevorzugter, wobei -Y_{C2}-R_{C2} ausgewählt ist aus -(C₀₋₃-Alkylen)-Heterocycloalkyl, -CO-(C₀₋₃-Alkylen)-Heterocycloalkyl, -(C₀₋₃-Alkylen)-CO-Heterocycloalkyl, -CONH-(C₀₋₃-Alkylen)-Heterocycloalkyl, -(C₀₋₃-Alkylen)-CONH-Heterocycloalkyl, -NHCO-(C₀₋₃-Alkylen)-Heterocycloalkyl, -(C₀₋₃-Alkylen)-NHCO-Heterocycloalkyl, -NH-(C₀₋₃-Alkylen)-Heterocycloalkyl, -(C₀₋₃-Alkylen)-NH-Heterocycloalkyl, -O-(C₀₋₃-Alkylen)-Heterocycloalkyl, -(C₀₋₃-Alkylen)-O-Cycloalkyl, -SO₂-(C₀₋₃-Alkylen)-Heterocycloalkyl, -(C₀₋₃-Alkylen)-SO₂-Heterocycloalkyl, -CONH-Heterocycloalkyl, -NHCO-Heterocycloalkyl, -NH-Heterocycloalkyl, -O-Heterocycloalkyl, -CO-Heterocycloalkyl, -SO₂-Heterocycloalkyl, -(C₀₋₃-Alkylen)-Aryl, -CO-(C₀₋₃-Alkylen)-Aryl, -(C₀₋₃-Alkylen)-CO-Aryl, -CONH-(C₀₋₃-Alkylen)-Aryl, -(C₀₋₃-Alkylen)-CONH-Aryl, -NHCO-(C₀₋₃-Alkylen)-Aryl, -(C₀₋₃-Alkylen) -NHCO-Aryl, -NH-(C₀₋₃-Alkylen)-Aryl, -(C₀₋₃-Alkylen)-NH-Aryl, -O-(C₀₋₃-Alkylen)-Aryl, -(C₀₋₃-Alkylen)-O-Aryl, -SO₂-(C₀₋₃-Alkylen)-Aryl, -(C₀₋₃-Alkylen)-SO₂-Aryl, -CONH-Aryl, - NHCO-Aryl, NH-Aryl, -O-Aryl, -CO-Aryl, -SO₂-Aryl, -(C₀₋₃-Alkylen)-Heteroaryl, -CO-(C₀₋₃-Alkylen)-Heteroaryl, - (C₀₋₃-Alkylen)-CO-Heteroaryl, -CONH-(C₀₋₃-Alkylen)-Heteroaryl, - (C₀₋₃-Alkylen)-CONH-Heteroaryl, -NHCO-(C₀₋₃-Alkylen)-Heteroaryl, -(C₀₋₃-Alkylen)-NHCO-Heteroaryl, -NH-(C₀₋₃-Alkylen)-Heteroaryl, -(C₀₋₃-Alkylen)-NH-Heteroaryl, -O-(C₀₋₃-Alkylen)-Heteroaryl, -(C₀₋₃-Alkylen)-O-Heteroaryl, -SO₂-(C₀₋₃-Alkylen)-Heteroaryl, -(C₀₋₃-Alkylen)-SO₂-Heteroaryl, -CONH-Heteroaryl, -NHCO-Heteroaryl, -NH-Heteroaryl, -O-Heteroaryl, -CO-Heteroaryl und -SO₂-Heteroaryl, wobei das Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, -CONH(C₁₋₅-Alkyl) und -CON(C₁₋₅-Alkyl)(C₁₋₅-Alkyl),
sogar noch bevorzugter, wobei -Y_{C2}-R_{C2} ausgewählt ist aus -(C₀₋₃-Alkylen)-Heterocycloalkyl, -CONH-Heterocycloalkyl, -NHCO-Heterocycloalkyl, -NH-Heterocycloalkyl, -O-Heterocycloalkyl, -CO-Heterocycloalkyl, -SO₂-Heterocycloalkyl, -(C₀₋₃-Alkylen)aryl, - CONH-Aryl, -NHCO-Aryl, -NH-Aryl, -O-Aryl, -CO-Aryl, -SO₂-Aryl, -(C₀₋₃-Alkylen)-Heteroaryl, -CONH-Heteroaryl, -NHCO-Heteroaryl, -NH-Heteroaryl, -O-Heteroaryl, -CO-Heteroaryl und -SO₂-Heteroaryl, wobei das Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -NH₂, -NH(C₁₋₅-Alkyl), -N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, -CONH(C₁₋₅-Alkyl) und -CON(C₁₋₅-Alkyl)(C₁₋₅-Alkyl), erneut noch bevorzugter, wobei -Y_{C2}-R_{C2} ausgewählt ist aus -(C₀₋₃-Alkylen)-Heterocycloalkyl, -(C₀₋₃-Alkylen)-Aryl und -(C₀₋₃-Alkylen)-Heteroaryl, wobei das Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CO(C₁₋₅-Alkyl), -CONH₂, -CONH(C₁₋₅-Alkyl) und - CON(C₁₋₅-Alkyl)(C₁₋₅-Alkyl),
und noch bevorzugter, wobei -Y_{C2}-R_{C2} ausgewählt ist aus Heterocycloalkyl, Aryl und Heteroaryl, bevorzugt Heterocycloalkyl und Heteroaryl, noch bevorzugter Heterocycloalkyl,
wobei das Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Halogenalkyl), -S(C₁₋₅-Alkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CO(C₁₋₅-Alkyl), -CONH₂, -CONH(C₁₋₅-Alkyl) und - CON(C₁₋₅-Alkyl)(C₁₋₅-Alkyl).

5. Verbindung nach Anspruch 3 oder 4, wobei -Y_{C2}-R_{C2} optional substituiertes Aryl ist, bevorzugt wobei -Y_{C2}-R_{C2} Phenyl ist, das optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CO(C₁₋₅-Alkyl), -CONH₂, -CONH(C₁₋₅-Alkyl) und - CON(C₁₋₅-Alkyl)(C₁₋₅-Alkyl), oder wobei -Y_{C2}-R_{C2} ein optional substituiertes Heteroaryl ist, bevorzugt wobei -Y_{C2}-R_{C2} Imidazolyl, Pyridazinyl, Thiazolyl, Pyridinyl, Pyrimidinyl, Pyrazinyl oder Indazolyl ist, wobei Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CO(C₁₋₅-Alkyl), -CONH₂, -CONH(C₁₋₅-Alkyl) und - CON(C₁₋₅-Alkyl)(C₁₋₅-Alkyl),
oder wobei -Y_{C2}-R_{C2} optional substituiertes Heterocycloalkyl ist, bevorzugt wobei -Y_{C2}-R_{C2} Morpholinyl, 1,1-Dioxothiomorpholinyl, Azetinyl, Pyrrolidinyl, Piperidinyl, 6-Oxo-1,6-dihydropyridinyl oder Piperazinyl ist, wobei Heterocycloalkyl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), - S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CO(C₁₋₅-Alkyl), -CONH₂, -CONH(C₁₋₅-Alkyl) und -CON(C₁₋₅-Alkyl)(C₁₋₅-Alkyl),
bevorzugt, wobei -Y_{C2}-R_{C2} Piperazinyl ist, das optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CO(C₁₋₅-Alkyl), -CONH₂, -CONH(C₁₋₅-Alkyl) und - CON(C₁₋₅-Alkyl)(C₁₋₅-Alkyl), bevorzugt, wobei -Y_{C2}-R_{C2} Piperazinyl (bevorzugt N-Piperazinyl) ist, das optional (bevorzugt N-substituiert) mit CO(C₁₋₅-Alkyl), -CONH₂, - CONH(C₁₋₅-Alkyl) und -CON(C₁₋₅-Alkyl)(C₁₋₅-Alkyl) substituiert ist, noch bevorzugter, wobei -Y_{C2}-R_{C2} Piperazinyl (bevorzugt N-Piperazinyl) ist, noch bevorzugter, wobei -Y_{C2}-R_{C2} Piperazinyl (bevorzugt N-Piperazinyl) ist, das (bevorzugt N-substituiert, bevorzugt an einem anderen N-Atom als demjenigen, das an das Ringsystem gebunden ist, wie in Formel (I) gezeigt) mit -CON(C₁₋₅-Alkyl)(C₁₋₅-Alkyl), bevorzugt mit -CON(CH₃)₂, substituiert ist.

6. Verbindung nach einem der Ansprüche 1 bis 5, wobei X₄ C-R_{C4} ist, wobei R_{C4} ausgewählt ist aus Wasserstoff, Halogen, C₁₋₆-Alkyl, C₂₋₆-Alkinyl, -O(C₁₋₆-Alkyl), -S(C₁₋₆-Alkyl), -NH(C₁₋₆-Alkyl), -N(C₁₋₆-Alkyl)(C₁₋₆-Alkyl), -CO(C₁₋₆-Alkyl), C₁₋₆-Halogenalkyl, -O(C₁₋₆-Halogenalkyl), -S(C₁₋₆-Halogenalkyl), -NH(C₁₋₆-Halogenalkyl), -N(C₁₋₆-Halogenalkyl)₂, - CO(C₁₋₆-Halogenalkyl), -(C₀₋₃-Alkylen)cycloalkyl, -O-(C₀₋₃-Alkylen)-Cycloalkyl, -CO-(C₀₋₃-Alkylen)-Cycloalkyl, -(C₀₋₃-Alkylen)-Heterocycloalkyl, -O-(C₀₋₃-Alkylen)-Heterocycloalkyl, - CO-(C₀₋₃-Alkylen)-Heterocycloalkyl, -(C₀₋₃-Alkylen)-Aryl, -O-(C₀₋₃-Alkylen)-Aryl, -CO-(C₀₋₃-Alkylen)-Aryl, -(C₀₋₃-Alkylen)-Heteroaryl, -O-(C₀₋₃-Alkylen)-Heteroaryl und -CO-(C₀₋₃-Alkylen)-Heteroaryl;
wobei das Alkyl oder das Alkinyl optional mit einer oder mehreren Gruppen substituiert ist, die unabhängig voneinander ausgewählt sind aus Halogen, -CN, -OH, -O(C₁₋₅-Alkyl), -O(C₁₋₅-Halogenalkyl), C₁₋₅-Halogenalkyl, -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(*N*-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl) und -N(C₁₋₅-Alkyl)CON(C₁₋₅-Alkyl)(C₁₋₅-Alkyl), bevorzugt ausgewählt aus Halogen, -CN, -OH, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), - N(C₁₋₅-Alkyl)(C₁₋₅-Alkyl), -N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl), und
wobei das Cycloalkyl, Cycloalkenyl, Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -(*N*-Heterocycloalkyl), -CO(C₁₋₅-Alkyl), -CONH₂, -CONH(C₁₋₅-Alkyl), -CON(C₁₋₅-Alkyl)(C₁₋₅-Alkyl), -CO-(*N*-Heterocycloalkyl), -NHCO-(C₁₋₅-Alkyl), -N(C₁₋₅-Alkyl)-CO-(C₁₋₅-Alkyl), -NHCONH₂, -NHCONH-(C₁₋₅-Alkyl), -NHCON(C₁₋₅-Alkyl)(C₁₋₅-Alkyl), -N(C₁₋₅-Alkyl)CONH₂, -N(C₁₋₅-Alkyl)CONH-(C₁₋₅-Alkyl) und -N(C₁₋₅-Alkyl)CON(C₁₋₅-Alkyl)(C₁₋₅-Alkyl), -(C₁₋₅-Alkylen)-CN, -(C₁₋₅-Alkylen)-OH, -(C₁₋₅-Alkylen)-O(C₁₋₅-Alkyl), -(C₁₋₅-Alkylen)-O(C₁₋₅-Halogenalkyl), -(C₁₋₅-Alkylen)-SH, -(C₁₋₅-Alkylen)-S(C₁₋₅-Alkyl), -(C₁₋₅-Alkylen)-S(C₁₋₅-Halogenalkyl), -(C₁₋₅-Alkylen)-NH₂, -(C₁₋₅-Alkylen)-NH(C₁₋₅-Alkyl), -(C₁₋₅-Alkylen)-NH(C₁₋₅-Halogenalkyl), -(C₁₋₅-Alkylen)-N(C₁₋₅-Alkyl)(C₁₋₅-Alkyl), -(C₁₋₅-Alkylen)-N(C₁₋₅-Alkyl)(C₁₋₅-Halogenalkyl), -(C₁₋₅-Alkylen)-(*N*-Heterocycloalkyl), -(C₁₋₅-Alkylen)-N(C₁₋₅-Halogenalkyl)(C₁₋₅-Alkyl), -(C₁₋₅-Alkylen)-CO(C₁₋₅-Alkyl), -(C₁₋₅-Alkylen)-CONH₂, - (C₁₋₅-Alkylen)-CONH(C₁₋₅-Alkyl), -(C₁₋₅-Alkylen)-CON(C₁₋₅-Alkyl)(C₁₋₅-Alkyl), -(C₁₋₅-Alkylen)-CO-(*N*-Heterocycloalkyl), -(C₁₋₅-Alkylen)-NHCO-(C₁₋₅-Alkyl), -(C₁₋₅-Alkylen)-N(C₁₋₅-Alkyl)-CO-(C₁₋₅-Alkyl), -(C₁₋₅-Alkylen)-NHCONH₂, -(C₁₋₅-Alkylen)-NHCONH-(C₁₋₅-Alkyl), -(C₁₋₅-Alkylen)-NHCON(C₁₋₅-Alkyl)(C₁₋₅-Alkyl), -(C₁₋₅-Alkylen)-N(C₁₋₅-Alkyl)CONH₂, -(C₁₋₅-Alkylen)-N(C₁₋₅-Alkyl)CONH-(C₁₋₅-Alkyl) und -(C₁₋₅-Alkylen)-N(C₁₋₅-Alkyl)CON(C₁₋₅-Alkyl)(C₁₋₅-Alkyl), bevorzugt ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl),
oder
wobei X₄ C-R_{C4} ist, wobei R_{C4} ausgewählt ist aus Wasserstoff, Halogen, C₁₋₆-Alkyl, C₂₋₆-Alkinyl, -O-C₁₋₆-Alkyl, -S-C₁₋₆-Alkyl, -NH-C₁₋₆-Alkyl, C₁₋₆-Halogenalkyl, -(C₀₋₃-Alkylen)-Cycloalkyl, -(C₀₋₃-Alkylen)-Heterocycloalkyl, -(C₀₋₃-Alkylen)-Aryl und -(C₀₋₃-Alkylen)-Heteroaryl, wobei das Alkyl optional mit einer oder mehreren Gruppen substituiert ist, die unabhängig voneinander ausgewählt sind aus Halogen, -CN, -OH, -O(C₁₋₅-Alkyl), -O(C₁₋₅-Halogenalkyl), -SH, -S(C₁₋₅-Alkyl), -O(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CONH₂, - CONH(C₁₋₅-Alkyl) und -CON(C₁₋₅-Alkyl)(C₁₋₅-Alkyl), und wobei das Cycloalkyl, Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Halogenalkyl), -S(C₁₋₅-Alkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl), bevorzugt, wobei R_{C4} ausgewählt ist aus Wasserstoff, Halogen, C₁₋₆-Alkyl, C₂₋₅-Alkinyl, -O-C₁₋₆-Alkyl, -S-C₁₋₆-Alkyl, -NH-C₁₋₆-Alkyl und C₁₋₆-Halogenalkyl,
noch bevorzugter, wobei R_{C4} ausgewählt ist aus Wasserstoff, Halogen, C₁₋₂-Alkyl und C₂₋₃-Alkinyl, sogar noch bevorzugter, wobei R_{C4} ausgewählt ist aus Wasserstoff, Halogen und C₁₋₂-Alkyl, und sogar noch mehr bevorzugt, wobei R_{C4} Wasserstoff oder Halogen ist.

7. Verbindung nach einem der Ansprüche 1 bis 6, wobei X₅ C-R_{C5} ist, wobei R_{C5} ausgewählt ist aus Wasserstoff, Halogen, C₁₋₃-Alkyl, -O-C₁₋₃-Alkyl, -S-C₁₋₃-Alkyl, -NH-C₁₋₃-Alkyl und C₁₋₃-Halogenalkyl, bevorzugt wobei R_{C5} ausgewählt ist aus Wasserstoff, Halogen, C₁₋₃-Alkyl und C₁₋₃-Halogenalkyl.

8. Verbindung nach einem der Ansprüche 1 bis 7, wobei R_{R5} ausgewählt ist aus C₁₋₁₂-Alkyl, C₂₋₁₂-Alkenyl, C₂₋₁₂-Alkinyl, Cycloalkyl, Heterocycloalkyl, Aryl und Heteroaryl,
wobei das Alkyl, Alkenyl oder Alkinyl optional mit einer oder mehreren Gruppen substituiert ist, die unabhängig voneinander ausgewählt sind aus Halogen, -CN, -OH, -O(C₁₋₅-Alkyl), - O(C₁₋₅-Halogenalkyl), -SH, -S(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), - CONH₂, -CONH(C₁₋₅-Alkyl) und -CON(C₁₋₅-Alkyl)(C₁₋₅-Alkyl), und wobei das Cycloalkyl, Heterocycloalkyl, Aryl oder Heteroaryl optional mit einer oder mehreren Gruppen substituiert ist, die unabhängig voneinander ausgewählt sind aus Halogen, -CN, -OH, -C₁₋₅-Alkyl, -C₁₋₅-Halogenalkyl, -O(C₁₋₅-Alkyl), -O(C₁₋₅-Halogenalkyl), -SH, -S(C₁₋₅-Alkyl), -SO(C₁₋₅-Alkyl), - SO₂(C₁₋₅-Alkyl), -S(C₁₋₅-Halogenalkyl), -SO(C₁₋₅-Halogenalkyl), -SO₂(C₁₋₅-Halogenalkyl), - NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl), bevorzugt ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), - CONH₂, -CONH(C₁₋₅-Alkyl) und -CON(C₁₋₅-Alkyl)(C₁₋₅-Alkyl).

9. Verbindung nach einem der Ansprüche 1 bis 7, wobei R₄ ausgewählt ist aus -(C₀₋₂-Alkylen)-Cycloalkyl, -CO-(C₀₋₂-Alkylen)-Cycloalkyl, -(C₀₋₂-Alkylen)-CO-Cycloalkyl, -CONH-(C₀₋₂-Alkylen)-Cycloalkyl, -(C₀₋₂-Alkylen)-CONH-Cycloalkyl, -NHCO-(C₀₋₂-Alkylen)-Cycloalkyl, -(C₀₋₂-Alkylen)-NHCO-Cycloalkyl, -NH-(C₀₋₂-Alkylen)-Cycloalkyl, -(C₀₋₂-Alkylen)-NH-Cycloalkyl, -O-(C₀₋₂-Alkylen)-Cycloalkyl, -(C₀₋₂-Alkylen)-O-Cycloalkyl, -SO₂-(C₀₋₂-Alkylen)-Cycloalkyl, -(C₀₋₂-Alkylen)-SO₂-Cycloalkyl, -CONH-Cycloalkyl, -NHCO-Cycloalkyl, -NH-Cycloalkyl, -O-Cycloalkyl, -CO-Cycloalkyl, -SO₂-Cycloalkyl, -(C₀₋₂-Alkylen)-Cycloalkenyl, -CO-(C₀₋₂-Alkylen)-Cycloalkenyl, -(C₀₋₂-Alkylen)-CO-Cycloalkenyl, -CONH-(C₀₋₂-Alkylen)-Cycloalkenyl, -(C₀₋₂-Alkylen)-CONH-Cycloalkenyl, -NHCO-(C₀₋₂-Alkylen)-Cycloalkenyl, -(C₀₋₂-Alkylen)-NHCO-Cycloalkenyl, -NH-(C₀₋₂-Alkylen)-Cycloalkenyl, -(C₀₋₂-Alkylen)-NH-Cycloalkenyl, -O-(C₀₋₂-Alkylen)-Cycloalkenyl, -(C₀₋₂-Alkylen)-O-Cycloalkenyl, -SO₂-(C₀₋₂-Alkylen)-Cycloalkenyl, -(C₀₋₂-Alkylen)-SO₂-Cycloalkenyl, -CONH-Cycloalkenyl, -NHCO-Cycloalkenyl, -NH-Cycloalkenyl, -O-Cycloalkenyl, -CO-Cycloalkenyl, -SO₂-Cycloalkenyl, -(C₀₋₂-Alkylen)-Heterocycloalkyl, -CO-(C₀₋₂-Alkylen)-Heterocycloalkyl, -(C₀₋₂-Alkylen)-CO-Heterocycloalkyl, -CONH-(C₀₋₂-Alkylen)-Heterocycloalkyl, - (C₀₋₂-Alkylen)-CONH-Heterocycloalkyl, -NHCO-(C₀₋₂-Alkylen)-Heterocycloalkyl, -(C₀₋₂-Alkylen)-NHCO-Heterocycloalkyl, -NH-(C₀₋₂-Alkylen)-Heterocycloalkyl, -(C₀₋₂-Alkylen)-NH-Heterocycloalkyl, -O-(C₀₋₂-Alkylen)-Heterocycloalkyl, -(C₀₋₂-Alkylen)-O-Heterocycloalkyl, -SO₂-(C₀₋₂-Alkylen)-Heterocycloalkyl, -(C₀₋₂-Alkylen)-SO₂-Heterocycloalkyl, -CONH-Heterocycloalkyl, -NHCO-Heterocycloalkyl, -NH-Heterocycloalkyl, -O-Heterocycloalkyl, -CO-Heterocycloalkyl, -SO₂-Heterocycloalkyl, -(C₀₋₂-Alkylen)-Heterocycloalkenyl, -CO-(C₀₋₂-Alkylen)-Heterocycloalkenyl, -(C₀₋₂-Alkylen)-CO-Heterocycloalkenyl, -CONH-(C₀₋₂-Alkylen)-Heterocycloalkenyl, -(C₀₋₂-Alkylen)-CONH-Heterocycloalkenyl, -NHCO-(C₀₋₂-Alkylen)-Heterocycloalkenyl, -(C₀₋₂-Alkylen)-NHCO-Heterocycloalkenyl, -NH-(C₀₋₂-Alkylen)-Heterocycloalkenyl, -(C₀₋₂-Alkylen)-NH-Heterocycloalkenyl, -O-(C₀₋₂-Alkylen)-Heterocycloalkenyl, -(C₀₋₂-Alkylen)-O-Heterocycloalkenyl, -SO₂-(C₀₋₂-Alkylen)-Heterocycloalkenyl, -(C₀₋₂-Alkylen)-SO₂-Heterocycloalkenyl, -CONH-Heterocycloalkenyl, -NHCO-Heterocycloalkenyl, -NH-Heterocycloalkenyl, -O-Heterocycloalkenyl, -CO-Heterocycloalkenyl, -SO₂-Heterocycloalkenyl, -(C₀₋₂-Alkylen)-Aryl, -CO-(C₀₋₂-Alkylen)-Aryl, -(C₀₋₂-Alkylen)-CO-Aryl, -CONH-(C₀₋₂-Alkylen)-Aryl, -(C₀₋₂-Alkylen)-CONH-Aryl, -NHCO-(C₀₋₂-Alkylen)-Aryl, -(C₀₋₂-Alkylen)-NHCO-Aryl, -NH-(C₀₋₂-Alkylen)-Aryl, -(C₀₋₂-Alkylen)-NH-Aryl, -O-(C₀₋₂-Alkylen)-Aryl, -(C₀₋₂-Alkylen)-O-Aryl, -SO₂-(C₀₋₂-Alkylen)-Aryl, -(C₀₋₂-Alkylen)-SO₂-Aryl, - CONH-Aryl, -NHCO-Aryl, -NH-Aryl, -O-Aryl, -CO-Aryl, -SO₂-Aryl, -(C₀₋₂-Alkylen)-Heteroaryl, -CO-(C₀₋₂-Alkylen)-Heteroaryl, -(C₀₋₂-Alkylen)-CO-Heteroaryl, -CONH-(C₀₋₂-Alkylen)-Heteroaryl, -(C₀₋₂-Alkylen)-CONH-Heteroaryl, -NHCO-(C₀₋₂-Alkylen)-Heteroaryl, - (C₀₋₂-Alkylen)-NHCO-Heteroaryl, -NH-(C₀₋₂-Alkylen)-Heteroaryl, -(C₀₋₂-Alkylen)-NH-Heteroaryl, -O-(C₀₋₂-Alkylen)-Heteroaryl, -(C₀₋₂-Alkylen)-O-Heteroaryl, -SO₂-(C₀₋₂-Alkylen)-Heteroaryl, -(C₀₋₂-Alkylen)-SO₂-Heteroaryl, -CONH-Heteroaryl, -NHCO-Heteroaryl, -NH-Heteroaryl, -O-Heteroaryl, -CO-Heteroaryl und -SO₂-Heteroaryl,
wobei das Cycloalkyl, Cycloalkenyl, Heterocycloalkyl, Heterocycloalkenyl, Aryl oder Heteroaryl optional mit einer oder mehreren Gruppen substituiert ist, die unabhängig voneinander ausgewählt sind aus Halogen, -CN, -OH, -C₁₋₅-Alkyl, -C₁₋₅-Halogenalkyl, -O(C₁₋₅-Alkyl), -O(C₁₋₅-Halogenalkyl), -SH, -S(C₁₋₅-Alkyl), -SO(C₁₋₅-Alkyl), -SO₂(C₁₋₅-Alkyl), - S(C₁₋₅-Halogenalkyl), -SO(C₁₋₅-Halogenalkyl), -SO₂(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl), bevorzugt ausgewählt aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CONH₂, - CONH(C₁₋₅-Alkyl) und -CON(C₁₋₅-Alkyl)(C₁₋₅-Alkyl).

10. Verbindung nach einem der Ansprüche 1 bis 9, wobei R₄ ausgewählt ist aus -(C₀₋₂-Alkylen)-Cycloalkyl, -CO-(C₀₋₂-Alkylen)-Cycloalkyl, -(C₀₋₂-Alkylen)-CO-Cycloalkyl, -CONH-(C₀₋₂-Alkylen)-Cycloalkyl, -(C₀₋₂-Alkylen)-CONH-Cycloalkyl, -NHCO-(C₀₋₂-Alkylen)-Cycloalkyl, -(C₀₋₂-Alkylen)-NHCO-Cycloalkyl, -NH-(C₀₋₂-Alkylen)-Cycloalkyl, -(C₀₋₂-Alkylen)-NH-Cycloalkyl, -O-(C₀₋₂-Alkylen)-Cycloalkyl, -(C₀₋₂-Alkylen)-O-Cycloalkyl, -SO₂-(C₀₋₂-Alkylen)-Cycloalkyl, -(C₀₋₂-Alkylen)-SO₂-Cycloalkyl, -CONH-Cycloalkyl, -NHCO-Cycloalkyl, -NH-Cycloalkyl, -O-Cycloalkyl, -CO-Cycloalkyl, -SO₂-Cycloalkyl, -(C₀₋₂-Alkylen)-Heterocycloalkyl, -CO-(C₀₋₂-Alkylen)-Heterocycloalkyl, -(C₀₋₂-Alkylen)-CO-Heterocycloalkyl, -CONH-(C₀₋₂-Alkylen)-Heterocycloalkyl, -(C₀₋₂-Alkylen)-CONH-Heterocycloalkyl, -NHCO-(C₀₋₂-Alkylen)-Heterocycloalkyl, -(C₀₋₂-Alkylen)-NHCO-Heterocycloalkyl, -NH-(C₀₋₂-Alkylen)-Heterocycloalkyl, -(C₀₋₂-Alkylen)-NH-Heterocycloalkyl, -O-(C₀₋₂-Alkylen)-Heterocycloalkyl, -(C₀₋₂-Alkylen)-O-Heterocycloalkyl, - SO₂-(C₀₋₂-Alkylen)-Heterocycloalkyl, -(C₀₋₂-Alkylen)-SO₂-Heterocycloalkyl, -CONH-Heterocycloalkyl, -NHCO-Heterocycloalkyl, -NH-Heterocycloalkyl, -O-Heterocycloalkyl, - CO-Heterocycloalkyl, -SO₂-Heterocycloalkyl, -(C₀₋₂-Alkylen)-Aryl, -CO-(C₀₋₂-Alkylen)-Aryl, -(C₀₋₂-Alkylen)-CO-Aryl, -CONH-(C₀₋₂-Alkylen)-Aryl, -(C₀₋₂-Alkylen)-CONH-Aryl, -NHCO-(C₀₋₂-Alkylen)-Aryl, -(C₀₋₂-Alkylen)-NHCO-Aryl, -NH-(C₀₋₂-Alkylen)-Aryl, -(C₀₋₂-Alkylen)-NH-Aryl, -O-(C₀₋₂-Alkylen)-Aryl, -(C₀₋₂-Alkylen)-O-Aryl, -SO₂-(C₀₋₂-Alkylen)-Aryl, -(C₀₋₂-Alkylen)-SO₂-Aryl, -CONH-Aryl, -NHCO-Aryl, - NH-Aryl, -O-Aryl, -CO-Aryl, -SO₂-Aryl, - (C₀₋₂-Alkylen)-Heteroaryl, -CO-(C₀₋₂-Alkylen)-Heteroaryl, -(C₀₋₂-Alkylen)-CO-Heteroaryl, - CONH-(C₀₋₂-Alkylen)-Heteroaryl, -(C₀₋₂-Alkylen)-CONH-Heteroaryl, -NHCO-(C₀₋₂-Alkylen)-Heteroaryl, -(C₀₋₂-Alkylen)-NHCO-Heteroaryl, -NH-(C₀₋₂-Alkylen)-Heteroaryl, -(C₀₋₂-Alkylen)-NH-Heteroaryl, -O-(C₀₋₂-Alkylen)-Heteroaryl, -(C₀₋₂-Alkylen)-O-Heteroaryl, -SO₂-(C₀₋₂-Alkylen)heteroaryl, -(C₀₋₂-Alkylen)-SO₂-Heteroaryl, -CONH-Heteroaryl, -NHCO-Heteroaryl, -NH-Heteroaryl, -O-Heteroaryl, -CO-Heteroaryl und -SO₂-Heteroaryl, wobei das Cycloalkyl, Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl),
oder
wobei R₄ ausgewählt ist aus -(C₀₋₂-Alkylen)-Aryl, -CO-(C₀₋₂-Alkylen)-Aryl, -(C₀₋₂-Alkylen)-CO-Aryl, -CONH-(C₀₋₂-Alkylen)-Aryl, -(C₀₋₂-Alkylen)-CONH-Aryl, -NHCO-(C₀₋₂-Alkylen)-Aryl, -(C₀₋₂-Alkylen)-NHCO-Aryl, -NH-(C₀₋₂-Alkylen)-Aryl, -(C₀₋₂-Alkylen)-NH-Aryl, -O-(C₀₋₂-Alkylen)-Aryl, -(C₀₋₂-Alkylen)-O-Aryl, -SO₂-(C₀₋₂-Alkylen)-Aryl, -(C₀₋₂-Alkylen)-SO₂-Aryl, -CONH-Aryl, -NHCO-Aryl, -NH-Aryl, -O-Aryl, -CO-Aryl, -SO₂-Aryl, -(C₀₋₂-Alkylen)-Heteroaryl, -CO-(C₀₋₂-Alkylen)-Heteroaryl, -(C₀₋₂-Alkylen)-CO-Heteroaryl, -CONH-(C₀₋₂-Alkylen)-Heteroaryl, -(C₀₋₂-Alkylen)-CONH-Heteroaryl, -NHCO-(C₀₋₂-Alkylen)-Heteroaryl, - (C₀₋₂-Alkylen)-NHCO-Heteroaryl, -NH-(C₀₋₂-Alkylen)-Heteroaryl, -(C₀₋₂-Alkylen)-NH-Heteroaryl, -O-(C₀₋₂-Alkylen)-Heteroaryl, -(C₀₋₂-Alkylen)-O-Heteroaryl, -SO₂-(C₀₋₂-Alkylen)-Heteroaryl, -(C₀₋₂-Alkylen)-SO₂-Heteroaryl, -CONH-Heteroaryl, -NHCO-Heteroaryl, -NH-Heteroaryl, -O-Heteroaryl, -CO-Heteroaryl und -SO₂-Heteroaryl, wobei das Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), -N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl),
oder
wobei R₄ ausgewählt ist aus C₁₋₁₂-Alkyl, C₂₋₁₂-Alkenyl, C₂₋₁₂-Alkinyl, Cycloalkyl, Heterocycloalkyl, Aryl und Heteroaryl, bevorzugt wobei R₄ ausgewählt ist aus Cycloalkyl, Heterocycloalkyl, Aryl und Heteroaryl, noch bevorzugter, wobei R₄ ausgewählt ist aus Aryl und Heteroaryl, und sogar noch bevorzugter, wobei R₄ Heteroaryl ist, wobei das Alkyl, Alkenyl oder Alkinyl optional substituiert ist mit einer oder mehreren Gruppen, die unabhängig voneinander ausgewählt sind aus Halogen, -CN, -OH, -O(C₁₋₅-Alkyl), noch bevorzugter, wobei R₄ ausgewählt ist aus Aryl und Heteroaryl, und sogar noch bevorzugter, wobei R₄ Heteroaryl ist, wobei das Alkyl, Alkenyl oder Alkinyl optional substituiert ist mit einer oder mehreren Gruppen, die unabhängig voneinander ausgewählt sind aus Halogen, -CN, -OH, -O(C₁₋₅-Alkyl), -O(C₁₋₅-Halogenalkyl), SH, -S(C₁₋₅-Alkyl), -S(C₁₋₅-Halogenalkyl), -NH₂, -NH(C₁₋₅-Alkyl), - NH(C₁₋₅-Halogenalkyl), -N(C₁₋₅-Alkyl)(C₁₋₅-Alkyl), -N(C₁₋₅-Halogenalkyl)(C₁₋₅-Alkyl), - CONH₂, -CONH(C₁₋₅-Alkyl) und -CON(C₁₋₅-Alkyl)(C₁₋₅-Alkyl), und wobei das Cycloalkyl, Heterocycloalkyl, Aryl oder Heteroaryl optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl),
oder
wobei R₄ ein fünfgliedriges Heteroaryl ist, das optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -O(C₁₋₅-Halogenalkyl), -SH, -S(C₁₋₅-Alkyl), -S(C₁₋₅-Halogenalkyl), -NH₂, -NH(C₁₋₅-Alkyl), -NH(C₁₋₅-Halogenalkyl), -N(C₁₋₅-Alkyl)(C₁₋₅-Alkyl), - N(C₁₋₅-Halogenalkyl)(C₁₋₅-Alkyl), -CONH₂, -CONH(C₁₋₅-Alkyl) und -CON(C₁₋₅-Alkyl)(C₁₋₅-Alkyl),
bevorzugt, wobei das fünfgliedrige Heteroaryl ausgewählt ist aus Imidazolyl, Isoxazolyl, Pyrazolyl, 1,2,3-Triazolyl, 1,2,4-Triazolyl, Thiazolyl, 1,2,4-Oxadiazolyl, 1,3,4-Oxadiazolyl, 1,2,4-Thiadiazolyl oder 1,3,4-Thiadiazolyl, bevorzugt wobei das fünfgliedrige Heteroaryl 1,2,4-Thiadiazolyl ist, das optional mit einer oder mehreren Gruppen substituiert ist, die unabhängig voneinander ausgewählt sind aus Halogen, -CN, -OH, C₁₋₅-Alkyl, C₁₋₅-Halogenalkyl, -O(C₁₋₅-Alkyl), -SH, -S(C₁₋₅-Alkyl), -NH₂, -NH(C₁₋₅-Alkyl), -N(C₁₋₅-Alkyl)(C₁₋₅-Alkyl), -CONH₂, -CONH(C₁₋₅-Alkyl) und -CON(C₁₋₅-Alkyl)(C₁₋₅-Alkyl), bevorzugt optional substituiert mit C₁₋₅-Alkyl, C₁₋₅-Halogenalkyl, -O(C₁₋₅-Alkyl), -SH, -S(C₁₋₅-Alkyl), noch bevorzugter optional substituiert mit C₁₋₅-Alkyl, C₁₋₅-Halogenalkyl, sogar noch bevorzugter optional substituiert mit C₁₋₅-Halogenalkyl, bevorzugt ausgewählt aus -CH₂F, -CHF₂ und CF₃, am meisten bevorzugt optional substituiert mit -CHF₂.

11. Verbindung nach Anspruch 1, ausgewählt aus: 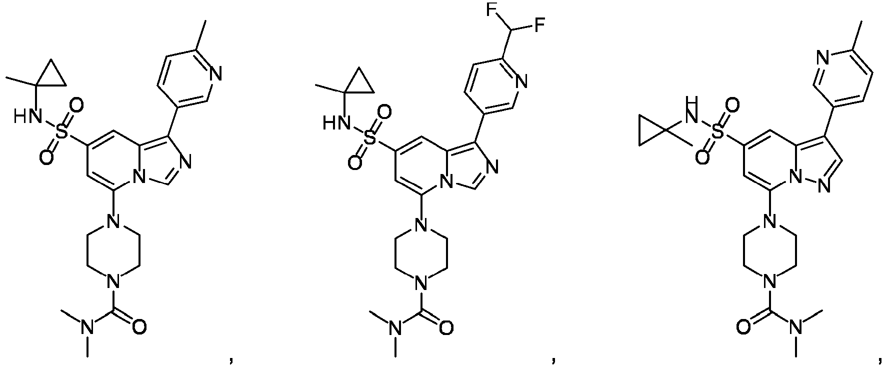 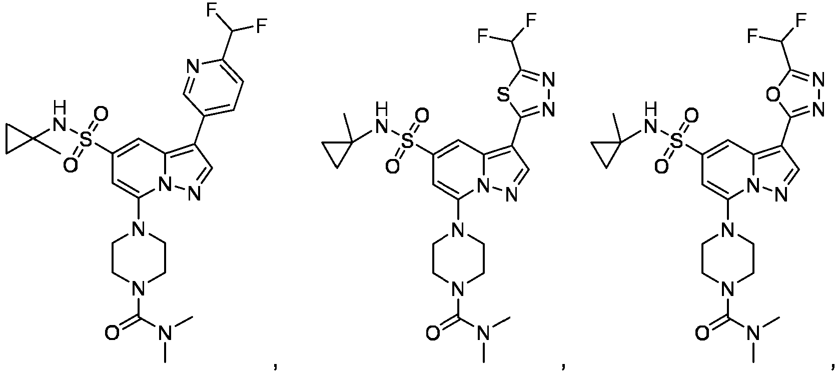 und 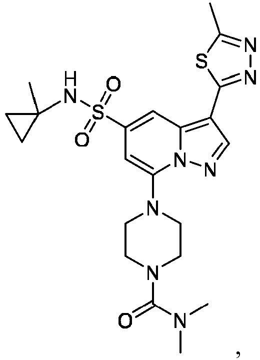 oder ein pharmazeutisch akzeptables Salz, Hydrat oder Solvat

12. Verbindung nach Anspruch 1, wobei die Verbindung eine Verbindung nach Formel (lap) ist: 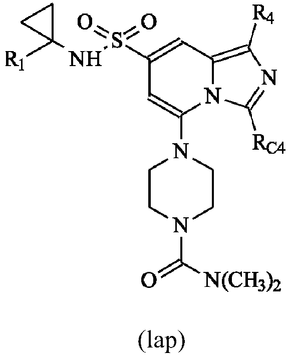 oder eine Verbindung nach Formel (Ibn): 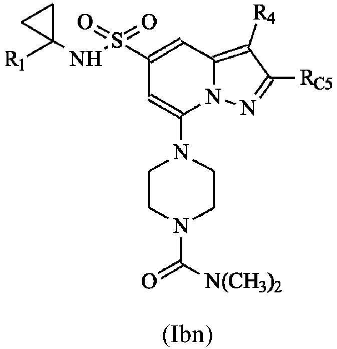 oder ein Enantiomer, Diastereomer, Tautomer, pharmazeutisch akzeptables Solvat, pharmazeutisch akzeptable Kristallform oder ein pharmazeutisch akzeptables Salz davon, wobei R₁, R₄, R_{C4} und R_{C5} jeweils wie in einem der Ansprüche 1 bis 10 definiert sind.

13. Pharmazeutische Zusammensetzung, umfassend die Verbindung nach einem der Ansprüche 1 bis 12 oder ein pharmazeutisch akzeptables Salz, Hydrat oder Solvat davon sowie einen pharmazeutisch akzeptablen Träger.

14. Verbindung nach einem der Ansprüche 1 bis 12 oder ein pharmazeutisch akzeptables Salz, Hydrat oder Solvat davon oder eine pharmazeutische Zusammensetzung nach Anspruch 13 zur Verwendung in Therapie.

15. Verbindung zur Verwendung oder pharmazeutische Zusammensetzung zur Verwendung nach Anspruch 14, zur Verwendung in einem Verfahren zum Behandeln einer proliferativen Erkrankung, bevorzugt wobei die proliferative Erkrankung Krebs ist, bevorzugt menschlicher Krebs, noch bevorzugter wobei der Krebs ausgewählt ist aus Lungen-, Darm-, Brust-, Eierstock-, Prostata-, Leber-, Bauchspeicheldrüsen-, Hirn- und Hautkrebs.

## Revendications

1. Composé de la formule (I) : 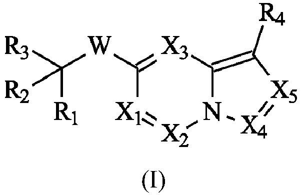 ou un énantiomère, un diastéréo-isomère, un tautomère, un solvate pharmaceutiquement acceptable, une forme cristalline pharmaceutiquement acceptable ou un sel pharmaceutiquement acceptable de celui-ci, dans lequel :
R₁ est choisi dans le groupe constitué par l'hydrogène, le chloro, le fluoro, le cyano, le formyle, l'alkyle en C₁₋₂, l'alcényle en C₂, l'alcynyle en C₂, l'haloalkyle en C₁₋₂, -(alkylène en C₁₋₂)-OH et -(alkylène en C₁₋₂)-O-(alkyle en C₁₋₂) ;
R₂ et R₃ forment, avec l'atome de carbone auquel ils sont attachés, un cyclopropyle ;
W est -NHS(O)₂-, dans lequel le côté gauche de W tel que défini ici est attaché à l'atome de carbone qui porte R₁, R₂ et R₃, et le côté droit de W tel que défini ici est attaché au système cyclique présenté dans la formule (I) ;
X₁ est CF ou CH et X₃ est CH ;
X₂ est C-Y_{C2}-R_{C2},
dans lequel Y_{C2} est choisi parmi une liaison covalente, alkylène en C₁₋₅, alcénylène en C₂₋₅, alcynylène en C₂₋₅, cycloalkylène, cycloalcénylène, hétérocycloalkylène et hétérocycloalcénylène, lesdits groupes alkylène, alcénylène et alcynylène pouvant chacun être éventuellement substitués par un ou plusieurs groupes choisis indépendamment parmi halogène, CN, OH, O(alkyle en C₁₋₅), -O(haloalkyle en C₁₋₅), un haloalkyle en C₁₋₅, SH, S(alkyle en C₁₋₅), -S(haloalkyle en C₁₋₅), NH₂, NH(alkyle en C₁₋₅), -NH(haloalkyle en C₁₋₅), N(alkyle en C₁₋₅)(alkyle en C₁₋₅), N(haloalkyle en C₁₋₅)(alkyle en C₁₋₅), -(N-hétérocycloalkyle), -CO(alkyle en C₁₋₅), CONH₂, CONH(alkyle en C₁₋₅), CON(alkyle en C₁₋₅)(alkyle en C₁₋₅), -CO-(N-hétérocycloalkyle), NHCO-(alkyle en C₁₋₅), N(alkyle en C₁₋₅)-CO-(alkyle en C₁₋₅), NHCONH₂, NHCONH-(alkyle en C₁₋₅), NHCON(alkyle en C₁₋₅)(alkyle en C₁₋₅), N(alkyle en C₁₋₅)CONH₂, N(alkyle en C₁₋₅)CONH-(alkyle en C₁₋₅) et N(alkyle en C₁₋₅)CON(alkyle en C₁₋₅)(alkyle en C₁₋₅), de préférence choisis parmi halogène, CN, OH, O(alkyle en C₁₋₅), SH, S(alkyle en C₁₋₅), NH₂, NH(alkyle en C₁₋₅) et N(alkyle en C₁₋₅)(alkyle en C₁₋₅), et dans lequel, en outre, une ou plusieurs unités -CH₂- comprises dans ledit alkylène, ledit alcénylène ou ledit alcynylène sont chacune éventuellement remplacées par un groupe choisi indépendamment parmi -O-, NH-, N(alkyle en C₁₋₅)-, CO-, S-, -SO- et SO₂-, et dans lequel, en outre, ledit cycloalkylène, ledit cycloalcénylène, ledit hétérocycloalkylène et ledit hétérocycloalcénylène sont chacun éventuellement substitués par un ou plusieurs groupes choisis indépendamment parmi halogène, CN, OH, alkyle en C₁₋₅, haloalkyle en C₁₋₅, O(alkyle en C₁₋₅), -O(haloalkyle en C₁₋₅), SH, S(alkyle en C₁₋₅), -S(haloalkyle en C₁₋₅), NH₂, NH(alkyle en C₁₋₅), -NH(haloalkyle en C₁₋₅), N(alkyle en C₁₋₅)(alkyle en C₁₋₅), N(haloalkyle en C₁₋₅)(alkyle en C₁₋₅), -(N-hétérocycloalkyle), -CO(alkyle en C₁₋₅), CONH₂, CONH(alkyle en C₁₋₅), CON(alkyle en C₁₋₅)(alkyle en C₁₋₅), -CO-(N-hétérocycloalkyle), NHCO-(alkyle en C₁₋₅), N(alkyle en C₁₋₅)-CO-(alkyle en C₁₋₅), NHCONH₂, NHCONH-(alkyle en C₁₋₅), NHCON(alkyle en C₁₋₅)(alkyle en C₁₋₅), N(alkyle en C₁₋₅)CONH₂, N(alkyle en C₁₋₅)CONH-(alkyle en C₁₋₅) et N(alkyle en C₁₋₅)CON(alkyle en C₁₋₅)(alkyle en C₁₋₅), -(alkylène en C₁₋₅)-CN, -(alkylène en C₁₋₅)OH, - (alkylène en C₁₋₅)O(alkyle en C₁₋₅), -(alkylène en C₁₋₅)-O(haloalkyle en C₁₋₅), -(alkylène en C₁₋₅)SH, -(alkylène en C₁₋₅)S(alkyle en C₁₋₅), -(alkylène en C₁₋₅)-S(haloalkyle en C₁₋₅), -(alkylène en C₁₋₅)NH₂, -(alkylène en C₁₋₅)NH(alkyle en C₁₋₅), -(alkylène en C₁₋₅)-NH(haloalkyle en C₁₋₅), -(alkylène en C₁₋₅)N(alkyle en C₁₋₅)(alkyle en C₁₋₅), -(alkylène en C₁₋₅)N(alkyle en C₁₋₅)(haloalkyle en C₁₋₅), -(alkylène en C₁₋₅)(N-hétérocycloalkyle), -(alkylène en C₁₋₅)N(haloalkyle en C₁₋₅)(alkyle en C₁₋₅), -(alkylène en C₁₋₅)-CO(alkyle en C₁₋₅), -(alkylène en C₁₋₅)CONH₂, -(alkylène en C₁₋₅)CONH(alkyle en C₁₋₅), -(alkylène en C₁₋₅)CON(alkyle en C₁₋₅)alkyle en C₁₋₅), -(alkylène en C₁₋₅)CO-(N-hétérocycloalkyle), -(alkylène en C₁₋₅)NHCO-(alkyle en C₁₋₅), -(alkylène en C₁₋₅)N(alkyle en C₁₋₅)-CO-(alkyle en C₁₋₅), -(alkylène en C₁₋₅)NHCONH₂, -(alkylène en C₁₋₅) NHCONH-(alkyle en C₁₋₅), -(alkylène en C₁₋₅)NHCON(alkyle en C₁₋₅)(alkyle en C₁₋₅), -(alkylène en C₁₋₅)N(alkyle en C₁₋₅)CONH₂, -(alkylène en C₁₋₅)N(alkyle en C₁₋₅) CONH-(alkyle en C₁₋₅), et -(alkylène en C₁₋₅)N(alkyle en C₁₋₅)CON(alkyle en C₁₋₅)(alkyle en C₁₋₅), de préférence choisis parmi halogène, CN, OH, alkyle en C₁₋₅, O(alkyle en C₁₋₅), SH, S(alkyle en C₁₋₅), NH₂, NH(alkyle en C₁₋₅) et N(alkyle en C₁₋₅)(alkyle en C₁₋₅), et R_{C2} est choisi parmi l'hydrogène, l'halo, -OH, -NH₂, -SH, -CN, l'alkyle en C₁₋₁₂, l'alcényle en C₂₋₁₂, l'alcynyle en C₂₋₁₂, le cycloalkyle, le cycloalcényle, l'hétérocycloalkyle, l'hétérocycloalcényle, l'aryle et l'hétéroaryle,
dans lequel lesdits groupes alkyle, alcényle ou alcynyle sont éventuellement substitués par un ou plusieurs groupes choisis indépendamment parmi halogène, -CN, -OH, -O(alkyle en C₁₋₅), - O(haloalkyle en C₁₋₅), un haloalkyle en C₁₋₅, -SH, -S(alkyle en C₁₋₅), -S(haloalkyle en C₁₋₅), - NH₂, -NH(alkyle en C₁₋₅), -NH(haloalkyle en C₁₋₅), -N(alkyle en C₁₋₅)(alkyle en C₁₋₅), - N(haloalkyle en C₁₋₅)(alkyle en C₁₋₅), -(*N*-hétérocycloalkyle), -CO(alkyle en C₁₋₅), -CONH₂, - CONH(alkyle en C₁₋₅), -CON(alkyle en C₁₋₅)(alkyle en C₁₋₅), -CO-(*N*-hétérocycloalkyle), - NHCO-(alkyle en C₁₋₅), -N(alkyle en C₁₋₅)-CO-(alkyle en C₁₋₅), -NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), - S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -P(O)(alkyle en C₁₋₅)(alkyle en C₁₋₅), -P(O)(O(alkyle en C₁₋₅))(O(alkyle en C₁₋₅)), et -P(O)(O(alkyle en C₁₋₅))(alkyle en C₁₋₅), de préférence choisis parmi halogène, -CN, -OH, -O(alkyle en C₁₋₅)-, -O(haloalkyle en C₁₋₅)-, un haloalkyle en C₁₋₅, -SH, -S(alkyle en C₁₋₅), -S(haloalkyle en C₁₋₅), -NH₂, -NH(alkyle en C₁₋₅), - NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅), de préférence choisis parmi halogène, -CN, -OH, -O(alkyle en C₁₋₅), -O(haloalkyle en C₁₋₅), haloalkyle en C₁₋₅, -SH, -S(alkyle en C₁₋₅), -S(haloalkyle en C₁₋₅), -NH₂, -NH(alkyle en C₁₋₅), - NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), -NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), - N(alkyle en C₁₋₅)CON(alkyle en C₁₋₅)(alkyle en C₁₋₅), -S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -P(O)(alkyle en C₁₋₅)(alkyle en C₁₋₅), -P(O)(O(alkyle en C₁₋₅))(O(alkyle en C₁₋₅)), et -P(O)(O(alkyle en C₁₋₅))(alkyle en C₁₋₅), de préférence choisis parmi halogène, - CN, -OH, -O(alkyle en C₁₋₅), -O(haloalkyle en C₁₋₅), un haloalkyle en C₁₋₅, -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, - CONH(alkyle en C₁₋₅) et -CON(alkyle en C₁₋₅)(alkyle en C₁₋₅), et
dans lequel lesdits groupes cycloalkyle, cycloalcényle, hétérocycloalkyle, hétérocycloalcényle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CO(haloalkyle en C₁₋₅), -CO-cycloalkyle, - COO(alkyle en C₁₋₅), -COO(haloalkyle en C₁₋₅), -COO-cycloalkyle, -CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), -NHCONH₂, -NHCONH-(alkyle en C₁₋₅), - NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -OCONH₂, -OCONH-(alkyle en C₁₋₅), -OCON(alkyle en C₁₋₅)(alkyle en C₁₋₅), -NHCOO(alkyle en C₁₋₅), -N(alkyle en C₁₋₅)COO-(alkyle en C₁₋₅), -P(O)(alkyle en C₁₋₅)(alkyle en C₁₋₅), -P(O)(O(alkyle en C₁₋₅))(O(alkyle en C₁₋₅)), -P(O)(O(alkyle en C₁₋₅))(alkyle en C₁₋₅), -(alkylène en C₁₋₅)-CN, - (alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-S(O)(alkyle en C₁₋₅), -(alkylène en C₁₋₅)-S(O)₂(alkyle en C₁₋₅), -(alkylène en C₁₋₅)-S(O)(NH)(alkyle en C₁₋₅), -(alkylène en C₁₋₅)-S(O)(N(alkyle en C₁₋₅))(alkyle en C₁₋₅), -(alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C15), -(alkylène en C₁₋₅)-CO(alkyle en C₁₋₅), -(alkylène en C₁₋₅)-CO(haloalkyle en C₁₋₅), -(alkylène en C₁₋₅)-CO-cycloalkyle, -(alkylène en C₁₋₅)-CONH₂, -(alkylène en C₁₋₅)-CONH(alkyle en C₁₋₅), -(alkylène en C₁₋₅)-CON(alkyle en C₁₋₅)(alkyle en C₁₋₅), -(alkylène en C₁₋₅)-CO-(N-hétérocycloalkyle), -(alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅) CONH₂, -(alkylène en C₁₋₅)-N(alkyle en C₁₋₅)CONH-(alkyle en C₁₋₅), -(alkylène en C₁₋₅)-N(alkyle en C₁₋₅)CON(alkyle en C₁₋₅)(alkyle en C₁₋₅), -(alkylène en C₁₋₅)-OCONH₂, -(alkylène en C₁₋₅)-OCONH-(alkyle en C₁₋₅), -(alkylène en C₁₋₅)-OCON(alkyle en C₁₋₅)(alkyle en C₁₋₅), -(alkylène en C₁₋₅)-NHCOO(alkyle en C₁₋₅) et -(alkylène en C₁₋₅)-N(alkyle en C₁₋₅)COO-(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -COO(alkyle en C₁₋₅), -CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), -NHCONH₂, -NHCONH-(alkyle en C₁₋₅), - NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -OCONH₂, -OCONH-(alkyle en C₁₋₅), -OCON(alkyle en C₁₋₅)(alkyle en C₁₋₅), -NHCOO(alkyle en C₁₋₅), -N(alkyle en C₁₋₅)COO-(alkyle en C₁₋₅), -P(O)(alkyle en C₁₋₅)(alkyle en C₁₋₅), -P(O)(O(alkyle en C₁₋₅))(O(alkyle en C₁₋₅)), -P(O)(O(alkyle en C₁₋₅))(alkyle en C₁₋₅), -(alkylène en C₁₋₅)-CN, - (alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-S(O)(alkyle en C₁₋₅), -(alkylène en C₁₋₅)-S(O)₂(alkyle en C₁₋₅), -(alkylène en C₁₋₅)-S(O)(NH)(alkyle en C₁₋₅), -(alkylène en C₁₋₅)-S(O)(N(alkyle en C₁₋₅))(alkyle en C₁₋₅), -(alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CONH₂, -(alkylène en C₁₋₅)-CONH(alkyle en C₁₋₅), -(alkylène en C₁₋₅)-CON(alkyle en C₁₋₅)(alkyle en C₁₋₅), -(alkylène en C₁₋₅)-CO-(*N*-hétérocycloalkyle), -(alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅)CONH₂, -(alkylène en C₁₋₅)-N(alkyle en C₁₋₅)CONH-(alkyle en C₁₋₅), -(alkylène en C₁₋₅)-N(alkyle en C₁₋₅)CON(alkyle en C₁₋₅)(alkyle en C₁₋₅), -(alkylène en C₁₋₅)-OCONH₂, -(alkylène en C₁₋₅)-OCONH-(alkyle en C₁₋₅), -(alkylène en C₁₋₅)-OCON(alkyle en C₁₋₅)(alkyle en C₁₋₅), -(alkylène en C₁₋₅)-NHCOO(alkyle en C₁₋₅), et -(alkylène en C₁₋₅)-N(alkyle en C₁₋₅) COO-(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(*N-*hétérocycloalkyle), -CO(alkyle en C₁₋₅), -CONH₂, -CONH(alkyle en C₁₋₅), -CON(alkyle en C₁₋₅)(alkyle en C₁₋₅), -CO-(*N*-hétérocycloalkyle), -NHCO-(alkyle en C₁₋₅), -N(alkyle en C₁₋₅)-CO-(alkyle en C₁₋₅), -NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -P(O)(alkyle en C₁₋₅)(alkyle en C₁₋₅), -P(O)(O(alkyle en C₁₋₅))(O(alkyle en C₁₋₅)), -P(O)(O(alkyle en C₁₋₅))(alkyle en C₁₋₅), -(alkylène en C₁₋₅)-CN, - (alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-S(O)(alkyle en C₁₋₅), -(alkylène en C₁₋₅)-S(O)₂(alkyle en C₁₋₅), -(alkylène en C₁₋₅)-S(O)(NH)(alkyle en C₁₋₅), -(alkylène en C₁₋₅)-S(O)(N(alkyle en C₁₋₅))(alkyle en C₁₋₅), -(alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(*N*-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CONH₂, -(alkylène en C₁₋₅)-CONH(alkyle en C₁₋₅), -(alkylène en C₁₋₅)-CON(alkyle en C₁₋₅)(alkyle en C₁₋₅), -(alkylène en C₁₋₅)-CO-(*N*-hétérocycloalkyle), -(alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅)CONH₂, -(alkylène en C₁₋₅)-N(alkyle en C₁₋₅)CONH-(alkyle en C₁₋₅), et -(alkylène en C₁₋₅)-N(alkyle en C₁₋₅)CON(alkyle en C₁₋₅)(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -(alkylène en C₁₋₅)-OH, -(alkylène en C₁₋₅)-O(alkyle en C₁₋₅), -(alkylène en C₁₋₅)-O(haloalkyle en C₁₋₅), -SH, -S(alkyle en C₁₋₅), - S(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(alkylène en C₁₋₅)-NH₂, - (alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, - NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅) ;
X₄ représente N ou C-R_{C4}, où R_{C4} est choisi parmi hydrogène, halo, alkyle en C₁₋₆, alcynyle en C₂₋₆, -O(alkyle en C₁₋₆), -S(alkyle en C₁₋₆), -NH(alkyle en C₁₋₆), -N(alkyle en C₁₋₆)(alkyle en C₁₋₆), -CO(alkyle en C₁₋₆), haloalkyle en C₁₋₆, -O(haloalkyle en C₁₋₆), -S(haloalkyle en C₁₋₆), - NH(haloalkyle en C₁₋₆), -N(haloalkyle en C₁₋₆)₂, -CO(haloalkyle en C₁₋₆), -(alkylène en C₀₋₃)cycloalkyle, -O-(alkylène en C₀₋₃)cycloalkyle, -CO-(alkylène en C₀₋₃)cycloalkyle, -(alkylène en C₀₋₃)cycloalcényle, -O-(alkylène en C₀₋₃)-cycloalcényle, -CO-(alkylène en C₀₋₃)-cycloalcényle, -(alkylène en C₀₋₃)-hétérocycloalkyle, -O-(alkylène en C₀₋₃)-hétérocycloalkyle, -CO-(alkylène en C₀₋₃)-hétérocycloalkyle, -(alkylène en C₀₋₃)-hétérocycloalcényle, -O-(alkylène en C₀₋₃)-hétérocycloalcényle, -CO-(alkylène en C₀₋₃)-hétérocycloalcényle, -(alkylène en C₀₋₃)-aryle, -O-(alkylène en C₀₋₃)-aryle, -CO-(alkylène en C₀₋₃)-aryle, -(alkylène en C₀₋₃)-hétéroaryle, -O-(alkylène en C₀₋₃)-hétéroaryle et -CO-(alkylène en C₀₋₃)-hétéroaryle,
dans lequel lesdits groupes alkyle ou alcynyle sont éventuellement substitués par un ou plusieurs groupes choisis indépendamment parmi un halogène, -CN, -OH, -O(alkyle en C₁₋₅), - O(haloalkyle en C₁₋₅), un haloalkyle en C₁₋₅, -SH, -S(alkyle en C₁₋₅), -S(haloalkyle en C₁₋₅), - NH₂, -NH(alkyle en C₁₋₅), -NH(haloalkyle en C₁₋₅), -N(alkyle en C₁₋₅)(alkyle en C₁₋₅), - N(haloalkyle en C₁₋₅)(alkyle en C₁₋₅), -(N-hétérocycloalkyle), -CO(alkyle en C₁₋₅), -CONH₂, - CONH(alkyle en C₁₋₅), -CON(alkyle en C₁₋₅)(alkyle en C₁₋₅), -CO-(N-hétérocycloalkyle), - NHCO-(alkyle en C₁₋₅), -N(alkyle en C₁₋₅)-CO-(alkyle en C₁₋₅), -NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅) et -N(alkyle en C₁₋₅)CON(alkyle en C₁₋₅)(alkyle en C₁₋₅), de préférence choisis parmi halogène, -CN, -OH, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, - CONH(alkyle en C₁₋₅) et -CON(alkyle en C₁₋₅)(alkyle en C₁₋₅), et
dans lequel lesdits groupes cycloalkyle, cycloalcényle, hétérocycloalkyle, hétérocycloalcényle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), -NHCONH₂, -NHCONH-(alkyle en C₁₋₅), - NHCON(alkyle en C₁₋₅)(alkyle en C₁₋₅), -N(alkyle en C₁₋₅)CONH₂, -N(alkyle en C₁₋₅)CONH-(alkyle en C₁₋₅) et -N(alkyle en C₁₋₅)CON(alkyle en C₁₋₅)(alkyle en C₁₋₅), -(alkylène en C₁₋₅)-CN, -(alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CONH₂, -(alkylène en C₁₋₅)-CONH(alkyle en C₁₋₅), - (alkylène en C₁₋₅)-CON(alkyle en C₁₋₅)(alkyle en C₁₋₅), -(alkylène en C₁₋₅)-CO-(N-hétérocycloalkyle), -(alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅) CONH₂, -(alkylène en C₁₋₅)-N(alkyle en C₁₋₅)CONH-(alkyle en C₁₋₅) et - (alkylène en C₁₋₅)-N(alkyle en C₁₋₅)CON(alkyle en C₁₋₅)(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), - NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅) ;
X₅ est N ou C-R_{C5},
dans lequel R_{C5} est choisi parmi l'hydrogène, l'halo, l'alkyle en C₁₋₆, -O(alkyle en C₁₋₆), -S(alkyle en C₁₋₆), -NH(alkyle en C₁₋₆), -N(alkyle en C₁₋₆)(alkyle en C₁₋₆) et l'haloalkyle en C₁₋₆ ;
R₄ est Y_{R5}-R_{R5},
dans lequel Y_{R5} est choisi parmi une liaison covalente, alkylène en C₁₋₄, alcénylène en C₂₋₄ et alcynylène en C_{2-4,} lesdits groupes alkylène, alcénylène et alcynylène pouvant chacun être éventuellement substitués par un ou plusieurs groupes choisis indépendamment parmi halogène, -CN, -OH, -O(alkyle en C₁₋₅), -O(haloalkyle en C₁₋₅), -SH, -S(alkyle en C₁₋₅), - SO(alkyle en C₁₋₅), -SO₂(alkyle en C₁₋₅), -S(haloalkyle en C₁₋₅), -SO(haloalkyle en C₁₋₅), - SO₂(haloalkyle en C₁₋₅), -NH₂, -NH(alkyle en C₁₋₅), -NH(haloalkyle en C₁₋₅), -N(alkyle en C₁₋₅)(alkyle en C₁₋₅) et -N(haloalkyle en C₁₋₅)(alkyle en C₁₋₅), et dans lequel, en outre, une ou plusieurs unités -CH₂- comprises dans lesdits groupes alkylène, alcénylène ou alcynylène sont chacun éventuellement remplacés par un groupe choisi indépendamment parmi -O-, NH-, N(alkyle en C₁₋₅)-, CO-, -COO-, S-, -SO- et SO₂-, et
dans lequel R_{R5} est choisi parmi alkyle en C₁₋₁₂, alcényle en C₂₋₁₂, alcynyle en C₂₋₁₂, cycloalkyle, cycloalcényle, hétérocycloalkyle, hétérocycloalcényle, aryle et hétéroaryle,
dans lequel lesdits groupes alkyle, alcényle ou alcynyle sont éventuellement substitués par un ou plusieurs groupes choisis indépendamment parmi halogène, -CN, -OH, -O(alkyle en C₁₋₅), - O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅), et dans lequel lesdits groupes cycloalkyle, cycloalcényle, hétérocycloalkyle, hétérocycloalcényle, aryle ou hétéroaryle sont éventuellement substitués par un ou plusieurs groupes choisis indépendamment parmi halogène, -CN, -OH, -alkyle en C₁₋₅, -haloalkyle en C₁₋₅, -O(alkyle en C₁₋₅), -O(haloalkyle en C₁₋₅), -SH, -S(alkyle en C₁₋₅), -SO(alkyle en C₁₋₅), -SO₂(alkyle en C₁₋₅), -S(haloalkyle en C₁₋₅), -SO(haloalkyle en C₁₋₅), -SO₂(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅).

2. Composé selon la revendication 1, dans lequel R₁ est choisi dans le groupe constitué d'un groupe chloro, fluoro, cyano, formyle, alkyle en C₁₋₂, alcényle en C₂, alcynyle en C₂ et haloalkyle en C₁₋₂, de préférence dans lequel R₁ est choisi parmi le groupe constitué d'un groupe cyano, alkyle en C₁₋₂ et haloalkyle en C₁₋₂, de préférence dans lequel R₁ est choisi parmi le groupe constitué du cyano, du méthyle et du fluorométhyle, et de manière encore plus préférentielle
dans lequel R₁ est un groupe cyano, dans lequel R₁ est un groupe méthyle, ou
dans lequel R₁ est un groupe fluorométhyle.

3. Composé selon la revendication 1 ou 2, dans lequel Y_{C2} est choisi parmi une liaison covalente, alkylène en C₁₋₅, alcénylène en C₂₋₅, alcynylène en C₂₋₅, cycloalkylène et hétérocycloalkylène, lesdits groupes alkylène, alcénylène et alcynylène pouvant chacun être éventuellement substitués par un ou plusieurs groupes choisis indépendamment parmi halogène, CN, OH, O(alkyle en C₁₋₅), -O(haloalkyle en C₁₋₅), haloalkyle en C₁₋₅, SH, S(alkyle en C₁₋₅), - S(haloalkyle en C₁₋₅), NH₂, NH(alkyle en C₁₋₅), -NH(haloalkyle en C₁₋₅), N(alkyle en C₁₋₅)(alkyle en C₁₋₅), N(haloalkyle en C₁₋₅)(alkyle en C₁₋₅), -(N-hétérocycloalkyle), -CO(alkyle en C₁₋₅), CONH₂, CONH(alkyle en C₁₋₅), CON(alkyle en C₁₋₅)(alkyle en C₁₋₅), -CO-(N-hétérocycloalkyle), NHCO-(alkyle en C₁₋₅), N(alkyle en C₁₋₅)-CO-(alkyle en C₁₋₅), NHCONH₂, NHCONH-(alkyle en C₁₋₅), NHCON(alkyle en C₁₋₅)(alkyle en C₁₋₅), N(alkyle en C₁₋₅)CONH₂, N(alkyle en C₁₋₅)CONH-(alkyle en C₁₋₅) et N(alkyle en C₁₋₅)CON(alkyle en C₁₋₅)(alkyle en C₁₋₅), de préférence choisis parmi halogène, CN, OH, O(alkyle en C₁₋₅), SH, S(alkyle en C₁₋₅), NH₂, NH(alkyle en C₁₋₅) et N(alkyle en C₁₋₅)(alkyle en C₁₋₅), et dans lequel, en outre, une ou plusieurs unités -CH₂- comprises dans lesdits groupes alkylène, alcénylène ou alcynylène sont chacun éventuellement remplacés par un groupe choisi indépendamment parmi -O-, NH-, N(alkyle en C₁₋₅)-, CO-, S-, -SO- et SO₂-, et dans lequel, en outre, lesdits groupes cycloalkylène et hétérocycloalkylène sont chacun éventuellement substitués par un ou plusieurs groupes choisis indépendamment parmi halogène, CN, OH, alkyle en C₁₋₅, haloalkyle en C₁₋₅, O(alkyle en C₁₋₅), -O(haloalkyle en C₁₋₅), SH, S(alkyle en C₁₋₅), -S(haloalkyle en C₁₋₅), NH₂, NH(alkyle en C₁₋₅), -NH(haloalkyle en C₁₋₅), N(alkyle en C₁₋₅)(alkyle en C₁₋₅), N(haloalkyle en C₁₋₅)(alkyle en C₁₋₅), -(N-hétérocycloalkyle), -CO(alkyle en C₁₋₅), CONH₂, CONH(alkyle en C₁₋₅), CON(alkyle en C₁₋₅)(alkyle en C₁₋₅), -CO-(N-hétérocycloalkyle), NHCO-(alkyle en C₁₋₅), N(alkyle en C₁₋₅)-CO-(alkyle en C₁₋₅), NHCONH₂, NHCONH-(alkyle en C₁₋₅), NHCON(alkyle en C₁₋₅)(alkyle en C₁₋₅), N(alkyle en C₁₋₅)CONH₂, N(alkyle en C₁₋₅)CONH-(alkyle en C₁₋₅) et N(alkyle en C₁₋₅)CON(alkyle en C₁₋₅)(alkyle en C₁₋₅), -(alkylène en C₁₋₅)-CN, -(alkylène en C₁₋₅)OH, -(alkylène en C₁₋₅)O(alkyle en C₁₋₅), -(alkylène en C₁₋₅)-O(haloalkyle en C₁₋₅), -(alkylène en C₁₋₅)SH, -(alkylène en C₁₋₅)S(alkyle en C₁₋₅), -(alkylène en C₁₋₅)-S(haloalkyle en C₁₋₅), - (alkylène en C₁₋₅)NH₂, -(alkylène en C₁₋₅)NH(alkyle en C₁₋₅), -(alkylène en C₁₋₅)-NH(haloalkyle en C₁₋₅), -(alkylène en C₁₋₅)N(alkyle en C₁₋₅)(alkyle en C₁₋₅), -(alkylène en C₁₋₅)N(alkyle en C₁₋₅)(haloalkyle en C₁₋₅), -(alkylène en C₁₋₅)(N-hétérocycloalkyle), -(alkylène en C₁₋₅)N(haloalkyle en C₁₋₅)(alkyle en C₁₋₅), -(alkylène en C₁₋₅)-CO(alkyle en C₁₋₅), -(alkylène en C₁₋₅) CONH₂, -(alkylène en C₁₋₅)CONH(alkyle en C₁₋₅), -(alkylène en C₁₋₅)CON(alkyle en C₁₋₅)(alkyle en C₁₋₅), -(alkylène en C₁₋₅)CO-(N-hétérocycloalkyle), -(alkylène en C₁₋₅) NHCO-(alkyle en C₁₋₅), -(alkylène en C₁₋₅)N(alkyle en C₁₋₅)-CO-(alkyle en C₁₋₅), -(alkylène en C₁₋₅)NHCONH₂, -(alkylène en C₁₋₅)NHCONH-(alkyle en C₁₋₅), -(alkylène en C₁₋₅) NHCON(alkyle en C₁₋₅) (alkyle en C₁₋₅), -(alkylène en C₁₋₅)N(alkyle en C₁₋₅)CONH₂, -(alkylène en C₁₋₅)N(alkyle en C₁₋₅)CONH-(alkyle en C₁₋₅), et -(alkylène en C₁₋₅)N(alkyle en C₁₋₅)CON(alkyle en C₁₋₅)(alkyle en C₁₋₅), de préférence choisis parmi halogène, CN, OH, alkyle en C₁₋₅, O(alkyle en C₁₋₅), SH, S(alkyle en C₁₋₅), NH₂, NH(alkyle en C₁₋₅) et N(alkyle en C₁₋₅)(alkyle en C₁₋₅), ou dans lequel -Y_{C2}-R_{C2} est choisi parmi -O-alkyle en C₁₋₁₂, -NH-alkyle en C₁₋₁₂, -N(alkyle en C₁₋₅)-alkyle en C₁₋₁₂, -O-alcényle en C₂₋₁₂, -NH-alcényle en C₂₋₁₂, -N(alkyle en C₁₋₅)-alcényle en C₂₋₁₂, -O-alcynyle en C₂₋₁₂, -NH-alcynyle en C₂₋₁₂, -N(alkyle en C₁₋₅)-alkynyle en C₂₋₁₂, (alkylène en C₀₋₃)-cycloalkyle, -CO-(alkylène en C₀₋₃)-cycloalkyle, (alkylène en C₀₋₃)-CO-cycloalkyle, -CONH-(alkylène en C₀₋₃)cycloalkyle, -(alkylène en C₀₋₃)-CONH-cycloalkyle, - NHCO-(alkylène en C₀₋₃)cycloalkyle, (alkylène en C₀₋₃)-NHCO-cycloalkyle, -NH-(alkylène en C₀₋₃)cycloalkyle, -(alkylène en C₀₋₃)-NH-cycloalkyle, -O-(alkylène en C₀₋₃)cycloalkyle, - (alkylène en C₀₋₃)-O-cycloalkyle, -SO₂-(alkylène en C₀₋₃)cycloalkyle, -(alkylène en C₀₋₃)-SO₂-cycloalkyle, -CONH-cycloalkyle, -NHCO-cycloalkyle, -NH-cycloalkyle, -O-cycloalkyle, -CO-cycloalkyle, -SO₂-cycloalkyle, (alkylène en C₀₋₃)-cycloalcényle, -CO-(alkylène en C₀₋₃)cycloalcényle, (alkylène en C₀₋₃)-CO-cycloalcényle, -CONH-(alkylène en C₀₋₃)cycloalcényle, -(alkylène en C₀₋₃)-CONH-cycloalcényle, -NHCO-(alkylène en C₀₋₃)cycloalcényle, (alkylène en C₀₋₃)-NHCO-cycloalcényle, -NH-(alkylène en C₀₋₃)cycloalcényle, -(alkylène en C₀₋₃)-NH-cycloalcényle, -O-(alkylène en C₀₋₃)cycloalcényle, -(alkylène en C₀₋₃)-O-cycloalcényle, -SO₂-(alkylène en C₀₋₃)cycloalcényle, -(alkylène en C₀₋₃)-SO₂-cycloalcényle, -CONH-cycloalcényle, -NHCO-cycloalcényle, -NH-cycloalcényle, -O-cycloalcényle, -CO-cycloalcényle, -SO₂-cycloalcényle, -(alkylène en C₀₋₃)-hétérocycloalkyle, -CO-(alkylène en C₀₋₃)-hétérocycloalkyle, -(alkylène en C₀₋₃)-CO-hétérocycloalkyle, -CONH-(alkylène en C₀₋₃)-hétérocycloalkyle, - (alkylène en C₀₋₃)-CONH-hétérocycloalkyle, -NHCO-(alkylène en C₀₋₃)hétérocycloalkyle, - (alkylène en C₀₋₃)-NHCO-hétérocycloalkyle, -NH-(alkylène en C₀₋₃)hétérocycloalkyle, - (alkylène en C₀₋₃)-NH-hétérocycloalkyle, -O-(alkylène en C₀₋₃)hétérocycloalkyle, -(alkylène en C₀₋₃)-O-hétérocycloalkyle, -SO₂-(C₀₋₃ alkylène)hétérocycloalkyle, -(C₀₋₃ alkylène)-SO₂-hétérocycloalkyle, -CONH-hétérocycloalkyle, -NHCO-hétérocycloalkyle, -NH-hétérocycloalkyle, -O-hétérocycloalkyle, -CO-hétérocycloalkyle, -SO₂-hétérocycloalkyle, - (alkylène en C₀₋₃)-hétérocycloalcényle, -CO-(alkylène en C₀₋₃)-hétérocycloalcényle, -(alkylène en C₀₋₃)-CO-hétérocycloalcényle, -CONH-(alkylène en C₀₋₃)hétérocycloalcényle, -(alkylène en C₀₋₃)-CONH-hétérocycloalcényle, -NHCO-(alkylène en C₀₋₃)hétérocycloalcényle, -(alkylène en C₀₋₃)-NHCO-hétérocycloalcényle, -NH-(alkylène en C₀₋₃)hétérocycloalcényle, -(alkylène en C₀₋₃)-NH-hétérocycloalcényle, -O-(alkylène en C₀₋₃)hétérocycloalcényle, -(alkylène en C₀₋₃)-O-hétérocycloalcényle, -SO₂-(alkylène en C₀₋₃)hétérocycloalcényle, -(alkylène en C₀₋₃)-SO₂-hétérocycloalcényle, -CONH-hétérocycloalcényle, -NHCO-hétérocycloalcényle, -NH-hétérocycloalcényle, -O-hétérocycloalcényle, -CO-hétérocycloalcényle, -SO₂-hétérocycloalcényle, (alkylène en C₀₋₃)aryle, -CO-(alkylène en C₀₋₃)aryle, -(alkylène en C₀₋₃)-CO-aryle, -CONH-(alkylène en C₀₋₃)aryle, -(alkylène en C₀₋₃)-CONH-aryle, -NHCO-(alkylène en C₀₋₃)aryle, -(alkylène en C₀₋₃)-NHCO-aryle, -NH-(alkylène en C₀₋₃)aryle, -(alkylène en C₀₋₃)-NH-aryle, -O-(alkylène en C₀₋₃)aryle, -(alkylène en C₀₋₃)-O-aryle, -SO₂-(alkylène en C₀₋₃)aryle, -(alkylène en C₀₋₃)-SO₂-aryle, -CONH-aryle, -NHCO-aryle, -NH-aryle, -O-aryle, -CO-aryle, -SO₂-aryle, -(alkylène en C₀₋₃)hétéroaryle, -CO-(alkylène en C₀₋₃)hétéroaryle, -(alkylène en C₀₋₃)-CO-hétéroaryle, -CONH-(alkylène en C₀₋₃)hétéroaryle, -(alkylène en C₀₋₃)-CONH-hétéroaryle, -NHCO-(C₀₋₃ alkylène)hétéroaryle, -(C₀₋₃ alkylène)-NHCO-hétéroaryle, -NH-(C₀₋₃ alkylène)hétéroaryle, (C₀₋₃ alkylène)-NH-hétéroaryle, -O-(alkylène en C₀₋₃)hétéroaryle, - (alkylène en C₀₋₃)-O-hétéroaryle, -SO2-(alkylène en C₀₋₃)hétéroaryle, -(alkylène en C₀₋₃)-SO2-hétéroaryle, -CONH-hétéroaryle, -NHCO-hétéroaryle, -NH-hétéroaryle, -O-hétéroaryle, -CO-hétéroaryle et -SO₂-hétéroaryle, de préférence -YC₂-RC₂ est choisi parmi -O-alkyle en C₁₋₁₂, - NH-alkyle en C₁₋₁₂, -N(alkyle en C₁₋₅)-alkyle en C₁₋₁₂, -O-alcényle en C₂₋₁₂, -NH-alcényle en C₂₋₁₂, -N(alkyle en C₁₋₅)-alcényle en C₂₋₁₂, -O-alcynyle en C₂₋₁₂, -NH-alcynyle en C₂₋₁₂, - N(alkyle en C₁₋₅)-alkynyle en C₂₋₁₂, (alkylène en C₀₋₃)-cycloalkyle, -CO-(alkylène en C₀₋₃)-cycloalkyle, (alkylène en C₀₋₃)-CO-cycloalkyle, -CONH-(alkylène en C₀₋₃)cycloalkyle, - (alkylène en C₀₋₃)-CONH-cycloalkyle, -NHCO-(alkylène en C₀₋₃)cycloalkyle, (alkylène en C₀₋₃)-NHCO-cycloalkyle, -NH-(alkylène en C₀₋₃)cycloalkyle, -(alkylène en C₀₋₃)-NH-cycloalkyle, -O-(alkylène en C₀₋₃)cycloalkyle, -(alkylène en C₀₋₃)-O-cycloalkyle, -SO₂-(alkylène en C₀₋₃)cycloalkyle, -(alkylène en C₀₋₃)-SO₂-cycloalkyle, -CONH-cycloalkyle, -NHCO-cycloalkyle, -NH-cycloalkyle, -O-cycloalkyle, -CO-cycloalkyle, -SO₂-cycloalkyle, -(alkylène en C₀₋₃)-hétérocycloalkyle, -CO-(alkylène en C₀₋₃)-hétérocycloalkyle, -(alkylène en C₀₋₃)-CO-hétérocycloalkyle, -CONH-(alkylène en C₀₋₃)hétérocycloalkyle, -(alkylène en C₀₋₃)-CONH-hétérocycloalkyle, -NHCO-(alkylène en C₀₋₃)hétérocycloalkyle, -(alkylène en C₀₋₃)-NHCO-hétérocycloalkyle, -NH-(alkylène en C₀₋₃)hétérocycloalkyle, -(alkylène en C₀₋₃)-NH-hétérocycloalkyle, -O-(alkylène en C₀₋₃)hétérocycloalkyle, -(alkylène en C₀₋₃)-O-cycloalkyle, - SO₂-(C₀₋₃ alkylène)hétérocycloalkyle, -(C₀₋₃ alkylène)-SO₂-hétérocycloalkyle, -CONH-hétérocycloalkyle, -NHCO-hétérocycloalkyle, -NH-hétérocycloalkyle, -O-hétérocycloalkyle, - CO-hétérocycloalkyle, -SO₂-hétérocycloalkyle, (alkylène en C₀₋₃)aryle, -CO-(alkylène en C₀₋₃)aryle, -(alkylène en C₀₋₃)-CO-aryle, -CONH-(alkylène en C₀₋₃)aryle, -(alkylène en C₀₋₃)-CONH-aryle, -NHCO-(alkylène en C₀₋₃)aryle, -(alkylène en C₀₋₃)-NHCO-aryle, -NH-(alkylène en C₀₋₃)aryle, -(alkylène en C₀₋₃)-NH-aryle, -O-(alkylène en C₀₋₃)aryle, -(alkylène en C₀₋₃)-O-aryle, -SO₂-(alkylène en C₀₋₃)aryle, -(alkylène en C₀₋₃)-SO₂-aryle, -CONH-aryle, -NHCO-aryle, -NH-aryle, -O-aryle, -CO-aryle, -SO₂-aryle, -(alkylène en C₀₋₃)hétéroaryle, -CO-(alkylène en C₀₋₃)hétéroaryle, -(alkylène en C₀₋₃)-CO-hétéroaryle, -CONH-(alkylène en C₀₋₃)hétéroaryle, - (alkylène en C₀₋₃)-CONH-hétéroaryle, -NHCO-(alkylène en C₀₋₃)hétéroaryle, -(alkylène en C₀₋₃)-NHCO-hétéroaryle, -NH-(alkylène en C₀₋₃)hétéroaryle, (alkylène en C₀₋₃)-NH-hétéroaryle, - O-(alkylène en C₀₋₃)hétéroaryle, -(alkylène en C₀₋₃)-O-hétéroaryle, -SO₂-(alkylène en C₀₋₃)hétéroaryle, -(alkylène en C₀₋₃)-SO₂-hétéroaryle, -CONH-hétéroaryle, -NHCO-hétéroaryle, - NH-hétéroaryle, -O-hétéroaryle, -CO-hétéroaryle et -SO₂-hétéroaryle, dans lequel lesdits groupes alkyle, alcényle ou alcynyle sont éventuellement substitués par un ou plusieurs groupes choisis indépendamment parmi halogène, -CN, -OH, -O(alkyle en C₁₋₅), -O(haloalkyle en C₁₋₅), un haloalkyle en C₁₋₅, -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CONH₂, -CONH(alkyle en C₁₋₅), - CON(alkyle en C₁₋₅)(alkyle en C₁₋₅), -CO-(N-hétérocycloalkyle), -NHCO-(alkyle en C₁₋₅), - N(alkyle en C₁₋₅)-CO-(alkyle en C₁₋₅), -NHCONH₂, -NHCONH-(alkyle en C₁₋₅), - NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -S(O)(alkyle en C₁₋₅), - S(O)₂(alkyle en C₁₋₅), -S(O)(NH)(alkyle en C₁₋₅), -S(O)(N(alkyle en C₁₋₅))(alkyle en C₁₋₅), - N=S(O)(alkyle en C₁₋₅)(alkyle en C₁₋₅), -P(O)(alkyle en C₁₋₅)(alkyle en C₁₋₅), -P(O)(O(alkyle en C₁₋₅))(O(alkyle en C₁₋₅)), et -P(O)(O(alkyle en C₁₋₅))(alkyle en C₁₋₅), de préférence choisis parmi halogène, -CN, -OH, -O(alkyle en C₁₋₅), -O(haloalkyle en C₁₋₅), -haloalkyle en C₁₋₅, -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), - CONH₂, -CONH(alkyle en C₁₋₅) et -CON(alkyle en C₁₋₅)(alkyle en C₁₋₅) et dans lequel lesdits groupes cycloalkyle, cycloalcényle, hétérocycloalkyle, hétérocycloalcényle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -S(haloalkyle en C₁₋₅), -NH2, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CO(haloalkyle en C₁₋₅), -CO-cycloalkyle, - COO(alkyle en C₁₋₅), -COO(haloalkyle en C₁₋₅), -COO-cycloalkyle, -CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), -NHCONH₂, -NHCONH-(alkyle en C₁₋₅), - NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -OCONH₂, -OCONH-(alkyle en C₁₋₅), -OCON(alkyle en C₁₋₅)(alkyle en C₁₋₅), -NHCOO(alkyle en C₁₋₅), -N(alkyle en C₁₋₅)COO-(alkyle en C₁₋₅), -P(O)(alkyle en C₁₋₅)(alkyle en C₁₋₅), -P(O)(O(alkyle en C₁₋₅))(O(alkyle en C₁₋₅)), -P(O)(O(alkyl en C_{1- 5}))(alkyle en C₁₋₅), -(alkylène en C₁₋₅)-CN, - (alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-S(O)(alkyle en C₁₋₅), -(alkylène en C₁₋₅)-S(O)₂(alkyle en C₁₋₅), -(alkylène en C₁₋₅)-S(O)(NH)(alkyle en C₁₋₅), -(alkylène en C₁₋₅)-S(O)(N(alkyle en C₁₋₅))(alkyle en C₁₋₅), -(alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CO(haloalkyle en C₁₋₅), -(alkylène en C₁₋₅)-CO-cycloalkyle, -(alkylène en C₁₋₅)-CONH₂, -(alkylène en C₁₋₅)-CONH(alkyle en C₁₋₅), -(alkylène en C₁₋₅)-CON(alkyle en C₁₋₅)(alkyle en C₁₋₅), -(alkylène en C₁₋₅)-CO-(N-hétérocycloalkyle), -(alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅)CONH₂, -(alkylène en C₁₋₅)-N(alkyle en C₁₋₅)CONH-(alkyle en C₁₋₅), -(alkylène en C₁₋₅)-N(alkyle en C₁₋₅)CON(alkyle en C₁₋₅)(alkyle en C₁₋₅), -(alkylène en C₁₋₅)-OCONH₂, -(alkylène en C₁₋₅)-OCONH-(alkyle en C₁₋₅), -(alkylène en C₁₋₅)-OCON(alkyle en C₁₋₅)(alkyle en C₁₋₅), -(alkylène en C₁₋₅)-NHCOO(alkyle en C₁₋₅) et -(alkylène en C₁₋₅)-N(alkyle en C₁₋₅)COO-(alkyle en C₁₋₅),
ou
dans lequel -Y_{C2}-R_{C2} est choisi parmi -(alkylène en C₀₋₃)-hétérocycloalkyle, -CO-(alkylène en C₀₋₃)-hétérocycloalkyle, -(alkylène en C₀₋₃)-CO-hétérocycloalkyle, -CONH-(alkylène en C₀₋₃) hétérocycloalkyle, -(alkylène en C₀₋₃)-CONH-hétérocycloalkyle, -NHCO-(alkylène en C₀₋₃)hétérocycloalkyle, -(alkylène en C₀₋₃)-NHCO-hétérocycloalkyle, -NH-(alkylène en C₀₋₃)hétérocycloalkyle, -(alkylène en C₀₋₃)-NH-hétérocycloalkyle, -O-(alkylène en C₀₋₃)hétérocycloalkyle, (alkylène en C₀₋₃)-O-cycloalkyle, (alkylène en C₀₋₃)-O-hétérocycloalkyle, -SO₂-(alkylène en C₀₋₃)hétérocycloalkyle, -(alkylène en C₀₋₃)-SO₂-hétérocycloalkyle, -CONH-hétérocycloalkyle, -NHCO-hétérocycloalkyle, -NH-hétérocycloalkyle, -O-hétérocycloalkyle, - CO-hétérocycloalkyle, -SO₂-hétérocycloalkyle, -(alkylène en C₀₋₃)-hétérocycloalcényle, -CO-(alkylène en C₀₋₃)-hétérocycloalcényle, -(alkylène en C₀₋₃)-CO-hétérocycloalcényle, -CONH-(alkylène en C₀₋₃)hétérocycloalcényle, -(alkylène en C₀₋₃) -CONH-hétérocycloalcényle, - NHCO-(C₀₋₃ alkylène)hétérocycloalcényle, -(C₀₋₃ alkylène)-NHCO-hétérocycloalcényle, -NH-(C₀₋₃ alkylène)hétérocycloalcényle, -(C₀₋₃ alkylène)-NH-hétérocycloalcényle, -O-(alkylène en C₀₋₃)hétérocycloalcényle, (alkylène en C₀₋₃)-O-hétérocycloalcényle, -SO₂-(alkylène en C₀₋₃)hétérocycloalcényle, -(alkylène en C₀₋₃)-SO₂-hétérocycloalcényle, -CONH-hétérocycloalcényle, - NHCO-hétérocycloalcényle, -NH-hétérocycloalcényle, -O-hétérocycloalcényle, -CO-hétérocycloalcényle, -SO₂-hétérocycloalcényle, -(alkylène en C₀₋₃)aryle, -CO-(alkylène en C₀₋₃)aryle, -(alkylène en C₀₋₃)-CO-aryle, -CONH-(alkylène en C₀₋₃)aryle, -(alkylène en C₀₋₃)-CONH-aryle, -NHCO-(alkylène en C₀₋₃)aryle, -(alkylène en C₀₋₃)-NHCO-aryle, -NH-(alkylène en C₀₋₃)aryle, -(alkylène en C₀₋₃)-NH-aryle, -O-(alkylène en C₀₋₃)aryle, -(alkylène en C₀₋₃)-O-aryle, -SO₂-(alkylène en C₀₋₃)aryle, -(alkylène en C₀₋₃)-SO₂-aryle, -CONH-aryle, -NHCO-aryle, -NH-aryle, -O-aryle, -CO-aryle, -SO₂-aryle, -(alkylène en C₀₋₃)hétéroaryle, -CO-(alkylène en C₀₋₃)hétéroaryle, -(alkylène en C₀₋₃)-CO-hétéroaryle, -CONH-(alkylène en C₀₋₃)hétéroaryle, -(alkylène en C₀₋₃)-CONH-hétéroaryle, -NHCO-(alkylène en C₀₋₃)hétéroaryle, -(alkylène en C₀₋₃)-NHCO-hétéroaryle, -NH-(alkylène en C₀₋₃)hétéroaryle, - (alkylène en C₀₋₃)-NH-hétéroaryle, -O-(alkylène en C₀₋₃)hétéroaryle, -(alkylène en C₀₋₃)-O-hétéroaryle, -SO₂-(alkylène en C₀₋₃)hétéroaryle, -(alkylène en C₀₋₃)-SO₂-hétéroaryle, -CONH-hétéroaryle, -NHCO-hétéroaryle, -NH-hétéroaryle, -O-hétéroaryle, -CO-hétéroaryle et -SO₂-hétéroaryle, de préférence -Y_{C2}-R_{C2} est choisi parmi -(alkylène en C₀₋₃)-hétérocycloalkyle, - CO-(alkylène en C₀₋₃)hétérocycloalkyle, -(alkylène en C₀₋₃)-CO-hétérocycloalkyle, -CONH-(alkylène en C₀₋₃)hétérocycloalkyle, -(alkylène en C₀₋₃)-CONH-hétérocycloalkyle, -NHCO-(alkylène en C₀₋₃)hétérocycloalkyle, -(alkylène en C₀₋₃)-NHCO-hétérocycloalkyle, -NH-(alkylène en C₀₋₃) hétérocycloalkyle, -(alkylène en C₀₋₃)-NH-hétérocycloalkyle, -O-(alkylène en C₀₋₃)-hétérocycloalkyle, (alkylène en C₀₋₃)-O-cycloalkyle, -SO₂-(alkylène en C₀₋₃)hétérocycloalkyle, -(alkylène en C₀₋₃)-SO₂-hétérocycloalkyle, -CONH-hétérocycloalkyle, - NHCO-hétérocycloalkyle, -NH-hétérocycloalkyle, -O-hétérocycloalkyle, -CO-hétérocycloalkyle, -SO₂-hétérocycloalkyle, -(alkylène en C₀₋₃)aryle, -CO-(alkylène en C₀₋₃)aryle, -(alkylène en C₀₋₃)-CO-aryle, -CONH-(alkylène en C₀₋₃)aryle, -(alkylène en C₀₋₃)-CONH-aryle, -NHCO-(alkylène en C₀₋₃)aryle, -(alkylène en C₀₋₃)-NHCO-aryle, -NH-(alkylène en C₀₋₃)aryle, -(alkylène en C₀₋₃)-NH-aryle, -O-(alkylène en C₀₋₃)aryle, -(alkylène en C₀₋₃)-O-aryle, -SO₂-(alkylène en C₀₋₃)aryle, -(alkylène en C₀₋₃)-SO₂-aryle, -CONH-aryle, -NHCO-aryle, -NH-aryle, -O-aryle, -CO-aryle, -SO₂-aryle, -(alkylène en C₀₋₃)hétéroaryle, -CO-(alkylène en C₀₋₃)hétéroaryle, -(alkylène en C₀₋₃)-CO-hétéroaryle, -CONH-(alkylène en C₀₋₃)hétéroaryle, - (alkylène en C₀₋₃)-CONH-hétéroaryle, -NHCO-(alkylène en C₀₋₃)hétéroaryle, -(alkylène en C₀₋₃)-NHCO-hétéroaryle, -NH-(alkylène en C₀₋₃)hétéroaryle, -(alkylène en C₀₋₃)-NH-hétéroaryle, -O-(alkylène en C₀₋₃)hétéroaryle, -(alkylène en C₀₋₃)-O-hétéroaryle, -SO₂-(alkylène en C₀₋₃)hétéroaryle, -(alkylène en C₀₋₃)-SO₂-hétéroaryle, -CONH-hétéroaryle, -NHCO-hétéroaryle, - NH-hétéroaryle, -O-hétéroaryle, - CO-hétéroaryle et -SO₂-hétéroaryle, dans lesquels lesdits groupes hétérocycloalkyle, hétérocycloalcényle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CO(haloalkyle en C₁₋₅), -CO-cycloalkyle, -COO(alkyle en C₁₋₅), -COO(haloalkyle en C₁₋₅), -COO-cycloalkyle, - CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), - NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -OCONH₂, -OCONH-(alkyle en C₁₋₅), -OCON(alkyle en C₁₋₅)(alkyle en C₁₋₅), -NHCOO(alkyle en C₁₋₅), -N(alkyle en C₁₋₅)COO-(alkyle en C₁₋₅), -P(O)(alkyle en C₁₋₅)(alkyle en C₁₋₅), -P(O)(O)alkyle en C₁₋₅))(O(alkyle en C₁₋₅)), -P(O)(O(alkyle en C₁₋₅))(alkyle en C₁₋₅), -(alkylène en C₁₋₅)-CN, -(alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-S(O)(alkyle en C₁₋₅), - (alkylène en C₁₋₅)-S(O)₂(alkyle en C₁₋₅), -(alkylène en C₁₋₅)-S(O) (NH)(alkyle en C₁₋₅), - (alkylène en C₁₋₅)-S(O)(N(alkyle en C₁₋₅))(alkyle en C₁₋₅), -(alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)- (N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CO(haloalkyle en C₁₋₅), -(alkylène en C₁₋₅)-CO-cycloalkyle, -(alkylène en C₁₋₅)-CONH₂, -(alkylène en C₁₋₅)-CONH(alkyle en C₁₋₅), -(alkylène en C₁₋₅)-CON(alkyle en C₁₋₅)(alkyle en C₁₋₅), -(alkylène en C₁₋₅)-CO-(N-hétérocycloalkyle), - (alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅)CONH₂, -(alkylène en C₁₋₅)-N(alkyle en C₁₋₅)CONH-(alkyle en C₁₋₅), -(alkylène en C₁₋₅)-N(alkyle en C₁₋₅)CON(alkyle en C₁₋₅)(alkyle en C₁₋₅), -(alkylène en C₁₋₅)-OCONH₂, -(alkylène en C₁₋₅)-OCONH-(alkyle en C₁₋₅), -(alkylène en C₁₋₅)-OCON(alkyle en C₁₋₅)(alkyle en C₁₋₅), -( alkylène en C₁₋₅)-NHCOO(alkyle en C₁₋₅), et -(alkylène en C₁₋₅)-N(alkyle en C₁₋₅)COO-(alkyle en C₁₋₅), ou
dans lequel -Y_{C2}-R_{C2} est choisi parmi -(alkylène en C₀₋₃)-hétérocycloalkyle, -CONH-hétérocycloalkyle, - NHCO-hétérocycloalkyle, -NH-hétérocycloalkyle, -O-hétérocycloalkyle, - CO-hétérocycloalkyle, -SO₂- hétérocycloalkyle, -(alkylène en C₀₋₃)-hétérocycloalcényle, - CONH-hétérocycloalcényle, -NHCO- hétérocycloalcényle, -NH-hétérocycloalcényle, -O-hétérocycloalcényle, -CO-hétérocycloalcényle, -SO₂- hétérocycloalcényle, -(alkylène en C₀₋₃)aryle, -CONH-aryle, -NHCO-aryle, -NH-aryle, -O-aryle, -CO-aryle, -SO₂-aryl, -(alkylène en C₀₋₃)hétréoaryle, -CONH-hétéroaryle, -NHCO-hétéroaryle, -NH-hétéroaryle, - O-hétéroaryle, - CO-hétéroaryle et -SO₂-hétéroaryle, de préférence -YC₂-RC₂ est choisi parmi -(alkylène en C₀₋₃)-hétérocycloalkyle, -CONH-hétérocycloalkyle, -NHCO-hétérocycloalkyle, -NH-hétérocycloalkyle, -O-hétérocycloalkyle, -CO-hétérocycloalkyle, -SO₂-hétérocycloalkyle, - (alkylène en C₀₋₃)aryle, -CONH-aryle, -NHCO-aryle, -NH-aryle, -O-aryle, -CO-aryle, -SO₂-aryle, -(alkylène en C₀₋₃)hétéroaryle, -CONH-hétéroaryle, -NHCO-hétéroaryle, -NH-hétéroaryle, -O-hétéroaryle, -CO- hétéroaryle et -SO₂-hétéroaryle, dans lequel ledit hétérocycloalkyle, hétérocycloalcényle, aryle ou hétéroaryle est éventuellement substitué par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), - S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -S(haloalkyle en C₁₋₅), -NH₂, - NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CO(haloalkyle en C₁₋₅), -CO-cycloalkyl, -COO(alkyle en C₁₋₅), -COO(haloalkyle en C₁₋₅), -COO- cycloalkyle, - CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), - NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -OCONH₂, -OCONH-(alkyle en C₁₋₅), -OCON(alkyle en C₁₋₅)(alkyle en C₁₋₅), -NHCOO(alkyle en C₁₋₅), -N(alkyle en C₁₋₅)COO-(alkyle en C₁₋₅), -P(O)(alkyle en C₁₋₅)(alkyle en C₁₋₅), -P(O)(O(alkyle en C₁₋₅))(O(alkyle en C₁₋₅)), -P(O)(O(alkyle en C₁₋₅))(alkyle en C₁₋₅), -(alkylène en C₁₋₅)-CN, -(alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-S(O)(alkyle en C₁₋₅), - (alkylène en C₁₋₅)-S(O)₂(alkyle en C₁₋₅), -(alkylène en C₁₋₅ )-S(O)(NH)(alkyle en C₁₋₅), - (alkylène en C₁₋₅)-S(O)(N(alkyle en C₁₋₅))(alkyle en C₁₋₅), -(alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CO(haloalkyle en C₁₋₅), -(alkylpne en C₁₋₅)-CO-cycloalkyle, -(alkylène en C₁₋₅)-CONH₂, -(alkylène en C₁₋₅)-CONH(alkyle en C₁₋₅), -(alkylène C₁₋₅)-CON(alkyle en C₁₋₅)(alkyle en C₁₋₅), -(alkylène en C₁₋₅)-CO-(N-hétérocycloalkyle), - (alkylène en C₁₋₅)-NHCO-(alkyle en C₁₋₅), -(alkylène en C₁₋₅)-N(alkyle en C₁₋₅)-CO-(Alkyle en C₁₋₅), -(alkylène en C₁₋₅)-NHCONH₂, -(alkylène C₁₋₅)-NHCONH-(alkyle en C₁₋₅), -(alkyène en C₁₋₅)-NHCON(alkyle en C₁₋₅)(alkyle en C₁₋₅), -(alkylène en C₁₋₅)-N(alkyle en C₁₋₅)CONH₂, - (alkylène en C₁₋₅)-N(alkyle en C₁₋₅)CONH-(alkyle en C₁₋₅), -(alkylène en C₁₋₅)-N(alkyle en C₁₋₅)CON(alkyle en C₁₋₅)(alkyle en C₁₋₅), -(alkylène en C₁₋₅)-OCONH₂, -(alkylène en C₁₋₅)-OCONH-(alkyle en C₁₋₅), -(alkylène en C₁₋₅)-OCON(alkyle en C₁₋₅)(alkyle en C₁₋₅), -(alkylène en C₁₋₅)-NHCOO(alkyle en C₁₋₅), et -(alkylène en C₁₋₅)-N(alkyle en C₁₋₅)COO-(alkyle en C₁₋₅), ou
dans lequel -Y_{C2}-R_{C2} est choisi parmi les groupes -(alkylène en C₀₋₃)-hétérocycloalkyle, - (alkylène en C₀₋₃)-hétérocycloalcényle, -(alkylène en C₀₋₃)-aryle et -(alkylène en C₀₋₃)-hétéroaryle ; de préférence, -Y_{C2}-R_{C2} est choisi parmi les groupes - (alkylène en C₀₋₃)-hétérocycloalkyle, -(alkylène en C₀₋₃)-aryle et -(alkylène en C₀₋₃)-hétéroaryle, lesdits groupes hétérocycloalkyle, hétérocycloalcényle, aryle ou hétéroaryle étant éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), - S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -S(haloalkyle en C₁₋₅), -NH₂, - NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CO(haloalkyle en C₁₋₅), -CO-cycloalkyle, -COO(alkyle en C₁₋₅), -COO(haloalkyle en C₁₋₅), -COO-cycloalkyle, - CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), - NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -OCONH₂, -OCONH-(alkyle en C₁₋₅), -OCON(alkyle en C₁₋₅)(alkyle en C₁₋₅), -NHCOO(alkyle en C₁₋₅), -N(alkyle en C₁₋₅)COO-(alkyle en C₁₋₅), -P(O)(alkyle en C₁₋₅)(alkyle en C₁₋₅), -P(O)(O(alkyle en C₁₋₅))(O(alkyle en C₁₋₅)), -P(O)(O(alkyle en C₁₋₅))(alkyle en C₁₋₅), -(alkylène en C₁₋₅)-CN, -(alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-S(O)(alkyle en C₁₋₅), - (alkylène en C₁₋₅)-S(O)₂(alkyle en C₁₋₅), -(alkylène en C₁₋₅)-S(O)(NH)(alkyle en C₁₋₅), - (alkylène en C₁₋₅)-S(O)(N(alkyle en C₁₋₅))(alkyle en C₁₋₅), -(alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CO(haloalkyle en C₁₋₅), -(alkylène en C₁₋₅)-CO-cycloalkyle, -(alkylène en C₁₋₅)-CONH₂, -(alkylène en C₁₋₅)-CONH(alkyle en C₁₋₅), -(alkylène en C₁₋₅)-CON(alkyle en C₁₋₅)(alkyle en C₁₋₅), -(alkylène en C₁₋₅)-CO-(N-hétérocycloalkyle), - (alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅) CONH₂, -(alkylène en C₁₋₅)-N(alkyle en C₁₋₅)CONH-(alkyle en C₁₋₅), -(alkylène en C₁₋₅)-N(alkyle en C₁₋₅)CON(alkyle en C₁₋₅)(alkyle en C₁₋₅), -(alkylène en C₁₋₅)-OCONH₂, -(alkylène en C₁₋₅)-OCONH-(alkyle en C₁₋₅), -(alkylène en C₁₋₅)-OCON(alkyle en C₁₋₅)(alkyle en C₁₋₅), -(alkylène en C₁₋₅)-NHCOO(alkyle en C₁₋₅), et -(alkylène en C₁₋₅)-N(alkyle en C₁₋₅)COO-(alkyle en C₁₋₅).

4. Composé selon la revendication 1 ou 2,
dans lequel Y_{C2} est choisi parmi une liaison covalente, alkylène en C₁₋₅, alcénylène en C₂₋₅ et alcynylène en C₂₋₅, lesdits alkylène, alcénylène et alcynylène pouvant chacun être éventuellement substitués par un ou plusieurs groupes choisis indépendamment parmi halogène, -CN, -OH, -O(alkyle en C₁₋₅), -SH, -S(alkyle en C₁₋₅), -NH₂, -NH(alkyle en C₁₋₅) et - N(alkyle en C₁₋₅)(alkyle en C₁₋₅), et dans lequel, en outre, une ou plusieurs unités -CH₂-comprises dans lesdits alkylène, alcénylène ou alcynylène sont chacune éventuellement remplacées par un groupe choisi indépendamment parmi -O-, -NH-, -N(alkyle en C₁₋₅)-, -CO-, -S-, -SO- et -SO₂-, et dans lequel R_{C2} est choisi parmi l'hydrogène, un halogène, -OH, -NH₂, - SH, -CN, alkyle en C₁₋₁₂, alcényle en C₂₋₁₂, alcynyle en C₂₋₁₂, cycloalkyle, hétérocycloalkyle, aryle et hétéroaryle,
dans lequel lesdits groupes alkyle, alcényle ou alcynyle sont éventuellement substitués par un ou plusieurs groupes choisis indépendamment parmi halogène, -CN, -OH, -O(alkyle en C₁₋₅), - O(haloalkyle en C₁₋₅), un haloalkyle en C₁₋₅, -SH, -S(alkyle en C₁₋₅), -S(haloalkyle en C₁₋₅), - NH₂, -NH(alkyle en C₁₋₅), -NH(haloalkyle en C₁₋₅), -N(alkyle en C₁₋₅)(alkyle en C₁₋₅), - N(haloalkyle en C₁₋₅)(alkyle en C₁₋₅), -(N-hétérocycloalkyle), -CO(alkyle en C₁₋₅), -CONH₂, - CONH(alkyle en C₁₋₅), -CON(alkyle en C₁₋₅)(alkyle en C₁₋₅), -CO-(N-hétérocycloalkyle), - NHCO-(alkyle en C₁₋₅), -N(alkyle en C₁₋₅)-CO-(alkyle en C₁₋₅), -NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), - S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), -P(O)(alkyle en C₁₋₅)(alkyle en C₁₋₅), -P(O)(O(alkyle en C₁₋₅))(O(alkyle en C₁₋₅)), et -P(O)(O(alkyle en C₁₋₅))(alkyle en C₁₋₅), de préférence choisis parmi halogène, -CN, -OH, -O(alkyle en C₁₋₅), -O(haloalkyle en C₁₋₅), un haloalkyle en C₁₋₅, -SH, -S(alkyle en C₁₋₅), -S(haloalkyle en C₁₋₅), -NH₂, -NH(alkyle en C₁₋₅), - NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅), et
dans lequel lesdits groupes cycloalkyle, hétérocycloalkyle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(O)(alkyle en C₁₋₅), -S(O)₂(alkyle en C₁₋₅), -S(O)(NH)(alkyle en C₁₋₅), -S(O)(N(alkyle en C₁₋₅))(alkyle en C₁₋₅), -N=S(O)(alkyle en C₁₋₅)(alkyle en C₁₋₅), - S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), - NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅), -N(alkyle en C₁₋₅)CON(alkyle en C₁₋₅)(alkyle en C₁₋₅), -P(O)(alkyle en C₁₋₅)(alkyle en C₁₋₅), -P(O)(O(alkyle en C₁₋₅))(O(alkyle en C₁₋₅)), -P(O)(O(alkyle en C₁₋₅))(alkyle en C₁₋₅), -(alkylène en C₁₋₅)-CN, -(alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), - (alkylène en C₁₋₅)-S(O)(alkyle en C₁₋₅), -(alkylène en C₁₋₅)-S(O)₂(alkyle en C₁₋₅), -(alkylène en C₁₋₅)-S(O)(NH)(alkyle en C₁₋₅), -(alkylène en C₁₋₅)-S(O)(N(alkyle en C₁₋₅))(alkyle en C₁₋₅), - (alkylène en C₁₋₅)-P(O)(alkyle en C₁₋₅)(alkyle en C₁₋₅), -(alkylène en C₁₋₅)-P(O)(O(alkyle en C₁₋₅))(O(alkyle en C₁₋₅)), -(alkylène en C₁₋₅)-P(O)(O(alkyle en C₁₋₅))(alkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CONH₂, -(alkylène en C₁₋₅)-CONH(alkyle en C₁₋₅), -(alkylène en C₁₋₅)-CON(alkyle en C₁₋₅)(alkyle en C₁₋₅), -(alkylène en C₁₋₅)-CO-(N-hétérocycloalkyle), -(alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅)CONH₂, -(alkylène en C₁₋₅)-N(alkyle en C₁₋₅) CONH-(alkyle en C₁₋₅), et -(alkylène en C₁₋₅)-N(alkyle en C₁₋₅)CON(alkyle en C₁₋₅)(alkyle en C₁₋₅), de préférence choisis parmi halogène, -CN, -OH, alkyle en C₁₋₅, un haloalkyle en C₁₋₅, -O(alkyle en C₁₋₅), -O(haloalkyle en C₁₋₅), -(alkylène en C₁₋₅)-OH, -(alkylène en C₁₋₅)-O(alkyle en C₁₋₅), -(alkylène en C₁₋₅)-O(haloalkyle en C₁₋₅), -SH, -S(alkyle en C₁₋₅), - S(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(alkylène en C₁₋₅)-NH₂, - (alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, - NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH_{2,} -CONH(alkyle en C₁₋₅) et -CON(alkyle en C₁₋₅)(alkyle en C₁₋₅), de préférence où -Y_{C2}-R_{C2} est choisi parmi -O-alkyle en C₁₋₁₂, -NH-alkyle en C₁₋₁₂, -N(alkyle en C₁₋₅)-alkyle en C₁₋₁₂, -O-alcényle en C₂₋₁₂, -NH-alcényle en C₂₋₁₂, -N(alkyle en C₁₋₅)-alcényle en C₂₋₁₂, -O-alkynyle en C₂₋₁₂, -NH-alkynyle en C₂₋₁₂, -N(alkyle en C₁₋₅)-alkynyle en C₂₋₁₂, -(alkylène en C₀₋₃)-cycloalkyle, -CO-(alkylène en C₀₋₃)-cycloalkyle, - (alkylène en C₀₋₃)-CO-cycloalkyle, -CONH-(alkylène en C₀₋₃)-cycloalkyle, (alkylène en C₀₋₃)-CONH-cycloalkyle, -NHCO-(alkylène en C₀₋₃)-cycloalkyle, -(alkylène en C₀₋₃)-NHCO-cycloalkyle, -NH-(alkylène en C₀₋₃)-cycloalkyle, -(alkylène en C₀₋₃)-NH-cycloalkyle, -O-(alkylène en C₀₋₃)-cycloalkyle, -(alkylène en C₀₋₃)-O-cycloalkyle, -SO₂-(alkylène en C₀₋₃)-cycloalkyle, -(alkylène en C₀₋₃)-SO₂-cycloalkyle, -CONH-cycloalkyle, -NHCO-cycloalkyle, - NH-cycloalkyle, -O-cycloalkyle, -CO-cycloalkyle, -SO₂-cycloalkyle, -(alkylène en C₀₋₃)-hétérocycloalkyle, -CO-(alkylène en C₀₋₃)-hétérocycloalkyle, -(alkylène en C₀₋₃)-CO-hétérocycloalkyle, -CONH-(alkylène en C₀₋₃)-hétérocycloalkyle, -(alkylène en C₀₋₃)-CONH-hétérocycloalkyle, -NHCO-(alkylène en C₀₋₃)-hétérocycloalkyle, -(alkylène en C₀₋₃)-NHCO-hétérocycloalkyle, -NH-(C₀₋₃ alkylène)-hétérocycloalkyle, -(C₀₋₃ alkylène)-NH-hétérocycloalkyle, -O-(C₀₋₃ alkylène)-hétérocycloalkyle, -(C₀₋₃ alkylène)-O-cycloalkyle, -SO₂-(C₀₋₃ alkylène)-hétérocycloalkyle, -(C₀₋₃ alkylène)-SO₂-hétérocycloalkyle, -CONH-hétérocycloalkyle, -NHCO-hétérocycloalkyle, -NH-hétérocycloalkyle, -O-hétérocycloalkyle, - CO-hétérocycloalkyle, -SO₂-hétérocycloalkyle, -(alkylène en C₀₋₃)-aryle, -CO-(alkylène en C₀₋₃)-aryle, -(alkylène en C₀₋₃)-CO-aryle, -CONH-(alkylène en C₀₋₃)-aryle, -(alkylène en C₀₋₃)-CONH-aryle, -NHCO-(alkylène en C₀₋₃)-aryle, -(alkylène en C₀₋₃) -NHCO-aryle, -NH-(alkylène en C₀₋₃)-aryle, -(alkylène en C₀₋₃)-NH-aryle, -O-(alkylène en C₀₋₃)-aryle, -(alkylène en C₀₋₃)-O-aryle, -SO₂-(alkylène en C₀₋₃)-aryle, -(alkylène en C₀₋₃)-SO₂-aryle, -CONH-aryle, - NHCO-aryle, -NH-aryle, -O-aryle, -CO-aryle, -SO₂-aryle, -(alkylène en C₀₋₃)-hétéroaryle, -CO-(alkylène en C₀₋₃)-hétéroaryle, -(alkylène en C₀₋₃)-CO-hétéroaryle, -CONH-(alkylène en C₀₋₃)-hétéroaryle, -(alkylène en C₀₋₃)-CONH-hétéroaryle, -NHCO-(alkylène en C₀₋₃)-hétéroaryle, - (alkylène en C₀₋₃)-NHCO-hétéroaryle, -NH-(alkylène en C₀₋₃)-hétéroaryle, -(alkylène en C₀₋₃)-NH-hétéroaryle, -O-(alkylène en C₀₋₃)-hétéroaryle, -(alkylène en C₀₋₃)-O-hétéroaryle, -SO2-(alkylène en C₀₋₃)-hétéroaryle, -(alkylène en C₀₋₃)-SO2-hétéroaryle, -CONH-hétéroaryle, - NHCO-hétéroaryle, -NH-hétéroaryle, -O-hétéroaryle, -CO-hétéroaryle et -SO₂-hétéroaryle, dans lequel lesdits groupes alkyle, alcényle ou alcynyle sont éventuellement substitués par un ou plusieurs groupes choisis indépendamment parmi halogène, -CN, -OH, -O(alkyle en C₁₋₅), - O(haloalkyle en C₁₋₅), -haloalkyle en C₁₋₅, -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅) et dans lequel lesdits groupes cycloalkyle, hétérocycloalkyle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅),
de préférence, dans lequel -Y_{C2}-R_{C2} est choisi parmi les groupes -(alkylène en C₀₋₃)-hétérocycloalkyle, -CO-(alkylène en C₀₋₃)-hétérocycloalkyle, -(alkylène en C₀₋₃)-CO-hétérocycloalkyle, -CONH-(alkylène en C₀₋₃)-hétérocycloalkyle, -(alkylène en C₀₋₃)-CONH-hétérocycloalkyle, -NHCO-(alkylène en C₀₋₃)-hétérocycloalkyle, -(alkylène en C₀₋₃)-NHCO-hétérocycloalkyle, -NH-(C₀₋₃ alkylène)-hétérocycloalkyle, -(C₀₋₃ alkylène)-NH-hétérocycloalkyle, -O-(C₀₋₃ alkylène)-hétérocycloalkyle, -(C₀₋₃ alkylène)-O-cycloalkyle, -SO₂-(C₀₋₃ alkylène)-hétérocycloalkyle, -(C₀₋₃ alkylène)-SO₂-hétérocycloalkyle, -CONH-hétérocycloalkyle, -NHCO-hétérocycloalkyle, -NH-hétérocycloalkyle, -O-hétérocycloalkyle, - CO-hétérocycloalkyle, -SO₂-hétérocycloalkyle, -(alkylène en C₀₋₃)-aryle, -CO-(alkylène en C₀₋₃)-aryle, -(alkylène en C₀₋₃)-CO-aryle, -CONH-(alkylène en C₀₋₃)-aryle, -(alkylène en C₀₋₃)-CONH-aryle, -NHCO-(alkylène en C₀₋₃)-aryle, -(alkylène en C₀₋₃) -NHCO-aryle, -NH-(C₀₋₃ alkylène)-aryle, -(C₀₋₃ alkylène)-NH-aryle, -O-(C₀₋₃ alkylène)-aryle, -(C₀₋₃ alkylène)-O-aryle, - SO₂-(alkylène en C₀₋₃)-aryle, -(alkylène en C₀₋₃)-SO₂-aryle, -CONH-aryle, -NHCO-aryle, -NH-aryle, -O-aryle, -CO-aryle, -SO₂-aryle, -(alkylène en C₀₋₃)-hétéroaryle, -CO-(alkylène en C₀₋₃)-hétéroaryle, -(alkylène en C₀₋₃)-CO-hétéroaryle, -CONH-(alkylène en C₀₋₃)-hétéroaryle, - (alkylène en C₀₋₃)-CONH-hétéroaryle, -NHCO-(alkylène en C₀₋₃)-hétéroaryle, -(alkylène en C₀₋₃)-NHCO-hétéroaryle, -NH-(alkylène en C₀₋₃)-hétéroaryle, -(alkylène en C₀₋₃)-NH-hétéroaryle, -O-(alkylène en C₀₋₃)-hétéroaryle, -(alkylène en C₀₋₃) -O-hétéroaryle, -SO₂-(alkylène en C₀₋₃)-hétéroaryle, -(alkylène en C₀₋₃)-SO₂-hétéroaryle, -CONH-hétéroaryle, -NHCO-hétéroaryle, - NH-hétéroaryle, -O-hétéroaryle, -CO-hétéroaryle et -SO₂-hétéroaryle, dans lequel lesdits groupes hétérocycloalkyle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -NH₂, - NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅),
de préférence encore, dans lequel -Y_{C2}-R_{C2} est choisi parmi les groupes -(alkylène en C₀₋₃)-hétérocycloalkyle, -CONH-hétérocycloalkyle, -NHCO-hétérocycloalkyle, -NH-hétérocycloalkyle, -O-hétérocycloalkyle, -CO-hétérocycloalkyle, -SO₂-hétérocycloalkyle, - (alkylène en C₀₋₃)aryle, -CONH-aryle, -NHCO-aryle, -NH-aryle, -O-aryle, -CO-aryle, -SO₂-aryle, -(alkylène en C₀₋₃)-hétéroaryle, -CONH-hétéroaryle, -NHCO-hétéroaryle, -NH-hétéroaryle, -O-hétéroaryle, -CO-hétéroaryle et -SO₂-hétéroaryle, dans lequel lesdits groupes hétérocycloalkyle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -NH₂, -NH(alkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅), de préférence encore lorsque -Y_{C2}-R_{C2} est choisi parmi -(alkylène en C₀₋₃)-hétérocycloalkyle, -(alkylène en C₀₋₃)-aryle et -(alkylène en C₀₋₃)-hétéroaryle, lesdits groupes hétérocycloalkyle, aryle ou hétéroaryle étant éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅),
de préférence encore, dans lequel -Y_{C2}-R_{C2} est choisi parmi les groupes hétérocycloalkyle, aryle et hétéroaryle, de préférence hétérocycloalkyle et hétéroaryle, et plus préférentiellement hétérocycloalkyle, lesdits groupes hétérocycloalkyle, aryle ou hétéroaryle étant éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(haloalkyle en C₁₋₅), -S(alkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, - CONH(alkyle en C₁₋₅) et -CON(alkyle en C₁₋₅)(alkyle en C₁₋₅).

5. Composé selon les revendications 3 ou 4, dans lequel -Y_{C2}-R_{C2} est un groupe aryle éventuellement substitué, de préférence -Y_{C2}-R_{C2} est un groupe phényle, éventuellement substitué par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, - CONH(alkyle en C₁₋₅) et -CON(alkyle en C₁₋₅)(alkyle en C₁₋₅), ou dans lequel -Y_{C2}-R_{C2} est un groupe hétéroaryle éventuellement substitué, de préférence dans lequel -Y_{C2}-R_{C2} est un groupe imidazolyle, pyridazinyle, thiazolyle, pyridinyle, pyrimidinyle, pyrazinyle ou indazolyle, le groupe hétéroaryle étant éventuellement substitué par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅),
ou dans lequel -Y_{C2}-R_{C2} est un groupe hétérocycloalkyle éventuellement substitué, de préférence dans lequel - Y_{C2}-R_{C2} est un groupe morpholinyle, 1,1-dioxothiomorpholinyle, azétinyle, pyrrolidinyle, pipéridinyle, 6-oxo-1,6-dihydropyridinyle ou pipérazinyle, le groupe hétérocycloalkyle étant éventuellement substitué par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, -CONH(alkyle en C₁₋₅) et -CON(alkyle en C₁₋₅)(alkyle en C₁₋₅),
de préférence dans lequel -Y_{C2}-R_{C2} est un groupe pipérazinyle, éventuellement substitué par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), - S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CO(alkyle en C₁₋₅), -CONH₂, - CONH(alkyle en C₁₋₅) et -CON(alkyle en C₁₋₅)(alkyle en C₁₋₅), de préférence dans lequel -Y_{C2}-R_{C2} est un groupe pipérazinyle (de préférence N-pipérazinyle) éventuellement substitué (de préférence N-substitué) par CO(alkyle en C₁₋₅), -CONH₂, -CONH(alkyle en C₁₋₅) et - CON(alkyle en C₁₋₅)(alkyle en C₁₋₅), de préférence -Y_{C2}-R_{C2} étant un groupe pipérazinyle (de préférence N-pipérazinyle) substitué (de préférence N-substitué, de préférence sur un atome d'azote différent de celui lié au système cyclique tel qu'illustré dans la formule (I)), par - CON(alkyle en C₁₋₅)(alkyle en C₁₋₅), de préférence par -CON(CH3)2.

6. Composé selon l'une quelconque des revendications 1 à 5, dans lequel X₄ représente C-R_{C4}, où R_{C4} est choisi parmi hydrogène, halo, alkyle en C₁₋₆, alcynyle en C2-6, -O(alkyle en C₁₋₆), - S(alkyle en C₁₋₆), -NH(alkyle en C₁₋₆), -N(alkyle en C₁₋₆)(alkyle en C₁₋₆), -CO(alkyle en C₁₋₆), haloalkyle en C₁₋₆, -O(haloalkyle en C₁₋₆), -S(haloalkyle en C₁₋₆), -NH(haloalkyle en C₁₋₆), - N(haloalkyle en C₁₋₆)₂, -CO(haloalkyle en C₁₋₆), -(alkylène en C₀₋₃)cycloalkyle, -O-(alkylène en C₀₋₃)-cycloalkyle, -CO-(alkylène en C₀₋₃)-cycloalkyle, -(alkylène en C₀₋₃)-hétérocycloalkyle, -O-(alkylène en C₀₋₃)-hétérocycloalkyle, -CO-(alkylène en C₀₋₃)-hétérocycloalkyle, -(alkylène en C₀₋₃)-aryle, -O-(alkylène en C₀₋₃)-aryle, -CO-(alkylène en C₀₋₃)-aryle, -(alkylène en C₀₋₃)-hétéroaryle, -O-(alkylène en C₀₋₃)-hétéroaryle et -CO-(alkylène en C₀₋₃)-hétéroaryle ;
dans lequel lesdits groupes alkyle ou alcynyle sont éventuellement substitués par un ou plusieurs groupes choisis indépendamment parmi un halogène, -CN, -OH, -O(alkyle en C₁₋₅), - O(haloalkyle en C₁₋₅), un haloalkyle en C₁₋₅, -SH, -S(alkyle en C₁₋₅), -S(haloalkyle en C₁₋₅), - NH₂, -NH(alkyle en C₁₋₅), -NH(haloalkyle en C₁₋₅), -N(alkyle en C₁₋₅)(alkyle en C₁₋₅), - N(haloalkyle en C₁₋₅)(alkyle en C₁₋₅), -(N-hétérocycloalkyle), -CO(alkyle en C₁₋₅), -CONH₂, - CONH(alkyle en C₁₋₅), -CON(alkyle en C₁₋₅)(alkyle en C₁₋₅), -CO-(N-hétérocycloalkyle), - NHCO-(alkyle en C₁₋₅), -N(alkyle en C₁₋₅)-CO-(alkyle en C₁₋₅), -NHCONH₂, -NHCONH-(alkyle en C₁₋₅), -NHCON(alkyle en C₁₋₅)(alkyle en C₁₋₅), -N(alkyle en C₁₋₅)CONH₂, -N(alkyle en C₁₋₅)CONH-(alkyle en C₁₋₅) et -N(alkyle en C₁₋₅)CON(alkyle en C₁₋₅)(alkyle en C₁₋₅), de préférence choisis parmi halogène, -CN, -OH, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, - CONH(alkyle en C₁₋₅) et -CON(alkyle en C₁₋₅)(alkyle en C₁₋₅), et
dans lequel lesdits groupes cycloalkyle, cycloalcényle, hétérocycloalkyle, hétérocycloalcényle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -(N-hétérocycloalkyle), -CO(alkyle en C₁₋₅), -CONH₂, -CONH(alkyle en C₁₋₅), -CON(alkyle en C₁₋₅)(alkyle en C₁₋₅), -CO-(N-hétérocycloalkyle), -NHCO-(alkyle en C₁₋₅), -N(alkyle en C₁₋₅)-CO-(alkyle en C₁₋₅), -NHCONH₂, -NHCONH-(alkyle en C₁₋₅), - NHCON(alkyle en C₁₋₅)(alkyle en C₁₋₅), -N(alkyle en C₁₋₅)CONH₂, -N(alkyle en C₁₋₅)CONH-(alkyle en C₁₋₅) et -N(alkyle en C₁₋₅)CON(alkyle en C₁₋₅)(alkyle en C₁₋₅), -(alkylène en C₁₋₅)-CN, -(alkylène en C₁₋₅)-OH, -(alkylène en C₁₋₅)-O(alkyle en C₁₋₅), -(alkylène en C₁₋₅)-O(haloalkyle en C₁₋₅), -(alkylène en C₁₋₅)-SH, -(alkylène en C₁₋₅)-S(alkyle en C₁₋₅), -(alkylène en C₁₋₅)-S(haloalkyle en C₁₋₅), -(alkylène en C₁₋₅)-NH₂, -(alkylène en C₁₋₅)-NH(alkyle en C₁₋₅), -(alkylène en C₁₋₅)-NH(haloalkyle en C₁₋₅), -(alkylène en C₁₋₅)-N(alkyle en C₁₋₅)(alkyle en C₁₋₅), -(alkylène en C₁₋₅)-N(alkyle en C₁₋₅)(haloalkyle en C₁₋₅), -(alkylène en C₁₋₅)-(N-hétérocycloalkyle), -(alkylène en C₁₋₅)-N(haloalkyle en C₁₋₅)(alkyle en C₁₋₅), -(alkylène en C₁₋₅)-CO(alkyle en C₁₋₅), -(alkylène en C₁₋₅)-CONH₂, -(alkylène en C₁₋₅)-CONH(alkyle en C₁₋₅), - (alkylène en C₁₋₅)-CON(alkyle en C₁₋₅)(alkyle en C₁₋₅), - (alkylène en C₁₋₅)-CO-(N-hétérocycloalkyle), -(alkylène en C₁₋₅)-NHCO-(alkyle en C₁₋₅), -(alkylène en C₁₋₅)-N(alkyle en C₁₋₅)-CO-(alkyle en C₁₋₅), -(alkylène en C₁₋₅)-NHCONH₂, -(alkylène en C₁₋₅)-NHCONH-(alkyle en C₁₋₅), -(alkylène en C₁₋₅)-NHCON(alkyle en C₁₋₅)(alkyle en C₁₋₅), -(alkylène en C₁₋₅)-N(alkyle en C₁₋₅) CON₂₂, -(alkylène en C₁₋₅)-N(alkyle en C₁₋₅)CONH-(alkyle en C₁₋₅) et - (alkylène en C₁₋₅)-N(alkyle en C₁₋₅)CON(alkyle en C₁₋₅)(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), - NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅),
ou
dans lequel X4 est un groupe C-R_{C4}, où R_{C4} est choisi parmi hydrogène, halo, alkyle en C₁₋₆, alkynyle en C₂₋₆,-O-alkyle en C₁₋₆, -S-alkyle en C₁₋₆, -NH-alkyle en C₁₋₆, haloalkyle en C₁₋₆, - (alkylène en C₀₋₃)-cycloalkyle, -(alkylène en C₀₋₃)-hétérocycloalkyle, -(alkylène en C₀₋₃)-aryle et -(alkylène en C₀₋₃)-hétéroaryle, ledit groupe alkyle étant éventuellement substitué par un ou plusieurs groupes choisis indépendamment parmi halogène, -CN, -OH, -O(alkyle en C₁₋₅), - O(haloalkyle en C₁₋₅), -SH, -S(alkyle en C₁₋₅), -O(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅), et dans lequel lesdits groupes cycloalkyle, hétérocycloalkyle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(haloalkyle en C₁₋₅), -S(alkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅), de préférence où R_{C4} est choisi parmi hydrogène, halo, alkyle en C₁₋₆, alkynyle en C2-5, -O-alkyle en C₁₋₆, -S-alkyle en C₁₋₆, -NH-alkyle en C₁₋₆ et haloalkyle en C₁₋₆,
de préférence, dans lequel R_{C4} est choisi parmi hydrogène, halo, alkyle en C₁₋₂ et alkynyle en C₂₋₃ ; de manière encore plus préférable, dans lequel R_{C4} est choisi parmi hydrogène, halo et alkyle en C₁₋₂ ; et de manière encore plus préférable, dans lequel R_{C4} est hydrogène ou halo.

7. Composé selon l'une quelconque des revendications 1 à 6, dans lequel X₅ est C-R_{C5}, où R_{C5} est choisi parmi hydrogène, halo, alkyle en C₁₋₃, -O-alkyle en C₁₋₃, -S-alkyle en C₁₋₃, -NH-alkyle en C₁₋₃ et haloalkyle en C₁₋₃, de préférence, dans lequel R_{C5} est choisi parmi hydrogène, halo, alkyle en C₁₋₃ et haloalkyle en C₁₋₃.

8. Composé selon l'une quelconque des revendications 1 à 7, dans lequel R_{R5} est choisi parmi alkyle en C₁₋₁₂, alcényle en C₂₋₁₂, alkynyle en C₂₋₁₂, cycloalkyle, hétérocycloalkyle, aryle et hétéroaryle,
dans lequel lesdits groupes alkyle, alcényle ou alkynyle sont éventuellement substitués par un ou plusieurs groupes choisis indépendamment parmi halogène, -CN, -OH, -O(alkyle en C₁₋₅), - O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅), et dans lequel lesdits groupes cycloalkyle, hétérocycloalkyle, aryle ou hétéroaryle sont éventuellement substitués par un ou plusieurs groupes choisis indépendamment parmi halogène, -CN, -OH, - alkyle en C₁₋₅, -haloalkyle en C₁₋₅, -O(alkyle en C₁₋₅), -O(haloalkyle en C₁₋₅), -SH, -S(alkyle en C₁₋₅), -SO(alkyle en C₁₋₅), -SO2(alkyle en C₁₋₅), -S(haloalkyle en C₁₋₅), -SO(haloalkyle en C₁₋₅), -SO₂(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅), de préférence choisis parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), - S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et - CON(alkyle en C₁₋₅)(alkyle en C₁₋₅).

9. Composé selon l'une quelconque des revendications 1 à 7, dans lequel R₄ est choisi parmi les groupes -(alkylène en C₀₋₂)-cycloalkyle, -CO-(alkylène en C₀₋₂)-cycloalkyle, -(alkylène en C₀₋₂)-CO-cycloalkyle, -CONH-(alkylène en C₀₋₂)-cycloalkyle, -(alkylène en C₀₋₂)-CONH-cycloalkyle, -NHCO-(alkylène en C₀₋₂)-cycloalkyle, -(alkylène en C₀₋₂)-NHCO-cycloalkyle, - NH-(alkylène en C₀₋₂)-cycloalkyle, -(alkylène en C₀₋₂)-NH-cycloalkyle, -O-(alkylène en C₀₋₂)-cycloalkyle, -(alkylène en C₀₋₂)-O-cycloalkyle, -SO₂-(alkylène en C₀₋₂)-cycloalkyle, -(alkylène en C₀₋₂)-SO₂-cycloalkyle, -CONH-cycloalkyle, -NHCO-cycloalkyle, -NH-cycloalkyle, -O-cycloalkyle, -CO-cycloalkyle, -SO₂-cycloalkyle, -(alkylène en C₀₋₂)-cycloalcényle, -CO-(alkylène en C₀₋₂)-cycloalcényle, -(alkylène en C₀₋₂)-CO-cycloalcényle, -CONH-(alkylène en C₀₋₂)-cycloalcényle, -(alkylène en C₀₋₂)-CONH-cycloalcényle, -NHCO-(alkylène en C₀₋₂)-cycloalcényle, -(alkylène en C₀₋₂)-NHCO-cycloalcényle, -NH-(alkylène en C₀₋₂)-cycloalcényle, -(alkylène en C₀₋₂)-NH-cycloalcényle, -O-(alkylène en C₀₋₂)-cycloalcényle, -(alkylène en C₀₋₂)-O-cycloalcényle, -SO₂-(C₀₋₂ alkylène)-cycloalcényle, -(C₀₋₂ alkylène)-SO₂-cycloalcényle, - CONH-cycloalcényle, -NHCO-cycloalcényle, -NH-cycloalcényle, -O-cycloalcényle, -CO-cycloalcényle, -SO₂-cycloalcényle, -(alkylène en C₀₋₂)-hétérocycloalkyle, -CO-(alkylène en C₀₋₂)-hétérocycloalkyle, -(alkylène en C₀₋₂)-CO-hétérocycloalkyle, -CONH-(alkylène en C₀₋₂)-hétérocycloalkyle, -(alkylène en C₀₋₂)-CONH-hétérocycloalkyle, -NHCO-(alkylène en C₀₋₂)-hétérocycloalkyle, -(alkylène en C₀₋₂)-NHCO-hétérocycloalkyle, -NH-(alkylène en C₀₋₂)-hétérocycloalkyle, -(alkylène en C₀₋₂)-NH-hétérocycloalkyle, -O-(alkylène en C₀₋₂)-hétérocycloalkyle, -(alkylène en C₀₋₂)-O-hétérocycloalkyle, -SO₂-(alkylène en C₀₋₂)-hétérocycloalkyle, -(alkylène en C₀₋₂)-SO₂-hétérocycloalkyle, -CONH-hétérocycloalkyle, - NHCO-hétérocycloalkyle, -NH-hétérocycloalkyle, -O-hétérocycloalkyle, -CO-hétérocycloalkyle, -SO₂-hétérocycloalkyle, -(alkylène en C₀₋₂)-hétérocycloalcényle, -CO-(alkylène en C₀₋₂)-hétérocycloalcényle, -(alkylène en C₀₋₂)-CO-hétérocycloalcényle, -CONH-(alkylène en C₀₋₂)-hétérocycloalcényle, -(alkylène en C₀₋₂)-CONH-hétérocycloalcényle, - NHCO-(alkylène en C₀₋₂)-hétérocycloalcényle, -(alkylène en C₀₋₂)-NHCO-hétérocycloalcényle, -NH-(alkylène en C₀₋₂)-hétérocycloalcényle, -(alkylène en C₀₋₂)-NH-hétérocycloalcényle, -O-(alkylène en C₀₋₂)-hétérocycloalcényle, -(alkylène en C₀₋₂)-O-hétérocycloalcényle, -SO₂-(alkylène en C₀₋₂)-hétérocycloalcényle, -(alkylène en C₀₋₂)-SO₂-hétérocycloalcényle, -CONH-hétérocycloalcényle, -NHCO-hétérocycloalcényle, -NH-hétérocycloalcényle, -O-hétérocycloalcényle, -CO-hétérocycloalcényle, -SO₂-hétérocycloalcényle, -(alkylène en C₀₋₂)-aryle, -CO-(alkylène en C₀₋₂)-aryle, -(alkylène en C₀₋₂)-CO-aryle, -CONH-(alkylène en C₀₋₂)-aryle, -(alkylène en C₀₋₂)-CONH-aryle, -NHCO-(alkylène en C₀₋₂)-aryle, -(alkylène en C₀₋₂)-NHCO-aryle, -NH-(alkylène en C₀₋₂)-aryle, - (alkylène en C₀₋₂)-NH-aryle, -O-(alkylène en C₀₋₂)-aryle, -(alkylène en C₀₋₂)-O-aryle, -SO₂-(alkylène en C₀₋₂)-aryle, -(alkylène en C₀₋₂)-SO₂-aryle, -CONH-aryle, -NHCO-aryle, -NH-aryle, -O-aryle, -CO-aryle, -SO₂-aryle, -(alkylène en C₀₋₂)-hétéroaryle, -CO-(alkylène en C₀₋₂)-hétéroaryle, -(alkylène en C₀₋₂)-CO-hétéroaryle, -CONH-(alkylène en C₀₋₂)-hétéroaryle, - (alkylène en C₀₋₂)-CONH-hétéroaryle, -NHCO-(alkylène en C₀₋₂)-hétéroaryle, -(alkylène en C₀₋₂)-NHCO-hétéroaryle, -NH-(alkylène en C₀₋₂)-hétéroaryle, -(alkylène en C₀₋₂)-NH-hétéroaryle, -O-(alkylène en C₀₋₂)-hétéroaryle, -(alkylène en C₀₋₂)-O-hétéroaryle, -SO₂-(alkylène en C₀₋₂)-hétéroaryle, -(alkylène en C₀₋₂)-SO₂-hétéroaryle, -CONH-hétéroaryle, -NHCO-hétéroaryle, - NH-hétéroaryle, -O-hétéroaryle, -CO-hétéroaryle et -SO₂-hétéroaryle,
dans lequel lesdits groupes cycloalkyle, cycloalcényle, hétérocycloalkyle, hétérocycloalcényle, aryle ou hétéroaryle sont éventuellement substitués par un ou plusieurs groupes choisis indépendamment parmi halogène, -CN, -OH, -alkyle en C₁₋₅, -haloalkyle en C₁₋₅, -O(alkyle en C₁₋₅), -O(haloalkyle en C₁₋₅), -SH, -S(alkyle en C₁₋₅), -SO(alkyle en C₁₋₅), -SO₂(alkyle en C₁₋₅), -S(haloalkyle en C₁₋₅), -SO(haloalkyle en C₁₋₅), -SO₂(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et -CON(alkyle en C₁₋₅)(alkyle en C₁₋₅), de préférence choisis parmi un atome d'halogène, -CN, -OH, un groupe alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), - N(haloalkyle en C₁₋₅)(alkyle en C₁₋₅), -CONH₂, -CONH(alkyle en C₁₋₅) et -CON(alkyle en C₁₋₅)(alkyle en C₁₋₅).

10. Composé selon l'une quelconque des revendications 1 à 9, dans lequel R₄ est choisi parmi les groupes -(alkylène en C₀₋₂)-cycloalkyle, -CO-(alkylène en C₀₋₂)-cycloalkyle, -(alkylène en C₀₋₂)-CO-cycloalkyle, -CONH-(alkylène en C₀₋₂)-cycloalkyle, -(alkylène en C₀₋₂)-CONH-cycloalkyle, -NHCO-(alkylène en C₀₋₂)-cycloalkyle, -(alkylène en C₀₋₂)-NHCO-cycloalkyle, - NH-(alkylène en C₀₋₂)-cycloalkyle, -(alkylène en C₀₋₂)-NH-cycloalkyle, -O-(alkylène en C₀₋₂)-cycloalkyle, -(alkylène en C₀₋₂)-O-cycloalkyle, -SO₂-(alkylène en C₀₋₂)-cycloalkyle, -(alkylène en C₀₋₂)-SO₂-cycloalkyle, -CONH-cycloalkyle, -NHCO-cycloalkyle, -NH-cycloalkyle, -O-cycloalkyle, -CO-cycloalkyle, -SO₂-cycloalkyle, -(alkylène en C₀₋₂)-hétérocycloalkyle, -CO-(alkylène en C₀₋₂)-hétérocycloalkyle, -(alkylène en C₀₋₂)-CO-hétérocycloalkyle, -CONH-(alkylène en C₀₋₂)-hétérocycloalkyle, -(alkylène en C₀₋₂) -CONH-hétérocycloalkyle, -NHCO-(alkylène en C₀₋₂)-hétérocycloalkyle, -(alkylène en C₀₋₂)-NHCO-hétérocycloalkyle, -NH-(alkylène en C₀₋₂)-hétérocycloalkyle, -(alkylène en C₀₋₂)-NH-hétérocycloalkyle, -O-(alkylène en C₀₋₂)-hétérocycloalkyle, -(alkylène en C₀₋₂)-O-hétérocycloalkyle, -SO2-(alkylène en C₀₋₂)-hétérocycloalkyle, -(alkylène en C₀₋₂)-SO2-hétérocycloalkyle, -CONH-hétérocycloalkyle, - NHCO-hétérocycloalkyle, -NH-hétérocycloalkyle, -O-hétérocycloalkyle, -CO-hétérocycloalkyle, -SO₂-hétérocycloalkyle, -(alkylène en C₀₋₂)-aryle, -CO-(alkylène en C₀₋₂)-aryle, -(alkylène en C₀₋₂)-CO-aryle, -CONH-(alkylène en C₀₋₂)-aryle, -(alkylène en C₀₋₂)-CONH-aryle, -NHCO-(alkylène en C₀₋₂)-aryle, -(alkylène en C₀₋₂) -NHCO-aryle, -NH-(alkylène en C₀₋₂)-aryle, -(alkylène en C₀₋₂)-NH-aryle, -O-(alkylène en C₀₋₂)-aryle, -(alkylène en C₀₋₂)-O-aryle, -SO₂-(alkylène en C₀₋₂)-aryle, -(alkylène en C₀₋₂)-SO₂-aryle, -CONH-aryle, - NHCO-aryle, -NH-aryle, -O-aryle, -CO-aryle, -SO₂-aryle, -(alkylène en C₀₋₂)-hétéroaryle, -CO-(alkylène en C₀₋₂)-hétéroaryle, -(alkylène en C₀₋₂)-CO-hétéroaryle, -CONH-(alkylène en C₀₋₂)-hétéroaryle, -(C₀₋₂ alkylène)-CONH-hétéroaryle, -NHCO-(alkylène en C₀₋₂)-hétéroaryle, - (alkylène en C₀₋₂)-NHCO-hétéroaryle, -NH-(alkylène en C₀₋₂)hétéroaryle, -(alkylène en C₀₋₂)-NH-hétéroaryle, -O-(alkylène en C₀₋₂)hétéroaryle, -(alkylène en C₀₋₂)-O-hétéroaryle, -SO₂-(alkylène en C₀₋₂)hétéroaryle, -(alkylène en C₀₋₂)-SO₂-hétéroaryle, -CONH-hétéroaryle, - NHCO-hétéroaryle, -NH-hétéroaryle, -O-hétéroaryle, -CO-hétéroaryle et -SO₂-hétéroaryle, dans lequel lesdits groupes cycloalkyle, hétérocycloalkyle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, - CONH(alkyle en C₁₋₅) et -CON(alkyle en C₁₋₅)(alkyle en C₁₋₅),
ou
dans lequel R₄ est choisi parmi les groupes -(alkylène en C₀₋₂)-aryle, -CO-(alkylène en C₀₋₂)-aryle, -(alkylène en C₀₋₂)-CO-aryle, -CONH-(alkylène en C₀₋₂)-aryle, -(alkylène en C₀₋₂)-CONH-aryle, -NHCO-(alkylène en C₀₋₂)-aryle, -(alkylène en C₀₋₂)-NHCO-aryle, -NH-(alkylène en C₀₋₂)-aryle, -(alkylène en C₀₋₂)-NH-aryle, -O-(alkylène en C₀₋₂)-aryle, - (alkylène en C₀₋₂)-O-aryle, -SO₂-(alkylène en C₀₋₂)-aryle, -(alkylène en C₀₋₂)-SO₂-aryle, -CONH-aryle, - NHCO-aryle, -NH-aryle, -O-aryle, -CO-aryle, -SO₂-aryle, -(alkylène en C₀₋₂)-hétéroaryle, -CO-(alkylène en C₀₋₂)-hétéroaryle, -(alkylène en C₀₋₂)-CO-hétéroaryle, -CONH-(alkylène en C₀₋₂)-hétéroaryle, -(alkylène en C₀₋₂) -CONH-hétéroaryle, -NHCO-(alkylène en C₀₋₂)-hétéroaryle, - (alkylène en C₀₋₂)-NHCO-hétéroaryle, -NH-(alkylène en C₀₋₂)-hétéroaryle, -(alkylène en C₀₋₂)-NH-hétéroaryle, -O-(alkylène en C₀₋₂)-hétéroaryle, -(alkylène en C₀₋₂)-O-hétéroaryle, -SO2-(alkylène en C₀₋₂)-hétéroaryle, -(alkylène en C₀₋₂)-SO2-hétéroaryle, -CONH-hétéroaryle, - NHCO-hétéroaryle, -NH-hétéroaryle, -O-hétéroaryle, -CO-hétéroaryle et -SO₂-hétéroaryle, dans lequel lesdits groupes aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), - S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et - CON(alkyle en C₁₋₅)(alkyle en C₁₋₅),
ou
dans lequel R₄ est choisi parmi alkyle en C₁₋₁₂, alcényle en C₂₋₁₂, alcynyle en C₂₋₁₂, cycloalkyle, hétérocycloalkyle, aryle et hétéroaryle, de préférence parmi les groupes cycloalkyle, hétérocycloalkyle, aryle et hétéroaryle, de préférence R₄ est choisi parmi aryle et hétéroaryle, et de manière encore plus préférentielle R₄ est hétéroaryle, lesdits groupes alkyle, alcényle ou alcynyle étant éventuellement substitués par un ou plusieurs groupes choisis indépendamment parmi halogène, -CN, -OH, -O(alkyle en C₁₋₅), -O(haloalkyle en C₁₋₅), SH, -S(alkyle en C₁₋₅), - S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et - CON(alkyle en C₁₋₅)(alkyle en C₁₋₅), et dans lequel lesdits groupes cycloalkyle, hétérocycloalkyle, aryle ou hétéroaryle sont éventuellement substitués par un ou plusieurs groupes choisis indépendamment parmi halogène, -CN, -OH, alkyle en C₁₋₅, haloalkyle en C₁₋₅, -O(alkyle en C₁₋₅), -O(haloalkyle en C₁₋₅), -SH, -S(alkyle en C₁₋₅), -S(haloalkyle en C₁₋₅), - NH₂, -NH(alkyle en C₁₋₅), -NH(haloalkyle en C₁₋₅), -N(alkyle en C₁₋₅)(alkyle en C₁₋₅), - N(haloalkyle en C₁₋₅)(alkyle en C₁₋₅), -CONH₂, -CONH(alkyle en C₁₋₅) et -CON(alkyle en C₁₋₅)(alkyle en C₁₋₅),
ou
dans lequel R₄ est un hétéroaryle à cinq chaînons, éventuellement substitué par un ou plusieurs groupes choisis indépendamment parmi les groupes suivants : halogène, CN, -OH, alkyle en C₁₋₅, haloalkyle en C₁₋₅, -O(alkyle en C₁₋₅), -O(haloalkyle en C₁₋₅), -SH, -S(alkyle en C₁₋₅), - S(haloalkyle en C₁₋₅), -NH₂, -NH(alkyle en C₁₋₅), -NH(haloalkyle en C₁₋₅), -N(alkyle en C₁₋₅)(alkyle en C₁₋₅), -N(haloalkyle en C₁₋₅)(alkyle en C₁₋₅), -CONH₂, -CONH(alkyle en C₁₋₅) et - CON(alkyle en C₁₋₅)(alkyle en C₁₋₅),
de préférence, l'hétéroaryle à cinq chaînons est choisi parmi les imidazoles, les isoxazoles, les pyrazoles, les 1,2,3-triazoles, les 1,2,4-triazoles, les thiazoles, les 1,2,4-oxadiazoles, les 1,3,4-oxadiazoles, les 1,2,4-thiadiazoles ou les 1,3,4-thiadiazoles, de préférence dans lequel l'hétéroaryle à cinq chaînons est un 1,2,4-thiadiazolyle, éventuellement substitué par un ou plusieurs groupes choisis indépendamment parmi les groupes suivants : halogène, -CN, -OH, alkyle en C₁₋₅, haloalkyle en C₁₋₅, -O(alkyle en C₁₋₅), -SH, -S(alkyle en C₁₋₅), -NH2, -NH(alkyle en C₁₋₅), -N(alkyle en C₁₋₅)(alkyle en C₁₋₅), -CONH2, -CONH(alkyle en C₁₋₅), et -CON(alkyle en C₁₋₅)(alkyle en C₁₋₅), de préférence éventuellement substitué par un alkyle en C₁₋₅, un haloalkyle en C₁₋₅, -O(alkyle en C₁₋₅), -SH, -S(alkyle en C₁₋₅), de préférence éventuellement substitué par un alkyle en C₁₋₅, un haloalkyle en C₁₋₅, de préférence encore éventuellement substitué par un haloalkyle en C₁₋₅, de préférence choisi parmi -CH₂F, -CHF₂ et CF₃, de préférence éventuellement substitué par -CHF₂.

11. Composé selon la revendication 1, choisi parmi : 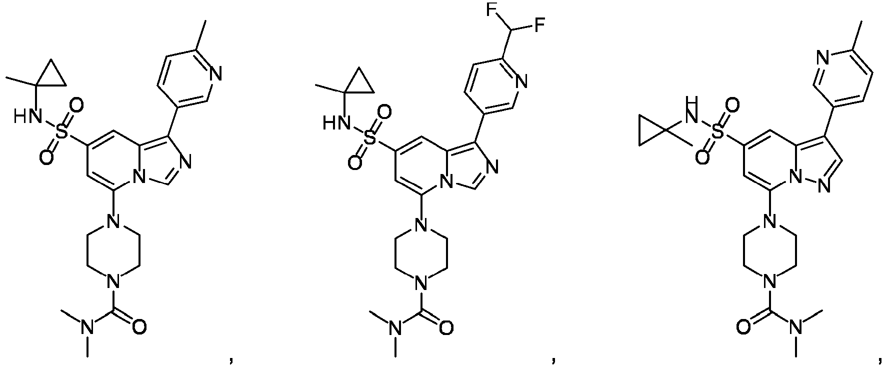 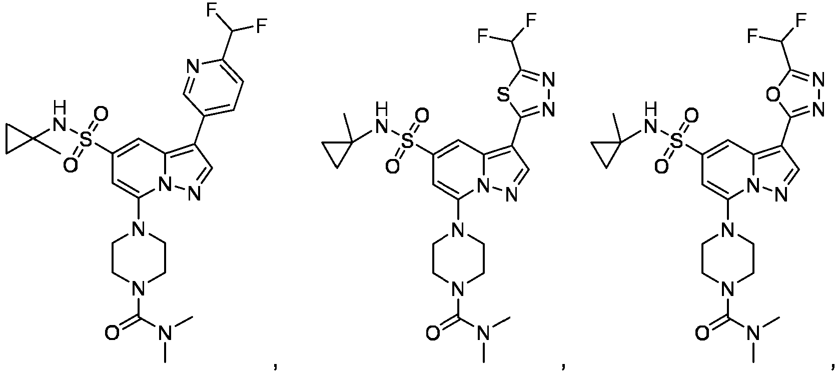 et 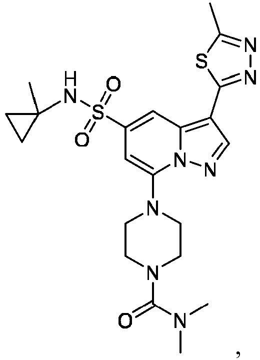 ou un sel, un hydrate ou un solvant pharmaceutiquement

12. Composé selon la revendication 1, dans lequel le composé est un composé selon la formule (lap) : 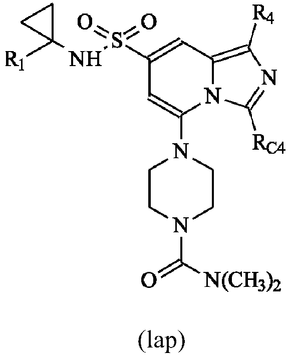 ou un composé selon la formule (Ibn) : 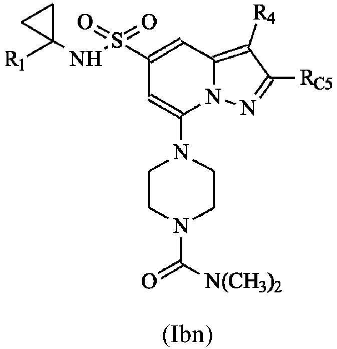 ou un énantiomère, un diastéréo-isomère, un tautomère, un solvate pharmaceutiquement acceptable, une forme cristalline pharmaceutiquement acceptable ou un sel pharmaceutiquement acceptable de celui-ci, dans lequel R₁, R₄, R_{C4} et R_{C5} sont chacun définis comme dans l'une quelconque des revendications 1 à 10.

13. Composition pharmaceutique comprenant le composé de l'une quelconque des revendications 1 à 12 ou l'un de ses sels, hydrates ou solvates pharmaceutiquement acceptables, et un support pharmaceutiquement acceptable.

14. Composé selon l'une quelconque des revendications 1 à 12 ou sel, hydrate ou solvate pharmaceutiquement acceptable de celui-ci, ou composition pharmaceutique selon la revendication 13, pour une utilisation en thérapie.

15. Composé à utiliser ou composition pharmaceutique à utiliser selon la revendication 14, pour une utilisation dans un procédé de traitement d'un trouble prolifératif, de préférence dans lequel le trouble prolifératif est un cancer, de préférence un cancer humain, plus préférentiellement dans lequel le cancer est choisi parmi les cancers du poumon, du côlon, du sein, des ovaires, de la prostate, du foie, du pancréas, du cerveau et de la peau.
